(19) Europäisches Patentamt
European Patent Office
Office européen des brevets

(11) EP 3 360 567 A1

(12) EUROPEAN PATENT APPLICATION

(43) Date of publication:
15.08.2018 Bulletin 2018/33

(21) Application number: 17208162.2

(22) Date of filing: 07.11.2008

(51) Int Cl.:
*A61K 38/20* (2006.01) *A61K 38/17* (2006.01)
*A61K 38/16* (2006.01) *A61P 31/00* (2006.01)

(84) Designated Contracting States:
**AT BE BG CH CY CZ DE DK EE ES FI FR GB GR HR HU IE IS IT LI LT LU LV MC MT NL NO PL PT RO SE SI SK TR**
Designated Extension States:
**AL BA MK RS**

(30) Priority: **07.11.2007 US 986170 P**
**13.12.2007 US 13620 P**
**20.12.2007 US 15620 P**

(62) Document number(s) of the earlier application(s) in accordance with Art. 76 EPC:
**12169572.0 / 2 514 436**
**08848478.7 / 2 217 263**

(71) Applicant: **Genentech, Inc.**
**South San Francisco, CA 94080 (US)**

(72) Inventors:
- **ABBAS, Alexander, R.**
  **San Carlos, CA California 94070 (US)**
- **DANILENKO, Dimitry, M.**
  **Millbrae, CA California 94030 (US)**
- **DE SAUVAGE, Frederic, J.**
  **Foster City, CA California 94404 (US)**
- **GHILARDI, Nico, P.**
  **South San Francisco, CA 94080 (US)**
- **MODRUSAN, Zora**
  **Fremont, CA California 94536 (US)**
- **OUYANG, Wenjun**
  **Foster City, CA California 94404 (US)**
- **VALDEZ, Patricia, A.**
  **Kensington, MD Maryland 20895 (US)**
- **ZHENG, Yan**
  **Redwood City, CA 94065 (US)**

(74) Representative: **Walton, Seán Malcolm et al**
**Mewburn Ellis LLP**
**City Tower**
**40 Basinghall Street**
**London EC2V 5DE (GB)**

Remarks:
•Claims filed after the date of filing of the application (Rule 68(4) EPC).
•This application was filed on 18-12-2017 as a divisional application to the application mentioned under INID code 62.

# (54) AMP FOR USE IN TREATING MICROBIAL DISORDERS

(57) The present invention relates to compositions and methods for treatment of microbial disorder by modulation of the host immune response. More particularly, the present invention relates to compositions that mediate an anti-microbial immune response, and methods of treating a microbial infection using such compositions.

EP 3 360 567 A1

## Description

### FIELD OF THE INVENTION

The present invention relates generally to the treatment of microbial disorders by modulation of the host immune response.

### BACKGROUND

Infection by microbial pathogens represents a major cause of death worldwide and continues to pose a serious threat to global health (WHO, The World Health Report (2004)). For example, Attaching and effacing (A/E) bacterial pathogens, such as enterohemorrhagic *Escherichia coli* (EHEC) and enteropathogenic *E. coli* (EPEC) are among the bacteria that cause diarrhea, morbidity and mortality, especially among infants and children in the developing world (2). *E. coli* O157:H7, one of the EHEC strains, caused many people to be hospitalized and 3 mortalities last year in the United States (MMWR Morb Mortal Wkly Rep 55, 1045 (Sep 29, 2006)). It is also believed that more than 90% of all cases of post-diarrhea hemolytic uremic syndrome (HUS) in industrialized countries were caused by *E. coli* O157:H7 infection (R. L. Siegler, Pediatr Clin North Am 42, 1505 (Dec, 1995)). Other EPEC strains such as *E. coli* O55:H7 also cause intestinal illness among infants world wide (T. S. Whittam et al., Infect Immun 61, 1619 (May, 1993)). Much of our knowledge on how hosts control the infection of A/E pathogens comes from the study of infection by *Citrobacter rodentium*, a natural pathogen in mice (L. Eckmann, Ann NY Acad Sci 1072, 28 (August 1, 2006)). Similar to the pathogenesis of EHEC or EHPC in human, intimately attaching of *C. rodentium* to murine colonic epithelial cells results in effacement of brush border microvilli, termed as attaching and effacing (A/E) lesion, and colonic hyperplasia (D. B. Schauer, S. Falkow, Infect Immun 61, 2486 (Jun, 1993)).

Both intestinal epithelial and immune cells play critical roles in host defense against A/E pathogens. The tight junctions of intestinal epithelial cells present the first barrier to prevent microbes leaving the intestinal lumen (T. T. MacDonald, G. Monteleone, Science 307, 1920 (March 25, 2005)). Additionally, epithelial cells secrete anti-microbial peptides to control pathogens in the gastrointestinal (GI) tract (A. Takahashi et al., FEBS Lett 508, 484 (Nov 23, 2001)). Studies with immune deficient mouse strains during *C. rodentium* infection established that $CD4^+$ T cells, B cells, and anti-*C. rodentium* specific antibody responses are all essential components of the adaptive immunity to contain and eradicate infection (L. Bry, M. B. Brenner, J Immunol 172, 433 (January 1, 2004)). Many cytokines produced by lymphocytes during infection can enhance the innate immune responses of epithelial cells. The specific functions of these cytokines, however, remain unclear during A/E pathogen infection.

IL-22, an IL-10 family cytokine, is produced by lymphocytes, particularly Th17 cells (Y. Zheng et al., Nature 445, 648 (Feb 8, 2007)). Th17 cells belong to a recently discovered $CD4^+$ T helper subset that also produces IL-17. IL-17 has important functions in the control of extracellular bacterial infections (K. I. Happel et al., J. Exp. Med. 202, 761 (September 19, 2005)). The role of IL-22, however, in host defense is still largely unknown. Tumor Necrosis Factor (TNF)-related proteins are recognized in the art as a large family of proteins having a variety of activities ranging from host defense to immune regulation to apoptosis. TNF was first identified as a serum-derived factor that was cytotoxic for several transformed cell lines *in vitro* and caused necrosis of certain tumors *in vivo.* A similar factor in the superfamily was identified and referred to as lymphotoxin ("LT"). Due to observed similarities between TNF and LT in the early 1980's, it was proposed that TNF and LT be referred to as TNF-$\alpha$ and TNF-$\beta$, respectively. Scientific literature thus makes reference to both nomenclatures. As used in the present application, the term "TNF" refers to TNF-$\alpha$. Later research revealed two forms of lymphotoxin, referred to as LT$\alpha$ and LT$\beta$. US 2005-0129614 describes another polypeptide member of the TNF ligand super-family based on structural and biological similarities, designated TL-5. Members of the TNF family of proteins exist in membrane-bound forms that act locally through cell-cell contact, or as secreted proteins. A family of TNF-related receptors react with these proteins and trigger a variety of signalling pathways including cell death or apoptosis, cell proliferation, tissue differentiation, and proinflammatory responses. TNF-$\alpha$ by itself has been implicated in inflammatory diseases, autoimmune diseases, viral, bacterial, and parasitic infections, malignancies, and/or neurodegenerative diseases and is a useful target for specific biological therapy in diseases such as RA and Crohn's disease.

### SUMMARY OF THE INVENTION

The present invention provides compositions and methods for treatment of microbial disorders by modulation of the host immune response. For example, an anti-microbial immune response in a host can be enhanced or inhibited by increasing or decreasing an activity of one or more anti-microbial polypeptides (AMPs) that mediate the anti-microbial immune response.

More particularly, the present invention provides AMPs, modulators thereof, and methods of using such compositions for treatment of microbial disorders. Such microbial disorders include, but are not limited to, infectious diseases, for example, EHEC- and EPEC-caused diarrhea , Inflammatory Bowel Disease (IBD) and, more particularly, Ulcerative Colitis (UC) and Crohn's Disease (CD).

AMPs of the present invention are polypeptides that mediate an anti-microbial immune response, and include, but are not limited to, LT, IL-6, IL-18, IL-22, IL-23 (including e.g., IL-23 p19 or IL-23 p40), and Reg or Reg-related proteins encoded by the genes of the Reg super family. The Reg super family includes Reg and Reg-related genes from human, rat, and mouse and are grouped into four subclasses, types I, II, III, and IV. For example, type I includes human *REG Iα*, human *REG Iβ*, rat *RegI*, and mouse *RegI*; type II includes mouse *RegII*; type III includes human REG III, human *HIP/PAP* (gene expressed in hepatocellular carcinoma-intestine-pancreas/gene encoding pancreatitis-associated protein), rat *PAP/Peptide23*, rat *RegIII/PAPII*, rat *PAP III*, mouse *RegIIIα*, *RegIIIβ*, *RegIIIγ*, mouse *RegIIIδ*, and hamster *INGAP* (islet neogenesis-associated protein). Type IV contains human *REG IV*. In one aspect, the REG protein is encoded by a member of the human REG gene family which includes, but is not limited to, REG Iα, REG Iβ, HIP/PAP, REG III, REG IV, and Reg-related sequence (RS).

In some aspects, the amino acid sequence of an AMP of the present invention comprises an amino acid sequence selected from the following group: SEQ ID NO: 2, SEQ ID NO: 4, SEQ ID NO: 6, SEQ ID NO: 8, SEQ ID NO: 10, SEQ ID NO: 12, SEQ ID NO: 14, SEQ ID NO: 16, SEQ ID NO: 18, SEQ ID NO: 20, SEQ ID NO: 22, SEQ ID NO: 24, SEQ ID NO: 26, SEQ ID NO: 28, SEQ ID NO: 30, SEQ ID NO: 32, SEQ ID NO: 34, SEQ ID NO: 36, SEQ ID NO: 38, SEQ ID NO: 40, SEQ ID NO: 42, SEQ ID NO: 44, SEQ ID NO: 46, SEQ ID NO: 48, SEQ ID NO: 50, SEQ ID NO: 52, SEQ ID NO: 54, and SEQ ID NO: 56.

In other aspects, the nucleic acid sequence encoding an AMP of the present invention comprises a nucleic acid sequence selected from the following group: SEQ ID NO: 1, SEQ ID NO: 3, SEQ ID NO: 5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, SEQ ID NO: 23, SEQ ID NO: 25, SEQ ID NO: 27, SEQ ID NO: 29, SEQ ID NO: 31, SEQ ID NO: 33, SEQ ID NO: 35, SEQ ID NO: 37, SEQ ID NO: 39, SEQ ID NO: 41, SEQ ID NO: 43, SEQ ID NO: 45, SEQ ID NO: 47, SEQ ID NO: 49, SEQ ID NO: 51, SEQ ID NO: 53, and SEQ ID NO: 55.

An activity of an AMP of the present invention can be increased or decreased and/or differentially regulated relative to the activity of another AMP or the same AMP. Examples of an activity of an AMP of the present invention, includes, but is not limited to, AMP expression, binding to a binding partner, signal transduction, anti-microbial activity, or other biological or immunological activity thereof.

In one aspect, an increase in the activity of one or more AMPs of the present invention results in an enhanced anti-microbial immune response in a subject.

In one aspect, AMPs of the present invention include, but are not limited to, polypeptides that directly or indirectly interact with IL-22, e.g., polypeptides that are upstream or downstream of an IL-22 signal transduction pathway that mediates host resistance to infection by a microbial pathogen (e.g., a bacteria or virus). Examples of such AMPs include, but are not limited to, LT, IL-6, IL-18,, and IL-23 (including e.g., IL-23 p19 or IL-23 p40).

Modulators of the present invention include, but are not limited to, polypeptides and nucleic acid molecules (e.g., a DNA molecule or RNA molecule) that directly or indirectly modulate an activity of an AMP. Examples of such modulation include, but are not limited to, an increase, decrease, induction or activation, inhibition, or regulation (e.g., up or down regulation) of an activity of an AMP of the present invention.

In one aspect, the modulator indirectly modulates IL-22 activity by decreasing or inhibiting IL-22 Binding Protein (BP) activity and thereby, increasing IL-22 activity. In a particular aspect, the modulator decreases or inhibits binding of IL-22 BP to IL-22 and thereby, increases IL-22 activity.

In some aspects, the modulator is a polypeptide e.g., a polypeptide that binds to or otherwise interacts with an AMP to increase, induce, or regulate an activity of an AMP. In one aspect, the modulator is a fusion polypeptide that modulates an activity of an AMP.

In one aspect, the modulator is an antibody that binds to an AMP. In a particular aspect, the antibody is a monoclonal antibody. In another aspect, the antibody is an antibody fragment selected from a Fab, Fab'-SH, Fv, scFv, or $(Fab')_2$ fragment. In another aspect, the antibody is a fusion polypeptide (e.g., an Fc fusion polypeptide). In another aspect, the antibody is a chimeric antibody. In a particular aspect, the antibody is humanized. In another aspect, the antibody is a human antibody. In another aspect, the antibody binds to the same epitope as an antibody selected from a human, non-human primate, or other mammal (e.g., pig, sheep, rabbit, marmot, rat, or mouse). In a particular aspect, the antibody is an AMP agonist.

In another particular aspect, the modulator is a recombinant AMP or nucleic acid molecule encoding an AMP (e.g., a DNA or RNA molecule).

The present invention further provides methods of treating a microbial disorder by modulating an anti-microbial immune response. In one aspect, the present invention provides a method of treating a microbial disorder, in a subject, comprising administering to the subject an effective amount of pharmaceutical composition comprising an AMP or modulator of the AMP, wherein the AMP is selected from a group consisting of: LT, IL-6, IL-18, IL-22, IL-23, REG Iα, REG Iβ, HIP/PAP, REG III, REG IV and Reg-related sequence (RS). In one aspect the disorder is an infectious disease, for example, EHEC- or EPEC-caused diarrhea, Inflammatory Bowel Disease (IBD) or, more particularly, Ulcerative Colitis (UC) or Crohn's Disease (CD).

In particular aspects, the present invention provides methods of modulating an anti-microbial immune response by stimulating or inhibiting an AMP-mediated signaling pathway and/or $Th_{IL-17}$ cell function. Such methods are useful for treatment of microbial disorders. For example, in one aspect, the present invention provides a method of enhancing an anti-microbial immune response by stimulating an AMP-mediated signaling pathway, e.g., and IL-22 and/or IL-23 mediated signaling pathway. In another aspect, the present invention provides methods of modulating an anti-microbial immune response by stimulating or inhibiting a cytokine-mediated signaling pathway. For example, in one aspect, the present invention provides methods of enhancing an anti-microbial immune response by stimulating a cytokine-mediated signaling pathway, e.g., an IL-22 and/or IL-23 signaling pathway. Moreover, the present invention provides methods of modulating an anti-microbial immune response by stimulating or inhibiting a $Th_{IL-17}$ cell function.

In one aspect, the present invention provides a method of stimulating an AMP-mediated signaling pathway in a biological system, the method comprising providing an AMP agonist to the biological system. Examples of such a biological system include, but are not limited to, mammalian cells in an in vitro cell culture system or in an organism in vivo. In another aspect, the present invention provides a method of inhibiting an AMP-mediated signaling pathway in a biological system, the method comprising providing an AMP antagonist to the biological system.

In a particular aspect, the present invention provides a method of enhancing an anti-microbial immune response in a biological system by stimulating an IL-23 and/or IL-22 mediated signaling pathway in a biological system, the method comprising providing an IL-22 or IL-22 agonist to the biological system. In one aspect, an IL-22 agonist is IL-22. In another aspect, the IL-22 agonist is an antibody that binds to IL-22.

In another aspect, a method of inhibiting an IL-23-mediated signaling pathway in a biological system is provided, the method comprising providing an IL-22 antagonist to the biological system. In one aspect, the antagonist of IL-22 is an antibody, e.g., a neutralizing anti-IL-22 antibody and/or a neutralizing anti-IL-22R antibody.

In another aspect, the present invention provides a method of stimulating a $Th_{IL-17}$ cell function, the method comprising exposing a $Th_{IL-17}$ cell to an agonist of an AMP that mediates the IL-23 mediated signaling pathway (e.g., IL-23, IL-6, or IL-22). Such methods are useful for treating a microbial disorder. In one aspect, an IL-22 agonist is IL-22. In another aspect, the IL-22 agonist is an antibody that binds to IL-22.

In another aspect, a method of inhibiting a $Th_{IL-17}$ cell function is provided, the method comprising exposing a $Th_{IL-17}$ cell to an antagonist of an AMP that mediates the IL-23 mediated signaling pathway (e.g., IL-23, IL-6, or IL-22). In one aspect the antagonist is an anti-IL-22 antibody, e.g., a neutralizing anti-IL-22 antibody.

Exemplary $Th_{IL-17}$ cell functions include, but are not limited to, stimulation of cell-mediated immunity (delayed-type hypersensitivity); recruitment of innate immune cells, such as myeloid cells (e.g., monocytes and neutrophils) to sites of inflammation; and stimulation of inflammatory cell infiltration into tissues. In one aspect, a $Th_{IL-17}$ cell function is mediated by IL-23 and/or IL-22.

In a further aspect, the present invention provides a method of treating an infection by a microbial pathogen (e.g., a bacteria or virus), in a subject, comprising administering to the subject an effective amount of pharmaceutical composition comprising an AMP or modulator of the AMP, wherein the AMP is selected from a group consisting of: LT, IL-6, IL-18,, IL-22, IL-23, REG Iα, REG Iβ, HIP/PAP, REG III, REG IV and Reg-related sequence (RS).

In another aspect, the present invention provides a method of treating a microbial disorder, in a subject, comprising contacting cells of the subject with a nucleic acid molecule (e.g., a DNA or RNA molecule) encoding an AMP or modulator of the AMP, wherein the AMP is selected from a group consisting of: LT, IL-6, IL-18, IL-22, IL-23, REG Iα, REG Iβ, HIP/PAP, REG III, REG IV and Reg-related sequence (RS). In one aspect the disorder is an infectious disease, for example, EHEC- or EPEC-caused diarrhea, inflammatory Bowel Disease (IBD) or, more particularly, Ulcerative Colitis (UC) or Crohn's Disease (CD).

In another aspect, the present invention provides a method of modulating the activity of an AMP in cells of a subject infected with a microbial pathogen (e.g., a bacteria or virus), comprising contacting the cells with a nucleic acid molecule (e.g., a DNA or RNA molecule) encoding an AMP or modulator of the AMP, wherein the AMP is selected from a group consisting of: LT, IL-6, IL-18, IL-22, IL-23, REG Iα, REG Iβ, HIP/PAP, REG III (e.g., REG IIIβ or REGIIIγ), REG IV, and Reg-related sequence (RS).

Examples of a microbial pathogen include, but are not limited to, a bacteria or virus. In one aspect, the microbial pathogen is a bacteria e.g., a gram-negative or gram-positive bacteria. In a particular aspect, the bacteria is a gram-negative bacteria. In another aspect, the bacteria is an attaching or effacing (A/E) bacteria and, more particularly, an enterohemorrhagic Escherichia coli (EHEC) or enteropathogenic E. Coli (EPEC). In one aspect, the bacteria is enteropathogenic E. coli (EHEC) is E. coli 0157:H7 or E. coli 055:H7.

In another aspect, the present invention provides polynucleotides encoding an AMP of the present invention, or modulator thereof. In another aspect, the invention provides a vector comprising the polynucleotide. In another aspect, the invention provides a host cell comprising the vector. In one aspect, the host cell is a eukaryotic cell. In another aspect, the host cell is a CHO cell, yeast cell, or bacterial cell (e.g., E. coli).

In one aspect, the present invention provides a method of making an antibody that binds to an AMP of the present invention, wherein the method comprises culturing the host cell under conditions suitable for expression of the polynucleotide encoding the antibody, and isolating the antibody. In a particular aspect, the invention provides a method of making an antibody that is an agonist of an AMP of the present invention.

In one aspect, the present invention provides a method of detecting the presence of an AMP in a biological sample, comprising contacting the biological sample with an antibody to the AMP, under conditions permissive for binding of the antibody to the AMP, and detecting whether a complex is formed between the antibody and AMP.

In another aspect, the present invention provides a kit comprising one or more AMPs of the present invention and/or modulators thereof. In another aspect, the present invention provides a kit comprising one or more one or more pharmaceutical compositions each comprising an AMP of the present invention or modulator thereof.

## BRIEF DESCRIPTION OF THE DRAWINGS

**Fig. 1** depicts data demonstrating host defense against *C. rodentium* infection. Fig. 1(A) depicts the results of a real-time RT-PCR analysis on receptor subunits for IL-22 in uninfected wildtype mouse GI track; Fig. 1(B-F) depicts a real-time RT-PCR analysis on various cytokine expressions in wildtype mouse colons upon *C. rodentium* infection; Fig. 1(G) depicts survival of C57B1/6 (n=5), IL-23p40$^{-/-}$ (n=5), and IL-6$^{-/-}$ (n=5) mice after *C. rodentium* infection; and Fig. 1(H) depicts a time course real-time RT-PCR analysis on IL-22 and IL-17 expressions in C57B1/6, IL-23p40$^{-/-}$, and IL-6$^{-/-}$ mouse colons upon C. *rodentium* infection. For C. *rodentium* infection, the mice were orally inoculated with 2x10$^9$ CFU of bacteria. All of the above data are representative of two independent experiments.

**Fig. 2** depicts data demonstrating that IL-22 deficiency renders mice susceptible to *C. rodentium* infection. 6-7 weeks old IL-22$^{-/-}$ (Fig. 2(A-C)), IL-17RC$^{-/-}$ (D), IL-20R$\beta^{-/-}$ mice (Fig. 2(F)) or wildtype mice (Fig. 2(A-C, E)) were orally inoculated with 2x10$^9$ CFU of *C. rodentium* and weighed at indicated time points. Histologic analysis of colons from IL-22$^{-/-}$ and wildtype mice 8 days post inoculation using hematoxylin-and-eosin (H&E) staining (Fig. 2(B and C)). Arrows indicate submucosal inflammation (Fig. 2(B)), and bacterial invasion into mucosal glands (Fig. 2(C)). Representative data are shown (bars = 100 $\mu$m for Fig. 2(B) and bars = 25 $\mu$m for Fig. 2(C)). Wildtype C57B1/6 mice received 150 $\mu$g of anti-IL-22 mAb or isotype control IgG1 mAb intraperitoneally, every other day, starting on day 0 or day 8 post inoculation (Fig. 2(E)). * $p < 0.05$, ** $p < 0.01$, *** $p < 0.001$. All data are representative of two independent experiments.

**Fig. 3** depicts data demonstrating the effect of IL-22 deficiency in mice during *C. rodentium* infection. C57B1/6 mice (Fig. 3(A, B, and F)), IL-22$^{-/-}$ and wildtype mice (Fig. 3(C-E, and G)) were orally inoculated with 2x10$^9$ CFU of *C. rodentium.* Mice also received 150 $\mu$g of anti-IL-22 mAb or isotype control IgG1 mAb intraperitoneally every other day starting from the same day as *C. rodentium* inoculation (Fig. 3(A and B)). On day 10, colons were photographed and individual colon length was measured (Fig. 3(A)). Histologic analysis of colons was performed using hematoxylin-and-eosin (H&E) staining (Fig. 3(B)). Histologic analysis of colons and livers (day 8) from infected IL-22$^{-/-}$ and wildtype mice was performed using H&E staining (Fig. 3(C and E)). Arrows in Fig. 3(C) indicate colonic transmural inflammation and ulceration. Fig. 3(E) depicts a hepatic septic microabscess in the IL-22$^{-/-}$ mouse. Representative data are shown in Fig. 3(C), where the bars = 500 $\mu$m for the upper panels and bars = 100 $\mu$m for the lower panels. In Fig.3(E), the bars = 25 $\mu$m. Fig. 3(D) depicts the $\log_{10}$ CFU of *C. rodentium* in colon, liver, spleen, and mesenteric lymph node. Fig. 3(F-G) depicts the serum anti-*C. rodentium* IgG levels by ELISA. * $p < 0.05$. All of the above data are representative of two independent experiments.

**Fig. 4** depicts data demonstrating that IL-22 induces anti-microbial RegIII family protein expression upon *C. rodentium* infection. *In vitro* culture of C57B1/6 mouse colons were treated with 10 $\mu$g of IL-22 for 24 hours, RNA were isolated and used for microarray analysis (Fig.4(A)) and real-time RT-PCR analysis (Fig. 4(B)). In Fig. 4(C), IL-22$^{-/-}$ mice and wildtype littermates were orally inoculated with 2x10$^9$ CFU of *C. rodentium*, and real-time RT-PCR was performed on RNA isolated from individual mouse colon collected on indicated time points. All data are representative of two independent experiments.

**Fig. 5** depicts data demonstrating the targeted disruption of the murine *IL-17RC* gene. Fig. 5(A) depicts the strategy for generation of IL-17RC knockout mice. Exons 1-5 (open boxes) encompassing the *IL-17RC* coding sequence was replaced with a neomycin resistance cassette. Fig. 5(B) depicts the genotyping of offspring from wildtype (WT) and knockout (KO) mice using the indicated primer sets (P1, P2 and P3). Tail tip fibroblasts from WT and KO mice were generated and stimulated with various concentrations of IL-17A and IL-17F *in vitro* for 24 hours, and culture supernatant were collected for IL-6 ELISA (Fig. 5(C)).

**Fig. 6** depicts data of a real-time RT-PCR analysis on IL-19, IL-20 and IL-24 expression in wildtype mouse colons upon *C. rodentium* infection, over time. C57B1/6 mice were orally inoculated with 2x10$^9$ CFU of *C. rodentium.* Colons were collected at indicated time points and isolated RNAs were used for real-time RT-PCR analysis.

**Fig. 7** depicts data demonstrating IL-20Rα and IL-20Rβ expression in the GI tract. Real-time RT-PCR analysis on receptor subunits for IL-19, IL-20 and IL-24 in uninfected wildtype mouse GI tract.

**Fig. 8** depicts data demonstrating targeted disruption of the murine *IL-20Rβ* gene. Fig. 8(A) depicts the strategy for generation of IL-20Rβ knockout mice. Exon 1 (open boxes) was replaced with a neomycin resistance cassette. Fig. 8(B) depicts the phenotyping of offspring from wildtype (WT), heterozygous (HET) and knockout (KO) mice using the indicated primer sets (p1, p2 and p3). Fig. 8(C) WT and KO mouse ears were injected intradermally with 500 ng recombinant IL-20 in 20 μl PBS or with 20 μl PBS alone. 24 hours later, mouse ears were collected for RNA isolation. Isolated RNAs were used for real-time RT-PCR analysis for genes known to be upregulated upon IL-20 signaling.

**Fig. 9** depicts data of a histologic analysis of mouse colons from anti-IL-22 mAb treated wildtype mice inoculated with *C. rodentium.* C57B1/6 mice were orally inoculated with $2x10^9$ CFU of *C. rodentium.* Mice also received 150 μg of anti-IL-22 mAb or isotype control IgG1 mAb intraperitoneally every other day starting from the same day as C. *rodentium* inoculation. On day 10, routine histologic analysis of colons was performed using hematoxylin-and-eosin (H&E) staining. Arrows indicate mucosal ulceration with transmural inflammation. Representative images are shown, bars = 500 μm for the upper panels and bars = 250 μm for the lower panels.

**Fig. 10** depicts data demonstrating serum Ig levels in IL-22$^{-/-}$ mice and wildtype littermates during C. *rodentium* infection. IL-22$^{-/-}$ and wild type littermates mice were orally inoculated with $2x10^9$ CFU of *C. rodentium.* On indicated time points, mouse blood were collected. Levels of total serum IgM and IgG (Fig. 10(A)) and serum anti-*C. rodentium* IgG2a, IgG2b, IgG2c and IgG3 (Fig. 10(B)) were determined by ELISA. All data are representative of two independent experiments.

**Fig. 11** depicts data demonstrating an *ex vivo* colon culture ELISA of IL-22 (Fig. 11(A)) and IL-17 (Fig.11(B)) expression in C57B1/6, IL-23p19$^{-/-}$, and IL-6$^{-/-}$ mouse colons after *C. rodentium* infection. For *C. rodentium* infection, mice were orally inoculated with $2x10^9$ CFU of bacteria. All data are representative of at least two independent experiments.

**Fig. 12** depicts a FACS analysis of IL-22R expression on isolated mouse IEL, LPMCs and colonic epithelial cells (Fig. 12 (A)), and a FACS analysis of IL-22R expression on primary human colonic epithelial cells (Fig. 12 (B)). All data are representative of at least two independent experiments.

**Fig. 13** depicts data demonstrating that IL-22, produced by dendritic cells (DCs), is critical for innate immune responses against *C. rodentium* infection. In Fig. 13(A), Rag2$^{-/-}$ and wildtype Balb/c mice were orally inoculated with $2x10^9$ CFU of *C. rodentium.* In Fig. 13(B and C), the mice also received 150 μg of isotype control IgG1 mAb or anti-IL-22 mAb intraperitoneally every other day starting at the same day as bacteria inoculation and were weighed at the indicated time points. Fig. 13(B) depicts a time course real-time RT-PCR analysis, and Fig. (13(C) depicts an *ex vivo* colon culture ELISA of IL-22 and IL-17 expression in colons of wildtype Balb/c and Rag2$^{-/-}$ mice following *C. rodentium* infection. Fig. 13(D) depicts the immunohistochemical staining for IL-22, CD11c, and DAPI in day 4 colons from *C. rodentium* infected Rag2$^{-/-}$ miceMagnification: 400x. Fig. 13(E) depicts data demonstrating that IL-23 directly induces IL-22 production, as measured by ELISA, from isolated murine CD11c$^+$ DCs *in vitro.* All data are representative of two independent experiments.

**Fig. 14** depicts data demonstrating that IL-22 can induce STAT3 activation in human colon cells lines. In Fig. 14(A), IL-22$^{-/-}$ mice and wildtype littermates were orally inoculated with $2x10^9$ CFU of *C. rodentium.* One group of IL-22$^{-/-}$ mice also received mRegIIIγ-Ig fusion protein. Animals were weighed and monitored at the indicated time points. * $p < 0.05$, ** $p < 0.01$. In Fig. 14(B), IL-23 directly induces IL-22 production from isolated human DCs, measured by ELISA. Fig. 14(C) depicts IL-22R expression by FACS on human colon cell lines. Fig. 14(D) depicts a Western blotting showing that IL-22 can induce STAT3 activation in human colon cell lines. Fig.14(E) depicts a real-time RT-PCR analysis for RegIIIβ and RegIIIγ expression in human colonic epithelial cell lines treated with IL-22. All data are representative of two independent experiments.

**Fig. 15** depicts the characterization of anti-IL-22 mAb for immunohistochemisty. Fig. 15(A) depicts colon sections from day4 *C. rodentium* infected IL-22-/- and wildtype mice or uninfected wildtype mice, stained with Alexa555 conjugated anti-IL-22 mAb (8E11) or isotype control. Fig 15(B) depicts cell pellets of IL-22-expressing 293 cells stained with Alexa555 conjugated anti-IL-22 mAb (8E11) or isotype control. The magnification is at 200x.

**Fig. 16** depicts a time-course analysis on RegIIIγ and RegIIIβ expression in C57B1/6 and IL-23p19-/- mouse colons following *C. rodentium* infection. C57B1/6 and IL-23p19-/- mice were orally inoculated with $2x10^9$ CFU of *C. rodentium.* At the indicated time points, mouse colons were collected for RNA extraction and subsequently real-time RT-PCR analysis on mouse RegIIIγ and RegIIIβ expression.

**Fig. 17** depicts a time-course analysis on other Reg family members expressions in IL-22-/-and wildtype mouse colons following *C. rodentium* infection. IL-22-/- and wild type littermates mice were orally inoculated with $2x10^9$ CFU of *C. rodentium.* At the indicated time points, mouse colons were collected for RNA extraction and subsequently real-time RT-PCR analysis.

**Fig. 18** depicts data demonstrating that recombinant human RegIII_ fusion protein can partially protect IL-22-/- following *C. rodentium* infection. IL-22-/- mice and wildtype littermates were orally inoculated with $2x10^9$ CFU of *C. rodentium.* One group of IL-22-/- mice also received human RegIII_-cFlag fusion proteins. Animals were weighed and monitored at the indicated time points. * $p < 0.05$.

**Fig. 19** A-C depicts 161 genes differentially expressed in colon, from IL-22 treatment.

**Fig. 20** depicts the 2D hierarchical clustering of 161 genes differentially expressed in colon from IL-22 treatment, where selected genes were clustered by iterative agglomeration of vectors most highly linked by Pearson correlation coefficient, with data for agglomerated vectors summarized by average linkage.

**Fig. 21** depicts data demonstrating LTbRFc and anti-IL-22 mAb both lead to mortality after C. rodentium infection.

**Fig. 22** depicts data demonstrating LT pathway regulation of multiple upstream aspects involved in IL-22 production.

**Fig. 23** depicts data demonstrating IL-22 partially rescues the defects seen in LTbR treated mice.

**Fig. 24** depicts data demonstrating anti-IL-22 mAb treatment leads to reduced colon follicles, compromised B/T organization, and reduced DC, T cell and B cell numbers in the colon.

## DETAILED DESCRIPTION OF THE INVENTION

The present invention provides compositions and methods for treatment of microbial disorders by modulation of the host immune response.

The present inventors discovered a novel cytokine pathway that mediates immune response and resistance of mammals to infectious microbial pathogens. In particular, the present inventors discovered that IL-22 is one of the key cytokines that bridges adaptive immune response and innate epithelial defense during early infection of an attaching or effacing (A/E) bacterial pathogen.

As shown herein, cytokines such as IL-22 that are produced by immune cells during the early stages of infection are necessary for intestinal epithelial cells to elicit a full-anti-microbial response and wound-healing response in order to prevent systemic invasion of pathogenic microbes into the host. The studies herein show that IL-22 protects the integrity of the intestinal epithelial barrier and prevents bacterial invasion with systemic spread. Further, the studies herein indicate that IL-22 is involved in the elicitation of the early anti-bacterial IgG responses, and is indespensable for the induction of anti-microbial lectins, such as RegIII$\beta$ and RegIII$\gamma$, from colonic epithelial cells during bacterial infection. The lack of either or both of these mechanisms may contribute to the compromised host defense response with increased systemic spread and mortality in IL-$22^{-/-}$ mice during C. rodentium infection.

As shown herein, the induction of RegIIIβ and RegIIIγ indicates that IL-22 may have broader functions in controlling various bacterial infections. The studies herein further support the role of $Th_{IL-17}$ cells and their effector cytokines in infectious disorders and autoimmune disorders. Further, the studies herein indicate that IL-22 and its downstream products, such as RegIIIβ and RegIIIγ, may be beneficial for the treatment of infectious disorders.

Therefore, the present invention provides methods of treating such microbial disorders by modulation of the host immune response. For example, an anti-microbial immune response in a subject can be enhanced or inhibited by increasing or decreasing an activity of one or more anti-microbial polypeptides (AMPs) that mediate the anti-microbial immune response.

More particularly, the present invention provides AMPs, modulators thereof, and methods of using such compositions for treatment of microbial disorders. Such microbial disorders include, but are not limited to, infectious diseases, for example, EHEC- and EPEC-caused diarrhea, Inflammatory Bowel Disease (IBD) and, more particularly, Ulcerative Colitis (UC) and Crohn's Disease (CD).

All references, including patents, applications, and scientific literature, cited herein are hereby incorporated by reference, in their entirety.

## GENERAL TECHNIQUES

The techniques and procedures described or referenced herein are generally well understood and commonly employed using conventional methodology by those skilled in the art, such as, for example, the widely utilized methodologies described in Sambrook et al., Molecular Cloning: A Laboratory Manual 3rd. edition (2001) Cold Spring Harbor Laboratory Press, Cold Spring Harbor, N.Y.; Current Protocols in Molecular Biology (F. M. Ausubel, et al. eds., (2003)); the series Methods in Enzymology (Academic Press, Inc.): PCR 2: A Practical Approach (M. J. MacPherson, B. D. Hames and G. R. Taylor eds. (1995)), Harlow and Lane, eds. (1988) Antibodies, A Laboratory Manual, and Animal Cell Culture (R. I. Freshney, ed. (1987)); Oligonucleotide Synthesis (M. J. Gait, ed., 1984); Methods in Molecular Biology, Humana Press; Cell Biology: A Laboratory Notebook (J. E. Cellis, ed., 1998) Academic Press; Animal Cell Culture (R. I. Freshney), ed., 1987); Introduction to Cell and Tissue Culture (J. P. Mather and P. E. Roberts, 1998) Plenum Press; Cell and Tissue Culture: Laboratory Procedures (A. Doyle, J. B. Griffiths, and D. G. Newell, eds., 1993-8) J. Wiley and Sons; Handbook of Experimental Immunology (D. M. Weir and C. C. Blackwell, eds.); Gene Transfer Vectors for Mammalian Cells (J. M. Miller and M. P. Calos, eds., 1987); PCR: The Polymerase Chain Reaction, (Mullis et al., eds., 1994); Current Protocols in Immunology (J. E. Coligan et al., eds., 1991); Short Protocols in Molecular Biology (Wiley and Sons, 1999); Immunobiology (C. A. Janeway and P. Travers, 1997); Antibodies (P. Finch, 1997); Antibodies: A Practical Approach (D. Catty., ed., IRL Press, 1988-1989); Monoclonal Antibodies: A Practical Approach (P. Shepherd and C. Dean, eds., Oxford University Press, 2000); Using Antibodies: A Laboratory Manual (E. Harlow and D. Lane (Cold Spring Harbor Laboratory Press, 1999); The Antibodies (M. Zanetti and J. D. Capra, eds., Harwood Academic Publishers, 1995); and Cancer: Principles and Practice of Oncology (V. T. DeVita et al., eds., J.B. Lippincott Company, 1993).

## I. DEFINITIONS

For purposes of interpreting this specification, the following definitions will apply and whenever appropriate, terms used in the singular will also include the plural and vice versa. In the event that any definition set forth below conflicts with any document incorporated herein by reference, the definition set forth below shall control.

An "anti-microbial polypeptide" or "AMP" is a polypeptide that mediates, or otherwise effects, an anti-microbial immune response to a microbial pathogen, and encompasses encompasses a fragment, variant, analog, derivative or mimetic thereof that retains an AMP activity, e.g., an anti-microbial activity, or activity for modulating an anti-microbial immune response. These methods can be used to treat subjects that are infected with or at risk for infection with an infectious microbial pathogen, e.g., a virus or bacterium. The activity of the AMP can be modulated or differentially regulated (e.g., up or down regulated) relative to another AMP or the same AMP.

An AMP of the present invention encompasses a native AMP and variant forms thereof (which are further defined herein), and may be isolated from a variety of sources, such as from human tissue or from another source, or prepared by recombinant or synthetic methods. A native AMP may be from any species, e.g., murine or human. AMPs of the present invention include, but are not limited to, LT, IL-6, IL-18, IL-22, IL-23 (including e.g., IL-23 p19 or IL-23 p40), and Reg or Reg-related proteins encoded by the genes of the Reg super family. The Reg super family includes Reg and Reg-related genes from human, rat, and mouse and are grouped into four subclasses, types I, II, III, and IV. For example, type I includes human *REG Iα*, human *REG Iβ*, rat *RegI*, and mouse *RegI*; type II includes mouse *RegII*; type III includes human REG III, human *HIP*/*PAP* (gene expressed in hepatocellular carcinoma-intestine-pancreas/gene encoding pancreatitis-associated protein), rat *PAP*/*Peptide23*, rat *RegIII*/*PAPII*, rat *PAP III,* mouse *RegIIIα*, *RegIIIβ*, *RegIIIγ*, mouse *RegIIIδ*, and hamster *INGAP* (islet neogenesis-associated protein). Type IV contains human *REG IV.* Additionally, human Reg-related Sequence (RS) is reportedly a pseudogene. In one embodiment, the REG protein is encoded by a member of the human REG gene family which includes, but is not limited to, REG Iα, REG Iβ, HIP/PAP, REG III, REG IV, and Reg-related sequence (RS).

Lymphotoxin (LT) is a trimeric cytokine in the tumor necrosis family; expressed by activated T, B, and NK cells; and involved in inflammatory response signaling and secondary lymphoid organ architecture. "Lymphotoxin-" or "LT" is defined herein as a biologically active polypeptide having the amino acid sequence shown in FIG. 2A of US Pat. No. 5,824,509. "LT" is defined to specifically exclude human TNFα or its natural animal analogues (Pennica et al., Nature 312:20/27 : 724-729 (1984) and Aggarwal et al., J. Biol. Chem. 260: 2345-2354 (1985)). As used herein, "LT" refers to one or more LT subunits as described herein.

"Lymphotoxin-a" or "LTα" is defined to specifically exclude human LTβ as defined, for example, in US 5,661,004. "Lymphotoxinα-3 trimer" or "LTα3" refers to a homotrimer of LTα monomers. This homotrimer is anchored to the cell surface by the LTβ, transmembrane and cytoplasmic domains.

"Lymphotoxin-αβ" or "LTαβ" or "LTαβ complex" refers to a heterotrimer of LTα with LTβ. These heterotrimers contain either two subunits of LTα and one subunit of LTβ (LTα2β1), or one subunit of LTα and two of LTβ (LTα1β2). The term "LTαβ" or "LTab" as used herein refers to a heterotrimer made up of one subunit of LTα and two of LTβ (LTα1β2).

"Tumor necrosis factor receptor-I" or "TNFRI" and "tumor necrosis factor receptor-II" or "TNFRII" refer to cell-surface TNF receptors for the LTα3 homotrimer, also known as p55 and p75, respectively.

"Lymphotoxin-β receptor" or "LTβ-K" refers to the receptor to which the LTαβ heterotrimers bind.

In some embodiments, the amino acid sequence of an AMP of the present invention comprises an amino acid sequence selected from the following group: SEQ ID NO: 2 (human IL-6), SEQ ID NO: 4 (human IL-12B), SEQ ID NO: 6 (human IL-18), SEQ ID NO: 8 (human IL-22), SEQ ID NO: 10 (human IL-23 p19 or IL-23A), SEQ ID NO: 12 (human REG1A), SEQ ID NO: 14 (human REG1B), SEQ ID NO: 16 (human REG3A, variant 1), SEQ ID NO: 18 (human REG3A, variant 2), SEQ ID NO: 20 (human REG3A, variant 3), SEQ ID NO: 22 (human REG3G, variant 2), SEQ ID NO: 24 (human REG3G, variant 1), SEQ ID NO: 26 (human REG4), SEQ ID NO: 28 (murine IL-6), SEQ ID NO: 30 (murine IL-12B), SEQ ID NO: 32 (murine IL-18), SEQ ID NO: 34 (murine IL-22), SEQ ID NO: 36 (murine IL-23 p19 or IL-23A), SEQ ID NO: 38 (murine PAP), SEQ ID NO: 40 (murine REG1), SEQ ID NO: 42 (murine REG2), SEQ ID NO: 44 (murine REG3A), SEQ ID NO: 46 (murine REG3D), SEQ ID NO: 48 (murine REG4), SEQ ID NO: 50 (human LTα), SEQ ID NO: 52 (human LTβ), SEQ ID NO: 54 (murine LTα), and SEQ ID NO: 56 (murine LTβ).

In other embodiments, the nucleic acid sequence encoding an AMP of the present invention comprises a nucleic acid sequence selected from the following group: SEQ ID NO: 1 (human IL-6), SEQ ID NO: 3 (human IL-12B), SEQ ID NO: 5 (human IL-18), SEQ ID NO: 7 (human IL-22), SEQ ID NO: 9 (human IL-23 p19 or IL-23A), SEQ ID NO: 11 (human REG1A), SEQ ID NO: 13 (human REG1B), SEQ ID NO: 15 (human REG3A, variant 1), SEQ ID NO: 17 (human REG3A, variant 2), SEQ ID NO: 19 (human REG3A, variant 3), SEQ ID NO: 21 (human REG3G, variant 2), SEQ ID NO: 23 (human REG3G, variant 1), SEQ ID NO: 25 (human REG4), SEQ ID NO: 27 (murine IL-6), SEQ ID NO: 29 (murine IL-12B), SEQ ID NO: 31 (murine IL-18), SEQ ID NO: 33 (murine IL-22), SEQ ID NO: 35 (murine IL-23 p19 or IL-23A), SEQ ID NO: 37 (murine PAP), SEQ ID NO: 39 (murine REG1), SEQ ID NO: 41 (murine REG2), SEQ ID NO: 43 (murine REG3A), SEQ ID NO: 45 (murine REG3D), SEQ ID NO: 47 (murine REG4), SEQ ID NO: 49 (human LT$\alpha$), SEQ ID NO: 51 (human LT$\beta$), SEQ ID NO: 53 (murine LT$\alpha$), and SEQ ID NO: 55 (murine LT$\beta$).

A "native sequence AMP polypeptide" or a "native sequence AMP polypeptide" refers to a polypeptide comprising the same amino acid sequence as a corresponding AMP polypeptide derived from nature. Such native sequence AMP polypeptides can be isolated from nature or can be produced by recombinant or synthetic means. The terms specifically encompass naturally-occurring truncated or secreted forms of the specific AMP polypeptide (*e.g.*, an IL-22 lacking its associated signal peptide), naturally-occurring variant forms (*e.g.*, alternatively spliced forms), and naturally-occurring allelic variants of the polypeptide. In various embodiments of the invention, the native sequence AMP polypeptides disclosed herein are mature or full-length native sequence polypeptides.

A "variant " polypeptide, refers to an active polypeptide having at least about 80% amino acid sequence identity with a full-length native polypeptide sequence. Ordinarily, a variant polypeptide will have at least about 80% amino acid sequence identity, alternatively at least about 81% amino acid sequence identity, alternatively at least about 82% amino acid sequence identity, alternatively at least about 83% amino acid sequence identity, alternatively at least about 84% amino acid sequence identity, alternatively at least about 85% amino acid sequence identity, alternatively at least about 86% amino acid sequence identity, alternatively at least about 87% amino acid sequence identity, alternatively at least about 88% amino acid sequence identity, alternatively at least about 89% amino acid sequence identity, alternatively at least about 90% amino acid sequence identity, alternatively at least about 91% amino acid sequence identity, alternatively at least about 92% amino acid sequence identity, alternatively at least about 93% amino acid sequence identity, alternatively at least about 94% amino acid sequence identity, alternatively at least about 95% amino acid sequence identity, alternatively at least about 96% amino acid sequence identity, alternatively at least about 97% amino acid sequence identity, alternatively at least about 98% amino acid sequence identity, and alternatively at least about 99% amino acid sequence identity to a full-length or mature native polypeptide sequence.

"Percent (%) amino acid sequence identity," is defined as the percentage of amino acid residues in a candidate sequence that are identical with the amino acid residues in a specific or reference polypeptide sequence, after aligning the sequences and introducing gaps, if necessary, to achieve the maximum percent sequence identity, and not considering any conservative substitutions as part of the sequence identity. Alignment for purposes of determining percent amino acid sequence identity can be achieved in various ways that are within the skill in the art, for instance, using publicly available computer software such as BLAST, BLAST-2, ALIGN or Megalign (DNASTAR) software. Those skilled in the art can determine appropriate parameters for measuring alignment, including any algorithms needed to achieve maximal alignment over the full length of the sequences being compared. For amino acid sequence comparisons, the % amino acid sequence identity of a given amino acid sequence A to, with, or against a given amino acid sequence B (which can alternatively be phrased as a given amino acid sequence A that has or comprises a certain % amino acid sequence identity to, with, or against a given amino acid sequence B) is calculated as follows:

$$100 \text{ times the fraction } X/Y$$

where X is the number of amino acid residues scored as identical matches by the sequence alignment program in that program's alignment of A and B, and where Y is the total number of amino acid residues in B. It will be appreciated that where the length of amino acid sequence A is not equal to the length of amino acid sequence B, the % amino acid sequence identity of A to B will not equal the % amino acid sequence identity of B to A. As examples of % amino acid sequence identity calculations using this method, Tables 1 and 2 below demonstrate how to calculate the % amino acid sequence identity of the amino acid sequence designated "Reference Protein" to the amino acid sequence designated "IL-22", wherein "IL-22" represents the amino acid sequence of an IL-22 polypeptide of interest, "Reference Protein" represents the amino acid sequence of a polypeptide against which the "IL-22" polypeptide of interest is being compared, and "X, "Y" and "Z" each represent different amino acid residues.

**Table 1**

| | |
|---|---|
| IL-22 | XXXXXXXXXXXXXXX (Length = 15 amino acids) |

(continued)

| | |
|---|---|
| Reference Protein | XXXXXYYYYYYY (Length = 12 amino acids) |
| % amino acid sequence identity = | (the number of identically matching amino acid residues between the two polypeptide sequences) divided by (the total number of amino acid residues of the IL-22 polypeptide) = 5 divided by 15 = 33.3% |

**Table 2**

| | |
|---|---|
| IL-22 | XXXXXXXXXX (Length = 10 amino acids) |
| Reference Protein | XXXXXYYYYYYZZYZ (Length = 15 amino acids) |
| % amino acid sequence identity = | (the number of identically matching amino acid residues between the two polypeptide sequences) divided by (the total number of amino acid residues of the IL-22 polypeptide) = 5 divided by 10 = 50% |

An "isolated" biological molecule, such as the various polypeptides, polynucleotides, and antibodiesdisclosed herein, refers to a biological molecule that has been identified and separated and/or recovered from at least one component of its natural environment.

"Active" or "activity," with reference to a polypeptide, refers to a biological and/or an immunological activity of a native polypeptide, wherein "biological" activity refers to a biological function of a native polypeptide other than the ability to induce the production of an antibody against an antigenic epitope possessed by the native polypeptide. An "immunological" activity refers to the ability to induce the production of an antibody against an antigenic epitope possessed by a native polypeptide.

The term "antagonist" is used in the broadest sense, and includes any molecule that partially or fully blocks, inhibits, or neutralizes a biological activity of a polypeptide. Also encompassed by "antagonist" are molecules that fully or partially inhibit the transcription or translation of mRNA encoding the polypeptide. Suitable antagonist molecules include, e.g., antagonist antibodies or antibody fragments; fragments or amino acid sequence variants of a native polypeptide; peptides; antisense oligonucleotides; small organic molecules; and nucleic acids that encode polypeptide antagonists or antagonist antibodies.. Reference to "an" antagonist encompasses a single antagonist or a combination of two or more different antagonists.

The term "agonist" is used in the broadest sense and includes any molecule that partially or fully mimics a biological activity of a polypeptide, e.g., a native AMP. Also encompassed by "agonist" are molecules that stimulate the transcription or translation of mRNA encoding the polypeptide. Suitable agonist molecules include, e.g., agonist antibodies or antibody fragments; a native polypeptide; fragments or amino acid sequence variants of a native polypeptide; peptides; antisense oligonucleotides; small organic molecules; and nucleic acids that encode polypeptides agonists or antibodies. Reference to "an" agonist encompasses a single agonist or a combination of two or more different agonists.

An "anti-microbial immune response" includes, but is not limited to, resistance or defense to infection by a microbial pathogen. Such resistance or defense can result in an inhibition or decrease in microbial infectivity, replication, proliferation or other activity of a microbial pathogen. In particular, treatment resulting in an anti-microbial immune response can result in the alleviation of a microbial disorder or symptom of a microbial disorder.

"Alleviation", "alleviating" or equivalents thereof, refers to both therapeutic treatment and prophylactic or preventative measures, wherein the object is to ameliorate, prevent, slow down (lessen), decrease or inhibit the targeted microbial disorder or symptom thereof. Those in need of treatment include those already with the disorder as well as those prone to having the disorder or those in whom the disorder is to be prevented.

With reference to treating a microbial disorder, "treatment", "treating", or equivalents thereof, refers to alleviating a microbial disorder or a symptom of a microbial disorder, in a subject having the disorder.

"Chronic" administration refers to administration of an agent(s) in a continuous mode as opposed to an acute mode, so as to maintain the initial therapeutic effect for an extended period of time. "Intermittent" administration is treatment that is not consecutively done without interruption, but rather is cyclic in nature.

"Mammal" for purposes of treatment refers to any animal classified as a mammal, including humans, rodents (e.g., mice and rats), and monkeys; domestic and farm animals; and zoo, sports, laboratory, or pet animals, such as dogs, cats, cattle, horses, sheep, pigs, goats, rabbits, etc. In some embodiments, the mammal is selected from a human, rodent, or monkey. Similarly, "subject" for the purposes of treatment, refers to a mammalian subject, and includes both human and veterinary subjects.

Administration "in combination with" one or more further therapeutic agents includes simultaneous (concurrent) and consecutive administration in any order.

"Carriers" as used herein include pharmaceutically acceptable carriers, excipients, or stabilizers which are nontoxic to the cell or mammal being exposed thereto at the dosages and concentrations employed. Often the physiologically acceptable carrier is an aqueous pH buffered solution. Examples of physiologically acceptable carriers include buffers such as phosphate, citrate, and other organic acids; antioxidants including ascorbic acid; low molecular weight (less than about 10 residues) polypeptide; proteins, such as serum albumin, gelatin, or immunoglobulins; hydrophilic polymers such as polyvinylpyrrolidone; amino acids such as glycine, glutamine, asparagine, arginine or lysine; monosaccharides, disaccharides, and other carbohydrates including glucose, mannose, or dextrins; chelating agents such as EDTA; sugar alcohols such as mannitol or sorbitol; salt-forming counterions such as sodium; and/or nonionic surfactants such as TWEEN™, polyethylene glycol (PEG), and PLURONICS™.

"Antibodies" (Abs) and "immunoglobulins" (Igs) are glycoproteins having similar structural characteristics. While antibodies exhibit binding specificity to a specific antigen, immunoglobulins include both antibodies and other antibody-like molecules which generally lack antigen specificity. Polypeptides of the latter kind are, for example, produced at low levels by the lymph system and at increased levels by myelomas.

The terms "antibody" and "immunoglobulin" are used interchangeably in the broadest sense and include monoclonal antibodies (*e.g.*, full length or intact monoclonal antibodies), polyclonal antibodies, monovalent antibodies, multivalent antibodies, multispecific antibodies (*e.g.*, bispecific antibodies so long as they exhibit the desired biological activity) and may also include certain antibody fragments (as described in greater detail herein). An antibody can be chimeric, human, humanized and/or affinity matured.

An antibody that specifically binds to a particular antigen refers to an antibody that is capable of binding the antigen with sufficient affinity such that the antibody is useful as a diagnostic and/or therapeutic agent in targeting the antigen. Preferably, the extent of binding of such an antibody to a non-target polypeptide is less than about 10% of the binding of the antibody to the target antigen as measured, e.g., by a radioimmunoassay (RIA). In certain embodiments, an antibody that binds to a target antigen has a dissociation constant (Kd) of $\leq 1 \mu M$, $\leq 100$ nM, $\leq 10$ nM, $\leq 1$ nM, or $\leq 0.1$ nM.

The "variable region" or "variable domain" of an antibody refers to the amino-terminal domains of the heavy or light chain of the antibody. The variable domain of the heavy chain may be referred to as "VH." The variable domain of the light chain may be referred to as "VL." These domains are generally the most variable parts of an antibody and contain the antigen-binding sites.

The term "variable" refers to the fact that certain portions of the variable domains differ extensively in sequence among antibodies and are used in the binding and specificity of each particular antibody for its particular antigen. However, the variability is not evenly distributed throughout the variable domains of antibodies. It is concentrated in three segments called complementarity-determining regions (CDRs) or hypervariable regions (HVRs) both in the light-chain and the heavy-chain variable domains. The more highly conserved portions of variable domains are called the framework regions (FR). The variable domains of native heavy and light chains each comprise four FR regions, largely adopting a beta-sheet configuration, connected by three CDRs, which form loops connecting, and in some cases forming part of, the beta-sheet structure. The CDRs in each chain are held together in close proximity by the FR regions and, with the CDRs from the other chain, contribute to the formation of the antigen-binding site of antibodies (see Kabat et al., Sequences of Proteins of Immunological Interest, Fifth Edition, National Institute of Health, Bethesda, MD (1991)). The constant domains are not involved directly in the binding of an antibody to an antigen, but exhibit various effector functions, such as participation of the antibody in antibody-dependent cellular toxicity.

The "light chains" of antibodies (immunoglobulins) from any vertebrate species can be assigned to one of two clearly distinct types, called kappa ($\kappa$) and lambda ($\lambda$), based on the amino acid sequences of their constant domains.

Depending on the amino acid sequences of the constant domains of their heavy chains, antibodies (immunoglobulins) can be assigned to different classes. There are five major classes of immunoglobulins: IgA, IgD, IgE, IgG and IgM, and several of these may be further divided into subclasses (isotypes), e.g., $IgG_1$, $IgG_2$, $IgG_3$, $IgG_4$, $IgA_1$, and $IgA_2$. The heavy chain constant domains that correspond to the different classes of immunoglobulins are called $\alpha$, $\delta$, $\varepsilon$, $\gamma$, and $\mu$, respectively. The subunit structures and three-dimensional configurations of different classes of immunoglobulins are well known and described generally in, for example, Abbas et al. Cellular and Mol. Immunology, 4th ed. (2000). An antibody may be part of a larger fusion molecule, formed by covalent or non-covalent association of the antibody with one or more other proteins or peptides.

The terms "full length antibody," "intact antibody" and "whole antibody" are used herein interchangeably to refer to an antibody in its substantially intact form, not antibody fragments as defined below. The terms particularly refer to an antibody with heavy chains that contain the Fc region.

"Antibody fragments" comprise only a portion of an intact antibody, wherein the portion retains at least one, and as many as most or all, of the functions normally associated with that portion when present in an intact antibody. In one embodiment, an antibody fragment comprises an antigen binding site of the intact antibody and thus retains the ability to bind antigen. In another embodiment, an antibody fragment, for example one that comprises the Fc region, retains at least one of the biological functions normally associated with the Fc region when present in an intact antibody, such as FcRn binding, antibody half life modulation, ADCC function and complement binding. In one embodiment, an antibody fragment is a monovalent antibody that has an *in vivo* half life substantially similar to an intact antibody. For example, such an antibody fragment may comprise on antigen binding arm linked to an Fc sequence capable of conferring *in vivo* stability to the fragment.

Papain digestion of antibodies produces two identical antigen-binding fragments, called "Fab" fragments, each with a single antigen-binding site, and a residual "Fc" fragment, whose name reflects its ability to crystallize readily. Pepsin treatment yields an $F(ab')_2$ fragment that has two antigen-combining sites and is still capable of cross-linking antigen.

"Fv" is the minimum antibody fragment which contains a complete antigen-binding site. In one embodiment, a two-chain Fv species consists of a dimer of one heavy- and one light-chain variable domain in tight, non-covalent association. In a single-chain Fv (scFv) species, one heavy- and one light-chain variable domain can be covalently linked by a flexible peptide linker such that the light and heavy chains can associate in a "dimeric" structure analogous to that in a two-chain Fv species. It is in this configuration that the three CDRs of each variable domain interact to define an antigen-binding site on the surface of the VH-VL dimer. Collectively, the six CDRs confer antigen-binding specificity to the antibody. However, even a single variable domain (or half of an Fv comprising only three CDRs specific for an antigen) has the ability to recognize and bind antigen, although at a lower affinity than the entire binding site.

The Fab fragment contains the heavy- and light-chain variable domains and also contains the constant domain of the light chain and the first constant domain (CH1) of the heavy chain. Fab' fragments differ from Fab fragments by the addition of a few residues at the carboxy terminus of the heavy chain CH1 domain including one or more cysteines from the antibody hinge region. Fab'-SH is the designation herein for Fab' in which the cysteine residue(s) of the constant domains bear a free thiol group. $F(ab')_2$ antibody fragments originally were produced as pairs of Fab' fragments which have hinge cysteines between them. Other chemical couplings of antibody fragments are also known.

"Single-chain Fv" or "scFv" antibody fragments comprise the VH and VL domains of antibody, wherein these domains are present in a single polypeptide chain. Generally, the scFv polypeptide further comprises a polypeptide linker between the VH and VL domains which enables the scFv to form the desired structure for antigen binding. For a review of scFv see Pluckthun, in The Pharmacology of Monoclonal Antibodies, vol. 113, Rosenburg and Moore eds., Springer-Verlag, New York, pp. 269-315 (1994).

The term "diabodies" refers to small antibody fragments with two antigen-binding sites, which fragments comprise a heavy-chain variable domain (VH) connected to a light-chain variable domain (VL) in the same polypeptide chain (VH-VL). By using a linker that is too short to allow pairing between the two domains on the same chain, the domains are forced to pair with the complementary domains of another chain and create two antigen-binding sites. Diabodies may be bivalent or bispecific. Diabodies are described more fully in, for example, EP 404,097; WO93/1161; Hudson et al. (2003) Nat. Med. 9:129-134; and Hollinger et al., Proc. Natl. Acad. Sci. USA 90: 6444-6448 (1993). Triabodies and tetrabodies are also described in Hudson et al. (2003) Nat. Med. 9:129-134.

The term "monoclonal antibody" as used herein refers to an antibody obtained from a population of substantially homogeneous antibodies, i.e., the individual antibodies comprising the population are identical except for possible mutations, e.g., naturally occurring mutations, that may be present in minor amounts. Thus, the modifier "monoclonal" indicates the character of the antibody as not being a mixture of discrete antibodies. In certain embodiments, such a monoclonal antibody typically includes an antibody comprising a polypeptide sequence that binds a target, wherein the target-binding polypeptide sequence was obtained by a process that includes the selection of a single target binding polypeptide sequence from a plurality of polypeptide sequences. For example, the selection process can be the selection of a unique clone from a plurality of clones, such as a pool of hybridoma clones, phage clones, or recombinant DNA clones. It should be understood that a selected target binding sequence can be further altered, for example, to improve affinity for the target, to humanize the target binding sequence, to improve its production in cell culture, to reduce its immunogenicity *in vivo,* to create a multispecific antibody, etc., and that an antibody comprising the altered target binding sequence is also a monoclonal antibody of this invention. In contrast to polyclonal antibody preparations which typically include different antibodies directed against different determinants (epitopes), each monoclonal antibody of a monoclonal antibody preparation is directed against a single determinant on an antigen. In addition to their specificity, monoclonal antibody preparations are advantageous in that they are typically uncontaminated by other immunoglobulins.

The modifier "monoclonal" indicates the character of the antibody as being obtained from a substantially homogeneous population of antibodies, and is not to be construed as requiring production of the antibody by any particular method. For example, the monoclonal antibodies to be used in accordance with the present invention may be made by a variety of techniques, including, for example, the hybridoma method (e.g., Kohler et al., Nature, 256: 495 (1975); Harlow et al., Antibodies: A Laboratory Manual, (Cold Spring Harbor Laboratory Press, 2nd ed. 1988); Hammerling et al., in: Monoclonal Antibodies and T-Cell Hybridomas 563-681 (Elsevier, N.Y., 1981)), recombinant DNA methods (see, e.g., U.S. Patent No. 4,816,567), phage display technologies (see, e.g., Clackson et al., Nature, 352: 624-628 (1991); Marks et al., J. Mol. Biol. 222: 581-597 (1992); Sidhu et al., J. Mol. Biol. 338(2): 299-310 (2004); Lee et al., J. Mol. Biol. 340(5): 1073-1093 (2004); Fellouse, Proc. Natl. Acad. Sci. USA 101(34): 12467-12472 (2004); and Lee et al., J. Immunol. Methods 284(1-2): 119-132(2004), and technologies for producing human or human-like antibodies in animals that have parts or all of the human immunoglobulin loci or genes encoding human immunoglobulin sequences (see, e.g.,

WO98/24893; WO96/34096; WO96/33735; WO91/10741; Jakobovits et al., Proc. Natl. Acad. Sci. USA 90: 2551 (1993); Jakobovits et al., Nature 362: 255-258 (1993); Bruggemann et al., Year in Immunol. 7:33 (1993); U.S. Patent Nos. 5,545,807; 5,545,806; 5,569,825; 5,625,126; 5,633,425; 5,661,016; Marks et al., Bio.Technology 10: 779-783 (1992); Lonberg et al., Nature 368: 856-859 (1994); Morrison, Nature 368: 812-813 (1994); Fishwild et al., Nature Biotechnol. 14: 845-851 (1996); Neuberger, Nature Biotechnol. 14: 826 (1996) and Lonberg and Huszar, Intern. Rev. Immunol. 13: 65-93 (1995).

The monoclonal antibodies herein specifically include "chimeric" antibodies in which a portion of the heavy and/or light chain is identical with or homologous to corresponding sequences in antibodies derived from a particular species or belonging to a particular antibody class or subclass, while the remainder of the chain(s) is identical with or homologous to corresponding sequences in antibodies derived from another species or belonging to another antibody class or subclass, as well as fragments of such antibodies, so long as they exhibit the desired biological activity (U.S. Patent No. 4,816,567; and Morrison et al., Proc. Natl. Acad. Sci. USA 81:6851-6855 (1984)).

"Humanized" forms of non-human (*e.g.*, murine) antibodies are chimeric antibodies that contain minimal sequence derived from non-human immunoglobulin. In one embodiment, a humanized antibody is a human immunoglobulin (recipient antibody) in which residues from a hypervariable region of the recipient are replaced by residues from a hypervariable region of a non-human species (donor antibody) such as mouse, rat, rabbit, or nonhuman primate having the desired specificity, affinity, and/or capacity. In some instances, framework region (FR) residues of the human immunoglobulin are replaced by corresponding non-human residues. Furthermore, humanized antibodies may comprise residues that are not found in the recipient antibody or in the donor antibody. These modifications may be made to further refine antibody performance. In general, a humanized antibody will comprise substantially all of at least one, and typically two, variable domains, in which all or substantially all of the hypervariable loops correspond to those of a non-human immunoglobulin, and all or substantially all of the FRs are those of a human immunoglobulin sequence. The humanized antibody optionally will also comprise at least a portion of an immunoglobulin constant region (Fc), typically that of a human immunoglobulin. For further details, see Jones et al., Nature 321:522-525 (1986); Riechmann et al., Nature 332:323-329 (1988); and Presta, Curr. Op. Struct. Biol. 2:593-596 (1992). See also the following review articles and references cited therein: Vaswani and Hamilton, Ann. Allergy, Asthma & Immunol. 1:105-115 (1998); Harris, Biochem. Soc. Transactions 23:1035-1038 (1995); Hurle and Gross, Curr. Op. Biotech. 5:428-433 (1994).

A "human antibody" is one which possesses an amino acid sequence which corresponds to that of an antibody produced by a human and/or has been made using any of the techniques for making human antibodies as disclosed herein. This definition of a human antibody specifically excludes a humanized antibody comprising non-human antigen-binding residues.

Depending on the amino acid sequence of the constant domain of their heavy chains, immunoglobulins can be assigned to different classes. There are five major classes of immunoglobulins: IgA, IgD, IgE, IgG, and IgM, and several of these may be further divided into subclasses (isotypes), e.g., IgG1, IgG2, IgG3, IgG4, IgA, and IgA2.

An "affinity matured" antibody is one with one or more alterations in one or more HVRs thereof which result in an improvement in the affinity of the antibody for antigen, compared to a parent antibody which does not possess those alteration(s). In one embodiment, an affinity matured antibody has nanomolar or even picomolar affinities for the target antigen. Affinity matured antibodies may be produced by procedures known in the art. Marks et al. Bio/Technology 10:779-783 (1992) describes affinity maturation by VH and VL domain shuffling. Random mutagenesis of HVR and/or framework residues is described by: Barbas et al. Proc Nat. Acad. Sci. USA 91:3809-3813 (1994); Schier et al. Gene 169:147-155 (1995); Yelton et al. J. Immunol. 155:1994-2004 (1995); Jackson et al., J. Immunol. 154(7):3310-9 (1995); and Hawkins et al, J. Mol. Biol. 226:889-896 (1992).

A "blocking" antibody, "neutralizing" antibody, or "antagonist" antibody is one which inhibits or reduces a biological activity of the antigen it binds. Such antibodies may substantially or completely inhibit the biological activity of the antigen.

An "agonist antibody," as used herein, is an antibody which partially or fully mimics a biological activity of a polypeptide of interest.

Antibody "effector functions" refer to those biological activities attributable to the Fc region (a native sequence Fc region or amino acid sequence variant Fc region) of an antibody, and vary with the antibody isotype. Examples of antibody effector functions include: C1q binding and complement dependent cytotoxicity; Fc receptor binding; antibody-dependent cell-mediated cytotoxicity (ADCC); phagocytosis; down regulation of cell surface receptors (*e.g.* B cell receptor); and B cell activation.

"Binding affinity" generally refers to the strength of the sum total of noncovalent interactions between a single binding site of a molecule (e.g., an antibody) and its binding partner (e.g., an antigen). Unless indicated otherwise, as used herein, "binding affinity" refers to intrinsic binding affinity which reflects a 1:1 interaction between members of a binding pair (e.g., antibody and antigen). The affinity of a molecule X for its partner Y can generally be represented by the dissociation constant (Kd). Affinity can be measured by common methods known in the art, including those described herein. Low-affinity antibodies generally bind antigen slowly and tend to dissociate readily, whereas high-affinity antibodies generally bind antigen faster and tend to remain bound longer. A variety of methods of measuring binding affinity are known in the art, any of which can be used for purposes of the present invention. Specific illustrative embodiments are described in the following.

In one embodiment, the "Kd" or "Kd value" according to this invention is measured by a radiolabeled antigen binding assay (RIA) performed with the Fab version of an antibody of interest and its antigen as described by the following assay. Solution binding affinity of Fabs for antigen is measured by equilibrating Fab with a minimal concentration of ($^{125}$I)-labeled antigen in the presence of a titration series of unlabeled antigen, then capturing bound antigen with an anti-Fab antibody-coated plate (Chen, et al., (1999) J. Mol. Biol. 293:865-881). To establish conditions for the assay, microtiter plates (Dynex) are coated overnight with 5 μg/ml of a capturing anti-Fab antibody (Cappel Labs) in 50 mM sodium carbonate (pH 9.6), and subsequently blocked with 2% (w/v) bovine serum albumin in PBS for two to five hours at room temperature (approximately 23°C). In a non-adsorbent plate (Nunc #269620), 100 pM or 26 pM [$^{125}$I]-antigen are mixed with serial dilutions of a Fab of interest (e.g., consistent with assessment of the anti-VEGF antibody, Fab-12, in Presta et al., (1997) Cancer Res. 57:4593-4599). The Fab of interest is then incubated overnight; however, the incubation may continue for a longer period (e.g., about 65 hours) to ensure that equilibrium is reached. Thereafter, the mixtures are transferred to the capture plate for incubation at room temperature (e.g., for one hour). The solution is then removed and the plate washed eight times with 0.1% Tween-20 in PBS. When the plates have dried, 150 μl/well of scintillant (MicroScint-20; Packard) is added, and the plates are counted on a Topcount gamma counter (Packard) for ten minutes. Concentrations of each Fab that give less than or equal to 20% of maximal binding are chosen for use in competitive binding assays.

According to another embodiment, the Kd or Kd value is measured by surface plasmon resonance assays using a BIAcore™-2000 or a BIAcore™-3000 (BIAcore, Inc., Piscataway, NJ) at 25°C with immobilized antigen CM5 chips at ~10 response units (RU). Briefly, carboxymethylated dextran biosensor chips (CM5, BIAcore Inc.) are activated with *N*-ethyl-*N*'- (3-dimethylaminopropyl)-carbodiimide hydrochloride (EDC) and *N*-hydroxysuccinimide (NHS) according to the supplier's instructions. Antigen is diluted with 10 mM sodium acetate, pH 4.8, to 5 μg/ml (~0.2 μM) before injection at a flow rate of 5 μl/minute to achieve approximately 10 response units (RU) of coupled protein. Following the injection of antigen, 1 M ethanolamine is injected to block unreacted groups. For kinetics measurements, two-fold serial dilutions of Fab (0.78 nM to 500 nM) are injected in PBS with 0.05% Tween 20 (PBST) at 25°C at a flow rate of approximately 25 μl/min. Association rates ($k_{on}$) and dissociation rates ($k_{off}$) are calculated using a simple one-to-one Langmuir binding model (BIAcore Evaluation Software version 3.2) by simultaneously fitting the association and dissociation sensorgrams. The equilibrium dissociation constant (Kd) is calculated as the ratio $k_{off}/k_{on.}$ See, e.g., Chen, Y., et al., (1999) J. Mol. Biol. 293:865-881. If the on-rate exceeds $10^6$ $M^{-1}$ $s^{-1}$ by the surface plasmon resonance assay above, then the on-rate can be determined by using a fluorescent quenching technique that measures the increase or decrease in fluorescence emission intensity (excitation = 295 nm; emission = 340 nm, 16 nm band-pass) at 25°C of a 20 nM anti-antigen antibody (Fab form) in PBS, pH 7.2, in the presence of increasing concentrations of antigen as measured in a spectrometer, such as a stop-flow equipped spectrophometer (Aviv Instruments) or a 8000-series SLM-Aminco spectrophotometer (ThermoSpectronic) with a stirred cuvette.

An "on-rate," "rate of association," "association rate," or "$k_{on}$" according to this invention can also be determined as described above using a BIAcore™-2000 or a BIAcore™-3000 system (BIAcore, Inc., Piscataway, NJ).

An "isolated" antibody is one which has been identified and separated and/or recovered from a component of its natural environment. Contaminant components of its natural environment are materials which would interfere with diagnostic or therapeutic uses for the antibody, and may include enzymes, hormones, and other proteinaceous or nonproteinaceous solutes. In preferred embodiments, the antibody will be purified (1) to greater than 95% by weight of antibody as determined by the Lowry method, and most preferably more than 99% by weight, (2) to a degree sufficient to obtain at least 15 residues ofN-terminal or internal amino acid sequence by use of a spinning cup sequenator, or (3) to homogeneity by SDS-PAGE under reducing or nonreducing conditions using Coomassie blue or, preferably, silver stain. Isolated antibody includes the antibody in situ within recombinant cells since at least one component of the antibody's natural environment will not be present. Ordinarily, however, isolated antibody will be prepared by at least one purification step.

The word "label" when used herein refers to a detectable compound or composition which is conjugated directly or indirectly to a molecule (such as a nucleic acid, polypeptide, or antibody) so as to generate a "labeled" molecule. The label may be detectable by itself (e.g. radioisotope labels or fluorescent labels) or, in the case of an enzymatic label, may catalyze chemical alteration of a substrate compound or composition, resulting in a detectable product.

By "solid phase" is meant a non-aqueous matrix to which a molecule (such as a nucleic acid, polypeptide, or antibody) can adhere. Examples of solid phases encompassed herein include those formed partially or entirely of glass (e.g., controlled pore glass), polysaccharides (e.g., agarose), polyacrylamides, polystyrene, polyvinyl alcohol and silicones. In certain embodiments, depending on the context, the solid phase can comprise the well of an assay plate; in others it is a purification column (e.g., an affinity chromatography column). This term also includes a discontinuous solid phase of discrete particles, such as those described in U.S. Patent No. 4,275,149.

A "liposome" is a small vesicle composed of various types of lipids, phospholipids and/or surfactant which is useful for delivery of a drug (such as a nucleic acid, polypeptide, antibody, agonist or antagonist) to a mammal. The components of the liposome are commonly arranged in a bilayer formation, similar to the lipid arrangement of biological membranes.

A "small molecule" or "small organic molecule" is defined herein as an organic molecule having a molecular weight below about 500 Daltons.

An "oligopeptide" that binds to a target polypeptide is an oligopeptide that is capable of binding the target polypeptide with sufficient affinity such that the oligopeptide is useful as a diagnostic and/or therapeutic agent in targeting the polypeptide. In certain embodiments, the extent of binding of an oligopeptide to an unrelated, non-target polypeptide is less than about 10% of the binding of the oligopeptide to the target polypeptide as measured, e.g., by a surface plasmon resonance assay. In certain embodiments, an oligopeptide bnds to a target polypeptide with a dissociation constant (Kd) of ≤ 1μM, ≤ 100 nM, ≤ 10 nM, ≤ 1 nM, or ≤ 0.1 nM.

An "organic molecule" that binds to a target polypeptide is an organic molecule other than an oligopeptide or antibody as defined herein that is capable of binding a target polypeptide with sufficient affinity such that the organic molecule is useful as a diagnostic and/or therapeutic agent in targeting the polypeptide. In certain embodiments, the extent of binding of an organic molecule to an unrelated, non-target polypeptide is less than about 10% of the binding of the organic molecule to the target polypeptide as measured, e.g., by a surface plasmon resonance assay. In certain embodiments, an organic molecule binds to a target polypeptide with a dissociation constant (Kd) of ≤ 1μM, ≤ 100 nM, ≤ 10 nM, ≤ 1 nM, or ≤ 0.1 nM.

A "biological system" is an in vitro, ex vivo, or in vivo system comprising mammalian cells that share a common signaling pathway.

"Microbial disorder" refers to a disease or condition wherein a microbial pathogen causes, mediates, or otherwise contributes to a morbidity of the disease or condition. Also included are diseases in which stimulation or intervention of an anti-microbial response has an ameliorative effect on progression of the disease. Included within this term are infectious diseases or conditions, and opportunistic diseases resulting from primary infection by a microbial pathogen. Examples of such infectious disease, include, but are not limited to, EHEC- and EPEC-caused diarrhea, Inflammatory Bowel Disease (IBD) and, more particularly, Ulcerative Colitis (UC) and Crohn's Disease (CD).

The term "T cell mediated disease" means a disease in which T cells directly or indirectly mediate or otherwise contribute to a morbidity in a mammal. The T cell mediated disease may be associated with cell mediated effects, lymphokine mediated effects, etc., and even effects associated with B cells if the B cells are stimulated, for example, by the lymphokines secreted by T cells.

An "autoimmune disorder" or "autoimmunity" refers to any condition in which a humoral or cell-mediated immune response is mounted against a body's own tissue. An "IL-23 mediated autoimmune disorder" is any autoimmune disorder that is caused by, maintained, or exacerbated by IL-23 activity.

"Inflammation" refers to the accumulation of leukocytes and the dilation of blood vessels at a site of injury or infection, typically causing pain, swelling, and redness,

"Chronic inflammation" refers to inflammation in which the cause of the inflammation persists and is difficult or impossible to remove.

"Autoimmune inflammation" refers to inflammation associated with an autoimmune disorder.

"Arthritic inflammation" refers to inflammation associated with arthritis.

"Inflammatory bowel disease" or "IBD" refers to a chronic disorder characterized by inflammation of the gastrointestinal tract. IBD encompasses ulcerative colitis, which affects the large intestine and/or rectum, and Crohn's disease, which may affect the entire gastrointestinal system but more commonly affects the small intestine (ileum) and possibly the large intestine.

The term "effective amount" is a concentration or amount of a molecule (e.g., a nucleic acid, polypeptide, agonist, or antagonist) that results in achieving a particular stated purpose. An "effective amount" may be determined empirically. A "therapeutically effective amount" is a concentration or amount of a molecule which is effective for achieving a stated therapeutic effect. This amount may also be determined empirically.

The term "cytotoxic agent" as used herein refers to a substance that inhibits or prevents the function of cells and/or causes destruction of cells. The term is intended to include radioactive isotopes (*e.g.*, $I^{131}$, $I^{125}$, $Y^{90}$ and $Re^{186}$), chemotherapeutic agents, and toxins such as enzymatically active toxins of bacterial, fungal, plant or animal origin, or fragments thereof.

A "growth inhibitory agent" when used herein refers to a compound or composition which inhibits growth of a cell, especially a cell overexpressing any of a gene, either *in vitro* or *in vivo.* Thus, a growth inhibitory agent is one which significantly reduces the percentage of cells overexpressing such genes in S phase. Examples of growth inhibitory agents include agents that block cell cycle progression (at a place other than S phase), such as agents that induce G1 arrest and M-phase arrest. Classical M-phase blockers include the vincas (vincristine and vinblastine), taxol, and topo II inhibitors such as doxorubicin, epirubicin, daunorubicin, etoposide, and bleomycin. Those agents that arrest G1 also spill over into S-phase arrest, for example, DNA alkylating agents such as tamoxifen, prednisone, dacarbazine, mechlorethamine, cisplatin, methotrexate, 5-fluorouracil, and ara-C. Further information can be found in The Molecular Basis of Cancer, Mendelsohn and Israel, eds., Chapter 1, entitled "Cell cycle regulation, oncogens, and antineoplastic drugs" by Murakami et al. (WB Saunders: Philadelphia, 1995), especially p. 13.

The term "cytokine" is a generic term for proteins released by one cell population which act on another cell population as intercellular mediators. Examples of such cytokines are lymphokines, monokines, and traditional polypeptide hormones. Included among the cytokines are growth hormone such as human growth hormone, N-methionyl human growth hormone, and bovine growth hormone; parathyroid hormone; thyroxine; insulin; proinsulin; relaxin; prorelaxin; glycoprotein hormones such as follicle stimulating hormone (FSH), thyroid stimulating hormone (TSH), and luteinizing hormone (LH); hepatic growth factor; fibroblast growth factor; prolactin; placental lactogen; tumor necrosis factor-$\alpha$ and -$\beta$; lymphotoxin-$\alpha$ and -$\beta$, mullerian-inhibiting substance; mouse gonadotropin-associated peptide; inhibin; activin; vascular endothelial growth factor; integrin; thrombopoietin (TPO); nerve growth factors such as NGF-$\beta$; platelet-growth factor; transforming growth factors (TGFs) such as TGF-$\alpha$ and TGF-$\beta$; insulin-like growth factor-I and -II; erythropoietin (EPO); osteoinductive factors; interferons such as interferon-$\alpha$, -$\beta$, and -$\gamma$; colony stimulating factors (CSFs) such as macrophage-CSF (M-CSF); granulocyte-macrophage-CSF (GM-CSF); and granulocyte-CSF (G-CSF); interleukins (ILs) such as IL-1, IL-1$\alpha$, IL-2, IL-3, IL-4, IL-5, IL-6, IL-7, IL-8, IL-9, IL-10, IL-11, IL-12, IL-6, IL-17, IL-18, IL-22, IL-23; a tumor necrosis factor such as TNF-$\alpha$ or TNF-$\beta$; and other polypeptide factors including LIF and kit ligand (KL). As used herein, the term cytokine includes proteins from natural sources or from recombinant cell culture and biologically active equivalents of the native sequence cytokines.

As used herein, the term "inflammatory cells" designates cells that enhance the inflammatory response such as mononuclear cells, eosinophils, macrophages, and polymorphonuclear neutrophils (PMN).

**II. Compositions and Methods of the Invention**

A. Anti-Microbial Polypeptides (AMP) and Modulators Thereof

Anti-microbial polypeptides (AMPs) of the present invention are polypeptides that mediate, or otherwise effect, an anti-microbial immune response to a microbial pathogen. AMPs of the present invention include, but are not limited to, LT, IL-6, IL-22, IL-23 (including e.g., IL-23 p19 or IL-23 p40), and Reg or Reg-related proteins encoded by the genes of the Reg super family. The Reg super family includes Reg and Reg-related genes from human, rat, and mouse and are grouped into four subclasses, types I, II, III, and IV. For example, type I includes human *REG I$\alpha$*, human *REG I$\beta$*, rat *RegI*, and mouse *RegI*; type II includes mouse *RegII*; type III includes human REG III, human *HIP/PAP* (gene expressed in hepatocellular carcinoma-intestine-pancreas/gene encoding pancreatitis-associated protein), rat *PAP/Peptide23*, rat *RegIII/PAPII*, rat *PAP III*, mouse *RegIII$\alpha$*, *RegIII$\beta$*, *RegIII$\gamma$*, mouse *RegIII$\delta$*, and hamster *INGAP* (islet neogenesis-associated protein). Type IV contains human *REG IV.* Additionally, human Reg-related Sequence (RS) is reportedly a pseudogene. In one embodiment, the REG protein is encoded by a member of the human REG gene family which includes, but is not limited to, REG I$\alpha$, REG I$\beta$, HIP/PAP, REG III, REG IV, and Reg-related sequence (RS).

In some aspects, the amino acid sequence of an AMP of the present invention comprises an amino acid sequence selected from the following group: SEQ ID NO: 2, SEQ ID NO: 4, SEQ ID NO: 6, SEQ ID NO: 8, SEQ ID NO: 10, SEQ ID NO: 12, SEQ ID NO: 14, SEQ ID NO: 16, SEQ ID NO: 18, SEQ ID NO: 20, SEQ ID NO: 22, SEQ ID NO: 24, SEQ ID NO: 26, SEQ ID NO: 28, SEQ ID NO: 30, SEQ ID NO: 32, SEQ ID NO: 34, SEQ ID NO: 36, SEQ ID NO: 38, SEQ ID NO: 40, SEQ ID NO: 42, SEQ ID NO: 44, SEQ ID NO: 46, SEQ ID NO: 48, SEQ ID NO: 50, SEQ ID NO: 52, SEQ ID NO: 54, and SEQ ID NO: 56.

In other aspects, the nucleic acid sequence encoding an AMP of the present invention comprises a nucleic acid sequence selected from the following group: SEQ ID NO: 1, SEQ ID NO: 3, SEQ ID NO: 5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, SEQ ID NO: 23, SEQ ID NO: 25, SEQ ID NO: 27, SEQ ID NO: 29, SEQ ID NO: 31, SEQ ID NO: 33, SEQ ID NO: 35, SEQ ID NO: 37, SEQ ID NO: 39, SEQ ID NO: 41, SEQ ID NO: 43, SEQ ID NO: 45, SEQ ID NO: 47, SEQ ID NO: 49, SEQ ID NO: 51, SEQ ID NO: 53, and SEQ ID NO: 55.

An activity of an AMP of the present invention can be increased or decreased and/or differentially regulated relative to the activity of another AMP or the same AMP. Examples of an activity of an AMP of the present invention, includes, but is not limited to, AMP expression, signal transduction, binding to a binding partner, anti-microbial response, or other biological or immunological activity thereof.

In one embodiment, an increase in the activity of one or more AMPs of the present invention results in an enhanced or induced anti-microbial immune response in a subject.

In one embodiment, AMPs of the present invention include, but are not limited to, polypeptides that directly or indirectly interact with IL-22, e.g., polypeptides that are upstream or downstream of an IL-22 signal transduction pathway that mediates host resistance to infection by a microbial pathogen (e.g., a bacteria or virus). Examples of such AMPs include, but are not limited to, LT, IL-6, IL-18, and IL-23 (including e.g., IL-23 p19 or IL-23 p40).

Modulators of the present invention include, but are not limited to, polypeptides and nucleic acid molecules (e.g., a DNA molecule or RNA molecule) that directly or indirectly modulate an activity of an AMP. Examples of such modulation include, but are not limited to, an increase, decrease, induction or activation, inhibition, or regulation (e.g., up or down regulation) of an activity of an AMP of the present invention.

In a particular embodiment, the modulator indirectly modulates IL-22 activity by decreasing or inhibiting IL-22 Binding Protein (BP) activity and thereby, increasing IL-22 activity. In a further embodiment, the modulator decreases or inhibits binding of IL-22 BP to IL-22 and thereby, increases IL-22 activity.

In some embodiments, the modulator is a polypeptide e.g., a polypeptide that binds to or otherwise interacts with an AMP to increase, induce, or regulate an activity of an AMP. In one embodiment, the modulator is a fusion polypeptide that modulates an activity of an AMP.

In one embodiment, the modulator is an antibody that binds to an AMP. In a particular embodiment, the antibody is a monoclonal antibody. In another embodiment, the antibody is an antibody fragment selected from a Fab, Fab'-SH, Fv, scFv, or $(Fab')_2$ fragment. In another embodiment, the antibody is a fusion polypeptide (e.g., an Fc fusion polypeptide). In another embodiment, the antibody is a chimeric antibody. In a particular embodiment, the antibody is humanized. In another embodiment, the antibody is a human antibody. In another embodiment, the antibody binds to the same epitope as an antibody selected from a human, non-human primate, or other mammal (e.g., pig, sheep, rabbit, marmot, rat, or mouse). In a particular embodiment, the antibody is an AMP agonist.

In a particular embodiment, the modulator is a recombinant AMP or nucleic acid molecule encoding an AMP (e.g., a DNA or RNA molecule).

In another particular embodiment, the modulator is a recombinant AMP or nucleic acid molecule encoding an AMP (e.g., a DNA or RNA molecule) that can be expressed in a cell.

AMPs of the present invention encompass native full-length or mature AMPs as well as variants thereof. AMP variants can be prepared by introducing appropriate nucleotide changes into the DNA encoding an AMP, and/or by synthesis of the desired anti-microbial polypeptide. Those skilled in the art will appreciate that amino acid changes may alter post-translational processing of a polypeptide of the present invention, such as changing the number or position of glycosylation sites or altering the membrane anchoring characteristics.

Variations in native AMP or in various domains of the AMP, as described herein, can be made, for example, using any of the techniques and guidelines for conservative and non-conservative mutations set forth, for instance, in U.S. Patent No. 5,364,934. Variations may be a substitution, deletion or insertion of one or more codons encoding the AMP that results in a change in the amino acid sequence of the AMP as compared with a native sequence AMP. Optionally, the variation is by substitution of at least one amino acid with any other amino acid in one or more domains of the AMP. Guidance in determining which amino acid residue may be inserted, substituted or deleted without adversely affecting the desired activity may be found by comparing the sequence of the AMP with that of homologous known protein molecules and minimizing the number of amino acid sequence changes made in regions of high homology. Amino acid substitutions can be the result of replacing one amino acid with another amino acid having similar structural and/or chemical properties, such as the replacement of a leucine with a serine, i.e., conservative amino acid replacements. Insertions or deletions may optionally be in the range of about 1 to 5 amino acids. The variation allowed may be determined by systematically making insertions, deletions or substitutions of amino acids in the sequence and testing the resulting variants for activity exhibited by the full-length or mature native sequence.

In particular embodiments, conservative substitutions of interest are shown in Table 3 under the heading of preferred substitutions. If such substitutions result in a change in biological activity, then more substantial changes, denominated exemplary substitutions in Table 6, or as further described below in reference to amino acid classes, are introduced and the products screened.

Table 3

| Original Residue | Exemplary Substitutions | Preferred Substitutions |
|---|---|---|
| Ala (A) | val; leu; ile | val |
| Arg (R) | lys; gln; asn | lys |
| Asn (N) | gln; his; lys; arg | gln |
| Asp (D) | glu | glu |
| Cys (C) | ser | ser |
| Gln (Q) | asn | asn |
| Glu (E) | asp | asp |
| Gly (G) | pro; ala | ala |
| His (H) | asn; gln; lys; arg | arg |

(continued)

| Original Residue | Exemplary Substitutions | Preferred Substitutions |
|---|---|---|
| Ile (I) | leu; val; met; ala; phe; norleucine | leu |
| Leu (L) | norleucine; ile; val; met; ala; phe | ile |
| Lys (K) | arg; gln; asn | arg |
| Met (M) | leu; phe; ile | leu |
| Phe (F) | leu; val; ile; ala; tyr | leu |
| Pro (P) | ala | ala |
| Ser (S) | thr | thr |
| Thr (T) | ser | ser |
| Trp (W) | tyr; phe | tyr |
| Tyr (Y) | trp; phe; thr; ser | phe |
| Val (V) | ile; leu; met; phe; ala; norleucine | leu |

Substantial modifications in function or immunological identity of the AMP polypeptide are accomplished by selecting substitutions that differ significantly in their effect on maintaining (a) the structure of the polypeptide backbone in the area of the substitution, for example, as a sheet or helical conformation, (b) the charge or hydrophobicity of the molecule at the target site, or (c) the bulk of the side chain. Naturally occurring residues are divided into groups based on common side-chain properties:

(1) hydrophobic: norleucine, met, ala, val, leu, ile;
(2) neutral hydrophilic: cys, ser, thr;
(3) acidic: asp, glu;
(4) basic: asn, gln, his, lys, arg;
(5) residues that influence chain orientation: gly, pro; and
(6) aromatic: trp, tyr, phe.

Non-conservative substitutions will entail exchanging a member of one of these classes for another class. Such substituted residues also may be introduced into the conservative substitution sites or, more preferably, into the remaining (non-conserved) sites.

The variations can be made using methods known in the art such as oligonucleotide-mediated (site-directed) mutagenesis, alanine scanning, and PCR mutagenesis. Site-directed mutagenesis [Carter et al., Nucl. Acids Res., 13:4331 (1986); Zoller et al., Nucl. Acids Res., 10:6487 (1987)], cassette mutagenesis [Wells et al., Gene, 34:315 (1985)], restriction selection mutagenesis [Wells et al., Philos. Trans. R. Soc. London SerA, 317:415 (1986)] or other known techniques can be performed on cloned DNA to produce a DNA encoding a variant AMP.

Fragments of an AMP or other polypeptides of the present invention are also provided herein. Such fragments may be truncated at the N-terminus or C-terminus, or may lack internal residues, for example, when compared with a full length native protein. Certain fragments lack amino acid residues that are not essential for a desired biological activity of an AMP or polypeptide of the present invention. Accordingly, in certain embodiments, a fragment of an AMP or other polypeptide of the present invention, is biologically active. In certain embodiments, a fragment of full length AMP lacks the N-terminal signal peptide sequence. In certain embodiments, a fragment of full-length AMP is a soluble form of a membrane-bound AMP. For example, a soluble form of AMP may lack all or a substantial portion of the transmembrane domain.

Covalent modifications of AMPs or other polypeptides of the present invention are included within the scope of this invention. One type of covalent modification includes reacting targeted amino acid residues of a polypeptide of the present invention with an organic derivatizing agent that is capable of reacting with selected side chains or the N- or C-terminal residues of the polypeptide. Derivatization with bifunctional agents is useful, for instance, for crosslinking the polypeptide to a water-insoluble support matrix or surface for use in the method for purifying antibodies to the polypeptide, and vice-versa. Commonly used crosslinking agents include, e.g., 1,1-bis(diazoacetyl)-2-phenylethane, glutaraldehyde, N-hydroxysuccinimide esters, for example, esters with 4-azidosalicylic acid, homobifunctional imidoesters, including disuccinimidyl esters such as 3,3'-dithiobis(succinimidylpropionate), bifunctional maleimides such as bis-N-maleimido-1,8-octane and agents such as methyl-3-[(p-azidophenyl)dithio]propioimidate.

Other modifications include deamidation of glutaminyl and asparaginyl residues to the corresponding glutamyl and aspartyl residues, respectively, hydroxylation of proline and lysine, phosphorylation of hydroxyl groups of seryl or threonyl residues, methylation of the $\alpha$-amino groups of lysine, arginine, and histidine side chains [T.E. Creighton, Proteins: Structure and Molecular Properties, W.H. Freeman & Co., San Francisco, pp. 79-86 (1983)], acetylation of the N-terminal amine, and amidation of any C-terminal carboxyl group.

Another type of covalent modification of a polypeptide of the present invention included within the scope of this invention comprises altering the native glycosylation pattern of the polypeptide. "Altering the native glycosylation pattern" is intended for purposes herein to mean deleting one or more carbohydrate moieties found in the native sequence of a polypeptide of the present invention (either by removing the underlying glycosylation site or by deleting the glycosylation by chemical and/or enzymatic means), and/or adding one or more glycosylation sites that are not present in the native sequence of the polypeptide. In addition, the phrase includes qualitative changes in the glycosylation of the native proteins, involving a change in the nature and proportions of the various carbohydrate moieties present.

A polypeptide of the present invention may also be modified in a way to form a chimeric molecule comprising the polypeptide fused to another, heterologous polypeptide or amino acid sequence. In one embodiment, a chimeric molecule comprises a fusion of the polypeptide with a tag polypeptide which provides an epitope to which an anti-tag antibody can selectively bind. The epitope tag is generally placed at the amino- or carboxyl- terminus of the polypeptide. The presence of such epitope-tagged forms of the polypeptide can be detected using an antibody against the tagged polypeptide. Also, provision of the epitope tag enables the AMP to be readily purified by affinity purification using an anti-tag antibody or another type of affinity matrix that binds to the epitope tag. Various tag polypeptides and their respective antibodies are well known in the art. Examples include poly-histidine (poly-his) or poly-histidine-glycine (poly-his-gly) tags; the flu HA tag polypeptide and its antibody 12CA5 [Field et al., Mol. Cell. Biol., 8:2159-2165 (1988)]; the c-myc tag and the 8F9, 3C7, 6E10, G4, B7 and 9E10 antibodies thereto [Evan et al., Molecular and Cellular Biology, 5:3610-3616 (1985)]; and the Herpes Simplex virus glycoprotein D (gD) tag and its antibody [Paborsky et al., Protein Engineering, 3(6):547-553 (1990)]. Other tag polypeptides include the Flag-peptide [Hopp et al., BioTechnology, 6:1204-1210 (1988)]; the KT3 epitope peptide [Martin et al., Science, 255:192-194 (1992)]; an alpha-tubulin epitope peptide [Skinner et al., J. Biol. Chem., 266:15163-15166 (1991)]; and the T7 gene 10 protein peptide tag [Lutz-Freyermuth et al., Proc. Natl. Acad. Sci. USA, 87:6393-6397 (1990)].

In another embodiment, a chimeric molecule may comprise a fusion of a polypeptide of the present invention with an immunoglobulin or a particular region of an immunoglobulin. For a bivalent form of the chimeric molecule (also referred to as an "immunoadhesin"), such a fusion could be to the Fc region of an IgG molecule. The Ig fusions preferably include the substitution of a soluble form of a polypeptide of the present invention (e.g., an AMP or polypeptide modulator thereof) in place of at least one variable region within an Ig molecule. In a particularly preferred embodiment, the immunoglobulin fusion includes the hinge, CH2 and CH3, or the hinge, CH1, CH2 and CH3 regions of an IgG1 molecule. For the production of immunoglobulin fusions see also US Patent No. 5,428,130 issued June 27, 1995.

### *1. Preparation of Polypeptides*

Polypeptides of the present invention may be prepared by routine recombinant methods, e.g., culturing cells transformed or transfected with a vector containing a nucleic acid encoding an AMP or polypeptide modulator thereof. Host cells comprising any such vector are also provided. By way of example, host cells may be CHO cells, *E. coli,* or yeast. A process for producing any of the herein described polypeptides is further provided and comprises culturing host cells under conditions suitable for expression of the desired polypeptide and recovering the desired polypeptide from the cell culture.

In other embodiments, the invention provides chimeric molecules comprising any of the herein described polypeptides fused to a heterologous polypeptide or amino acid sequence. Examples of such chimeric molecules include, but are not limited to, any of the herein described polypeptides fused to an epitope tag sequence or an Fc region of an immunoglobulin.

Alternative methods, which are well known in the art, may be employed to prepare a polypeptide of the present invention. For example, a sequence encoding a polypeptide or portion thereof, may be produced by direct peptide synthesis using solid-phase techniques [see, e.g., Stewart et al., Solid-Phase Peptide Synthesis, W.H. Freeman Co., San Francisco, CA (1969); Merrifield, J. Am. Chem. Soc., 85:2149-2154 (1963)]. *In vitro* protein synthesis may be performed using manual techniques or by automation. Automated synthesis may be accomplished, for instance, using an Applied Biosystems Peptide Synthesizer (Foster City, CA) using manufacturer's instructions. Various portions of a polypeptide of the present invention or portion thereof may be chemically synthesized separately and combined using chemical or enzymatic methods to produce the full-length polypeptide or portion thereof.

Recombinantly expressed polypeptides of the present invention may be recovered from culture medium or from host cell lysates. The following procedures are exemplary of suitable purification procedures: by fractionation on an ion-exchange column; ethanol precipitation; reverse phase HPLC; chromatography on silica or on a cation-exchange resin such as DEAE; chromatofocusing; SDS-PAGE; ammonium sulfate precipitation; gel filtration using, for example, Sephadex G-75; protein A Sepharose columns to remove contaminants such as IgG; and metalchelating columns to bind epitope-tagged forms of a polypeptide of the present invention. Various methods of protein purification may be employed and such methods are known in the art and described for example in Deutscher, Methods in Enzymology, 182 (1990); Scopes, Protein Purification: Principles and Practice, Springer-Verlag, New York (1982). The purification step(s) selected will depend, for example, on the nature of the production process used and the particular polypeptide produced. LT polypeptides may be purified by expressing a tagged LT polypeptide such as, for example, an LT$\alpha$-tagged polypeptide (SEQ ID NO:61).

### *2. Detection of Gene Expression*

Expression of a gene encoding a polypeptide of the present invention can be detected by various methods in the art, e.g, by detecting expression of mRNA encoding the polypeptide. As used herein, the term "detecting" encompasses quantitative or qualitative detection. By detecting gene expression of a polypeptide of the present invention, one can identify, e.g., those tissues that express this gene. Gene expression may be measured using certain methods known to those skilled in the art, e.g., Northern blotting, (Thomas, Proc. Natl. Acad. Sci. USA, 77:5201-5205 [1980]); quantitative PCR; or *in situ* hybridization, using an appropriately labeled probe, based on the sequences provided herein. Alternatively, gene expression may be measured by immunological methods, such as immunohistochemical staining of tissue sections and assay of cell culture or body fluids, to quantitate directly the expression of gene product. Antibodies useful for immunohistochemical staining and/or assay of sample fluids encompass any of the antibodies provided herein. Conveniently, the antibodies may be prepared against a native sequence encoding e.g., an AMP of the present invention; against a synthetic peptide comprising a fragment of the AMP sequence; or against an exogenous sequence fused to AMP polypeptide or fragment thereof (including a synthetic peptide).

### B. Antibodies

Antibodies that bind to any of the above- or below- described polypeptides are provided. In one embodiment, an isolated antibody that binds to an AMP of the present invention and thereby modulates AMP activity, e.g., increasing an activity of the AMP. Exemplary antibodies include polyclonal, monoclonal, humanized, human, bispecific, and heteroconjugate antibodies. An antibody may be an antibody fragment, e.g., a Fab, Fab'-SH, Fv, scFv, or (Fab')2 fragment. In one embodiment, an isolated antibody that binds to an IL-22 is provided. In one such embodiment, an antibody partially or completely increases the activity of an AMP of the present invention.

Exemplary monoclonal antibodies that bind an AMP of the present invention are described herein. These antibodies include the anti-IL-22 antibodies designated 3F11.3 ("3F11"), 11H4.4 ("11H4"), and 8E11.9 ("8E11"), and the anti-IL-22R antibodies designated 7E9.10.8 ("7E9"), 8A12.32 ("8A12"), 8H11.32.28 ("8H11"), and 12H5. In one embodiment, a hybridoma that produces any of those antibodies is provided. In one embodiment, monoclonal antibodies that compete with 3F11.3, 11H4.4, or 8E11.9 for binding to IL-22 are provided. In another embodiment, monoclonal antibodies that bind to the same epitope as 3F11.3, 11H4.4, or 8E11.9 are provided. In another embodiment, monoclonal antibodies that compete with 7E9, 8A12, 8H11, or 12H5 for binding to IL-22R are provided. In one embodiment, monoclonal antibodies that bind to the same epitope as 7E9, 8A12, 8H11, or 12H5 are provided. Various embodiments of antibodies are provided below:

### *1. Polyclonal Antibodies*

Antibodies may comprise polyclonal antibodies. Methods of preparing polyclonal antibodies are known to the skilled artisan. Polyclonal antibodies can be raised in a mammal, for example, by one or more injections of an immunizing agent and, if desired, an adjuvant. Typically, the immunizing agent and/or adjuvant will be injected in the mammal by multiple subcutaneous or intraperitoneal injections. The immunizing agent may include the polypeptide of interest or a fusion protein thereof. It may be useful to conjugate the immunizing agent to a protein known to be immunogenic in the mammal being immunized. Examples of such immunogenic proteins include but are not limited to keyhole limpet hemocyanin, serum albumin, bovine thyroglobulin, and soybean trypsin inhibitor. Examples of adjuvants which may be employed include Freund's complete adjuvant and MPL-TDM adjuvant (monophosphoryl Lipid A, synthetic trehalose dicorynomycolate). The immunization protocol may be selected by one skilled in the art without undue experimentation.

### *2. Monoclonal Antibodies*

Antibodies may, alternatively, be monoclonal antibodies. Monoclonal antibodies may be prepared using hybridoma methods, such as those described by Kohler and Milstein, Nature, 256:495 (1975). In a hybridoma method, a mouse, hamster, or other appropriate host animal, is typically immunized with an immunizing agent to elicit lymphocytes that produce or are capable of producing antibodies that will specifically bind to the immunizing agent. Alternatively, the lymphocytes may be immunized *in vitro.*

The immunizing agent will typically include the polypeptide of interest or a fusion protein thereof. Generally, either peripheral blood lymphocytes ("PBLs") are used if cells of human origin are desired, or spleen cells or lymph node cells are used if non-human mammalian sources are desired. The lymphocytes are then fused with an immortalized cell line using a suitable fusing agent, such as polyethylene glycol, to form a hybridoma cell [Goding, Monoclonal Antibodies: Principles and Practice, Academic Press, (1986) pp. 59-103]. Immortalized cell lines are usually transformed mammalian cells, particularly myeloma cells of rodent, bovine and human origin. Usually, rat or mouse myeloma cell lines are employed. The hybridoma cells may be cultured in a suitable culture medium that preferably contains one or more substances that inhibit the growth or survival of the unfused, immortalized cells. For example, if the parental cells lack the enzyme hypoxanthine guanine phosphoribosyl transferase (HGPRT or HPRT), the culture medium for the hybridomas typically will include hypoxanthine, aminopterin, and thymidine ("FIAT medium"), which substances prevent the growth of HGPRT-dcficient cells.

Preferred immortalized cell lines are those that fuse efficiently, support stable high level expression of antibody by the selected antibody-producing cells, and are sensitive to a medium such as HAT medium. More preferred immortalized cell lines are murine myeloma lines, which can be obtained, for instance, from the Salk Institute Cell Distribution Center, San Diego, California and the American Type Culture Collection, Manassas, Virginia. Human myeloma and mouse-human heteromyeloma cell lines also have been described for the production of human monoclonal antibodies [Kozbor, J. Immunol., 133:3001 (1984); Brodeur et al., Monoclonal Antibody Production Techniques and Applications, Marcel Dekker, Inc., New York, (1987) pp. 51-63].

The culture medium in which the hybridoma cells are cultured can then be assayed for the presence of monoclonal antibodies that bind to the polypeptide of interest. Preferably, the binding specificity of monoclonal antibodies produced by the hybridoma cells is determined by immunoprecipitation or by an *in vitro* binding assay, such as radioimmunoassay (RIA) or enzyme-linked immunoabsorbent assay (ELISA). Such techniques and assays are known in the art. The binding affinity of the monoclonal antibody can, for example, be determined by the Scatchard analysis of Munson and Pollard, Anal. Biochem., 107:220 (1980).

After the desired hybridoma cells are identified, the clones may be subcloned by limiting dilution procedures and grown by standard methods [Coding, supra]. Suitable culture media for this purpose include, for example, Dulbecco's Modified Eagle's Medium and RPMI-1640 medium. Alternatively, the hybridoma cells may be grown *in vivo* as ascites in a mammal.

The monoclonal antibodies secreted by the subclones may be isolated or purified from the culture medium or ascites fluid by conventional immunoglobulin purification procedures such as, for example, protein A-Sepharose, hydroxylapatite chromatography, gel electrophoresis, dialysis, or affinity chromatography.

Monoclonal antibodies can be made by using combinatorial libraries to screen for antibodies with the desired activity or activities. For example, a variety of methods are known in the art for generating phage display libraries and screening such libraries for antibodies possessing the desired binding characteristics. Such methods are described generally in Hoogenboom et al. (2001) in Methods in Molecular Biology 178:1-37 (O'Brien et al., ed., Human Press, Totowa, NJ), and in certain embodiments, in Lee et al. (2004) J. Mol. Biol. 340:1073-1093.

In principle, synthetic antibody clones are selected by screening phage libraries containing phage that display various fragments of antibody variable region (Fv) fused to phage coat protein. Such phage libraries are panned by affinity chromatography against the desired antigen. Clones expressing Fv fragments capable of binding to the desired antigen are adsorbed to the antigen and thus separated from the non-binding clones in the library. The binding clones are then eluted from the antigen, and can be further enriched by additional cycles of antigen adsorption/elution. Any of the antibodies of the invention can be obtained by designing a suitable antigen screening procedure to select for the phage clone of interest followed by construction of a full length antibody clone using the Fv sequences from the phage clone of interest and suitable constant region (Fc) sequences described in Kabat et al., Sequences of Proteins of Immunological Interest, Fifth Edition, NIH Publication 91-3242, Bethesda MD (1991), vols. 1-3.

The monoclonal antibodies may also be made by recombinant DNA methods, such as those described in U.S. Patent No. 4,816,567. DNA encoding the monoclonal antibodies of the invention can be readily isolated and sequenced using conventional procedures (e.g., by using oligonucleotide probes that are capable of binding specifically to genes encoding the heavy and light chains of murine antibodies). The hybridoma cells of the invention serve as a preferred source of such DNA. Once isolated, the DNA may be placed into expression vectors, which are then transfected into host cells such as simian COS cells, Chinese hamster ovary (CHO) cells, or myeloma cells that do not otherwise produce immunoglobulin protein, to obtain the synthesis of monoclonal antibodies in the recombinant host cells. The DNA also may be modified, for example, by substituting the coding sequence for human heavy and light chain constant domains in place of the homologous murine sequences [U.S. Patent No. 4,816,567; Morrison et al., supra] or by covalently joining to the immunoglobulin coding sequence all or part of the coding sequence for a non-immunoglobulin polypeptide. Such a non-immunoglobulin polypeptide can be substituted for the constant domains of an antibody of the invention, or can be substituted for the variable domains of one antigen-combining site of an antibody of the invention to create a chimeric bivalent antibody.

### *3. Monovalent Antibodies*

Monovalent antibodies are also provided. Methods for preparing monovalent antibodies are well known in the art. For example, one method involves recombinant expression of immunoglobulin light chain and modified heavy chain. The heavy chain is truncated generally at any point in the Fc region so as to prevent heavy chain crosslinking. Alternatively, the relevant cysteine residues are substituted with another amino acid residue or are deleted so as to prevent crosslinking.

*In vitro* methods are also suitable for preparing monovalent antibodies. Digestion of antibodies to produce fragments thereof, particularly, Fab fragments, can be accomplished using routine techniques known in the art.

### *4. Antibody Fragments*

Antibody fragments are also provided. Antibody fragments may be generated by traditional means, such as enzymatic digestion, or by recombinant techniques. In certain circumstances there are advantages of using antibody fragments, rather than whole antibodies. The smaller size of the fragments allows for rapid clearance, and may lead to improved access to solid tumors. For a review of certain antibody fragments, see Hudson et al. (2003) Nat. Med. 9:129-134.

Various techniques have been developed for the production of antibody fragments. Traditionally, these fragments were derived via proteolytic digestion of intact antibodies (see, e.g., Morimoto et al., Journal of Biochemical and Biophysical Methods 24:107-117 (1992); and Brennan et al., Science, 229:81 (1985)). However, these fragments can now be produced directly by recombinant host cells. Fab, Fv and ScFv antibody fragments can all be expressed in and secreted from E. coli, thus allowing the facile production of large amounts of these fragments. Antibody fragments can be isolated from the antibody phage libraries discussed above. Alternatively, Fab'-SH fragments can be directly recovered from E. coli and chemically coupled to form $F(ab')_2$ fragments (Carter et al., Bio/Technology 10:163-167 (1992)). According to another approach, $F(ab')_2$ fragments can be isolated directly from recombinant host cell culture. Fab and $F(ab')_2$ fragment with increased in vivo half-life comprising salvage receptor binding epitope residues are described in U.S. Pat. No. 5,869,046. Other techniques for the production of antibody fragments will be apparent to the skilled practitioner. In certain embodiments, an antibody is a single chain Fv fragment (scFv). See WO 93/16185; U.S. Pat. Nos. 5,571,894; and 5,587,458. Fv and scFv are the only species with intact combining sites that are devoid of constant regions; thus, they may be suitable for reduced nonspecific binding during in vivo use. scFv fusion proteins may be constructed to yield fusion of an effector protein at either the amino or the carboxy terminus of an scFv. See Antibody Engineering, ed. Borrebaeck, supra. The antibody fragment may also be a "linear antibody", e.g., as described in U.S. Pat. No. 5,641,870, for example. Such linear antibodies may be monospecific or bispecific.

### *5. Humanized Antibodies*

Humanized antibodies are also provided. Various methods for humanizing non-human antibodies are known in the art. For example, a humanized antibody can have one or more amino acid residues introduced into it from a source which is non-human. These non-human amino acid residues are often referred to as "import" residues, which are typically taken from an "import" variable domain. Humanization can be essentially performed following the method of Winter and co-workers (Jones et al. (1986) Nature 321:522-525; Riechmann et al. (1988) Nature 332:323-327; Verhoeyen et al. (1988) Science 239:1534-1536), by substituting hypervariable region sequences for the corresponding sequences of a human antibody. Accordingly, such "humanized" antibodies are chimeric antibodies (U.S. Patent No. 4,816,567) wherein substantially less than an intact human variable domain has been substituted by the corresponding sequence from a non-human species. In practice, humanized antibodies are typically human antibodies in which some hypervariable region residues and possibly some FR residues are substituted by residues from analogous sites in rodent antibodies.

The choice of human variable domains, both light and heavy, to be used in making the humanized antibodies can be important to reduce antigenicity. According to the so-called "best-fit" method, the sequence of the variable domain of a rodent antibody is screened against the entire library of known human variable-domain sequences. The human sequence which is closest to that of the rodent is then accepted as the human framework for the humanized antibody (Sims et al. (1993) J. Immunol. 151:2296; Chothia et al. (1987) J. Mol. Biol. 196:901. Another method uses a particular framework derived from the consensus sequence of all human antibodies of a particular subgroup of light or heavy chains. The same framework may be used for several different humanized antibodies (Carter et al. (1992) Proc. Natl. Acad. Sci. USA, 89:4285; Presta et a/. (1993) J. Immunol., 151:2623.

It is further generally desirable that antibodies be humanized with retention of high affinity for the antigen and other favorable biological properties. To achieve this goal, according to one method, humanized antibodies are prepared by a process of analysis of the parental sequences and various conceptual humanized products using three-dimensional models of the parental and humanized sequences. Three-dimensional immunoglobulin models are commonly available and are familiar to those skilled in the art. Computer programs are available which illustrate and display probable three-dimensional conformational structures of selected candidate immunoglobulin sequences. Inspection of these displays permits analysis of the likely role of the residues in the functioning of the candidate immunoglobulin sequence, *i.e.,* the analysis of residues that influence the ability of the candidate immunoglobulin to bind its antigen. In this way, FR residues can be selected and combined from the recipient and import sequences so that the desired antibody characteristic, such as increased affinity for the target antigen(s), is achieved. In general, the hypervariable region residues are directly and most substantially involved in influencing antigen binding.

### *6. Human Antibodies*

Human antibodies are also provided. Human antibodies can be constructed by combining Fv clone variable domain sequence(s) selected from human-derived phage display libraries with known human constant domain sequences(s) as described above. Alternatively, human monoclonal antibodies of the invention can be made by the hybridoma method. Human myeloma and mouse-human heteromyeloma cell lines for the production of human monoclonal antibodies have been described, for example, by Kozbor J. Immunol., 133: 3001 (1984); Brodeur et al., Monoclonal Antibody Production Techniques and Applications, pp. 51-63 (Marcel Dekker, Inc., New York, 1987); and Boerner et al., J. Immunol., 147: 86 (1991).

It is now possible to produce transgenic animals (e.g. mice) that are capable, upon immunization, of producing a full repertoire of human antibodies in the absence of endogenous immunoglobulin production. For example, it has been described that the homozygous deletion of the antibody heavy-chain joining region (JH) gene in chimeric and germ-line mutant mice results in complete inhibition of endogenous antibody production. Transfer of the human germ-line immunoglobulin gene array in such germ-line mutant mice will result in the production of human antibodies upon antigen challenge. See, e.g., Jakobovits et al., Proc. Natl. Acad. Sci USA, 90: 2551 (1993); Jakobovits et al., Nature, 362: 255 (1993); Bruggermann et al., Year in Immunol., 7: 33 (1993).

Gene shuffling can also be used to derive human antibodies from non-human, e.g. rodent, antibodies, where the human antibody has similar affinities and specificities to the starting non-human antibody. According to this method, which is also called "epitope imprinting", either the heavy or light chain variable region of a non-human antibody fragment obtained by phage display techniques as described herein is replaced with a repertoire of human V domain genes, creating a population of non-human chain/human chain scFv or Fab chimeras. Selection with antigen results in isolation of a non-human chain/human chain chimeric scFv or Fab wherein the human chain restores the antigen binding site destroyed upon removal of the corresponding non-human chain in the primary phage display clone, i.e. the epitope governs (imprints) the choice of the human chain partner. When the process is repeated in order to replace the remaining non-human chain, a human antibody is obtained (see PCT WO 93/06213 published April 1, 1993). Unlike traditional humanization of non-human antibodies by CDR grafting, this technique provides completely human antibodies, which have no FR or CDR residues of non-human origin.

### *7. Bispecific Antibodies*

Bispecific antibodies are also provided. Bispecific antibodies are monoclonal antibodies that have binding specificities for at least two different antigens. In certain embodiments, bispecific antibodies are human or humanized antibodies. In certain embodiments, one of the binding specificities is for a polypeptide of interest and the other is for any other antigen. In certain embodiments, bispecific antibodies may bind to two different epitopes of a polypeptide of interest. Bispecific antibodies may also be used to localize cytotoxic agents to cells which express a polypeptide of interest, such a cell surface polypeptide. These antibodies possess a TAT226-binding arm and an arm which binds a cytotoxic agent, such as, e.g., saporin, anti-interferon-a, vinca alkaloid, ricin A chain, methotrexate or radioactive isotope hapten. Bispecific antibodies can be prepared as full length antibodies or antibody fragments (*e.g.* $F(ab')_2$ bispecific antibodies).

Methods for making bispecific antibodies are known in the art. Traditionally, the recombinant production of bispecific antibodies is based on the co-expression of two immunoglobulin heavy chain-light chain pairs, where the two heavy chains have different specificities (Milstein and Cuello, Nature, 305: 537 (1983)). Because of the random assortment of immunoglobulin heavy and light chains, these hybridomas (quadromas) produce a potential mixture of 10 different antibody molecules, of which only one has the correct bispecific structure. The purification of the correct molecule, which is usually done by affinity chromatography steps, is rather cumbersome, and the product yields are low. Similar procedures are disclosed in WO 93/08829 published May 13, 1993, and in Traunecker et al., EMBO J., 10: 3655 (1991).

According to a different approach, antibody variable domains with the desired binding specificities (antibody-antigen combining sites) are fused to immunoglobulin constant domain sequences. The fusion, for example, is with an immunoglobulin heavy chain constant domain, comprising at least part of the hinge, CH2, and CH3 regions. In certain embodiments, the first heavy-chain constant region (CH1), containing the site necessary for light chain binding, is present in at least one of the fusions. DNAs encoding the immunoglobulin heavy chain fusions and, if desired, the immunoglobulin light chain, are inserted into separate expression vectors, and are co-transfected into a suitable host organism. This provides for great flexibility in adjusting the mutual proportions of the three polypeptide fragments in embodiments when unequal ratios of the three polypeptide chains used in the construction provide the optimum yields. It is, however, possible to insert the coding sequences for two or all three polypeptide chains in one expression vector when the expression of at least two polypeptide chains in equal ratios results in high yields or when the ratios are of no particular significance.

In one embodiment of this approach, the bispecific antibodies are composed of a hybrid immunoglobulin heavy chain with a first binding specificity in one arm, and a hybrid immunoglobulin heavy chain-light chain pair (providing a second binding specificity) in the other arm. It was found that this asymmetric structure facilitates the separation of the desired bispecific compound from unwanted immunoglobulin chain combinations, as the presence of an immunoglobulin light chain in only one half of the bispecific molecule provides for a facile way of separation. This approach is disclosed in WO 94/04690. For further details of generating bispecific antibodies see, for example, Suresh et al., Methods in Enzymology, 121:210 (1986).

According to another approach, the interface between a pair of antibody molecules can be engineered to maximize the percentage of heterodimers which are recovered from recombinant cell culture. The interface comprises at least a part of the $C_H3$ domain of an antibody constant domain. In this method, one or more small amino acid side chains from the interface of the first antibody molecule are replaced with larger side chains (*e.g.* tyrosine or tryptophan). Compensatory "cavities" of identical or similar size to the large side chain(s) are created on the interface of the second antibody molecule by replacing large amino acid side chains with smaller ones (*e.g.* alanine or threonine). This provides a mechanism for increasing the yield of the heterodimer over other unwanted end-products such as homodimers.

Bispecific antibodies include cross-linked or "heteroconjugate" antibodies. For example, one of the antibodies in the heteroconjugate can be coupled to avidin, the other to biotin. Such antibodies have, for example, been proposed to target immune system cells to unwanted cells (US Patent No. 4,676,980), and for treatment of HIV infection (WO 91/00360, WO 92/00373, and EP 03089). Heteroconjugate antibodies may be made using any convenient cross-linking method. Suitable cross-linking agents are well known in the art, and are disclosed in US Patent No. 4,676,980, along with a number of cross-linking techniques.

Techniques for generating bispecific antibodies from antibody fragments have also been described in the literature. For example, bispecific antibodies can be prepared using chemical linkage. Brennan et al., Science, 229: 81 (1985) describe a procedure wherein intact antibodies are proteolytically cleaved to generate $F(ab')_2$ fragments. These fragments are reduced in the presence of the dithiol complexing agent sodium arsenite to stabilize vicinal dithiols and prevent intermolecular disulfide formation. The Fab' fragments generated are then converted to thionitrobenzoate (TNB) derivatives. One of the Fab'-TNB derivatives is then reconverted to the Fab'-thiol by reduction with mercaptoethylamine and is mixed with an equimolar amount of the other Fab'-TNB derivative to form the bispecific antibody. The bispecific antibodies produced can be used as agents for the selective immobilization of enzymes.

Recent progress has facilitated the direct recovery of Fab'-SH fragments from *E. coli,* which can be chemically coupled to form bispecific antibodies. Shalaby et al, J. Exp. Med., 175: 217-225 (1992) describe the production of a fully humanized bispecific antibody $F(ab')_2$ molecule. Each Fab' fragment was separately secreted from *E. coli* and subjected to directed chemical coupling *in vitro* to form the bispecific antibody. The bispecific antibody thus formed was able to bind to cells overexpressing the HER2 receptor and normal human T cells, as well as trigger the lytic activity of human cytotoxic lymphocytes against human breast tumor targets.

Various techniques for making and isolating bispecific antibody fragments directly from recombinant cell culture have also been described. For example, bispecific antibodies have been produced using leucine zippers. Kostelny et al., J. Immunol., 148(5):1547-1 553 (1992). The leucine zipper peptides from the Fos and Jun proteins were linked to the Fab' portions of two different antibodies by gene fusion. The antibody homodimers were reduced at the hinge region to form monomers and then re-oxidized to form the antibody heterodimers. This method can also be utilized for the production of antibody homodimers. The "diabody" technology described by Hollinger et al, Proc. Natl. Acad. Sci. USA, 90:6444-6448 (1993) has provided an alternative mechanism for making bispecific antibody fragments. The fragments comprise a heavy-chain variable domain (VH) connected to a light-chain variable domain (VL) by a linker which is too short to allow pairing between the two domains on the same chain. Accordingly, the VH and VL domains of one fragment are forced to pair with the complementary VL and VH domains of another fragment, thereby forming two antigen-binding sites. Another strategy for making bispecific antibody fragments by the use of single-chain Fv (sFv) dimers has also been reported. See Gruber et al., J. Immunol., 152:5368 (1994).

Antibodies with more than two valencies are contemplated. For example, trispecific antibodies can be prepared. Tutt et al. J. Immunol. 147: 60 (1991).

### *8. Multivalent Antibodies*

Multivalent antibodies are also provided. A multivalent antibody may be internalized (and/or catabolized) faster than a bivalent antibody by a cell expressing an antigen to which the antibodies bind. The antibodies of the present invention can be multivalent antibodies (which are other than of the IgM class) with three or more antigen binding sites (e.g. tetravalent antibodies), which can be readily produced by recombinant expression of nucleic acid encoding the polypeptide chains of the antibody. The multivalent antibody can comprise a dimerization domain and three or more antigen binding sites. In certain embodiments, the dimerization domain comprises (or consists of) an Fc region or a hinge region. In this scenario, the antibody will comprise an Fc region and three or more antigen binding sites amino-terminal to the Fc region. In certain embodiments, a multivalent antibody comprises (or consists of) three to about eight antigen binding sites. In one such embodiment, a multivalent antibody comprises (or consists of) four antigen binding sites. The multivalent antibody comprises at least one polypeptide chain (for example, two polypeptide chains), wherein the polypeptide chain(s) comprise two or more variable domains. For instance, the polypeptide chain(s) may comprise VD1-(X1)n -VD2-(X2)n -Fc, wherein VD1 is a first variable domain, VD2 is a second variable domain, Fc is one polypeptide chain of an Fc region, X1 and X2 represent an amino acid or polypeptide, and n is 0 or 1. For instance, the polypeptide chain(s) may comprise: VH-CH1-flexible linker-VH-CH1-Fc region chain; or VH-CH1-VH-CH1-Fc region chain. The multivalent antibody herein may further comprise at least two (for example, four) light chain variable domain polypeptides. The multivalent antibody herein may, for instance, comprise from about two to about eight light chain variable domain polypeptides. The light chain variable domain polypeptides contemplated here comprise a light chain variable domain and, optionally, further comprise a CL domain.

### *9. Single-Domain Antibodies*

Single-domain antibodies are also provided. A single-domain antibody is a single polyeptide chain comprising all or a portion of the heavy chain variable domain or all or a portion of the light chain variable domain of an antibody. In certain embodiments, a single-domain antibody is a human single-domain antibody (Domantis, Inc., Waltham, MA; *see, e.g.,* U.S. Patent No. 6,248,516 B1). In one embodiment, a single-domain antibody consists of all or a portion of the heavy chain variable domain of an antibody.

### *10. Antibody Variants*

In some embodiments, amino acid sequence modification(s) of the antibodies described herein are contemplated. For example, it may be desirable to improve the binding affinity and/or other biological properties of the antibody. Amino acid sequence variants of the antibody may be prepared by introducing appropriate changes into the nucleotide sequence encoding the antibody, or by peptide synthesis. Such modifications include, for example, deletions from, and/or insertions into and/or substitutions of, residues within the amino acid sequences of the antibody. Any combination of deletion, insertion, and substitution can be made to arrive at the final construct, provided that the final construct possesses the desired characteristics. The amino acid alterations may be introduced in the subject antibody amino acid sequence at the time that sequence is made.

A useful method for identification of certain residues or regions of the antibody that are preferred locations for mutagenesis is called "alanine scanning mutagenesis" as described by Cunningham and Wells (1989) Science, 244:1081-1085. Here, a residue or group of target residues are identified (*e.g.*, charged residues such as arg, asp, his, lys, and glu) and replaced by a neutral or negatively charged amino acid (e.g., alanine or polyalanine) to affect the interaction of the amino acids with antigen. Those amino acid locations demonstrating functional sensitivity to the substitutions then are refined by introducing further or other variants at, or for, the sites of substitution. Thus, while the site for introducing an amino acid sequence variation is predetermined, the nature of the mutation *per se* need not be predetermined. For example, to analyze the performance of a mutation at a given site, ala scanning or random mutagenesis is conducted at the target codon or region and the expressed immunoglobulins are screened for the desired activity.

Amino acid sequence insertions include amino- and/or carboxyl-terminal fusions ranging in length from one residue to polypeptides containing a hundred or more residues, as well as intrasequence insertions of single or multiple amino acid residues. Examples of terminal insertions include an antibody with an N-terminal methionyl residue. Other insertional variants of the antibody molecule include the fusion to the N- or C-terminus of the antibody to an enzyme (*e.g.* for ADEPT) or a polypeptide which increases the serum half-life of the antibody.

In certain embodiments, an antibody of the invention is altered to increase or decrease the extent to which the antibody is glycosylated. Glycosylation of polypeptides is typically either N-linked or O-linked. N-linked refers to the attachment of a carbohydrate moiety to the side chain of an asparagine residue. The tripeptide sequences asparagine-X-serine and asparagine-X-threonine, where X is any amino acid except proline, are the recognition sequences for enzymatic attachment of the carbohydrate moiety to the asparagine side chain. Thus, the presence of either of these tripeptide sequences in a polypeptide creates a potential glycosylation site. O-linked glycosylation refers to the attachment of one of the sugars N-aceylgalactosamine, galactose, or xylose to a hydroxyamino acid, most commonly serine or threonine, although 5-hydroxyproline or 5-hydroxylysine may also be used.

Addition or deletion of glycosylation sites to the antibody is conveniently accomplished by altering the amino acid sequence such that one or more of the above-described tripeptide sequences (for N-linked glycosylation sites) is created or removed. The alteration may also be made by the addition, deletion, or substitution of one or more serine or threonine residues to the sequence of the original antibody (for O-linked glycosylation sites).

Where the antibody comprises an Fc region, the carbohydrate attached thereto may be altered. For example, antibodies with a mature carbohydrate structure that lacks fucose attached to an Fc region of the antibody are described in US Pat Appl No US 2003/0157108 (Presta, L.). See also US 2004/0093621 (Kyowa Hakko Kogyo Co., Ltd). Antibodies with a bisecting N-acetylglucosamine (GlcNAc) in the carbohydrate attached to an Fc region of the antibody are referenced in WO 2003/01 1878, Jean-Mairet et al. and US Patent No. 6,602,684, Umana et al. Antibodies with at least one galactose residue in the oligosaccharide attached to an Fc region of the antibody are reported in WO 1997/30087, Patel et al. See, also, WO 1998/58964 (Raju, S.) and WO 1999/22764 (Raju, S.) concerning antibodies with altered carbohydrate attached to the Fc region thereof. See also US 2005/0123546 (Umana et al.) on antigen-binding molecules with modified glycosylation.

In certain embodiments, a glycosylation variant comprises an Fc region, wherein a carbohydrate structure attached to the Fc region lacks fucose. Such variants have improved ADCC function. Optionally, the Fc region further comprises one or more amino acid substitutions therein which further improve ADCC, for example, substitutions at positions 298, 333, and/or 334 of the Fc region (Eu numbering of residues). Examples of publications related to "defucosylated" or "fucose-deficient" antibodies include: US 2003/0157108; WO 2000/61739; WO 2001/29246; US 2003/01 15614; US 2002/0164328; US 2004/0093621; US 2004/0132140; US 2004/0110704; US 2004/01 10282; US 2004/0109865; WO 2003/085119; WO 2003/084570; WO 2005/035586; WO 2005/035778; WO2005/053742; Okazaki et al. J. Mol. Biol. 336:1239-1249 (2004); Yamane-Ohnuki et al. Biotech. Bioeng 87: 614 (2004). Examples of cell lines producing defucosylated antibodies include Lec13 CHO cells deficient in protein fucosylation (Ripka et al. Arch. Biochem. Biophys. 249:533-545 (1986); US Pat Appl No US 2003/0157108 A1, Presta, L; and WO 2004/056312 A1, Adams et al*.,* especially at Example 11), and knockout cell lines, such as alpha-1,6-fucosyltransferase gene, *FUT8,* knockout CHO cells (Yamane-Ohnuki et al. Biotech. Bioeng. 87: 614 (2004)).

Another type of variant is an amino acid substitution variant. These variants have at least one amino acid residue in the antibody molecule replaced by a different residue. Sites of interest for substitutional mutagenesis include the hypervariable regions, but FR alterations are also contemplated. Conservative substitutions are shown in Table 3 above under the heading of "preferred substitutions." If such substitutions result in a desirable change in biological activity, then more substantial changes, denominated "exemplary substitutions" in Table 3, or as further described above in reference to amino acid classes, may be introduced and the resulting antibodies screened for the desired binding propeties.

One type of substitutional variant involves substituting one or more hypervariable region residues of a parent antibody (*e.g.* a humanized or human antibody). Generally, the resulting variant(s) selected for further development will have modified (e.g., improved) biological properties relative to the parent antibody from which they are generated. A convenient way for generating such substitutional variants involves affinity maturation using phage display. Briefly, several hypervariable region sites (*e.g.* 6-7 sites) are mutated to generate all possible amino acid substitutions at each site. The antibodies thus generated are displayed from filamentous phage particles as fusions to at least part of a phage coat protein (e.g., the gene III product of M13) packaged within each particle. The phage-displayed variants are then screened for their biological activity (*e.g.* binding affinity). In order to identify candidate hypervariable region sites for modification, scanning mutagenesis (e.g., alanine scanning) can be performed to identify hypervariable region residues contributing significantly to antigen binding. Alternatively, or additionally, it may be beneficial to analyze a crystal structure of the antigen-antibody complex to identify contact points between the antibody and antigen. Such contact residues and neighboring residues are candidates for substitution according to techniques known in the art, including those elaborated herein. Once such variants are generated, the panel of variants is subjected to screening using techniques known in the art, including those described herein, and antibodies with superior properties in one or more relevant assays may be selected for further development.

Nucleic acid molecules encoding amino acid sequence variants of the antibody are prepared by a variety of methods known in the art. These methods include, but are not limited to, isolation from a natural source (in the case of naturally occurring amino acid sequence variants) or preparation by oligonucleotide-mediated (or site-directed) mutagenesis, PCR mutagenesis, and cassette mutagenesis of an earlier prepared variant or a non-variant version of the antibody.

It may be desirable to introduce one or more amino acid modifications in an Fc region of antibodies of the invention, thereby generating an Fc region variant. The Fc region variant may comprise a human Fc region sequence (*e.g.,* a human IgG1, IgG2, IgG3 or IgG4 Fc region) comprising an amino acid modification (*e.g.* a substitution) at one or more amino acid positions including that of a hinge cysteine.

In accordance with this description and the teachings of the art, it is contemplated that in some embodiments, an antibody of the invention may comprise one or more alterations as compared to the wild type counterpart antibody, e.g. in the Fc region. These antibodies would nonetheless retain substantially the same characteristics required for therapeutic utility as compared to their wild type counterpart. For example, it is thought that certain alterations can be made in the Fc region that would result in altered (*i.e.,* either improved or diminished) Clq binding and/or Complement Dependent Cytotoxicity (CDC), e.g., as described in WO99/51642. See also Duncan & Winter Nature 322:738-40 (1988); U.S. Patent No. 5,648,260; U.S. Patent No. 5,624,821; and WO94/29351 concerning other examples of Fc region variants. WO00/42072 (Presta) and WO 2004/056312 (Lowman) describe antibody variants with improved or diminished binding to FcRs. The content of these patent publications are specifically incorporated herein by reference. See, also, Shields et al. J. Biol. Chem. 9(2): 6591-6604 (2001). Antibodies with increased half lives and improved binding to the neonatal Fc receptor (FcRn), which is responsible for the transfer of maternal IgGs to the fetus (Guyer et al., J. Immunol. 1 17:587 (1976) and Kim et al., J. Immunol. 24:249 (1994)), are described in US2005/0014934A1 (Hinton et al.). These antibodies comprise an Fc region with one or more substitutions therein which improve binding of the Fc region to FcRn. Polypeptide variants with altered Fc region amino acid sequences and increased or decreased Clq binding capability are described in US patent No. 6,194,551B1, WO99/51642. The contents of those patent publications are specifically incorporated herein by reference. See, also, Idusogie et al. J Immunol. 164: 4178-4184 (2000).

In one embodiment, the invention provides antibodies comprising modifications in the interface of Fc polypeptides comprising the Fc region, wherein the modifications facilitate and/or promote heterodimerization. These modifications comprise introduction of a protuberance into a first Fc polypeptide and a cavity into a second Fc polypeptide, wherein the protuberance is positionable in the cavity so as to promote complexing of the first and second Fc polypeptides. Methods of generating antibodies with these modifications are known in the art, e.g., as described in U.S. Pat. No. 5,731,168.

### *11. Antibody Derivatives*

Antibodies can be further modified to contain additional nonproteinaceous moieties that are known in the art and readily available. Preferably, the moieties suitable for derivatization of the antibody are water soluble polymers. Non-limiting examples of water soluble polymers include, but are not limited to, polyethylene glycol (PEG), copolymers of ethylene glycol/propylene glycol, carboxymethylcellulose, dextran, polyvinyl alcohol, polyvinyl pyrrolidone, poly-1, 3-dioxolane, po1y-1,3,6-trioxane, ethylene/maleic anhydride copolymer, polyaminoacids (either homopolymers or random copolymers), and dextran or poly(n-vinyl pyrrolidone)polyethylene glycol, propropylene glycol homopolymers, prolypropylene oxide/ethylene oxide co-polymers, polyoxyethylated polyols (e.g., glycerol), polyvinyl alcohol, and mixtures thereof. Polyethylene glycol propionaldehyde may have advantages in manufacturing due to its stability in water. The polymer may be of any molecular weight, and may be branched or unbranched. The number of polymers attached to the antibody may vary, and if more than one polymer are attached, they can be the same or different molecules. In general, the number and/or type of polymers used for derivatization can be determined based on considerations including, but not limited to, the particular properties or functions of the antibody to be improved, whether the antibody derivative will be used in a therapy under defined conditions, etc.

In another embodiment, conjugates of an antibody and nonproteinaceous moiety that may be selectively heated by exposure to radiation are provided. In one embodiment, the nonproteinaceous moiety is a carbon nanotube (Kam et al., Proc. Natl. Acad. Sci. 102: 11600-11605 (2005)). The radiation may be of any wavelength, and includes, but is not limited to, wavelengths that do not harm ordinary cells, but which heat the nonproteinaceous moiety to a temperature at which cells proximal to the antibody-nonproteinaceous moiety are killed.

In certain embodiments, an antibody may be labeled and/or may be immobilized on a solid support. In a further embodiment, an antibody is an anti-idiotypic antibody.

### *12. Heteroconjugate Antibodies*

Heteroconjugate antibodies are also provided. Heteroconjugate antibodies are composed of two covalently joined antibodies. Such antibodies have, for example, been proposed to target immune system cells to unwanted cells [U.S. Patent No. 4,676,980], and for treatment of HIV infection [WO 91/00360; WO 92/200373; EP 03089]. It is contemplated that the antibodies may be prepared *in vitro* using known methods in synthetic protein chemistry, including those involving crosslinking agents. For example, immunotoxins may be constructed using a disulfide exchange reaction or by forming a thioether bond. Examples of suitable reagents for this purpose include iminothiolate and methyl-4-mercaptobutyrimidate and those disclosed, for example, in U.S. Patent No. 4,676,980.

### *13. Effector Function Engineering*

It may be desirable to modify an antibody with respect to effector function, so as to enhance, *e.g.,* the effectiveness of the antibody in treating a microbial disorder. For example, cysteine residue(s) may be introduced into the Fc region, thereby allowing interchain disulfide bond formation in this region. Homodimeric antibodies with enhanced anti-anti-microbial activity may also be prepared using heterobifunctional cross-linkers. Alternatively, an antibody can be engineered that has dual Fc regions and may thereby have enhanced activity.

### *14. Vectors, Host Cells, and Recombinant Methods*

For recombinant production of an antibody, in one embodiment, the nucleic acid encoding it is isolated and inserted into a replicable vector for further cloning (amplification of the DNA) or for expression. DNA encoding the antibody is readily isolated and sequenced using conventional procedures (e.g., by using oligonucleotide probes that are capable of binding specifically to genes encoding the heavy and light chains of the antibody). Many vectors are available. The choice of vector depends in part on the host cell to be used. Generally, host cells are of either prokaryotic or eukaryotic (generally mammalian) origin. It will be appreciated that constant regions of any isotype can be used for this purpose, including IgG, IgM, IgA, IgD, and IgE constant regions, and that such constant regions can be obtained from any human or animal species.

a)Generating antibodies using prokaryotic host cells:

*(1) Vector Construction*

Polynucleotide sequences encoding polypeptide components of an antibody can be obtained using standard recombinant techniques. Desired polynucleotide sequences may be isolated and sequenced from antibody producing cells such as hybridoma cells. Alternatively, polynucleotides can be synthesized using nucleotide synthesizer or PCR techniques. Once obtained, sequences encoding the polypeptides are inserted into a recombinant vector capable of replicating and expressing heterologous polynucleotides in prokaryotic hosts. Many vectors that are available and known in the art can be used for the purpose of the present invention. Selection of an appropriate vector will depend mainly on the size of the nucleic acids to be inserted into the vector and the particular host cell to be transformed with the vector. Each vector contains various components, depending on its function (amplification or expression of heterologous polynucleotide, or both) and its compatibility with the particular host cell in which it resides. The vector components generally include, but are not limited to: an origin of replication, a selection marker gene, a promoter, a ribosome binding site (RBS), a signal sequence, the heterologous nucleic acid insert and a transcription termination sequence.

In general, plasmid vectors containing replicon and control sequences which are derived from species compatible with the host cell may be used in connection with these hosts. The vector ordinarily carries a replication site, as well as marking sequences which are capable of providing phenotypic selection in transformed cells. For example, E. coli is typically transformed using pBR322, a plasmid derived from an E. coli species. pBR322 contains genes encoding ampicillin (Amp) and tetracycline (Tet) resistance and thus provides easy means for identifying transformed cells. pBR322, its derivatives, or other microbial plasmids or bacteriophage may also contain, or be modified to contain, promoters which can be used by the microbial organism for expression of endogenous proteins. Examples of pBR322 derivatives used for expression of particular antibodies are described in detail in Carter et al., U.S. Patent No. 5,648,237.

In addition, phage vectors containing replicon and control sequences that are compatible with the host microorganism can be used as transforming vectors in connection with these hosts. For example, bacteriophage such as λGEM.TM.-11 may be utilized in making a recombinant vector which can be used to transform susceptible host cells such as E. coli LE392.

An expression vector of the invention may comprise two or more promoter-cistron pairs, encoding each of the polypeptide components. A promoter is an untranslated regulatory sequence located upstream (5') to a cistron that modulates its expression. Prokaryotic promoters typically fall into two classes, inducible and constitutive. Inducible promoter is a promoter that initiates increased levels of transcription of the cistron under its control in response to changes in the culture condition, e.g. the presence or absence of a nutrient or a change in temperature.

A large number of promoters recognized by a variety of potential host cells are well known. The selected promoter can be operably linked to cistron DNA encoding the light or heavy chain by removing the promoter from the source DNA via restriction enzyme digestion and inserting the isolated promoter sequence into the vector of the invention. Both the native promoter sequence and many heterologous promoters may be used to direct amplification and/or expression of the target genes. In some embodiments, heterologous promoters are utilized, as they generally permit greater transcription and higher yields of expressed target gene as compared to the native target polypeptide promoter.

Promoters suitable for use with prokaryotic hosts include the PhoA promoter, the β-galactamase and lactose promoter systems, a tryptophan (trp) promoter system and hybrid promoters such as the tac or the trc promoter. However, other promoters that are functional in bacteria (such as other known bacterial or phage promoters) are suitable as well. Their nucleotide sequences have been published, thereby enabling a skilled worker operably to ligate them to cistrons encoding the target light and heavy chains (Siebenlist et al. (1980) Cell 20: 269) using linkers or adaptors to supply any required restriction sites.

In one embodiment of the invention, each cistron within the recombinant vector comprises a secretion signal sequence component that directs translocation of the expressed polypeptides across a membrane. In general, the signal sequence may be a component of the vector, or it may be a part of the target polypeptide DNA that is inserted into the vector. The signal sequence selected for the purpose of this invention should be one that is recognized and processed (i.e. cleaved by a signal peptidase) by the host cell. For prokaryotic host cells that do not recognize and process the signal sequences native to the heterologous polypeptides, the signal sequence is substituted by a prokaryotic signal sequence selected, for example, from the group consisting of the alkaline phosphatase, penicillinase, Ipp, or heat-stable enterotoxin II (STII) leaders, LamB, PhoE, PelB, OmpA and MBP. In one embodiment of the invention, the signal sequences used in both cistrons of the expression system are STII signal sequences or variants thereof.

In another embodiment, the production of the immunoglobulins according to the invention can occur in the cytoplasm of the host cell, and therefore does not require the presence of secretion signal sequences within each cistron. In that regard, immunoglobulin light and heavy chains are expressed, folded and assembled to form functional immunoglobulins within the cytoplasm. Certain host strains (e.g., the E. coli trxB- strains) provide cytoplasm conditions that are favorable for disulfide bond formation, thereby permitting proper folding and assembly of expressed protein subunits. Proba and Pluckthun Gene, 159:203 (1995).

Antibodies of the invention can also be produced by using an expression system in which the quantitative ratio of expressed polypeptide components can be modulated in order to maximize the yield of secreted and properly assembled antibodies of the invention. Such modulation is accomplished at least in part by simultaneously modulating translational strengths for the polypeptide components.

One technique for modulating translational strength is disclosed in Simmons et al., U.S. Pat. No. 5,840,523. It utilizes variants of the translational initiation region (TIR) within a cistron. For a given TIR, a series of amino acid or nucleic acid sequence variants can be created with a range of translational strengths, thereby providing a convenient means by which to adjust this factor for the desired expression level of the specific chain. TIR variants can be generated by conventional mutagenesis techniques that result in codon changes which can alter the amino acid sequence. In certain embodiments, changes in the nucleotide sequence are silent. Alterations in the TIR can include, for example, alterations in the number or spacing of Shine-Dalgarno sequences, along with alterations in the signal sequence. One method for generating mutant signal sequences is the generation of a "codon bank" at the beginning of a coding sequence that does not change the amino acid sequence of the signal sequence (i.e., the changes are silent). This can be accomplished by changing the third nucleotide position of each codon; additionally, some amino acids, such as leucine, serine, and arginine, have multiple first and second positions that can add complexity in making the bank. This method of mutagenesis is described in detail in Yansura et al. (1992) METHODS: A Companion to Methods in Enzymol. 4:151-158.

In one embodiment, a set of vectors is generated with a range of TIR strengths for each cistron therein. This limited set provides a comparison of expression levels of each chain as well as the yield of the desired antibody products under various TIR strength combinations. TIR strengths can be determined by quantifying the expression level of a reporter gene as described in detail in Simmons et al. U.S. Pat. No. 5, 840,523. Based on the translational strength comparison, the desired individual TIRs are selected to be combined in the expression vector constructs of the invention.

Prokaryotic host cells suitable for expressing antibodies of the invention include Archaebacteria and Eubacteria, such as Gram-negative or Gram-positive organisms. Examples of useful bacteria include Escherichia (e.g., E. coli), Bacilli (e.g., B. subtilis), Enterobacteria, Pseudomonas species (e.g., P. aeruginosa), Salmonella typhimurium, Serratia marcescans, Klebsiella, Proteus, Shigella, Rhizobia, Vitreoscilla, or Paracoccus. In one embodiment, gram-negative cells are used. In one embodiment, E. coli cells are used as hosts for the invention. Examples of E. coli strains include strain W3110 (Bachmann, Cellular and Molecular Biology, vol. 2 (Washington, D.C.: American Society for Microbiology, 1987), pp. 1190-1219; ATCC Deposit No. 27,325) and derivatives thereof, including strain 33D3 having genotype W3110 ΔfhuA (ΔtonA) ptr3 lac Iq lacL8 ΔompTΔ(nmpc-fepE) degP41 kanR (U.S. Pat. No. 5,639,635). Other strains and derivatives thereof, such as E. coli 294 (ATCC 31,446), E. coli B, E. coliλ 1776 (ATCC 31,537) and E. coli RV308(ATCC 31,608) are also suitable. These examples are illustrative rather than limiting. Methods for constructing derivatives of any of the above-mentioned bacteria having defined genotypes are known in the art and described in, for example, Bass et al., Proteins, 8:309-314 (1990). It is generally necessary to select the appropriate bacteria taking into consideration replicability of the replicon in the cells of a bacterium. For example, E. coli, Serratia, or Salmonella species can be suitably used as the host when well known plasmids such as pBR322, pBR325, pACYC177, or pKN410 are used to supply the replicon. Typically the host cell should secrete minimal amounts of proteolytic enzymes, and additional protease inhibitors may desirably be incorporated in the cell culture .

*(2) Antibody Production*

Most cells are transformed with the above-described expression vectors and cultured in conventional nutrient media modified as appropriate for inducing promoters, selecting transform ants, or amplifying the genes encoding the desired sequences.

Transformation means introducing DNA into the prokaryotic host so that the DNA is replicable, either as an extrachromosomal element or by chromosomal integrant. Depending on the host cell used, transformation is done using standard techniques appropriate to such cells. The calcium treatment employing calcium chloride is generally used for bacterial cells that contain substantial cell-wall barriers. Another method for transformation employs polyethylene glycol/DMSO. Yet another technique used is electroporation.

Prokaryotic cells used to produce the polypeptides of the invention are grown in media known in the art and suitable for culture of the selected host cells. Examples of suitable media include luria broth (LB) plus necessary nutrient supplements. In some embodiments, the media also contains a selection agent, chosen based on the construction of the expression vector, to selectively permit growth of prokaryotic cells containing the expression vector. For example, ampicillin is added to media for growth of cells expressing ampicillin resistant gene.

Any necessary supplements besides carbon, nitrogen, and inorganic phosphate sources may also be included at appropriate concentrations introduced alone or as a mixture with another supplement or medium such as a complex nitrogen source. Optionally the culture medium may contain one or more reducing agents selected from the group consisting of glutathione, cysteine, cystamine, thioglycollate, dithioerythritol and dithiothreitol.

The prokaryotic host cells are cultured at suitable temperatures. In certain embodiments, for E. coli growth, growth temperatures range from about 20°C to about 39°C; from about 25°C to about 37°C; or about 30°C. The pH of the medium may be any pH ranging from about 5 to about 9, depending mainly on the host organism. In certain embodiments, for E. coli, the pH is from about 6.8 to about 7.4, or about 7.0.

If an inducible promoter is used in the expression vector of the invention, protein expression is induced under conditions suitable for the activation of the promoter. In one embodiment of the invention, PhoA promoters are used for controlling transcription of the polypeptides. Accordingly, the transformed host cells are cultured in a phosphate-limiting medium for induction. In certain embodiments, the phosphate-limiting medium is the C.R.A.P. medium (see, e.g., Simmons et al., J. Immunol. Methods (2002), 263: 133-147). A variety of other inducers may be used, according to the vector construct employed, as is known in the art.

In one embodiment, the expressed polypeptides of the present invention are secreted into and recovered from the periplasm of the host cells. Protein recovery typically involves disrupting the microorganism, generally by such means as osmotic shock, sonication or lysis. Once cells are disrupted, cell debris or whole cells may be removed by centrifugation or filtration. The proteins may be further purified, for example, by affinity resin chromatography. Alternatively, proteins can be transported into the culture media and isolated therein. Cells may be removed from the culture and the culture supernatant being filtered and concentrated for further purification of the proteins produced. The expressed polypeptides can be further isolated and identified using commonly known methods such as polyacrylamide gel electrophoresis (PAGE) and Western blot assay.

In one embodiment of the invention, antibody production is conducted in large quantity by a fermentation process. Various large-scale fed-batch fermentation procedures are available for production of recombinant proteins. Large-scale fermentations have at least 1000 liters of capacity, and in certain embodiments, about 1,000 to 100,000 liters of capacity. These fermentors use agitator impellers to distribute oxygen and nutrients, especially glucose (the preferred carbon/energy source). Small scale fermentation refers generally to fermentation in a fermentor that is no more than approximately 100 liters in volumetric capacity, and can range from about 1 liter to about 100 liters.

In a fermentation process, induction of protein expression is typically initiated after the cells have been grown under suitable conditions to a desired density, e.g., an OD550 of about 180-220, at which stage the cells are in the early stationary phase. A variety of inducers may be used, according to the vector construct employed, as is known in the art and described above. Cells may be grown for shorter periods prior to induction. Cells are usually induced for about 12-50 hours, although longer or shorter induction time may be used.

To improve the production yield and quality of the polypeptides of the invention, various fermentation conditions can be modified. For example, to improve the proper assembly and folding of the secreted antibody polypeptides, additional vectors overexpressing chaperone proteins, such as Dsb proteins (DsbA, DsbB, DsbC, DsbD and or DsbG) or FkpA (a peptidylprolyl cis,trans-isomerase with chaperone activity) can be used to co-transform the host prokaryotic cells. The chaperone proteins have been demonstrated to facilitate the proper folding and solubility of heterologous proteins produced in bacterial host cells. Chen et al. (1999) J. Biol. Chem. 274:19601-19605; Georgiou et al., U.S. Patent No. 6,083,715; Georgiou et al., U.S. Patent No. 6,027,888; Bothmann and Pluckthun (2000) J. Biol. Chem. 275:17100-17105; Ramm and Pluckthun (2000) J. Biol. Chem. 275:17106-171 13; Arie et al. (2001) Mol. Microbiol. 39:199-210.

To minimize proteolysis of expressed heterologous proteins (especially those that are proteolytically sensitive), certain host strains deficient for proteolytic enzymes can be used for the present invention. For example, host cell strains may be modified to effect genetic mutation(s) in the genes encoding known bacterial proteases such as Protease III, OmpT, DegP, Tsp, Protease I, Protease Mi, Protease V, Protease VI and combinations thereof. Some E. coli protease-deficient strains are available and described in, for example, Joly et al. (1998), *supra*; Georgiou et al., U.S. Patent No.

5,264,365; Georgiou et al., U.S. Patent No. 5,508,192; Hara et al., Microbial Drug Resistance, 2:63-72 (1996).

In one embodiment, E. coli strains deficient for proteolytic enzymes and transformed with plasmids overexpressing one or more chaperone proteins are used as host cells in the expression system of the invention.

*(3) Antibody Purification*

In one embodiment, an antibody produced herein is further purified to obtain preparations that are substantially homogeneous for further assays and uses. Standard protein purification methods known in the art can be employed. The following procedures are exemplary of suitable purification procedures: fractionation on immunoaffinity or ion-exchange columns, ethanol precipitation, reverse phase HPLC, chromatography on silica or on a cation-exchange resin such as DEAE, chromatofocusing, SDS-PAGE, ammonium sulfate precipitation, and get filtration using, for example, Sephadex G-75.

In one embodiment, Protein A immobilized on a solid phase is used for immunoaffinity purification of the antibody products of the invention. Protein A is a 41kD cell wall protein from *Staphylococcus aureas* which binds with a high affinity to the Fc region of antibodies. Lindmark et al (1983) J. Immunol. Meth. 62:1-13. The solid phase to which Protein A is immobilized can be a column comprising a glass or silica surface, or a controlled pore glass column or a silicic acid column. In some applications, the column is coated with a reagent, such as glycerol, to possibly prevent nonspecific adherence of contaminants.

As the first step of purification, a preparation derived from the cell culture as described above can be applied onto a Protein A immobilized solid phase to allow specific binding of the antibody of interest to Protein A. The solid phase would then be washed to remove contaminants non-specifically bound to the solid phase. Finally the antibody of interest is recovered from the solid phase by elution.

b) Generating antibodies using eukaryotic host cells:

A vector for use in a eukaryotic host cell generally includes one or more of the following non-limiting components: a signal sequence, an origin of replication, one or more marker genes, an enhancer element, a promoter, and a transcription termination sequence.

*(1) Signal sequence component*

A vector for use in a eukaryotic host cell may also contain a signal sequence or other polypeptide having a specific cleavage site at the N-terminus of the mature protein or polypeptide of interest. The heterologous signal sequence selected may be one that is recognized and processed (i.e., cleaved by a signal peptidase) by the host cell. In mammalian cell expression, mammalian signal sequences as well as viral secretory leaders, for example, the herpes simplex gD signal, are available. The DNA for such a precursor region is ligated in reading frame to DNA encoding the antibody.

*(2) Origin of replication*

Generally, an origin of replication component is not needed for mammalian expression vectors. For example, the SV40 origin may typically be used only because it contains the early promoter.

*(3) Selection gene component*

Expression and cloning vectors may contain a selection gene, also termed a selectable marker. Typical selection genes encode proteins that (a) confer resistance to antibiotics or other toxins, e.g., ampicillin, neomycin, methotrexate, or tetracycline, (b) complement auxotrophic deficiencies, where relevant, or (c) supply critical nutrients not available from complex media.

One example of a selection scheme utilizes a drug to arrest growth of a host cell. Those cells that are successfully transformed with a heterologous gene produce a protein conferring drug resistance and thus survive the selection regimen. Examples of such dominant selection use the drugs neomycin, mycophenolic acid and hygromycin.

Another example of suitable selectable markers for mammalian cells are those that enable the identification of cells competent to take up the antibody nucleic acid, such as DHFR, thymidine kinase, metallothionein-I and -II, preferably primate metallothionein genes, adenosine deaminase, ornithine decarboxylase, etc.

For example, in some embodiments, cells transformed with the DHFR selection gene are first identified by culturing all of the transformants in a culture medium that contains methotrexate (Mtx), a competitive antagonist of DHFR. In some embodiments, an appropriate host cell when wild-type DHFR is employed is the Chinese hamster ovary (CHO) cell line deficient in DHFR activity (e.g., ATCC CRL-9096).

Alternatively, host cells (particularly wild-type hosts that contain endogenous DHFR) transformed or co-transformed with DNA sequences encoding an antibody, wild-type DHFR protein, and another selectable marker such as aminoglycoside 3'-phosphotransferase (APH) can be selected by cell growth in medium containing a selection agent for the selectable marker such as an aminoglycosidic antibiotic, e.g., kanamycin, neomycin, or G418. See U.S. Patent No. 4,965,199.

*(4) Promoter component*

Expression and cloning vectors usually contain a promoter that is recognized by the host organism and is operably linked to nucleic acid encoding a polypeptide of interest (e.g., an antibody). Promoter sequences are known for eukaryotes. For example, virtually all eukaryotic genes have an AT-rich region located approximately 25 to 30 bases upstream from the site where transcription is initiated. Another sequence found 70 to 80 bases upstream from the start of transcription of many genes is a CNCAAT region where N may be any nucleotide. At the 3' end of most eukaryotic genes is an AATAAA sequence that may be the signal for addition of the poly A tail to the 3' end of the coding sequence. In certain embodiments, any or all of these sequences may be suitably inserted into eukaryotic expression vectors.

Transcription from vectors in mammalian host cells is controlled, for example, by promoters obtained from the genomes of viruses such as polyoma virus, fowlpox virus, adenovirus (such as Adenovirus 2), bovine papilloma virus, avian sarcoma virus, cytomegalovirus, a retrovirus, hepatitis-B virus and Simian Virus 40 (SV40), from heterologous mammalian promoters, e.g., the actin promoter or an immunoglobulin promoter, from heat-shock promoters, provided such promoters are compatible with the host cell systems.

The early and late promoters of the SV40 virus are conveniently obtained as an SV40 restriction fragment that also contains the SV40 viral origin of replication. The immediate early promoter of the human cytomegalovirus is conveniently obtained as a HindIII E restriction fragment. A system for expressing DNA in mammalian hosts using the bovine papilloma virus as a vector is disclosed in U.S. Patent No. 4,419,446. A modification of this system is described in U.S. Patent No. 4,601,978. See also Reyes et al., Nature 297:598-601 (1982), describing expression of human β-interferon cDNA in mouse cells under the control of a thymidine kinase promoter from herpes simplex virus. Alternatively, the Rous Sarcoma Virus long terminal repeat can be used as the promoter.

*(5) Enhancer element component*

Transcription of DNA encoding an antibody of this invention by higher eukaryotes is often increased by inserting an enhancer sequence into the vector. Many enhancer sequences are now known from mammalian genes (globin, elastase, albumin, α-fetoprotein, and insulin). Typically, however, one will use an enhancer from a eukaryotic cell virus. Examples include the SV40 enhancer on the late side of the replication origin (bp 100-270), the cytomegalovirus early promoter enhancer, the polyoma enhancer on the late side of the replication origin, and adenovirus enhancers. See also Yaniv, Nature 297:17-18 (1982) describing enhancer elements for activation of eukaryotic promoters. The enhancer may be spliced into the vector at a position 5' or 3' to the antibody polypeptide-encoding sequence, but is generally located at a site 5' from the promoter.

*(6) Transcription termination component*

Expression vectors used in eukaryotic host cells may also contain sequences necessary for the termination of transcription and for stabilizing the mRNA. Such sequences are commonly available from the 5' and, occasionally 3', untranslated regions of eukaryotic or viral DNAs or cDNAs. These regions contain nucleotide segments transcribed as polyadenylated fragments in the untranslated portion of the mRNA encoding an antibody. One useful transcription termination component is the bovine growth hormone polyadenylation region. See WO94/1 1026 and the expression vector disclosed therein.

*(7) Selection and transformation of host cells*

Suitable host cells for cloning or expressing the DNA in the vectors herein include higher eukaryote cells described herein, including vertebrate host cells. Propagation of vertebrate cells in culture (tissue culture) has become a routine procedure. Examples of useful mammalian host cell lines are monkey kidney CV1 line transformed by SV40 (COS-7, ATCC CRL 1651); human embryonic kidney line (293 or 293 cells subcloned for growth in suspension culture, Graham et al., J. Gen Virol. 36:59 (1977)) ; baby hamster kidney cells (BHK, ATCC CCL 10); Chinese hamster ovary cells/-DHFR (CHO, Urlaub et al., Proc. Natl. Acad. Sci. USA 77:4216 (1980)); mouse sertoli cells (TM4, Mather, Biol. Reprod. 23:243-251 (1980)); monkey kidney cells (CV1 ATCC CCL 70); African green monkey kidney cells (VERO-76, ATCC CRL-1587); human cervical carcinoma cells (HELA, ATCC CCL 2); canine kidney cells (MDCK, ATCC CCL 34);

buffalo rat liver cells (BRL 3A, ATCC CRL 1442); human lung cells (W138, ATCC CCL 75); human liver cells (Hep G2, HB 8065); mouse mammary tumor (MMT 060562, ATCC CCL51); TRI cells (Mather et al., Annals N.Y. Acad. Sci 383:44-68 (1982)); MRC 5 cells: FS4 cells; and a human hepatoma line (Hep G2).

Host cells are transformed with the above-described expression or cloning vectors for antibody production and cultured in conventional nutrient media modified as appropriate for inducing promoters, selecting transformants, or amplifying the genes encoding the desired sequences.

*(8) Culturing the host cells*

The host cells used to produce an antibody of this invention may be cultured in a variety of media. Commercially available media such as Ham's F10 (Sigma), Minimal Essential Medium ((MEM), (Sigma), RPMI-1640 (Sigma), and Dulbecco's Modified Eagle's Medium ((DMEM), Sigma) are suitable for culturing the host cells. In addition, any of the media described in Ham et al., Meth. Enz. 58:44 (1979), Barnes et al., Anal. Biochem. 102:255 (1980), U.S. Pat. Nos. 4,767,704; 4,657,866; 4,927,762; 4,560,655; or 5,122,469; WO 90/03430; WO 87/00195; or U.S. Patent Re. 30,985 may be used as culture media for the host cells. Any of these media may be supplemented as necessary with hormones and/or other growth factors (such as insulin, transferrin, or epidermal growth factor), salts (such as sodium chloride, calcium, magnesium, and phosphate), buffers (such as HEPES), nucleotides (such as adenosine and thymidine), antibiotics (such as GENTAMYCIN™ drug), trace elements (defined as inorganic compounds usually present at final concentrations in the micromolar range), and glucose or an equivalent energy source. Any other supplements may also be included at appropriate concentrations that would be known to those skilled in the art. The culture conditions, such as temperature, pH, and the like, are those previously used with the host cell selected for expression, and will be apparent to the ordinarily skilled artisan.

*(9) Purification of antibody*

When using recombinant techniques, the antibody can be produced intracellularly, or directly secreted into the medium. If the antibody is produced intracellularly, as a first step, the particulate debris, either host cells or lysed fragments, may be removed, for example, by centrifugation or ultrafiltration. Where the antibody is secreted into the medium, supernatants from such expression systems may be first concentrated using a commercially available protein concentration filter, for example, an Amicon or Millipore Pellicon ultrafiltration unit. A protease inhibitor such as PMSF may be included in any of the foregoing steps to inhibit proteolysis, and antibiotics may be included to prevent the growth of adventitious contaminants.

The antibody composition prepared from the cells can be purified using, for example, hydroxylapatite chromatography, gel electrophoresis, dialysis, and affinity chromatography, with affinity chromatography being a convenient technique. The suitability of protein A as an affinity ligand depends on the species and isotype of any immunoglobulin Fc domain that is present in the antibody. Protein A can be used to purify antibodies that are based on human $\gamma$1, $\gamma$2, or $\gamma$4 heavy chains (Lindmark et al., J. Immunol. Methods 62:1-13 (1983)). Protein G is recommended for all mouse isotypes and for human $\gamma$3 (Guss et al., EMBO J. 5:15671575 (1986)). The matrix to which the affinity ligand is attached may be agarose, but other matrices are available. Mechanically stable matrices such as controlled pore glass or poly(styrenedivinyl)benzene allow for faster flow rates and shorter processing times than can be achieved with agarose. Where the antibody comprises a CH3 domain, the Bakerbond ABX™resin (J. T. Baker, Phillipsburg, NJ) is useful for purification. Other techniques for protein purification such as fractionation on an ion-exchange column, ethanol precipitation, Reverse Phase HPLC, chromatography on silica, chromatography on heparin SEPHAROSE™ chromatography on an anion or cation exchange resin (such as a polyaspartic acid column), chromatofocusing, SDS-PAGE, and ammonium sulfate precipitation are also available depending on the antibody to be recovered.

Following any preliminary purification step(s), the mixture comprising the antibody of interest and contaminants may be subjected to further purification, for example, by low pH hydrophobic interaction chromatography using an elution buffer at a pH between about 2.5-4.5, preferably performed at low salt concentrations (e.g., from about 0-0.25M salt).

In general, various methodologies for preparing antibodies for use in research, testing, and clinical use are well-established in the art, consistent with the above-described methodologies and/or as deemed appropriate by one skilled in the art for a particular antibody of interest.

**C. Agonists and Antagonists**

Agonists and antagonists of an AMP of the present inventions are provided. Such AMP modulators are encompassed in the present invention and useful for treating a microbial disorder as provided herein.

In one embodiment, an agonist or antagonist of an AMP of the present invention is an antibody, e.g., and IL-22 antibody or an anti-IL-22R antibody. In certain embodiments, an anti-IL-22 antibody is an agonistic antibody that promotes the interaction of IL-22 with IL-22R. In another embodiment, an anti-IL-22 antibody is an antagonistic antibody that fully or partially blocks the interaction of IL-22 with IL-22R. In certain embodiments, an anti-IL-22R antibody binds to the extracellular ligand binding domain of an IL-22R. For example, an anti-IL-22R antibody may bind to the extracellular ligand binding domain of human IL-22R, which is found in SEQ ID NO:3 from about amino acids 18-228.

In a particular embodiment, an IL-22 agonist is an antibody that binds IL-22BP and blocks or inhibits binding of IL-22BP to IL-22, and thereby induces or increases an IL-22 activity (e.g., binding to IL-22R).

In another embodiment, an agonist or antagonist of an AMP of the present invention is an oligopeptide that binds to the AMP. In one embodiment, an oligopeptide binds to the extracellular ligand binding domain of IL-22R. Oligopeptides may be chemically synthesized using known oligopeptide synthesis methodology or may be prepared and purified using recombinant technology. Such oligopeptides are usually at least about 5 amino acids in length, alternatively at least about 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91 92, 93, 94, 95, 96, 97, 98, 99, or 100 amino acids in length. Such oligopeptides may be identified without undue experimentation using well known techniques. In this regard, it is noted that techniques for screening oligopeptide libraries for oligopeptides that are capable of specifically binding to a polypeptide target are well known in the art (see, e.g., U.S. Patent Nos. 5,556,762, 5,750,373, 4,708,871, 4,833,092, 5,223,409, 5,403,484, 5,571,689, 5,663,143; PCT Publication Nos. WO 84/03506 and WO84/03564; Geysen et al., Proc. Natl. Acad. Sci. U.S.A., 81:3998-4002 (1984); Geysen et al., Proc. Natl. Acad. Sci. USA, 82:178-182 (1985); Geysen et al., in Synthetic Peptides as Antigens, 130-149 (1986); Geysen et al., J. Immunol. Meth., 102:259-274 (1987); Schoofs et al., J. Immunol., 140:611-616 (1988), Cwirla, S. E. et al. (1990) Proc. Natl. Acad. Sci. USA, 87:6378; Lowman, H.B. et al. (1991) Biochemistry, 30:10832; Clackson, T. et al. (1991) Nature, 352: 624; Marks, J. D. et al. (1991), J. Mol. Biol., 222:581; Kang, A.S. et al. (1991) Proc. Natl. Acad. Sci. USA, 88:8363, and Smith, G. P. (1991) Current Opin. Biotechnol., 2:668).

In yet another embodiment, an agonist or antagoist of an AMP of the present invention is an organic molecule that binds to the AMP, other than an oligopeptide or antibody as described herein. An organic molecule may be, for example, a small molecule. In one embodiment, an organic molecule binds to the extracellular domain of an IL-22R. An organic molecule that binds to an AMP of the present invention may be identified and chemically synthesized using known methodology (see, e.g., PCT Publication Nos. WO00/00823 and WO00/39585). Such organic molecules are usually less than about 2000 daltons in size, alternatively less than about 1500, 750, 500, 250 or 200 daltons in size, wherein such organic molecules that are capable of binding to an AMP of the present invention may be identified without undue experimentation using well known techniques. In this regard, it is noted that techniques for screening organic molecule libraries for molecules that are capable of binding to a polypeptide target are well known in the art (see, e.g., PCT Publication Nos. WO00/00823 and WO00/39585).

In a particular embodiment, an IL-22 agonist is an organic molecule that binds IL-22BP and blocks or inhibits binding of IL-22BP to IL-22, and thereby induces or increases an IL-22 activity (e.g., binding to IL-22R.).

In a particular embodiment, an IL-22 antagonist is a soluble IL-22 receptor, e.g., a form of IL-22R that is not membrane bound. Such soluble forms of IL-22R may compete with membrane-bound IL-22R for binding to IL-22. In certain embodiments, a soluble form of IL-22R may comprise all or a ligand-binding portion of an extracellular domain of IL-22R, e.g., all or a ligand-binding portion of a polypeptide comprising amino acids 18-228 of SEQ ID NO:3. In certain embodiments, a soluble form of IL-22R lacks a transmembrane domain. For example, a soluble form of human IL-22R may lack all or a substantial portion of the transmembrane domain from about amino acids 229-251 of SEQ ID NO:3.

A naturally occurring, soluble receptor for IL-22 has been reported. *See* Dumoutier L. et al., "Cloning and characterization of IL-22 binding protein, a natural antagonist of IL-10-related T cell-derived inducible factor/IL-22," J. Immunol. 166:7090-7095 (2001); and Xu W. et al., "A soluble class 11 cytokine receptor, IL-22RA2, is a naturally occurring IL-22 antagonist," Proc. Natl. Acad. Sci. U.S.A. 98:9511-9516 (2001). That receptor is variously designated "IL-22BP" or "IL-22RA2" in the art. The sequence of a human IL-22BP is shown in Figure 4. The term "IL-22BP" or "IL-22 binding protein" as used herein refers to any native IL-22BP from any vertebrate source, including mammals such as primates (e.g. humans and monkeys) and rodents (e.g., mice and rats), unless otherwise indicated.

In yet another embodiment, an antagonist of IL-22 is an antisense nucleic acid that decreases expression of the IL-22 or IL-22R gene (i.e., that decreases transcription of the IL-22 or IL-22R gene and/or translation of IL-22 or IL-22R mRNA). In certain embodiments, an antisense nucleic acid binds to a nucleic acid (DNA or RNA) encoding IL-22 or IL-22R. In certain embodiments, an antisense nucleic acid is an oligonucleotide of about 10-30 nucleotides in length (including all points between those endpoints). In certain embodiments, an antisense oligonucleotide comprises a modified sugar-phosphodiester backbones (or other sugar linkages, including phosphorothioate linkages and linkages as described in WO 91/06629), wherein such modified sugar-phosphodiester backbones are resistant to endogenous nucleases. In one embodiment, an antisense nucleic acid is an oligodeoxyribonucleotide, which results in the degradation and/or reduced transcription or translation of mRNA encoding IL-22 or IL-22R. In certain embodiments, an antisense nucleic acid is an RNA that reduces expression of a target nucleic acid by "RNA interference" ("RNAi"). For review of RNAi, see, e.g., Novina et al. (2004) Nature 430:161-164. Such RNAs are derived from, for example, short interfering RNAs (siRNAs) and microRNAs. siRNAs, e.g., may be synthesized as double stranded oligoribonucleotides of about 18-26 nucleotides in length. *Id*.

In yet another embodiment, agonists of IL-22 are provided. Exemplary agonists include, but are not limited to, native IL-22 or IL-22R; fragments, variants, or modified forms of IL-22 or IL-22R that retain at least one activity of the native polypeptide; agents that are able to bind to and activate IL-22R; and agents that induce overexpression of IL-22 or IL-22R or nucleic acids encoding IL-22 or IL-22R.

**D. Pharmaceutical Formulations**

The invention provides pharmaceutical formulations. In one embodiment, a pharmaceutical formulation comprises 1) an active agent, e.g., any of the above-described polypeptides, antibodies, agonists, or antagonists; and 2) a pharmaceutically acceptable carrier. In a further embodiment, a pharmaceutical formulation further comprises at least one additional therapeutic agent.

Pharmaceutical formulations are prepared for storage by mixing an agent having the desired degree of purity with optional pharmaceutically acceptable carriers, excipients or stabilizers (Remington's Pharmaceutical Sciences 16th edition, Osol, A. Ed. [1980]), in the form of lyophilized formulations or aqueous solutions. Acceptable carriers, excipients, or stabilizers are nontoxic to recipients at the dosages and concentrations employed, and include buffers such as phosphate, citrate, and other organic acids; antioxidants including ascorbic acid and methionine; preservatives (such as octadecyldimethylbenzyl ammonium chloride; hexamethonium chloride; benzalkonium chloride, benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (*e.g.*, Zn-protein complexes); and/or non-ionic surfactants such as TWEEN™, PLURONICS™ or polyethylene glycol (PEG).

Lipofections or liposomes can also be used to deliver an agent into a cell. Where the agent is an antibody fragment, the smallest inhibitory fragment which specifically binds to the target protein is preferred. For example, based upon the variable region sequences of an antibody, peptide molecules can be designed which retain the ability to bind the target protein sequence. Such peptides can be synthesized chemically and/or produced by recombinant DNA technology (see, *e.g.,* Marasco et al., Proc. Natl. Acad. Sci. USA 90, 7889-7893 [1993]). Antibodies disclosed herein may also be formulated as immunoliposomes. Liposomes containing an antibody are prepared by methods known in the art, such as described in Epstein et al., Proc. Natl. Acad. Sci. USA, 82: 3688 (1985); Hwang et al., Proc. Natl Acad. Sci. USA, 77: 4030 (1980); and U.S. Pat. Nos. 4,485,045 and 4,544,545. Liposomes with enhanced circulation time are disclosed in U.S. Patent No. 5,013,556. Particularly useful liposomes can be generated by the reverse-phase evaporation method with a lipid composition comprising phosphatidylcholine, cholesterol, and PEG-derivatized phosphatidylethanolamine (PEG-PE). Liposomes are extruded through filters of defined pore size to yield liposomes with the desired diameter. Fab' fragments of an antibody of the present invention can be conjugated to liposomes as described in Martin et al., J. Biol. Chem., 257: 286-288 (1982) via a disulfide-interchange reaction. A chemotherapeutic agent (such as doxorubicin) is optionally contained within the liposome. See Gabizon et al., J. National Cancer Inst., 81(19): 1484 (1989).

An agent may also be entrapped in microcapsules prepared, for example, by coacervation techniques or by interfacial polymerization, for example, hydroxymethylcellulose or gelatin-microcapsules and poly-(methylmethacylate) microcapsules, respectively, in colloidal drug delivery systems (for example, liposomes, albumin microspheres, microemulsions, nano-particles and nanocapsules) or in macroemulsions. Such techniques are disclosed in Remington's Pharmaceutical Sciences 16th edition, Osol, A. Ed. (1980).

Sustained-release preparations of an agent may be prepared. Suitable examples of sustained-release preparations include semipermeable matrices of solid hydrophobic polymers containing the agent, which matrices are in the form of shaped articles, *e.g*., films, or microcapsules. Examples of sustained-release matrices include polyesters, hydrogels (for example, poly(2-hydroxyethylmethacrylate), or poly(vinylalcohol)), polylactides (U.S. Pat. No. 3,773,919), copolymers of L-glutamic acid and $\gamma$-ethyl-L-glutamate, non-degradable ethylene-vinyl acetate, degradable lactic acid-glycolic acid copolymers such as the LUPRON DEPOTTM (injectable microspheres composed of lactic acid-glycolic acid copolymer and leuprolide acetate), and poly-D-(-)-3-hydroxybutyric acid. While polymers such as ethylene-vinyl acetate and lactic acid-glycolic acid enable release of molecules for over 100 days, certain hydrogels release proteins for shorter time periods. When encapsulated antibodies remain in the body for a long time, they may denature or aggregate as a result of exposure to moisture at 37°C, resulting in a loss of biological activity and possible changes in immunogenicity. Rational strategies can be devised for stabilization depending on the mechanism involved. For example, if the aggregation mechanism is discovered to be intermolecular S-S bond formation through thio-disulfide interchange, stabilization may be achieved by modifying sulfhydryl residues, lyophilizing from acidic solutions, controlling moisture content, using appropriate additives, and developing specific polymer matrix compositions.

A pharmaceutical formulation herein may also contain more than one active compound as necessary for the particular indication being treated. For example, in one embodiment, a pharmaceutical formulation containing more than one active compound comprises 1) at least one agonist of IL-22, e.g., an antibody that binds to IL-22 and/or an antibody that binds to IL-22R; and 2) at least one antibody that binds to IL-6 or IL-23 (wherein any number of the antibodies listed in 2) may be selected in any combination). In another embodiment, a pharmaceutical formulation contains two or more active compounds having complementary activities.

## E. Methods of Treatment

The present invention further provides methods of treating a microbial disorder. In another embodiment, the present invention provides a method of treating a microbial disorder, in a subject, comprising administering to the subject an effective amount of pharmaceutical composition comprising an AMP or modulator of the AMP, wherein the AMP is selected from a group consisting of: LT, IL-6, IL-18, IL-22, IL-23, REG Iα, REG Iβ, HIP/PAP, REG III, REG IV and Reg-related sequence (RS). In one embodiment the disorder is EHEC- or EPEC-caused diarrhea, Inflammatory Bowel Disease (IBD) or, more particularly, Ulcerative Colitis (UC) and Crohn's Disease (CD).

In one embodiment, the present invention provides a method of treating an infection by a microbial pathogen (e.g., a bacteria or virus), in a subject, comprising administering to the subject an effective amount of pharmaceutical composition comprising an AMP or modulator of the AMP, wherein the AMP is selected from a group consisting of: LT, IL-6, IL-18, IL-22, IL-23, REG Iα, REG Iβ, HIP/PAP, REG III, REG IV and Reg-related sequence (RS).

In another embodiment, the present invention provides a method of modulating the activity of an AMP in cells of a subject infected with a microbial pathogen (e.g., a bacteria or virus), comprising contacting the cells with an AMP or modulator of the AMP, wherein the AMP is selected from a group consisting of: LT, IL-6, IL-18, IL-22, IL-23, REG Iα, REG Iβ, HIP/PAP, REG III (e.g., REG IIIβ or REGIIIγ), REG IV, and Reg-related sequence (RS).

In another embodiment, the present invention provides a method of treating a microbial disorder, in a subject, comprising contacting cells of the subject with a nucleic acid molecule (e.g., a DNA or RNA molecule) encoding an AMP or modulator of the AMP, wherein the AMP is selected from a group consisting of: LT, IL-6, IL-18, IL-22, IL-23, REG Iα, REG Iβ, HIP/PAP, REG III, REG IV and Reg-related sequence (RS). In one embodiment the disorder is EHEC- or EPEC-caused diarrhea, Inflammatory Bowel Disease (IBD) or, more particularly, Ulcerative Colitis (UC) or Crohn's Disease (CD).

In another embodiment, the present invention provides a method of modulating the activity of an AMP in cells of a subject infected with a microbial pathogen (e.g., a bacteria or virus), comprising contacting the cells with a nucleic acid molecule (e.g., a DNA or RNA molecule) encoding an AMP or modulator of the AMP, wherein the AMP is selected from a group consisting of: LT, IL-6, IL-18, IL-22, IL-23, REG Iα, REG Iβ, HIP/PAP, REG III (e.g., REG IIIβ or REGIIIγ), REG IV, and Reg-related sequence (RS).

Examples of a microbial pathogen include, but are not limited to, a bacteria or virus. In one embodiment, the microbial pathogen is a bacteria e.g., a gram-negative or gram-positive bacteria. In a particular embodiment, the bacteria is a gram-negative bacteria. In another embodiment, the bacteria is an attaching or effacing (A/E) bacteria and, more particularly, an enterohemorrhagic Escherichia coli (EHEC) or enteropathogenic E. Coli (EPEC). In one embodiment, the bacteria is enteropathogenic E. coli (EHEC) is E. coli 01 57:H7 or E. coli 055:H7.

The therapeutic methods of the present invention comprise one or more compositions or pharmaceutical formulations of the present invention. Such methods include in vitro, ex vivo, and in vivo therapeutic methods, unless otherwise indicated.

In various embodiments, the present invention provides methods of modulating an anti-microbial immune response by stimulating or inhibiting an AMP-mediated signaling pathway and/or $Th_{IL-17}$ cell function. Such methods are useful for treatment of microbial disorders. For example, in one embodiment, the present invention provides a method of enhancing an anti-microbial immune response by stimulating an AMP-mediated signaling pathway, e.g., and IL-22 and/or IL-23 mediated signaling pathway. In another embodiment, the present invention provides methods of modulating an anti-microbial immune response by stimulating or inhibiting a cytokine-mediated signaling pathway. For example, in one embodiment, the present invention provides methods of enhancing an anti-microbial immune response by stimulating a cytokine-mediated signaling pathway, e.g., an IL-22 and/or IL-23 signaling pathway. Moreover, the present invention provides methods of modulating an anti-microbial immune response by stimulating or inhibiting a $Th_{IL-17}$ cell function.

In one embodiment, the present invention provides a method of stimulating an AMP-mediated signaling pathway in a biological system, the method comprising providing an AMP agonist to the biological system. Examples of such a biological system include, but are not limited to, mammalian cells in an in vitro cell culture system or in an organism in vivo. In another embodiment, the present invention provides a method of inhibiting an AMP-mediated signaling pathway in a biological system, the method comprising providing an AMP antagonist to the biological system.

In a particular embodiment, the present invention provides a method of enhancing an anti-microbial immune response in a biological system by stimulating an IL-23 and/or IL-22 mediated signaling pathway in a biological system, the method comprising providing an IL-22 or IL-22 agonist to the biological system. In one embodiment, an IL-22 agonist is IL-22. In another embodiment, the IL-22 agonist is an antibody that binds to IL-22.

In another embodiment, a method of inhibiting an IL-23-mediated signaling pathway in a biological system is provided, the method comprising providing an IL-22 antagonist to the biological system. In one embodiment, the antagonist of IL-22 is an antibody, e.g., a neutralizing anti-IL-22 antibody and/or a neutralizing anti-IL-22R antibody.

In another embodiment, the present invention provides a method of stimulating a $Th_{IL-17}$ cell function, the method comprising exposing a $Th_{IL-17}$ cell to an agonist of an AMP that mediates the IL-23 mediated signaling pathway (e.g., IL-23, IL-6, or IL-22). Such methods are useful for treating a microbial disorder. In one embodiment, an IL-22 agonist is IL-22. In another embodiment, the IL-22 agonist is an antibody that binds to IL-22.

In another embodiment, a method of inhibiting a $Th_{IL-17}$ cell function is provided, the method comprising exposing a $Th_{IL-17}$ cell to an antagonist of an AMP that mediates the IL-23 mediated signaling pathway (e.g., IL-23, IL-6, or IL-22). In one embodiment the antagonist is an anti-IL-22 antibody, e.g., a neutralizing anti-IL-22 antibody.

Exemplary $Th_{IL-17}$ cell functions include, but are not limited to, stimulation of cell-mediated immunity (delayed-type hypersensitivity); recruitment of innate immune cells, such as myeloid cells (e.g., monocytes and neutrophils) to sites of inflammation; and stimulation of inflammatory cell infiltration into tissues. In one embodiment, a $Th_{IL-17}$ cell function is mediated by IL-23 and/or IL-22.

Compositions of the present invention are administered to a mammal, preferably a human, in accord with known methods, such as intravenous administration as a bolus or by continuous infusion over a period of time, by intramuscular, intraperitoneal, intracerobrospinal, subcutaneous, intra-articular, intrasynovial, intrathecal, oral, topical, or inhalation (intranasal, intrapulmonary) routes. Intravenous or inhaled administration of polypeptides and antibodies is preferred.

For the treatment or reduction in the severity of a microbial disorder, the appropriate dosage of a composition of the invention will depend on the type of disorder to be treated, as defined above, the severity and course of the disorder, whether the agent is administered for preventive or therapeutic purposes, previous therapy, the patient's clinical history and response to the compound, and the discretion of the attending physician. The compound is suitably administered to the patient at one time or over a series of treatments.

For example, depending on the type and severity of a disorder, about 1 μg/kg to 15 mg/kg (*e.g.*, 0.1-20 mg/kg) of a polypeptide or antibody is an initial candidate dosage for administration to a patient, whether, for example, by one or more separate administrations, or by continuous infusion. A typical daily dosage might range from about 1 μg/kg to 100 mg/kg or more, depending on the factors mentioned above. For repeated administrations over several days or longer, depending on the condition, the treatment is sustained until a desired suppression of disease symptoms occurs. However, other dosage regimens may be useful. The progress of this therapy is easily monitored by conventional techniques and assays.

## F. Diagnostic Methods and Methods of Detection

In one embodiment, the present invention provides a method of detecting the presence of an AMP in a biological sample, comprising contacting the biological sample with an antibody to the AMP, under conditions permissive for binding of the antibody to the AMP, and detecting whether a complex is formed between the antibody and AMP.

In one embodiment, the present invention provides a method of monitoring treatment of a microbial disorder in a subject, wherein the method comprises detecting the level of expression of a gene encoding an AMP in a test sample of tissue cells obtained from the subject in need of treatment, and the expression level in the test sample is detected. The detection may be qualitative or quantitative. In one embodiment, the test sample comprises blood or serum. In one embodiment, detecting the level of expression of a gene encoding an AMP comprises (a) contacting an anti-AMP antibody with a test sample obtained from the mammal, and (b) detecting the formation of a complex between the antibody and an AMP in the test sample. The antibody may be linked to a detectable label. Complex formation can be monitored, for example, by light microscopy, flow cytometry, fluorimetry, or other techniques known in the art. The test sample may be obtained from an individual suspected of having a microbial disorder.

In one embodiment, detecting the level of expression of a gene encoding an AMP polypeptide comprises detecting the level of mRNA transcription from the gene. Levels of mRNA transcription may be detected, either quantitatively or qualitatively, by various methods known to those skilled in the art. Levels of mRNA transcription may also be detected directly or indirectly by detecting levels of cDNA generated from the mRNA. Exemplary methods for detecting levels of mRNA transcription include, but are not limited to, real-time quantitative RT-PCR and hybridization-based assays, including microarray-based assays and filter-based assays such as Northern blots.

In another embodiment, the present invention provides a method of detecting the presence of an AMP in a biological sample, comprising contacting the biological sample with an antibody to the AMP, under conditions permissive for binding of the antibody to the AMP, and detecting whether a complex is formed between the antibody and AMP.

In another embodiment, the present invention concerns a diagnostic kit containing an anti-AMP in suitable packaging. The kit preferably contains instructions for using the antibody to detect an AMP. In one embodiment, the diagnostic kit is for diagnosing a microbial disorder. In one embodiment, the diagnostic kit is for diagnosing a microbial infection.

In another embodiment, the present invention provides a kit comprising one or more AMPs of the present invention and/or modulators thereof. In another embodiment, the present invention provides a kit comprising one or more one or more pharmaceutical compositions each comprising an AMP of the present invention or modulator thereof.

## G. Assays

### *1. Cell-Based Assays and Animal Models*

Cell-based assays and animal models for immune diseases are useful in practicing certain embodiments of the invention. Certain cell-based assays provided in the Examples below are useful, e.g., for testing the efficacy of IL-22 antagonists or agonists.

*In vivo* animal models are also useful in practicing certain embodiments of the invention. Exemplary animal models are also described in the Examples below. The *in vivo* nature of such models makes them predictive of responses in human patients. Animal models of immune related diseases include both non-recombinant and recombinant (transgenic) animals. Non-recombinant animal models include, for example, rodent, *e.g*., murine models. Such models can be generated by introducing cells into syngeneic mice using standard techniques, *e.g.*, subcutaneous injection, tail vein injection, spleen implantation, intraperitoneal implantation, implantation under the renal capsule, *etc.*

Graft-versus-host disease models provide a means of assessing T cell reactivity against MHC antigens and minor transplant antigens. Graft-versus-host disease occurs when immunocompetent cells are transplanted into immunosuppressed or tolerant patients. The donor cells recognize and respond to host antigens. The response can vary from life threatening severe inflammation to mild cases of diarrhea and weight loss. A suitable procedure for assessing graft-versus-host disease is described in detail in Current Protocols in Immunology, above, unit 4.3.

An animal model for skin allograft rejection is a means of testing the ability of T cells to mediate *in vivo* tissue destruction and a measure of their role in transplant rejection. The most common and accepted models use murine tail-skin grafts. Repeated experiments have shown that skin allograft rejection is mediated by T cells, helper T cells and killer-effector T cells, and not antibodies. Auchincloss, H. Jr. and Sachs, D. H., Fundamental Immunology, 2nd ed., W. E. Paul ed., Raven Press, NY, 1989, 889-992. A suitable procedure is described in detail in *Current Protocols in Immunology,* above, unit 4.4. Other transplant rejection models which can be used to test the compounds of the invention are the allogeneic heart transplant models described by Tanabe, M. et al, Transplantation (1994) 58:23 and Tinubu, S. A. et al, J. Immunol. (1994) 4330-4338.

Contact hypersensitivity is a simple *in vivo* assay for cell mediated immune function (delayed type hypersensitivity). In this procedure, cutaneous exposure to exogenous haptens which gives rise to a delayed type hypersensitivity reaction which is measured and quantitated. Contact sensitivity involves an initial sensitizing phase followed by an elicitation phase. The elicitation phase occurs when the T lymphocytes encounter an antigen to which they have had previous contact. Swelling and inflammation occur, making this an excellent model of human allergic contact dermatitis. A suitable procedure is described in detail in Current Protocols in Immunology, Eds. J. E. Cologan, A. M. Kruisbeek, D. H. Margulies, E. M. Shevach and W. Strober, John Wiley & Sons, Inc., 1994, unit 4.2. See also Grabbe, S. and Schwarz, T, Immun. Today 19 (1): 37-44 (1998).

Additionally, the compositions of the invention can be tested on animal models for psoriasis-like diseases. For example, compositions of the invention can be tested in the scid/scid mouse model described by Schon, M. P. et al, Nat. Med. (1997) 3:183, in which the mice demonstrate histopathologic skin lesions resembling psoriasis. Another suitable model is the human skin/scid mouse chimera prepared as described by Nickoloff, B. J. et al, Am. J. Path. (1995) 146:580. Another suitable model is described in Boyman et al., J Exp Med. (2004) 199(5):731-6, in which human prepsoriatic skin is grafted onto AGR129 mice, leading to the development of psoriatic skin lesions.

Knock out animals can be constructed which have a defective or altered gene encoding a polypeptide identified herein, as a result of homologous recombination between the endogenous gene encoding the polypeptide and a DNA molecule in which that gene has been altered. For example, cDNA encoding a particular polypeptide can be used to clone genomic DNA encoding that polypeptide in accordance with established techniques. A portion of the genomic DNA encoding a particular polypeptide can be deleted or replaced with another gene, such as a gene encoding a selectable marker which can be used to monitor integration. Typically, several kilobases of unaltered flanking DNA (both at the 5' and 3' ends) are included in the vector [see *e.g.,* Thomas and Capecchi, Cell, 51:503 (1987) for a description of homologous recombination vectors]. The vector is introduced into an embryonic stem cell line (*e.g.,* by electroporation) and cells in which the introduced DNA has homologously recombined with the endogenous DNA are selected [see *e.g.,* Li et al., Cell, 69:915 (1992)]. The selected cells are then injected into a blastocyst of an animal (*e.g.,* a mouse or rat) to form aggregation chimeras [see *e.g*., Bradley, in Teratocarcinomas and Embryonic Stem Cells: A Practical Approach, E. J. Robertson, cd. (IRL, Oxford, 1987), pp. 113-152]. A chimeric embryo can then be implanted into a suitable pseudopregnant female foster animal and the embryo brought to term to create a "knock out" animal. Progeny harboring the homologously recombined DNA in their germ cells can be identified by standard techniques and used to breed animals in which all cells of the animal contain the homologously recombined DNA. Knockout animals can be characterized for instance, for their ability to defend against certain pathological conditions and for their development of pathological conditions due to absence of the polypeptide.

### *2. Screening Assays for Drug Candidates*

Screening assays for drug candidates are designed to identify compounds that bind to or complex with a polypeptide identified herein or a biologically active fragment thereof, or otherwise interfere with the interaction of a polypeptide with other cellular proteins. Such screening assays will include assays amenable to high-throughput screening of chemical libraries, making them particularly suitable for identifying small molecule drug candidates. Small molecules contemplated include synthetic organic or inorganic compounds, including peptides, preferably soluble peptides, (poly)peptide-immunoglobulin fusions, and, in particular, antibodies including, without limitation, poly- and monoclonal antibodies and antibody fragments, single-chain antibodies, anti-idiotypic antibodies, and chimeric or humanized versions of such antibodies or fragments, as well as human antibodies and antibody fragments. The assays can be performed in a variety of formats, including protein-protein binding assays, biochemical screening assays, immunoassays and cell based assays, which are well characterized in the art. All assays are common in that they call for contacting a test compound with a polypeptide identified herein under conditions and for a time sufficient to allow the polypeptide to interact with the test compound.

In binding assays, the interaction is binding and the complex formed can be isolated or detected in the reaction mixture. In a particular embodiment, a polypeptide or the test compound is immobilized on a solid phase, *e.g.*, on a microtiter plate, by covalent or non-covalent attachments. Non-covalent attachment generally is accomplished by coating the solid surface with a solution of the polypeptide or test compound and drying. Alternatively, an immobilized antibody, *e.g.,* a monoclonal antibody specific for a polypeptide to be immobilized, can be used to anchor the polypeptide to a solid surface. The assay is performed by adding the non-immobilized component, which may be labeled by a detectable label, to the immobilized component, *e.g.*, the coated surface containing the anchored component. When the reaction is complete, the non-reacted components are removed, *e.g.*, by washing, and complexes anchored on the solid surface are detected. When the originally non-immobilized component carries a detectable label, the detection of label immobilized on the surface indicates that complexing occurred. Where the originally non-immobilized component does not carry a label, complexing can be detected, for example, by using a labelled antibody specifically binding the immobilized complex.

If the test compound interacts with but does not bind to a particular polypeptide identified herein, its interaction with that protein can be assayed by methods well known for detecting protein-protein interactions. Such assays include traditional approaches, such as, cross-linking, co-immunoprecipitation, and co-purification through gradients or chromatographic columns. In addition, protein-protein interactions can be monitored by using a yeast-based genetic system described by Fields and co-workers [Fields and Song, Nature (London) 340, 245-246 (1989); Chien et al., Proc. Natl. Acad. Sci. USA 88, 9578-9582 (1991)] as disclosed by Chevray and Nathans, Proc. Natl. Acad. Sci. USA 89, 5789-5793 (1991). Many transcriptional activators, such as yeast GAL4, consist of two physically discrete modular domains, one acting as the DNA-binding domain, while the other one functioning as the transcription activation domain. The yeast expression system described in the foregoing publications (generally referred to as the "two-hybrid system") takes advantage of this property, and employs two hybrid proteins, one in which the target protein is fused to the DNA-binding domain of GAL4, and another, in which candidate activating proteins are fused to the activation domain. The expression of a GAL1-*lac*Z reporter gene under control of a GAL4-activated promoter depends on reconstitution of GAL4 activity via protein-protein interaction. Colonies containing interacting polypeptides are detected with a chromogenic substrate for β-galactosidase. A complete kit (MATCHMAKER™) for identifying protein-protein interactions between two specific proteins using the two-hybrid technique is commercially available from Clontech. This system can also be extended to map protein domains involved in specific protein interactions as well as to pinpoint amino acid residues that are crucial for these interactions.

To identify compounds that interfere with the interaction of a polypeptide identified herein and other intra- or extracellular component(s), a reaction mixture may be prepared containing the polypeptide and the component under conditions allowing for the interaction of the polypeptide with the component. To test the ability of a test compound to inhibit the interaction, the reaction mixture is prepared in the absence and in the presence of the test compound. If there is a decrease in the interaction of the polypeptide with the component in the presence of the test compound, then the test compound is said to inhibit the interaction of the polypeptide with the component.

In certain embodiments, methods for identifying agonists or antagonists of an AMP comprise contacting an AMP with a candidate agonist or antagonist molecule and measuring a detectable change in one or more biological activities normally associated with the AMP. Such activities include, but are not limited to, those described in the Examples below.

In one embodiment, the present invention provides methods for identifying agonists of an IL-22 polypeptide comprise contacting an IL-22 polypeptide with a candidate agonist molecule and measuring a detectable change in one or more biological activities normally associated with the IL-22 polypeptide. Such activities include, but are not limited to, those described in the Examples below.

***3.Antibody binding assays***

Antibody binding studies may be carried out in any known assay method, such as competitive binding assays, direct and indirect sandwich assays, and immunoprecipitation assays. Zola, Monoclonal Antibodies: A Manual of Techniques, pp.147-158 (CRC Press, Inc., 1987).

Competitive binding assays rely on the ability of a labeled standard to compete with the test sample analyte for binding with a limited amount of antibody. The amount of target protein in the test sample is inversely proportional to the amount of standard that becomes bound to the antibodies. To facilitate determining the amount of standard that becomes bound, the antibodies preferably are insolubilized before or after the competition, so that the standard and analyte that are bound to the antibodies may conveniently be separated from the standard and analyte which remain unbound.

Sandwich assays involve the use of two antibodies, each capable of binding to a different immunogenic portion, or epitope, of the protein to be detected. In a sandwich assay, the test sample analyte is bound by a first antibody which is immobilized on a solid support, and thereafter a second antibody binds to the analyte, thus forming an insoluble three-part complex. See, *e.g.,* US Pat No. 4,376,110. The second antibody may itself be labeled with a detectable moiety (direct sandwich assays) or may be measured using an anti-immunoglobulin antibody that is labeled with a detectable moiety (indirect sandwich assay). For example, one type of sandwich assay is an ELISA assay, in which case the detectable moiety is an enzyme.

Immunhistochemistry may also be used to determine the cellular location of an antigen to which an antibody binds. For immunohistochemistry, the tissue sample may be fresh or frozen or may be embedded in paraffin and fixed with a preservative such as formalin, for example. Articles of Manufacture

In another embodiment, the present invention provides an article of manufacture comprising compositions useful for the diagnosis or treatment of the microbial disorders described herein. The article of manufacture comprises a container and an instruction. Suitable containers include, for example, bottles, vials, syringes, and test tubes. The containers may be formed from a variety of materials such as glass or plastic. The container holds a composition which is effective for diagnosing or treating the condition and may have a sterile access port (for example the container may be an intravenous solution bag or a vial having a stopper pierceable by a hypodermic injection needle). The active agent in the composition is usually a polypeptide, an antibody, an agonist, or an antagonist of the invention. An instruction or label on, or associated with, the container indicates that the composition is used for diagnosing or treating the condition of choice. The article of manufacture may further comprise a second container comprising a pharmaceutically-acceptable buffer, such as phosphate-buffered saline, Ringer's solution and dextrose solution. It may further include other materials desirable from a commercial and user standpoint, including other buffers, diluents, filters, needles, syringes, and package inserts with instructions for use.

In one embodiment, the invention provides an article of manufacture, comprising:

(a) a composition of matter comprising an AMP or modulator thereof (e.g., an IL-22 agonist);
(b) a container containing said composition; and
(c) a label affixed to said container, or a package insert included in said container, referring to the use of said agonist in the treatment of an microbial disorder. The composition may comprise an effective amount of the agonist.

**EXAMPLES**

The following are examples of methods and compositions of the invention, and are provided herein for illustrative purposes, and are not intended to limit the scope of the present invention. It is understood that various other embodiments may be practiced, given the general description provided herein.

Commercially available reagents referred to in the examples were used according to manufacturer's instructions unless otherwise indicated. The source of those cells identified in the following examples, and throughout the specification, by ATCC accession numbers is the American Type Culture Collection, Manassas, VA.

The data presented herein demonstrate for the first time that IL-22 is one of the key cytokines that bridges adaptive immune response and innate epithelial defense during early infection of an A/E bacterial pathogen. As shown herein, the induction of RegIIIβ and RegIIIγ also indicates that IL-22 may have broader functions in controlling various bacterial infections. The data further supports the role of Th17 cells and their effector cytokines in infectious diseases and autoimmune diseases. Finally, the present studies indicate that IL-22 and its downstream products, such as RegIIIβ and RegIIIγ, may be beneficial for the treatment of certain infectious diseases.

**EXAMPLE 1: IL-23 is essential for IL-22 regulation during an infectious disease process.**

The data herein demonstrate that IL-23 is essential for IL-22 regulation during an infectious disease process.

Both IL-22 receptor pairs, IL-22R and IL-10Rβ chains, are expressed in the GI tract of wildtype C57Bl/6 mice (Fig. 1A). Their expression in the duodenum, jejunum, ileum, and colon are higher than they are in the skin, a tissue where IL-22 has been shown to induce hyperplasia. Consistently both colonic epithelial cells and subepithelial myofibroblasts have been reported to respond to IL-22. During *C. rodentium* infection, IL-22 was induced in the colon of wildtype mice (Fig. 1B), as were cytokines that promote Th17 cell differentiation, including the p19 and p40 subunit of IL-23 (Fig. 1C-D), and IL-6 (Fig. 1E). All of these cytokines were rapidly induced, with peak expression around day 4 post inoculation. In contrast, IL-17 induction had slower kinetics and reached its maximum level at day l2 post inoculation (Fig. IF).

Since either IL-23 or IL-6 promotes IL-22 production from T cells *in vitro,* the present inventors sought to first define their role in regulation of IL-22 production during *C. rodentium* infection. When comparing the survival rate of wildtype, p19$^{-/-}$, p40$^{-/-}$, and IL-6$^{-/-}$ mice after *C. rodentium* infection, we consistently found all the mice from either the p40$^{-/-}$ group (Fig. 1G) or the p19$^{-/-}$ group (data not shown) died 10 days post inoculation. Interestingly, 60% mortality was also observed in the IL-6$^{-/-}$ group around day 12 (Fig. 1G), indicating that IL-6 is required, to a certain extent, for a total control of *C. rodentium* infection. Next we examined IL-22 and IL-17 expression in both p19$^{-/-}$ and IL-6$^{-/-}$ mice (Fig. 1H). While, IL-17 expression was not altered in p19$^{-/-}$ mice (*15*), induction of IL-22 was diminished in p19$^{-/-}$ mice compared to wildtype mice. In IL-6$^{-/-}$ mice, however, while the peak level of IL-22 was comparable to that of wildtype mice, its induction was significantly delayed (Fig 1H). Furthermore, in IL-6$^{-/-}$ mice, the induction of IL-17 was significantly reduced, consistent with an essential role of IL-6 for IL-17 production.

Directly measuring IL-22/IL-17 proteins by ELISA in *ex vivo* culture of colons from infected mice confirmed the kinetics of IL-22/IL-17 production and the absence of IL-22 induction from p19$^{-/-}$ mice during *C. rodentium* infection (Fig. 11). These data for the first time demonstrate that IL-23 is essential for IL-22 regulation during an infectious disease process.

These data for the first time demonstrate that IL-23 is essential for IL-22 regulation during an infectious disease process.

**Example 2: IL-22 is a key downstream effector cytokine that contributes to the biology of IL-23 in controlling microbial infection.**

The altered regulation of IL-22 in both IL-23 deficient and IL-6$^{-/-}$ mice indicated that IL-22 may play a critical role in the host defense against *C. rodentium* infection. To further examine the role of IL-22, IL-22$^{-/-}$ mice were inoculated with *C. rodentium.* While wildtype littermates transiently lost weight but were able to fully recover after day 6, IL-22$^{-/-}$ mice continued losing weight following *C. rodentium* infection (Fig. 2A). About 80% of IL-22$^{-/-}$ mice became moribund or died 12 days post *C. rodentium* inoculation (Fig. 2A). Histologic analysis of the colons from day 8 infected IL-22$^{-/-}$ mice demonstrated increased mucosal thickness when compared with that of WT mice (Fig. 2B). Coincidently, there was also increased submucosal inflammation (Arrow, Fig. 2B). Furthermore, while in control mice, *C. rodentium* infection was predominantly superficial, large numbers of bacteria penetrated deeply into colonic crypts in IL-22$^{-/-}$ mice (Arrows, Fig. 2C). FACS analysis with an anti-IL-22R antibody (Fig. 12) revealed that IL-22R was expressed by E-cadherin positive primary murine colonic epithelial cells, but not by CD45$^{+}$ intra-epithelial lymphocytes (IEL) or lamina propria mononuclear cells (LPMCs) (Fig. 12A). Similarly, primary human colonic epithelial cells also expressed IL-22R (Fig. 12B). These data suggest that colonic epithelial cells were directly targeted by IL-22.

These data support the importance of IL-22 in host defense against *C. rodentium* infection, and indicate that IL-22 may be one of the key downstream effector cytokines that contribute to the biology of IL-23 in controlling microbial infections.

**Example 3: IL-17A and IL-17F pathways are not required for host defense against *C. rodentium* infection.**

The partial impairment of host defense in IL-6$^{-/-}$ mice against *C. rodentium* could also be explained by the delayed induction of IL-22 in these mice (Fig. 1H, left panel). However, it is also possible that lethality in *C. rodentium* infected IL-6$^{-/-}$ mice may have been due to their inability to upregulate IL-17 (Fig. 1H, right panel). The IL-17 pathway is crucial for the control of many extracellular bacterial infections, such as *Klebsiella pneumoniae.* IL-17 signals through IL-17R and IL-17RC (D. Toy et al., J Immunol 177, 36 (July 1, 2006)), and induces proinflammatory responses from many cell types, including epithelial cells (J. Witowski, K. Ksiazek, A. Jorres, Cell Mol Life Sci 61, 567 (Mar, 2004)). To analyze the role of the IL-17 pathway during *C. rodentium* infection, IL-17RC$^{-/-}$ mice were generated (Fig. 5). Compared to wildtype littermates, there was no obvious defect in IL-17RC$^{-/-}$ mice in terms of development or composition of T cells, B cells and other immune cells (data not shown). However, fibroblasts generated from the tail tip (Fig. 5C) or lung tissue (data not shown) of IL-17RC$^{-/-}$ mice were completely incapable of producing IL-6 when stimulated with either IL-17A or IL-17F, indicating that IL-17RC is an essential receptor for both IL-17A and IL-17F mediated functions. Following *C. rodentium* inoculation, both IL-17RC$^{-/-}$ mice and wildtype littermates survived the course of infection without any significant loss of weight (Fig. 2D) or any histologic differences in the colon (data not shown).

These results indicate that IL-17A and IL-17F pathways were not required for host defense against *C. rodentium* infection, directly excluding the possibility that defective IL-17 production is the major cause of observed mortality in IL-6$^{-/-}$ mice. Thus, the delayed induction of IL-22 observed in IL-6$^{-/-}$ mice might be the reason that these mice were incapable of surviving the infection. Other factors downstream of IL-6, however, may also be important. The results from IL-6$^{-/-}$ mice imply that the early induction of IL-22 might be critical for the host to mount a sufficient response against *C. rodentium* infection in order to prevent lethality.

**Example 3: IL-22 plays a critical role in the early stage of bacterial infection.**

To determine whether early induction of IL-22 is critical for the host to mount a sufficient response against *C. rodentium* infection in order to prevent lethality, anti-IL-22 neutralizing antibody was administrated every other day starting either at day 0 or at day 8 post inoculation of *C. rodentium.* As expected, mice that received anti-IL-22 mAb at the same time as the bacterial inoculation continued to lose weight, and all became moribund or died 12 days post inoculation. In contrast, all isotype control antibody treated animals survived (Fig. 2E). Mice that received anti-IL-22 mAb starting 8 days post inoculation had a similar outcome as did isotype mAb treated mice, with full recovery from infection.

Therefore, these data indicate that IL-22 plays a critical role in the early stage of *C. rodentium* infection, but plays no role during the later phase of host defense when bacteria are being eradicated.

**Example 4: IL-19, IL-20, and IL-24 are dispensable for host defense against bacterial infection.**

Other IL-10 family cytokines, IL-19, IL-20, and IL-24, all induce similar biological functions as those induced by IL-22 in human epidermal keratinocytes (S. M. Sa et al., J Immunol 178, 2229 (February 15, 2007).). IL-19, IL-20, and IL-24 were all upregulated in wildtype mouse colon during *C. rodentium* infection (Fig. 6). They may, therefore, play similar role as does IL-22 during C. *rodentium* infection. IL-19 signals through IL-20R$\alpha$ and 1L-20R$\beta$ chains. IL-20 and IL-24 can signal through two different receptor pairs, IL-20R$\alpha$/IL-20R$\beta$ and IL-22R/IL-20R$\beta$ (J.-C. Renauld, Nature Reviews Immunology 3, 667 (2003)). Therefore, 1L-20R$\beta$ is the common receptor chain for these three cytokines. In the G1 tract, expression of IL-20R$\alpha$ and IL-20R$\beta$ chains was significantly lower than the expression of these chains in skin (Fig. 7).

To critically address the role of these three cytokines during *C. rodentium* infection, IL-20R$\beta^{-/-}$ mice were generated (Fig. 8). These mice exhibited normal development with similar lymphocyte composition and development in all major lymphoid organs when compared to wildtype mice (data not shown). The ear skin from these mice failed to upregulate S100 family proteins when treated with recombinant IL-20, indicating a defect in IL-20 signaling *in vivo* (Fig. 8C).

IL-20R$\beta^{-/-}$ mice survived *C. rodentium* infection as well as wildtype mice did (Fig. 2F), demonstrating that IL-19, IL-20, and IL-24 are dispensable for host defense against *C. rodentium.*

**Example 5: IL-22 deficiency may compromise epithelial integrity during the early stage of *C. rodentium* infection.**

The present inventors examined the downstream mechanisms of IL-22 during *C. rodentium* infection. Both IL-22$^{-/-}$ mice and wildtype mice treated with anti-IL-22 mAb on day 0 developed more severe bloody diarrhea and an increased incidence of rectal prolapse compared to control mice 8 days post inoculation of *C. rodentium* (data not shown). Colons from IL-22$^{-/-}$ mice (data not shown) or day 0 anti-IL-22 mAb treated mice were thickened and shortened 10 days post inoculation (Fig. 3A), as well as having a smaller cecum, compared to control mice. Histologic analysis further revealed increased inflammation in colons lacking IL-22 signaling (Fig. 3B). There were also marked multifocal mucosal ulceration and multiple foci of transmural inflammation in both IL-22$^{-/-}$ and anti-IL-22 mAb treated mice (Fig. 3C, and Fig. 9). Furthermore, the bacterial burdens in mesenteric lymph node, spleen, and liver of IL-22$^{-/-}$ mice were significantly increased compared to those of wildtype mice. Interestingly, the difference in bacterial burdens in colons of wildtype mice and IL-22$^{-/-}$ mice was negligible (Fig. 3D). Consistent with these results, there was also evidence of systemic bacterial spread, particularly in the livers of IL-22$^{-/-}$ mice, where multifocal hepatocellular necrosis with embolic microabscessation was evident (Fig. 3E).

In conclusion, these data indicate that the epithelial integrity is compromised in IL-22$^{-/-}$ mice during the early stage of *C. rodentium* infection.

**Example 6: IL-22 deficiency leads to a reduction in anti-bacterial IgG titers**

Previous studies established the essential role of anti-*C. rodentium* antibodies in the clearance of bacteria. Transferring serum from wildtype mice post-infection fully rescued CD4$^{-/-}$ mice from death following *C. rodentium* challenge (*10*). In our studies, IL-22 deficient mice became moribund or died starting around day 8, when antibody responses were not fully developed in wildtype mice (Fig. 3F). On day 8, anti-*C. rodentium* antibody titers were 50 fold less than those on day 16 in wildtype mice. However, when we compared the titers of anti-*C. rodentium* antibodies on day 8 from wildtype and IL-22$^{-/-}$ mice, there was an unexpected significant reduction in the anti-*C. nodentium* 1gG titer in IL-22$^{-/-}$ mice compared to that in wildtype mice (Fig. 3G). In contrast, there was no decrease in total IgG, IgM, IgA or anti-*C. rodentium* IgM and IgA titers in IL-22$^{-/-}$ mice (Fig. 10A and data not shown). Further IgG subtype analysis revealed that while there was no anti-*C. rodentium* IgG1 in either wildtype or IL-22$^{-/-}$ mice 8 days post inoculation, other anti-*C. rodentium* IgG subtypes, including IgG2a, IgG2b, IgG2c and IgG3, were all significantly reduced in IL-22$^{-/-}$ mice (Fig. 10B). It was unlikely that differences in anti-bacterial specific IgG contributed to clearance of *C. rodentium* from the colon at this time point, especially since IgG is not targeted to the colonic mucosal lumen, and colonic bacterial burdens in both wildtype and IL-22$^{-/-}$ mice were similar (Fig. 3D). It is possible, though that circulating anti-*C. rodentium* IgG may be important in controlling penetration of *C. rodentium* through the intestinal epithelial barrier, and preventing systemic spread, since a recent study demonstrated that circulating IgG, but not secretory IgA or IgM, was required for systemic clearance of *C. rodentium* (C. Maaser et al., Infect. Immun. 72, 3315 (June 1, 2004)). How IL-22 deficiency leads to a reduction in anti-bacterial IgG titers is unclear. It is unlikely that IL-22 directly acts on B cells, since the expression of IL-22R is not detectable on B cells (S. Lecart et al., Int. Immunol. 14, 1351 (November 1, 2002)). Nonetheless, reduced anti-*C. rodentium* IgG might be one of the factors that contribute to the defective host defense response in IL-22$^{-/-}$ mice during *C. rodentium* infection.

**Example 7: IL-22 was indispensable for the induction of anti-microbial lectins, such as RegIIIβ and RegIIIγ, from colonic epithelial cells during bacterial infection.**

IL-22 treatment of colon tissues from uninfected wildtype mice *ex vivo* upregulated many anti-microbial proteins, including S100A8, S100A9, RegIIIβ, RegIIIγ, haptoglobin, SAA3, and lactotransferrin by microarray analysis (Fig.s 4A, 19, and 20). The induction of these proteins was confirmed by real-time RT-PCR (Fig. 4B and data not shown). During *C. rodentium* infection, however, only S100A8, S100A9, RegIIIβ and RegIIIγ were differentially expressed in IL-22$^{-/-}$ mice compared to wildtype mice (Fig. 4C). All other genes were either not induced or were not different in colons of wildtype vs. IL-22$^{-/-}$ mice (data not shown). Expression of both S100A8 and S100A9 was slightly higher in the colons of IL-22$^{-/-}$ mice than it was in wildtype colon on day 4 and day 6, suggesting that differential expression of these proteins was most likely not responsible for the increased mortality observed in IL-22$^{-/-}$ mice during *C. rodentium* infection. Differences were not found in the expression of defensins, proteins that are important in host defense of infected epithelium (T. Ganz, Science 286, 420 (October 15, 1999)), between wildtype and IL-22$^{-/-}$ mice (data not shown). Interestingly, the upregulation of RegIIIβ and RegIIIγ observed in wild type mice was completely abolished in IL-22$^{-/-}$ mice post *C. rodentium* inoculation (Fig. 4C), indicating that these two proteins had potential functions in controlling C. *rodentium* infection. Both RegIIIβ and RegIIIγ belong to a family of secreted C-type lectin proteins (H. L. Cash, C. V. Whitham, L. V. Hooper, Protein Expression and Purification 48, 151 (2006)). RegIIIβ and RegIIIγ expression levels increase dramatically in response to bacterial colonization as well as following other inflammatory stimuli in mice (S. A. Keilbaugh et al., Gut 54, 623 (May 1, 2005) H. Ogawa et al., Inflammatory Bowel Diseases 9, 162 (2003) H. Ogawa, K. Fukushima, I. Sasaki, S. Matsuno, Am J Physiol Gastrointest Liver Physiol 279, G492 (Sep, 2000)).

RegIIIβ or RegIIIγ may prevent the invasion of *C. rodentium* deep into the colonic crypts, as we saw no differences in bacterial burdens from the colons of IL-22$^{-/-}$ vs. wildtype mice (Fig. 3D). Alternatively, RegIIIβ or RegIIIγ proteins may act as autocrine growth factors that play a role in epithelial repair and/or protection in the setting of intestinal inflammation (H. Ogawa et al., Inflammatory Bowel Diseases 9, 162 (2003); S. L. Pull, J. M. Doherty, J. C. Mills, J. I. Gordon, T. S. Stappenbeck, PNAS 102, 99 (January 4, 2005); V. Moucadel et al., Eur J Cell Biol 80, 156 (Feb, 2001)).

**Example 8: Adaptive immunity is not essential for IL-22 mediated early host defense against *C. rodentium* infection**

The above data suggested roles of IL-22 in both innate immunity and adaptive immunity. Therefore, we used recombination activating gene 2 deficient (Rag2$^{-/-}$) mice to critically examine the function of IL-22 in innate vs. adaptive immunity during *C. rodentium* infection. Rag2$^{-/-}$ mice gradually lost weight and eventually became moribund or died around day 30, due to their lack of B and T cells, and their consequent inability to mount anti-*C. rodentium* antibody responses (Fig. 13A). In contrast to p19$^{-/-}$ or IL-22$^{-/-}$ mice, none of the Rag2$^{-/-}$ mice lost more than 10% of their body weight or died during the first two weeks of infection. Furthermore, Rag2$^{-/-}$ mice treated with anti-IL-22 mAb lost weight very rapidly (Fig. 13A), similar to WT mice treated with anti-IL-22 mAb (Fig. 2E). All Rag2$^{-/-}$ mice treated with anti-IL-22 mAb became moribund or died around day 10 (Fig. 13). These data suggest that the IL-22 pathway is still active in Rag2$^{-/-}$ mice, and that IL-22 is essential to protect mice from death during the early phase of *C. rodentium* infection in the absence of adaptive immunity. These data also indicate that reduction in anti-*C. rodentium* IgG titers was insufficient cause for the morbidity and mortality observed in IL-22$^{-/-}$ mice following *C. rodentium* infection, as lack of antibody production in Rag2$^{-/-}$ mice alone did not cause rapid weight loss and early death following infection.

IL-22 production in Rag2$^{-/-}$ mice was comparable with that of WT mice following *C. rodentium* infection (Fig. 13B). In contrast, induction of IL-17A was significantly reduced in Rag2$^{-/-}$ mice (Fig. 13B and C). T cells and B cells, therefore, were not the sources of IL-22 in this model. Immunohistochemical staining with an anti-IL-22 mAb (Fig. 15) detected IL-22 positive cells in the colon of WT mice infected with *C. rodentium,* but not in uninfected colon or colon from infected IL-22$^{-/-}$ mice. IL-22 positive cells primarily co-localized with CD11c$^{+}$ cell clusters in the colon of Rag2$^{-/-}$ mice (Fig. 13D), but not with F4/80, Gr-1, or DX5 positive cells (data not shown). In addition, IL-23 induced IL-22 production directly from CD11c$^{+}$ DCs *in vitro* (Fig. 13E). Taken together, our data demonstrate that DCs are one of the major sources of IL-22 production during *C. rodentium* infection, and that IL-23 can directly promote IL-22 production from DCs.

**Example 9: RegIII plays an important role during bacterial infection.**

Interestingly, the upregulation of RegIIIβ and RegIIIγ observed in wild type mice was completely abolished in IL-22$^{-/-}$ mice (Fig. 4C), as well as in p19$^{-/-}$ mice, (Fig. 16) post *C. rodentium* inoculation. RegIIIβ and RegIIIγ belong to a family of secreted C-type lectin proteins. We found that other family members, including RegI, RegII, RegIIIα, and RegIIIδ (Fig. 17), but not RegIV (data not shown), were also upregulated in *C. rodentium* infected colons, and that their induction was completely abolished in IL-22$^{-/-}$ mice. Exogenous mouse RegIIIγ fusion protein (rmRegIIIγ) significantly protected IL-22$^{-/-}$ mice from the weight loss induced by the *C. rodentium* infection, and approximately 50% of rmRegIIIγ fusion protein treated animals survived the infection, whereas 100% of control treated IL-22$^{-/-}$ mice became moribund or died (Fig. 14A). These data support the hypothesis that Reg family proteins, such as RegIIIγ, mediate essential functions in controlling *C. rodentium* infection downstream of IL-22.

Finally, the presence of the IL-23/IL-22/Reg axis was also validated in a human system. Human IL-23 induces hIL-22 production from human DCs (Fig. 14B). Primary human colonic epithelial cells (Fig. 12B) and the human colonic epithelial cell lines, HT29 and HCT15, express IL-22R (Fig. 14C). *In vitro,* primary human colonic epithelial cells grew slowly, and gradually lost their expression of IL-22R during expansion (data not shown). Therefore, we used colonic epithelial cell lines to test their response to human IL-22. IL-22 induced STAT3 activation in these colonic epithelial cell lines (Fig. 14D), and both RegIIIβ and RegIIIγ were significantly induced by IL-22 (Fig. 14E). Importantly, human RegIIIγ fusion protein (rhRegIIIγ), like rmRegIIIγ. fusion protein, also reduced the mortality of IL-22$^{-/-}$ mice, to 40% following *C. rodentium* infection, versus 100% mortality in control treated IL-22$^{-/-}$ mice (Fig. 18). In conclusion, our data imply that the IL-22 pathway may play an essential role in controlling bacterial infections, particularly A/E bacterial infections, in the human GI tract.

**Summary**

The present inventors demonstrate herein that IL-22 plays an indispensable role in early host defense against attaching and effacing (A/E) bacterial pathogens.

The data herein indicate that IL-22 protects the integrity of the intestinal epithelial barrier and prevents bacterial invasion with systemic spread through two mechanisms. First, IL-22 is involved in the elicitation of the early anti-bacterial IgG responses. Second, IL-22 is indispensable for the induction of anti-microbial lectins, such as RegIIIβ and RegIIIγ, from colonic epithelial cells during bacterial infection. The lack of either or both of these mechanisms may contribute to the compromised host defense response with increased systemic spread and mortality in IL-22 mice during *C. rodentium* infection.

While adaptive immune responses are essential for clearance of these pathogens (L. Bry, M. B. Brenner, J Immunol 172, 433 (January 1, 2004)), cytokines such as IL-22 that are produced by immune cells during the early stages of infection are also necessary for intestinal epithelial cells to elicit a full anti-microbial response and wound healing response in order to prevent systemic invasion of pathogenic bacteria into the host. As shown herein, the induction of RegIIIβ and RegIIIγ also indicates that IL-22 may have broader functions in controlling various bacterial infections. The data further supports the role of Th17 cells and their effector cytokines in infectious diseases and autoimmune diseases. Finally, the present studies indicate that IL-22 and its downstream products, such as RegIIIβ and RegIIIγ, may be beneficial for the treatment of certain infectious diseases.

**Materials and Methods**

*Mice*

C57B1/6, IL-12p40$^{-/-}$, and IL-6$^{-/-}$ mice were purchased from the Jackson Laboratory. IL-22$^{-/-}$ mice and IL-12p19$^{-/-}$ were generated as described before (*11, Fig.5*). IL-17RC$^{-/-}$ and IL-20Rβ$^{-/-}$ mice were generated by Lexicon Pharmaceuticals (The Woodlands, TX) by using strategies as described (Fig.5 and Fig.8). Briefly, knockout mice were made by standard homologous recombination using depicted targeting vectors. Targeting vectors are electroporated into 129 strain ES cells and targeted clones are identified. Targeted clones are microinjected into host blastocysts to produce chimeras. Chimeras are bred with C57B1/6 animals to produce F1 heterozygotes. Heterozygotes are intercrossed to produce F2 wild type, heterozygote and homozygote cohorts. Mice used in these studies were genotyped by tail DNA via PCR using a pool of three primers. The primers used for wild-type allele amplification of IL-20Rβ$^{-/-}$ mice were 5'-GTG GAA GCT ACT TGA TGA GTA GGG-3' (p1) and 5'-AGA TGC GAA AAT GGA GAT TAA AAG-3' (p2), which yielded a 595 bp product. The primers used for mutant allele amplification of IL-20Rβ$^{-/-}$ mice were 5'-CTA CCC GTG ATA TTG CTG AAG AG-3' (p3) and p2, which yielded a 351 bp product. The primers used for wild-type allele amplification of IL-17RC$^{-/-}$ mice were 5'-GAG CCT GAA GAA GCT GGA AA-3' (P3) and 5'-CAA GTG TTG GCA GAG ATG GA-3' (P2), which yielded a 534 bp product. The primers used for mutant allele amplification of IL-17RC$^{-/-}$ mice were 5'-TCG CCT TCT TGA CGA GTT CT-3' (P1) and P2, which yielded a 404 bp product.

***Bacteria strain and infection of mice***

6-8 weeks old mice were fasted for 8h before oral inoculation with 2x10$^9$ *C. rodentium* strain DBS100 (ATCC 51459; American Type Culture Collection) in a total volume of 200 μl per mouse. While fasting, animals had access to water. Inoculation and all subsequent manipulations were conducted in BL-2 biosafety cabinets. Animals were allowed access to food after inoculation. Bacteria were prepared by incubation with shaking at 37°C overnight in LB broth. The relative concentration of bacteria was assessed by measuring absorbance at OD600 and each inoculation culture was serially diluted and plated to confirm CFU administered.

**Tissue collection, histology and CFU counts**

Control or infected mice were inoculated as described. Samples of whole blood, spleen, liver, mesenteric lymph node, and colon were removed under aseptic conditions. The colon was dissected to the anal canal, and the terminal 0.5-cm piece was used for CFU analysis. Proximal segments were fixed in 10% neutral buffered formalin. Sections were stained with H&E to evaluate tissue pathology. Spleen, liver, mesenteric lymph node, and colon were weighed and homogenized. Homogenates were serially diluted and plated in triplicates to MacConkey agar (Remel). *C. rodentium* colonies were identified as pink colonies. Colonies were counted after 24 h of incubation at 37°C to determine the $\log_{10}$ CFU per gram of tissues.

**RNA isolation and real-time RT-PCR**

Cell and tissue RNA were isolated by RNeasy Mini Kit (Qiagen) according to the manufacture's directions. Real-time RT-PCR was conducted on an ABI 7500 Real-Time PCR system (Applied Biosystems) with primers and probes using TaqMan one-step RT-PCR master mix reagents (Applied Biosystems). The sequences for primers and probes were as follows: mIL-22, forward, 5'-TCC GAG GAG TCA GTG CTA AA-3', reverse, 5'-AGA ACG TCT TCC AGG GTG AA-3', and probe, 5'-TGA GCA CCT GCT TCA TCA GGT AGC A-3' (FAM, TAMRA); mIL-17A, forward, 5'-GCT CCA GAA GGC CCT CAG A-3', reverse, 5'-CTT TCC CTC CGC ATT GAC A-3', and probe, 5'- ACC TCA ACC GTT CCA CGT CAC-3' (FAM, TAMRA); mouse ribosomal housekeeping gene RPL-19, forward, 5'-GCA TCC TCA TGG AGC ACA T-3', reverse, 5'-CTG GTC AGC CAG GAG CTT-3', and probe, 5'-CTT GCG GGC CTT GTC TGC CTT-3' (FAM, TAMRA); mIL-19, forward, 5'-AGC CTG GAT TGA CAG GAA TC-3', reverse, 5'-GAT AAT CAG ACG AGG CGT TTC-3', and probe, 5'-TCT GGA AAC TCC TGC AGC CTG ACA C-3' (FAM, TAMRA); mIL-20, forward, 5'-TTT GGG AGA ACT AGG CAT TCT T-3', reverse, 5'-TCT TGG ACA GGA GTG TTC TCA-3', and probe, 5'-CAG CCT CTC CAC TTT CAT CTA TAG CAT CTC C-3' (FAM,TAMRA); mIL-24, forward, 5'-GCT CTC CAT GCC ATT TCA A-3', reverse, 5'-TGG CCA AGG GTC TGA AGT-3', and probe, 5'-TGT ACA TCC CTG CTG TCC TCA AGG C-3' (FAM, TAMRA); mIL-6, forward, 5'-TCC AAT GCT CTC CTA ACA GAT AAG-3', reverse, 5'-CAA GAT GAA TTG GAT GGT CTT G-3', and probe, 5'-TCC TTA GCC ACT CCT TCT GTG ACT CCA-3' (FAM, TAMRA); mS100A8, forward, 5'-TGT CCT CAG TTT GTG CAG AAT ATA AA-3', reverse, 5'-TCA CCA TCG CAA GGA ACT CC-3', and probe 5'-CGA AAA CTT GTT CAG AGA ATT GGA CAT CAA TAG TGA-3' (FAM, TAMRA); mS100A9, forward, 5'-GGT GGA AGC ACA GTT GGC A-3', reverse, 5'-GTG TCC AGG TCC TCC ATG ATG-3', and probe, 5'-TGA AGA AAG AGA AGA GAA ATG AAG CCC TCA TAA ATG-3' (FAM, TAMRA); mRegIIIγ, forward, 5'-ATG GCT CCT ATT GCT ATG CC-3', reverse, 5'-GAT GTC CTG AGG GCC TCT T-3', and probe, 5'-TGG CAG GCC ATA TCT GCA TCA TAC C-3' (FAM, TAMRA); mPAP/HIP/RegIIIβ, forward, 5'- ATG GCT CCT ACT GCT ATG CC-3', reverse, 5'- GTG TCC TCC AGG CCT CTT T-3', and probe, 5'-TGA TGC AGA ACT GGC CTG CCA-3' (FAM, TAMRA); mIL-12p40, forward, 5'-ACA TCT ACC GAA GTC CAA TGC A-3', reverse, 5'-GGA ATT GTA ATA GCG ATC CTG AGC-3', and probe, 5'-TGC ACG CAG ACA TTC CCG CCT-3' (FAM, TAMRA); mIL-23p19, forward, 5'-GGT GGC TCA GGG AAA TGT-3', reverse, 5'-GAC AGA GCA GGC AGG TAC AG-3', and probe, 5'-CAG ATG CAC AGT ACT CCA GAC AGC AGC-3' (FAM, TAMRA); mIL-20Rβ, forward, 5'-CAG GTG CTT CCA GTC CGT CT-3', reverse, 5'-CTC TCC TGG AAT CCC CAA AGT-3', and probe, 5'-CAG CAC AGA TGC CAA CGG CCT CAT-3' (FAM, TAMRA); mIL-20Rα, forward, 5'-CTG GCC GCT TCG GGA CGC-3', reverse, 5'-AAC CAC AGA AGA CAC AAG GAA CTG-3', and probe, 5'-TCT GCT GCT GGC CGC TTC GG-3' (FAM, TAMRA); mIL-22R, forward, 5'-GCT GGA CTC CCT TGT GTG T-3', reverse, 5'-CAC ATG GCC TCA GTC TCA A-3', and probe, 5'-CGC GGG ACC CTC ATC CTT TG-3' (FAM, TAMRA); mIL-10Rβ, forward, 5'-TCC ACA GCA CCT GAA GGA GTT-3', reverse, 5'-GGA GGG AAG GAG AAC AGC AGA-3', and probe, 5'-TGG GCC ACC CCC ATC ACA GC-3' (FAM, TAMRA). Reactions were run in duplicates and samples were normalized to the control housekeeping gene RPL-19 and reported according to the $\Delta\Delta$Ct method: $\Delta\Delta Ct = \Delta Ct_{sample} - \Delta Ct_{reference}$.

### *Ig ELISA*

Analyses were performed on serum from collected whole blood as previously described (*10*). Briefly, ELISA plates (Nunc) were coated with heat-killed *C. rodentium* or with a goat anti-mouse Ig capture Ab diluted 1/1000 in PBS (SouthernBiotech). Coated plates were washed in PBS plus 0.05% Tween 20, blocked for 1 h with 300 μl of blocking buffer (PBS + 0.5 % BSA + 10 PPM Proclin), and washed before addition of serially diluted standards (mouse monoclonal IgA, IgG, IgG3, and IgM from SouthernBiotech; IgG1, IgG2a, and IgG2b isotypes from Sigma-Aldrich; mouse IgG2c obtained from Bethyl Laboratories) or unknowns. Samples were incubated for 4 hours at room temperature. Plates were washed five times and the Ig isotypes were detected with goat anti-mouse IgA, IgM, IgG, IgG1, IgG2a, IgG2b, IgG2c, and IgG3 (SouthernBiotech) conjugated to horseradish peroxidase (HRP), diluted 1/4,000 in assay diluent (PBS + 0.5 % BSA + 0.05 % Tween 20 + 10 PPM Proclin, pH 7.4), and incubated for 1 hour at room temperature. After washing, TMB peroxidase substrate was added to each well and allowed to develop for 15 minutes, then stop solution (1 M Phosphoric acid) were added to each well. Absorbance was read at 450 nm in a Molecular Devices (Sunnyvale, CA) plate reader at $OD_{450}$.

### *In vitro colon culture*

Colons were removed from C57Bl/6 mice. After cleaning with cold PBS, colons were cut longitudinally. Colons were placed in a 100mm Petri dish with 10ml HBSS (Mediatech) buffer containing 2.5 μg/ml of Fungizone-Amphotericin B, 10 μg/ml Gentamicin, 100 U/ml Penicillin and 100 μg/ml Streptomycin (all from GIBCO, Invitrogen). Colons were gently scraped to remove mucus at the edge of the Petri dish and were transferred to a new Petri dish with fresh HBSS buffer. Colons were cut into 1-2 mm pieces and transferred to a 24-well plate with 50mg colons/1ml/well in RPMI buffer containing 10% heat inactivated FCS (HyClone), 2.5 μg/ml of Fungizone-Amphotericin B, 10 μg/ml Gentamicin, 2 mM L-Glutamine, 100 U/ml Penicillin and 100 ng/ml Streptomycin. 10 μg/ml of IL-22 (R & D systems) were added to the culture and incubated in 37 °C for 24 hours.

### *Microarray analysis*

Quantity and quality of total RNA samples was determined using an ND-1000 spectrophotometer (Nanodrop Technologies) and Bioanalyzer 2100 (Agilent Technologies), respectively. The method for preparation of Cy-dye labeled cRNA and array hybridization was provided by Agilent Technologies. Briefly, total RNA sample was converted to double-stranded cDNA and then to Cy-dye labeled cRNA using Agilent's Low RNA Input Fluorescent Linear Amplification Kit. The labeled cRNA was purified using RNeasy mini kit (Qiagen). cRNA yield and Cy-dye incorporation was determined using ND-1000 spectrophotometer. 750 ng of the labeled cRNA was fragmented and hybridized to the Agilent's Whole Mouse Genome array as described in manufacturer's In situ Hybridization kit-plus. All samples were labeled with Cy5 and hybridized against Cy3 labeled universal mouse reference (Stratagene). Following hybridization, the arrays were washed, dried and scanned on Agilent's DNA microarray scanner. Agilent's Feature Extraction software 8.5 was used to analyze acquired array images. For microarray data clustering (Fig. 20), expression data was processed to Agilent log-ratio data by standard methods. Selected genes were clustered by iterative agglomeration of vectors most highly linked by Pearson correlation coefficient, with data for agglomerated vectors summarized by average linkage.

***In vitro mouse tail tip fibroblast culture and stimulation***

To establish tail tip fibroblasts (TTFs), the tails from IL-17RC$^{-/-}$ adult mice and wild type littermates were peeled, minced into 1 cm pieces, placed on culture dishes, and incubated in high glucose DMEM (containing 10% FCS, 2 nm glutamine, 100 U/ml Penicillin and 100 μg/ml Streptomycin) for 5 days. Cells that migrated out of the graft pieces were transferred to new plates (passage 2) and maintained in the same media. We used TTFs at passage 3 for stimulation experiments. TTFs were seeded into 24-well plate at a density of $1.2 \times 10^5$ per well. Twenty four hours after seeding, recombinant murine IL-17A and IL-17F (R&D Systems) were added to the culture medium at various concentrations. Cell culture supernatant was harvested 24 hours after addition of cytokines and levels of murine IL-6 was measured by enzyme linked immunosorbent assay (ELISA) by mouse IL-6 ELISA set (BD Biosciences) following manufacturer's instructions.

***Blockade of murine IL-22 in vivo***

Blocking anti-mouse IL-22 (Clone 8E11, isotype mouse IgG1) mAb (*11*) was intraperitoneally injected before (Day 0) or 8 days after (Day 8) *C. rodentium* infection at a dose of 150 μg/mouse every other day. Certain control group also received isotype control IgG1 mAb.

***Statistics***

Statistical significance was calculated by one -way or two-way *ANOVA* using Prism software (GraphPad). All p values $\leq 0.05$ are considered significant, and are indicated in the text. Unless otherwise specified, all studies for which data are presented are representative of at least two independent experiments.

Although the foregoing invention has been described in some detail by way of illustration and example for purposes of clarity of understanding, the descriptions and examples should not be construed as limiting the scope of the invention. The disclosures of all patent and scientific literatures cited herein are expressly incorporated in their entirety by reference.

**Example 10: The LT pathway is mediated by IL-22 during *Citrobacer rodentium* infection.**

To help determine if IL-22 is important for the mortality caused by LT blockade, we performed a rescue experiment in which we expressed IL-22 in the mouse at the same time of LTbR-Fc treatment. The method we used for IL-22 expression was hydrodynamic tail vein delivery of plasmid DNA encoding mouse IL-22. Human LTbR-Ig was constructed as follows: human LTbR encompassing the extracellular domain (position 1 through position 224; SEQ ID NO:57) was cloned into a modified pRK5 expression vector encoding the human IgG1 Fc region (SEQ ID NO:58) downstream of the LTbR sequence. Proteins were overexpressed in CHO cells and purified by protein A affinity chromatography. Murine LTbR.Ig was constructed as follows: murine LTbR encompassing the extracellular domain (position 1 through position 222; SEQ ID NO:59) was cloned into a modified pRK5 expression vector encoding the murine IgG2a Fc region (SEQ ID NO:60) downstream of the LTbR sequence.

In Figure 21, we find that LTbR-Fc produces a similar weight loss curve (Figure 21 right panel) and death curve (Figure 21 left panel) to IL-22 blockade which led us to examine the relationship between LT and IL-22. *C. rodetium* infection leads to early expression of IL-22 in the colon.

Fig. 21 shows the percent survival of mice inoculated with *Citrobacter rodentium.* 6-8 week old Balb/c mice were fasted for 8h before oral inoculation with 2x109 *C. rodentium* strain DBS100 (ATCC 5 1459; American Type Culture Collection) in a total volume of 200 μl per mouse. While fasting, animals had access to water. Inoculation and all subsequent manipulations were conducted in BL-2 biosafety cabinets. Animals were allowed access to food after inoculation. Bacteria were prepared by incubation with shaking at 37°C overnight in LB broth. The relative concentration of bacteria was assessed by measuring absorbance at OD600 and each inoculation culture was serially diluted and plated to confirm CFU administered. On the day of inoculation, mice were also injected with 150ug of anti-gp 120 mAb, anti-IL-22 8E11 mAb, or LTbR-Fc 3 times per week.

**LT pathway regulate multiple upstream aspects that important for IL-22 production.** Figure 22 provides data on the LT pathway after infection with *C. rodentium*. A, C, E. Colons were harvested at different timepoints after infection with *C. rodentium.* Mice were injected with 150ug anti-gp 120 or LTbR-Fc every other day. RNA was extracted using Qiagen RNeasy Kit. Taqman analysis was performed to determine expression of IL-22, RegIIg, p19, or p40 relative to the day 0 timepoint. B. On day 4 after infection, colons were collected. After cleaning with cold PBS, colons were cut longitudinally. Colons were placed in a 100 mm Petri dish with 10 ml HBSS (Mediatech) buffer containing 2.5 μg/ml of Fungizone-Amphotericin B, 10 μg/ml Gentamicin, 100 U/ml Penicillin and 100 μg/ml Streptomycin (all from GIBCO, Invitrogen). Colons were gently scraped to remove mucus at the edge of the Petri dish and were transferred to a new Petri dish with fresh HBSS buffer. Colons were cut into 1-2 mm pieces and transferred to a 24-well plate with 50 mg colons/1 ml/well in RPMI buffer containing 10 % heat inactivated FCS (HyClone), 2.5 μg/ml of Fungizone-Amphotericin 13, 10 μg/ml Gentamicin, 2 mM L-Glutamine, 100 U/ml Penicillin and 100 μg/ml Streptomycin. 10 ng/ml of rmIL-22 (R & D systems) were added to the culture and incubated in 37°C for 24 hours. Supernatants were collected for an IL-22 ELISA. D. Day 6 colon lamina propria cells. DC determined by CD11c+ and MHC I1+. Colons were harvested and flushed with HBSS without calcium and magnesium (Invitrogen) with 2% FBS and 10mM HEPES. Colons were cut longitudinally, and then sectioned into 2-4 cm pieces, and the pieces were transferred to a 10 cm dish with HBSS without calcium and magnesium, 2% FBS, 1mM EDTA, 10mM HEPES, and 1mM DTT (Sigma-Aldrich). 1EL fractions were collected and discarded after a 45 minute incubation at 37°C while shaking at 200 rpm. For LPMCs isolation, the remaining epithelial layer was peeled off and the colon pieces were diced and placed into RPMI containing 10% FCS, 20mM HEPES, and 0.5 mg/ml collagenase/dispase (Roche Diagnostics). Colon pieces were incubated for one hour at 37°C while shaking. Isolated epithelial cells were washed and used for FACS analysis.

In Figure 22, we find that LTbR-Fc blocked the induction of IL-22 as well as RegIIIg which has been shown to be induced by IL-22 (Figure 22A-C). Dendritic cells have previously been shown to produce IL-22 and we find a slight reduction of DC numbers in the lamina propria of the colon 6 days after infection (Figure 22D). The decrease in IL-22 caused by LTbR-Fc is most likely due the loss of IL-23, since both p19 and p40 expression is inhibited after LTbR-Fc treatment during infection (Figure 22E).

**IL-22 partially rescues the defects seen in LTbR treated mice.** Figure 23 provides data concerning the effect of IL-22 on LTbR treated mice. A. Test of expression of IL-22 in serum and RegIIIg in colon after tail vein injection of IL-22 plasmid. B. Rescue of LTbRFc effects with IL-22 plasmid.

On day-1, animals were weighed and grouped, extra mice were euthanized. After weighing, all animals were fasted 14 h. The next morning (day 0), all mice were orally inoculated with 2-4 X 10e9 CFU of C. *rodentium* in 200 ul PBS. 150ug control mAb or Fc fusion protein was injected i.p. in 200ul PBS three times per week for two weeks starting on the same day as bacteria inoculation. Food was replaced back by investigators after inoculation. Six hours later plasmid DNA was injected by tail vein. Tail vein injection experiments: 1) DNA construct (pRK vector or pRK-mIL-22) was diluted in Ringer's to a concentration to yield a final dose of 10 micrograms/mouse/injection. 2) Each mouse was injected intravenously in the tail vein with approximately 1.6ml of the solution containing DNA in Ringer's. 3) Doses were administered as a bolus intravenous injection (tail vein) over a period of 4-5 seconds (8 seconds maximum) for maximum DNA uptake. Mice were restrained without anesthesia in a conical acrylic restrainer with a heating element to increase body temperature and dilate blood vessels. 4) Disposable sterile syringes were used for each animal. Animals were continuously monitored until they are clinically normal. 5) Animals were observed for any adverse clinical signs for at least 20 minutes post dose. If animals were not clinically normal by 1 hour post dose, they were euthanized or they were monitored until they were clinically normal. Moribund animals were euthanized. All manipulations were performed in BL-2 biosafety cabinets. During infection, moribund animals or those showing unalleviated distress or rectal prolapse were euthanized.

The mice were monitored for 4 weeks everyday. Between day 5 to day 17 when LTbR-Fc treated mice might become moribund, the mice were monitored twice per day including weekends. Fecal pellets were collected every week to measure CFU of C. rodentium. Mice were weighed once per week during the study. If mice exhibited a weight loss of 15% or more, they were weighed daily. If the weight loss exceeds 20%, the mice were euthanized. At the end of the study, all mice were euthanized and spleen, and colon were collected for histology, RNA or FACS analysis.

As shown in Figure 23A, we can detect expression of IL-22 in the mouse serum beginning at 2 hours post-injection, with expression declining at 72 hours. We can also detect expression of RegIIIg in the colon, suggesting that active IL-22 can act on the colon when expressed in this manner. IL-22 could partially rescue mortality and weight loss induced by LTb-Fc treatment during infection (Figure 23B).

**Treatment of mice with IL-22 mAb (8E11).** Figure 24 shows data demonstrating that treatment with IL-22 mAb 8E11 leads to reduced colon follicles, compromised B/T cell organization, and reduced DC, T cell, and B cell numbers in the colon. A. Six days after infection, colons were harvested and cut longitudinally. After a 30 minute incubation in HBSS without calcium and magnesium, 2% FBS, 1mM EDTA, 10mM HEPES, and 1mM DTT, colons were gently scraped to remove epithelial cells. Follicles were identified as white, round masses. There were five mice per group and each colon was counted and plotted as total follicles found or total follicles greater than 1mm found. B. Six days after infection, colons were flushed with cold PBS and quick frozen in OCT. Six micron sections were cut, dried, then fixed in acetone. Sections were blocked with 10% serum, the incubated with anti-CD5 FITC and anti-B220 APC at 10ug/ml each. Images were capture on a NIKON BX61 microscope. C. Six days after infection colon lamina propria cells were isolated at described above. FAC analysis was performed to determine the number of dendritic cells, CD3 T cells, and B cells after 8E11 treatment.

As shown in Figure 24, we treated mice with either IL-22 blocking antibody or LTbR-Fc and counted lymphoid follicles in the colon after six days post infection in order to determine if IL-22 could have a role in formation of colon lymphoid structures. We found a decrease in follicles greater than 1mm, suggesting IL-22 and LT could be important for the increase in follicle size after infection (Figure 24A). Histological analysis shows that blocking IL-22 disrupted the T and B cell zones of the follicle while LT blockade had a similar effect (Figure 24B). We next determined whether blockade of IL-22 leads to a change in cell numbers in the colon lamina propria. We found that IL-22 blockade led to decreases in DC, T cell, and B cell numbers during infection. In conclusion, IL-22 appears to be important for lymphoid follicle formation and may be an important downstream component of the lymphotoxin pathway in the colon.

Additional statements of invention

1. A method of treating an infection by a microbial pathogen, in a subject, by modulating an anti-microbial immune response in said subject, comprising administering to said subject an effective amount of an anti-microbial polypeptide (AMP), wherein said AMP is IL-22.

2. A method of treating an infection by a microbial pathogen, in a subject, by modulating an anti-microbial immune response in said subject, comprising administering to said subject an effective amount of an anti-microbial polypeptide (AMP) or modulator thereof, wherein said AMP is selected from a group consisting of: IL-6, IL-18, IL-23, REG I$\alpha$, REG I$\beta$, HIP/PAP, REG III, REG IV, Reg-related sequence (RS) and LT.

3. A method of modulating the activity of an anti-microbial polypeptide (AMP) in cells of a subject infected with a microbial pathogen, comprising contacting said cells with an isolated AMP, wherein said AMP is IL-22.

4. A method of modulating the activity of an anti-microbial polypeptide (AMP) in cells of a subject infected with a microbial pathogen, comprising contacting said cells with an isolated AMP, wherein said AMP is selected from a group consisting of: IL-6, IL-18, IL-23, REG I$\alpha$, REG I$\beta$, HIP/PAP, REG III, REG IV, Reg-related sequence (RS) and LT.

5. The method of 1, wherein said infection is a microbial disorder.

6. The method of 5, wherein said microbial disorder is Inflammatory Bowel Disease (IBD).

7. The method of 5, wherein said microbial disorder is Crohn's or ulcerative colitis (UC).

8. The method of 1, wherein said microbial pathogen is a bacteria.

9. The method of 8, wherein said bacteria is gram negative.

10. The method of 8, wherein said bacteria is gram positive.

11. The method of 8, wherein said bacteria is an attaching or effacing (A/E) bacteria.

12. The method of 11, wherein said attaching or effacing (A/E) bacteria is an enterohemorrhagic Escherichia coli (EHEC) and enteropathogenic E. Coli (EPEC).

13. The method of 12, wherein said enteropathogenic E. coli (EHEC) is E. coli 0157:H7 or E. coli 055:117.

14. The method of 2, wherein said anti-microbial polypeptide (AMP) is RegIII$\beta$ and RegIII$\gamma$.

15. The method of 1, wherein said microbial pathogen is a virus.

16. A method of treating an infection by a microbial pathogen, in a subject, by modulating an anti-microbial immune response in said subject, comprising administering to said subject an effective amount of an anti-microbial polypeptide (AMP) modulator, wherein said AMP modulator is an IL-22 agonist.

17. The method of 16, wherein said agonist increases expression and/or activity of said IL-22.

18. The method of 16, wherein said agonist is a polypeptide or nucleic acid molecule.

19. The method of 16, wherein said agonist is a fusion polypeptide.

20. The method of 16, wherein said agonist is an Fc fusion polypeptide.

21. The method of 16, wherein said agonist is an antibody or biologically active fragment thereof.

22. The method of 16, wherein said agonist is a monoclonal antibody.

23. The method of 16, wherein said agonist is a humanized antibody.

24. The method of 1 or 3 wherein the amino acid sequence of said IL-22 comprises a sequence shown as SEQ ID NO:8.

25. The method of 2 or 4 wherein the amino acid sequence of said AMP comprises a sequence selected from a group of amino acid sequences consisting of SEQ ID NO: 2, SEQ ID NO: 4, SEQ ID NO: 6, SEQ ID NO: 10, SEQ ID NO: 12, SEQ ID NO: 14, SEQ ID NO: 16, SEQ ID NO: 18, SEQ ID NO: 20, SEQ ID NO: 22, SEQ ID NO: 24, SEQ ID NO: 26, SEQ ID NO: 50, and SEQ ID NO: 52.

26. The method of 1 or 3, wherein the nucleic acid sequence encoding said IL-22 is a sequence shown as SEQ ID NO:7.

27. The method of 1 or 3, wherein the nucleic acid sequence encoding said AMP comprises a sequence selected from a group of nucleic acid sequences consisting of SEQ ID NO: 1, SEQ ID NO: 3, SEQ ID NO: 5, SEQ ID NO: 9, SEQ ID NO: 11, SEQ ID NO: 13, SEQ ID NO: 15, SEQ ID NO: 17, SEQ ID NO: 19, SEQ ID NO: 21, SEQ ID NO: 23, SEQ ID NO: 25, SEQ ID NO: 49, and SEQ ID NO: 51.

28. A kit comprising a pharmaceutical composition for treatment of a microbial disorder, wherein said pharmaceutical composition comprises an anti-microbial polypeptide (AMP), wherein said AMP is IL-22.

29. A kit comprising one or more pharmaceutical compositions for treatment of a microbial disorder, wherein said pharmaceutical compositions each comprise a different anti-microbial polypeptide (AMP) or modulator thereof, and wherein said AMP is selected from a group consisting of: IL-6, IL-18, IL-23, REG Iα, REG Iβ, HIP/PAP, REG III, REG IV, Reg-related sequence (RS), and LT.

## Claims

1. An anti-microbial polypeptide (AMP) or modulator thereof for use in a method of treating a microbial disorder in a subject, the method comprising administering the AMP to the subject, wherein the AMP is selected from the group consisting of: REG III, REG Iα, REG Iβ, HIP/PAP, REG IV, and Reg-related sequence (RS).

2. The AMP for use according to claim 1, wherein the microbial disorder is an inflammatory bowel disease (IBD).

3. The AMP for use according to claim 2, wherein the IBD is Crohn's disease or ulcerative colitis (UC).

4. The AMP for use according to any one of claims 1-3, wherein the AMP is REG III.

5. The AMP for use according to claim 4, wherein the REG III is REG IIIβ and/or REG IIIγ.

6. The AMP for use according to any one of claims 1-5, wherein the AMP comprises an amino acid sequence selected from the group consisting of: SEQ ID NO: 2, SEQ ID NO: 4, SEQ ID NO: 6, SEQ ID NO: 10, SEQ ID NO: 12, SEQ ID NO: 14, SEQ ID NO: 16, SEQ ID NO: 18, SEQ ID NO: 20, SEQ ID NO: 22, SEQ ID NO: 24, SEQ ID NO: 26, SEQ ID NO: 50, and SEQ ID NO: 52, or a mature form of the AMP thereof.

7. A kit comprising one or more pharmaceutical compositions comprising an AMP or modulator thereof for treating a microbial disorder in a subject, wherein the one or more pharmaceutical compositions each comprise a different AMP or modulator thereof, and wherein the AMP is selected from the group consisting of: REG III, REG Iα, REG Iβ, HIP/PAP, REG IV, and Reg-related sequence (RS).

8. The kit of claim 7, wherein the REG III is REG IIIβ and/or REG IIIγ.

Fig.1

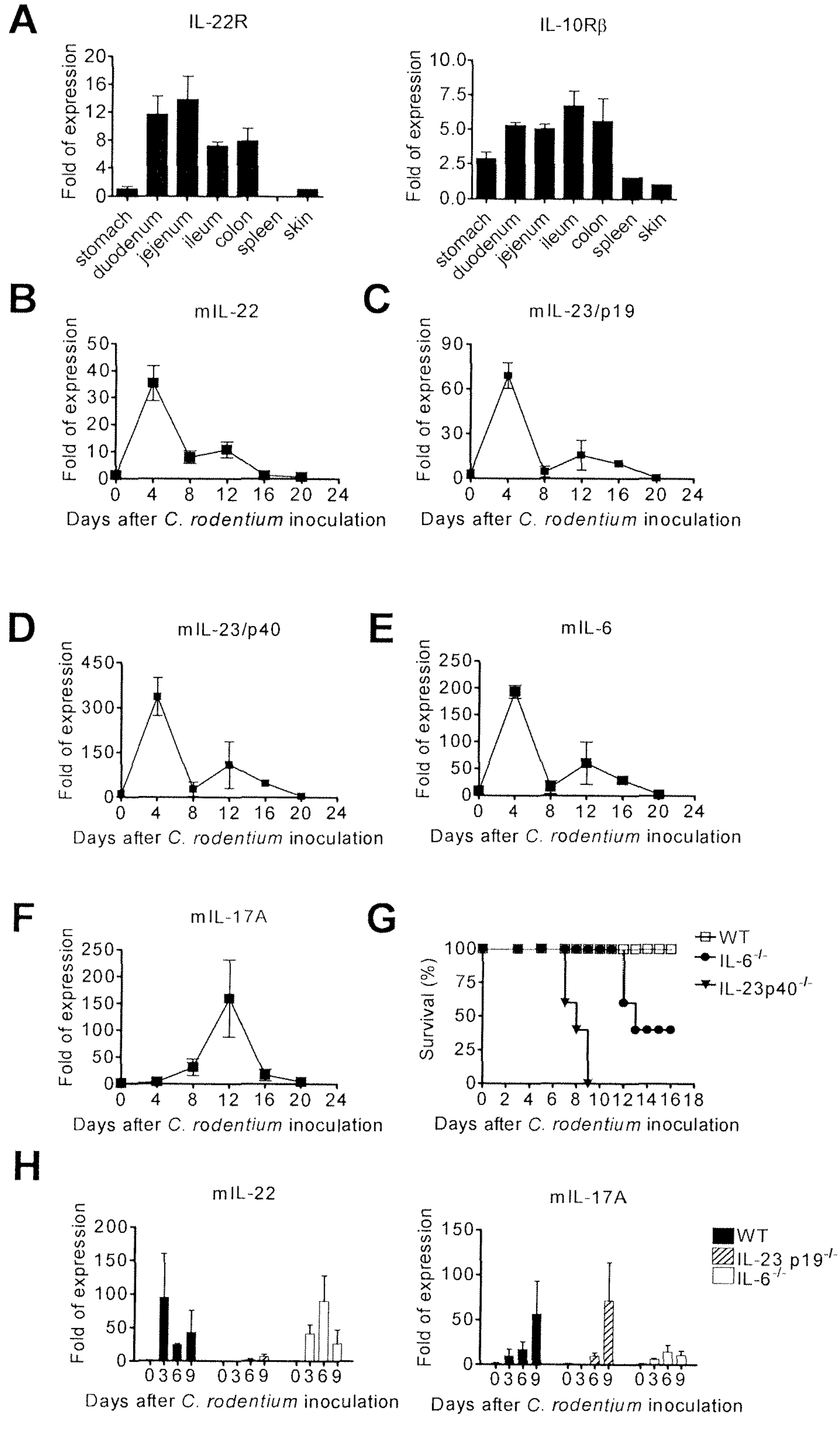
A
IL-22R
Fold of expression
20
16
12
8
4
0
stomach
duodenum
jejenum
ileum
colon
spleen
skin
IL-10Rβ
10.0
7.5
5.0
2.5
0.0
B
mIL-22
50
40
30
20
10
0
0 4 8 12 16 20 24
Days after C. rodentium inoculation
C
mIL-23/p19
90
60
30
0
D
mIL-23/p40
450
300
150
0
E
mIL-6
250
200
150
100
50
0
F
mIL-17A
G
Survival (%)
100
75
50
25
0
0 2 4 6 8 10 12 14 16 18
WT
IL-6-/-
IL-23p40-/-
H
mIL-22
0 3 6 9 0 3 6 9 0 3 6 9
mIL-17A
WT
IL-23 p19-/-
IL-6-/-

Fig.2

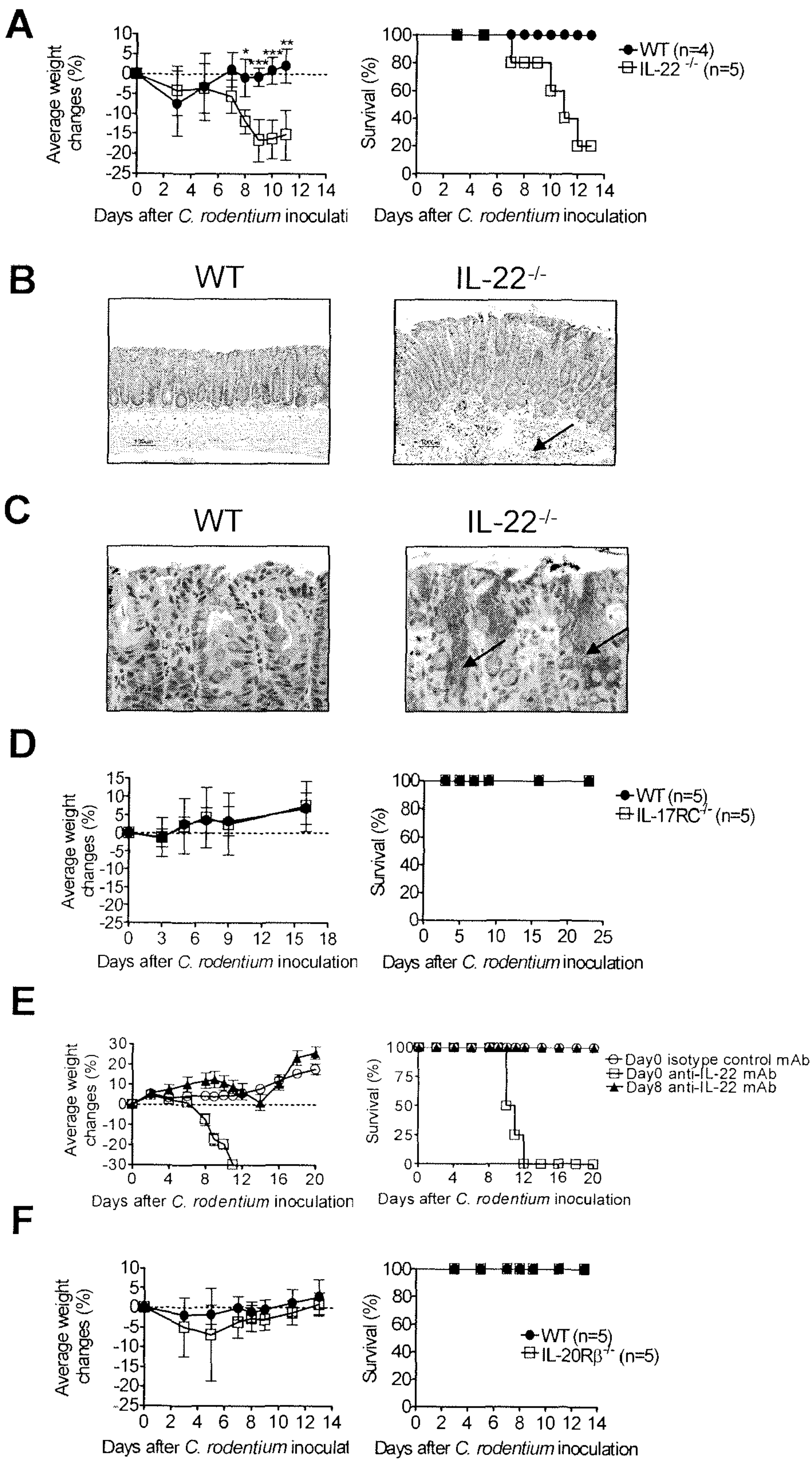
A
Average weight changes (%)
Days after C. rodentium inoculati
Survival (%)
Days after C. rodentium inoculation
WT (n=4)
IL-22 -/- (n=5)
B
WT
IL-22-/-
C
WT
IL-22-/-
D
Average weight changes (%)
Days after C. rodentium inoculation
Survival (%)
Days after C. rodentium inoculation
WT (n=5)
IL-17RC-/- (n=5)
E
Average weight changes (%)
Days after C. rodentium inoculation
Survival (%)
Days after C. rodentium inoculation
Day0 isotype control mAb
Day0 anti-IL-22 mAb
Day8 anti-IL-22 mAb
F
Average weight changes (%)
Days after C. rodentium inoculat
Survival (%)
Days after C. rodentium inoculation
WT (n=5)
IL-20Rβ-/- (n=5)

Fig.3

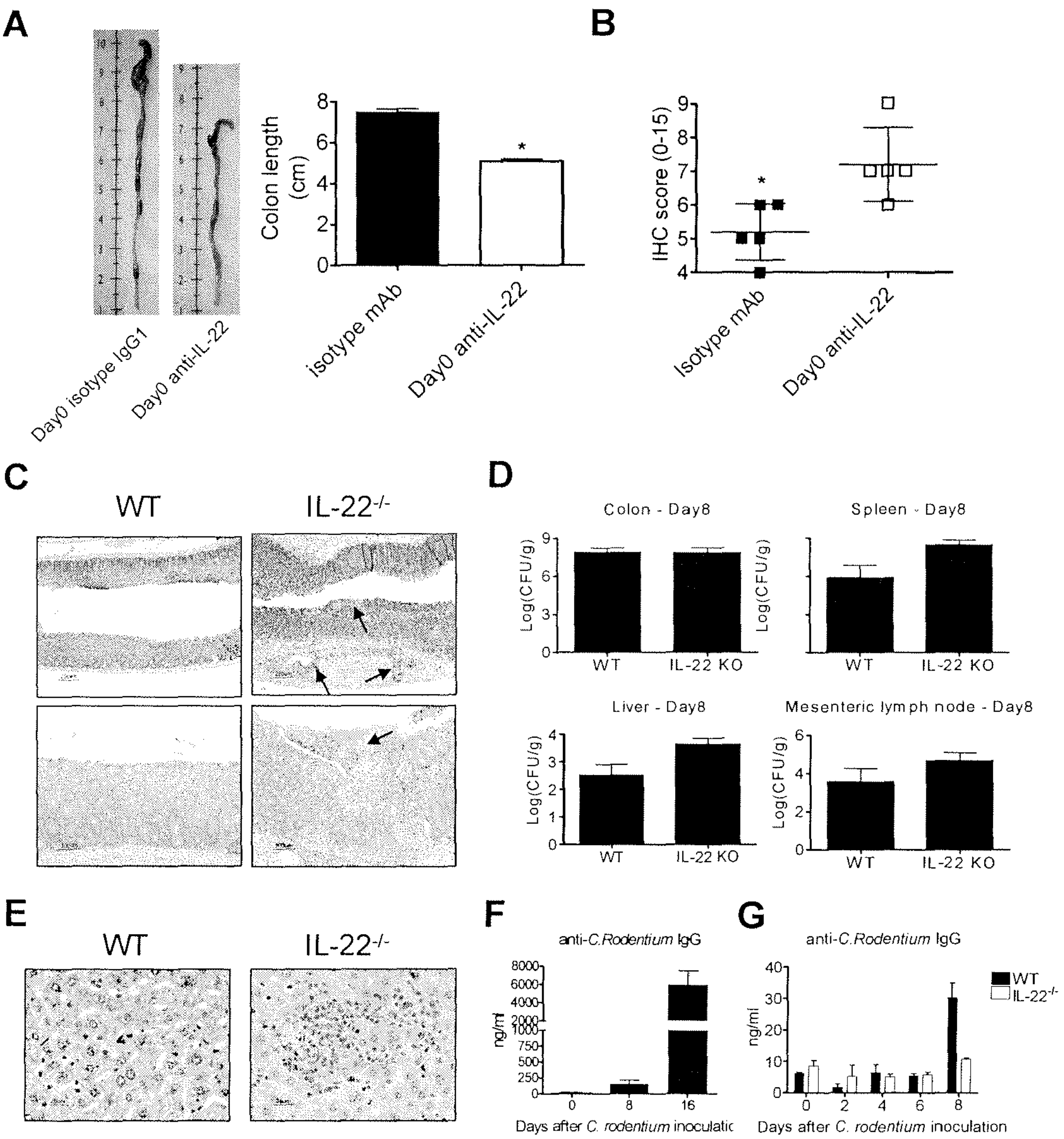
A
B
Day0 isotype IgG1
Day0 anti-IL-22
Colon length (cm)
isotype mAb
Day0 anti-IL-22
IHC score (0-15)
Isotype mAb
Day0 anti-IL-22
C
WT
IL-22-/-
D
Colon - Day8
Spleen - Day8
Liver - Day8
Mesenteric lymph node - Day8
Log(CFU/g)
WT
IL-22 KO
E
WT
IL-22-/-
F
anti-C.Rodentium IgG
ng/ml
Days after C. rodentium inoculatic
G
anti-C.Rodentium IgG
WT
IL-22-/-
ng/ml
Days after C. rodentium inoculation

Fig.4

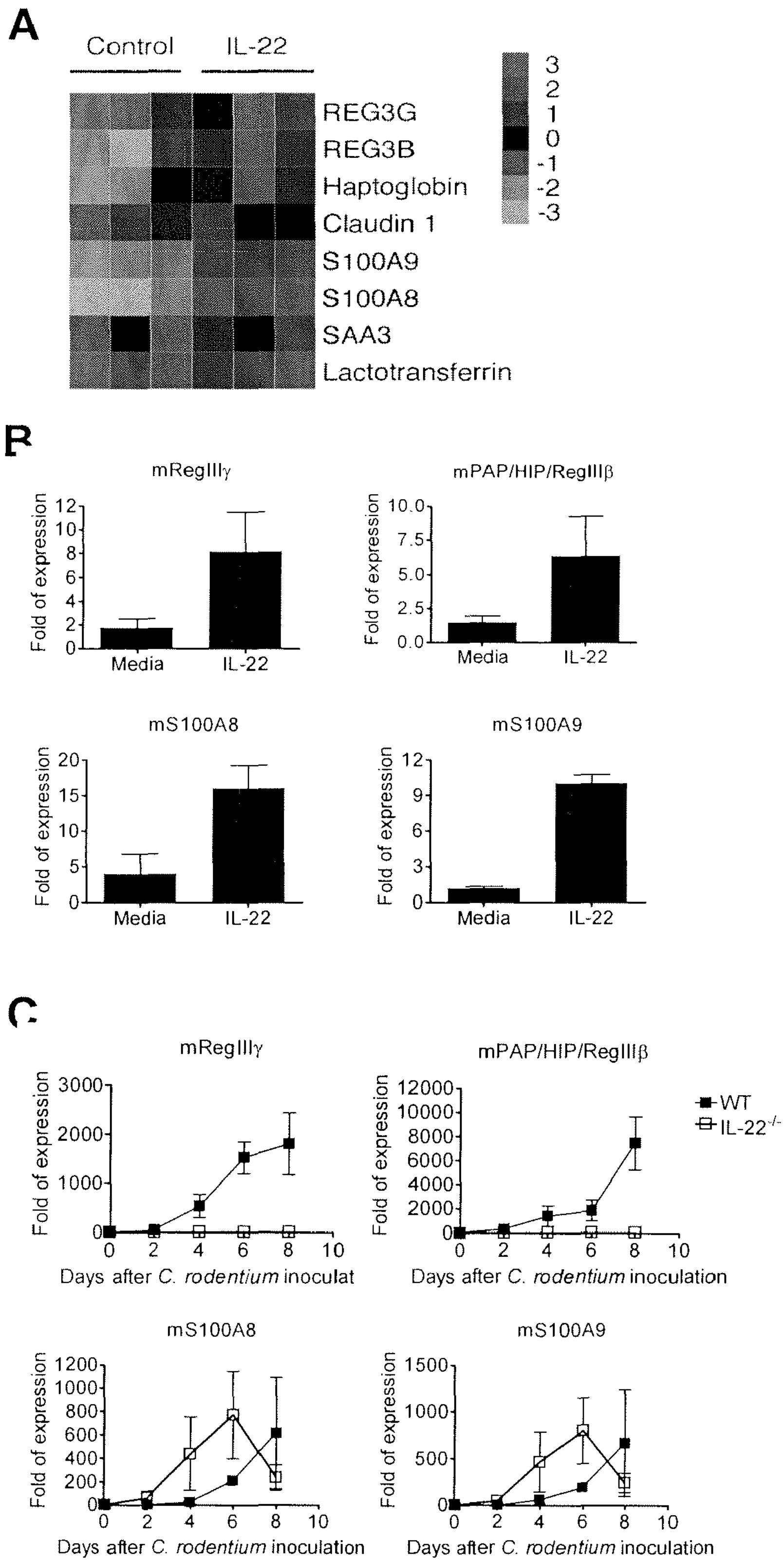
A
Control
IL-22
REG3G
REG3B
Haptoglobin
Claudin 1
S100A9
S100A8
SAA3
Lactotransferrin
3
2
1
0
-1
-2
-3
B
mRegIIIγ
mPAP/HIP/RegIIIβ
Fold of expression
Media
IL-22
mS100A8
mS100A9
C
mRegIIIγ
mPAP/HIP/RegIIIβ
WT
IL-22-/-
Days after C. rodentium inoculat
Days after C. rodentium inoculation
mS100A8
mS100A9

Fig. 5

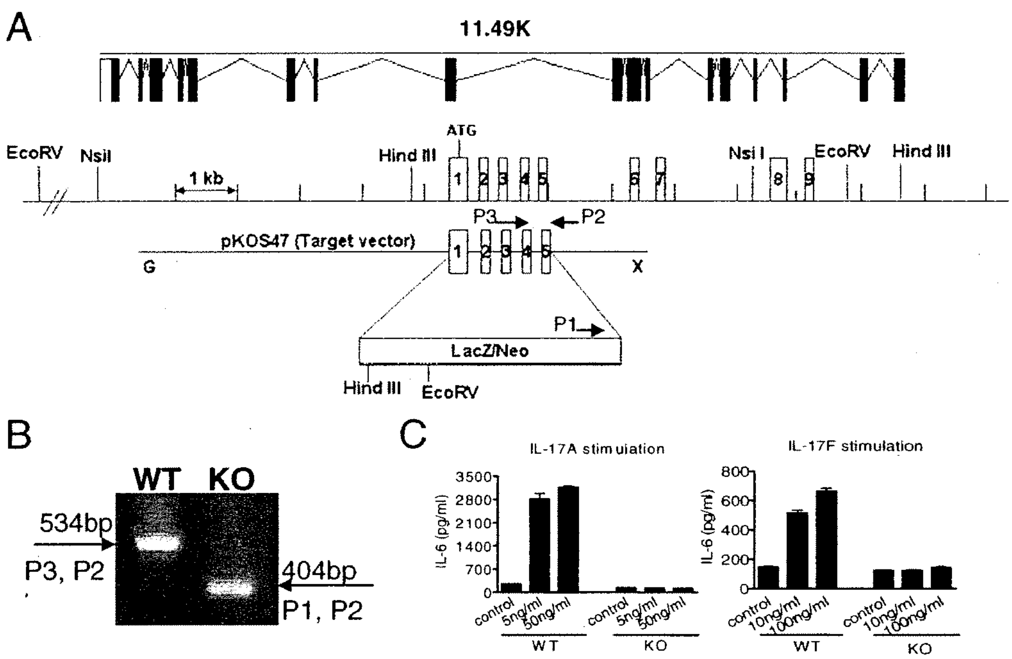
A
11.49K
ATG
EcoRV
NsiI
1 kb
Hind III
Nsi I
EcoRV
Hind III
P3
P2
pKOS47 (Target vector)
G
X
P1
LacZ/Neo
Hind III
EcoRV
B
WT
KO
534bp
P3, P2
404bp
P1, P2
C
IL-17A stimulation
IL-6 (pg/ml)
control
5ng/ml
50ng/ml
WT
KO
IL-17F stimulation
10ng/ml
100ng/ml

Fig. 6

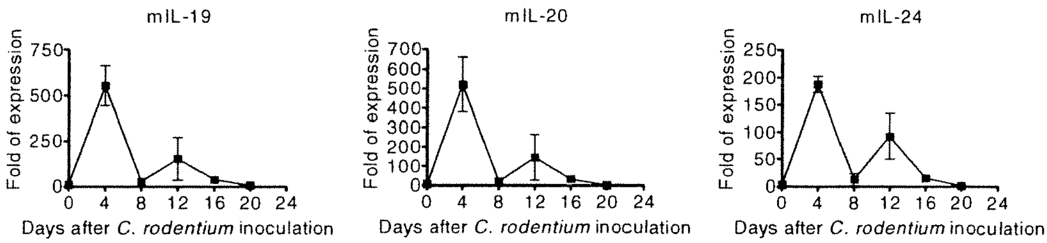
mIL-19
Fold of expression
750
500
250
0
0 4 8 12 16 20 24
Days after C. rodentium inoculation
mIL-20
Fold of expression
700
600
500
400
300
200
100
0
0 4 8 12 16 20 24
Days after C. rodentium inoculation
mIL-24
Fold of expression
250
200
150
100
50
0
0 4 8 12 16 20 24
Days after C. rodentium inoculation

Fig. 7

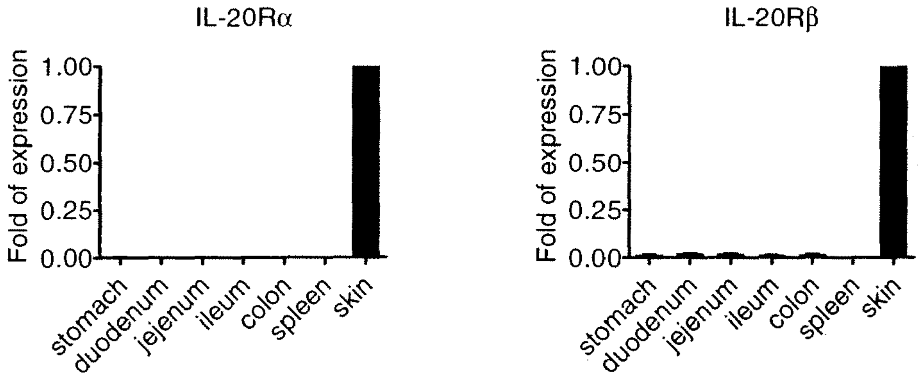
IL-20Rα
Fold of expression
1.00
0.75
0.50
0.25
0.00
stomach
duodenum
jejenum
ileum
colon
spleen
skin
IL-20Rβ
Fold of expression
1.00
0.75
0.50
0.25
0.00
stomach
duodenum
jejenum
ileum
colon
spleen
skin

Fig. 8

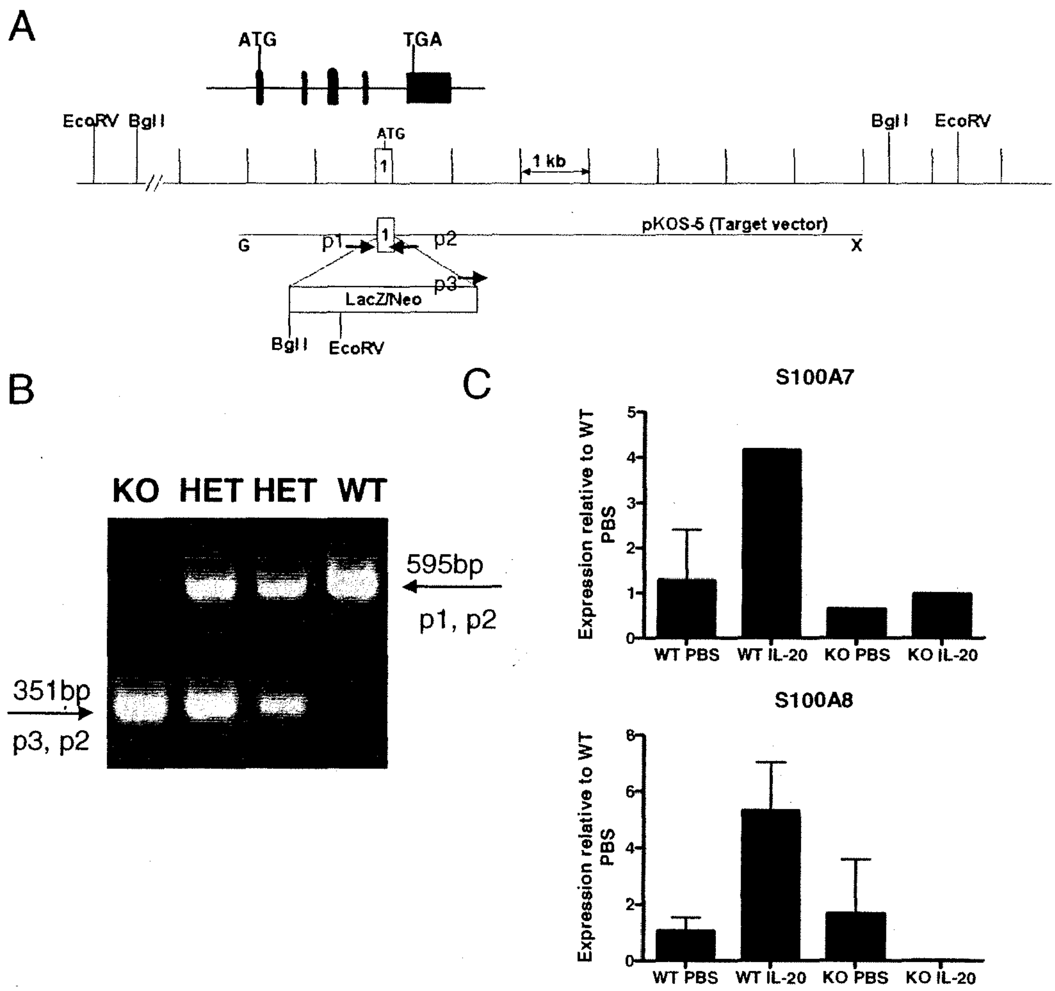
A
ATG
TGA
EcoRV BglI
ATG
1
1 kb
BglI EcoRV
pKOS-5 (Target vector)
G
p1
1
p2
X
p3
LacZ/Neo
BglI EcoRV
B
KO HET HET WT
595bp
p1, p2
351bp
p3, p2
C
S100A7
Expression relative to WT PBS
WT PBS WT IL-20 KO PBS KO IL-20
S100A8
Expression relative to WT PBS
WT PBS WT IL-20 KO PBS KO IL-20

Fig. 9

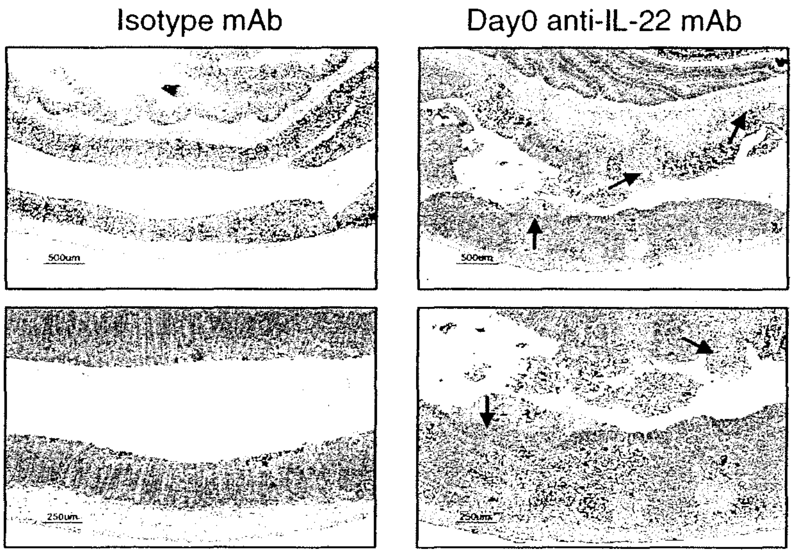

Fig. 10

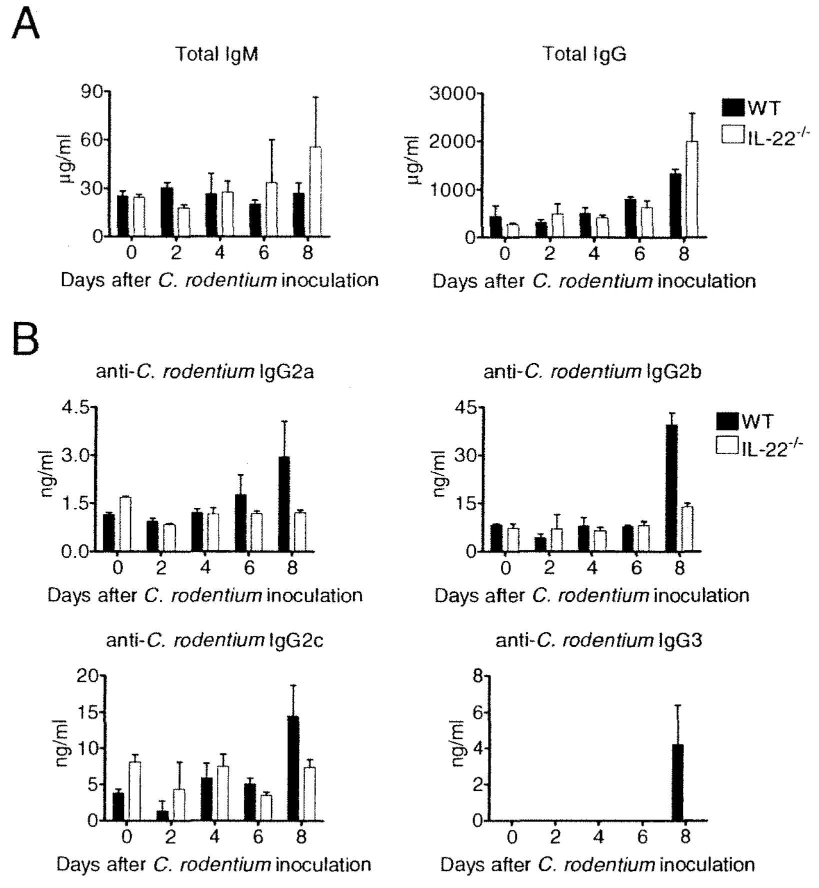
A
Total IgM
µg/ml
90
60
30
0
0 2 4 6 8
Days after C. rodentium inoculation
Total IgG
µg/ml
3000
2000
1000
0
0 2 4 6 8
Days after C. rodentium inoculation
WT
IL-22-/-
B
anti-C. rodentium IgG2a
ng/ml
4.5
3.0
1.5
0.0
0 2 4 6 8
Days after C. rodentium inoculation
anti-C. rodentium IgG2b
ng/ml
45
30
15
0
0 2 4 6 8
Days after C. rodentium inoculation
WT
IL-22-/-
anti-C. rodentium IgG2c
ng/ml
20
15
10
5
0
0 2 4 6 8
Days after C. rodentium inoculation
anti-C. rodentium IgG3
ng/ml
8
6
4
2
0
0 2 4 6 8
Days after C. rodentium inoculation

Fig. 11

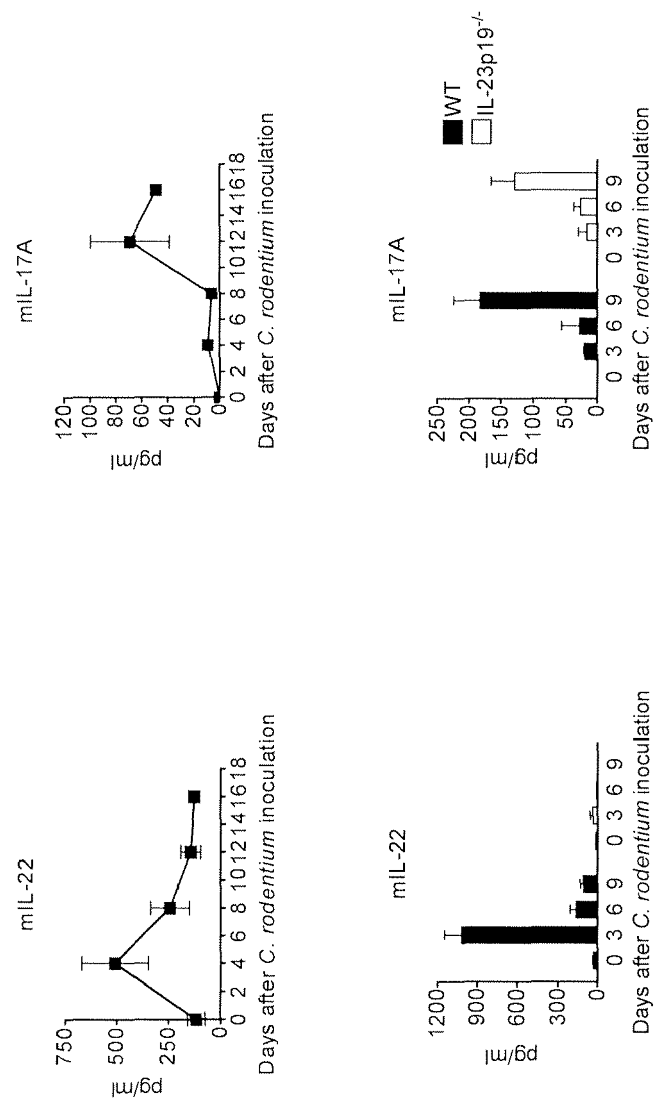
mIL-22
mIL-17A
pg/ml
Days after C. rodentium inoculation
WT
IL-23p19-/-

Fig. 12

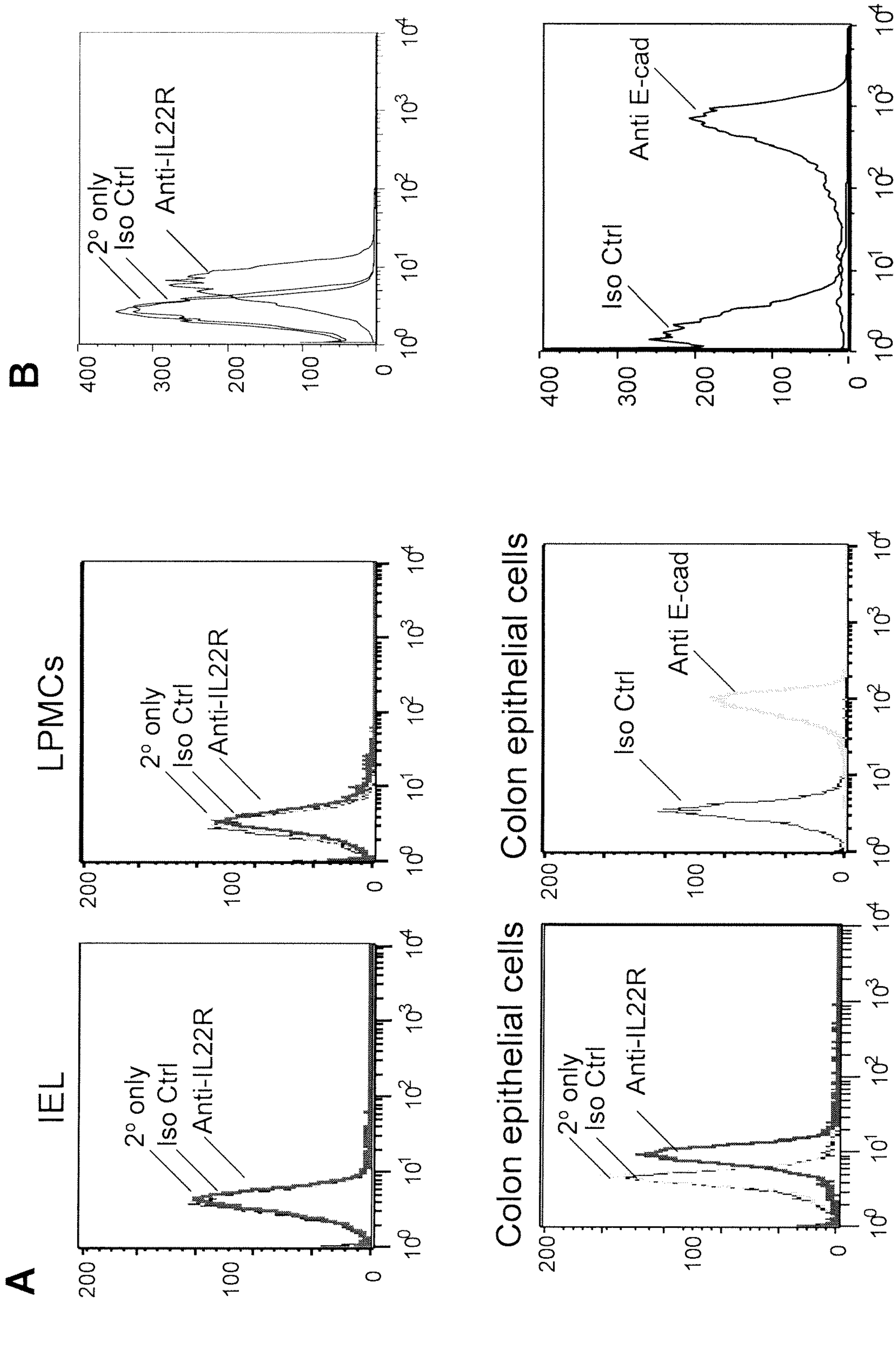
A
IEL
2° only
Iso Ctrl
Anti-IL22R
LPMCs
2° only
Iso Ctrl
Anti-IL22R
Colon epithelial cells
2° only
Iso Ctrl
Anti-IL22R
Colon epithelial cells
Iso Ctrl
Anti E-cad
B
2° only
Iso Ctrl
Anti-IL22R
Iso Ctrl
Anti E-cad

Fig. 13

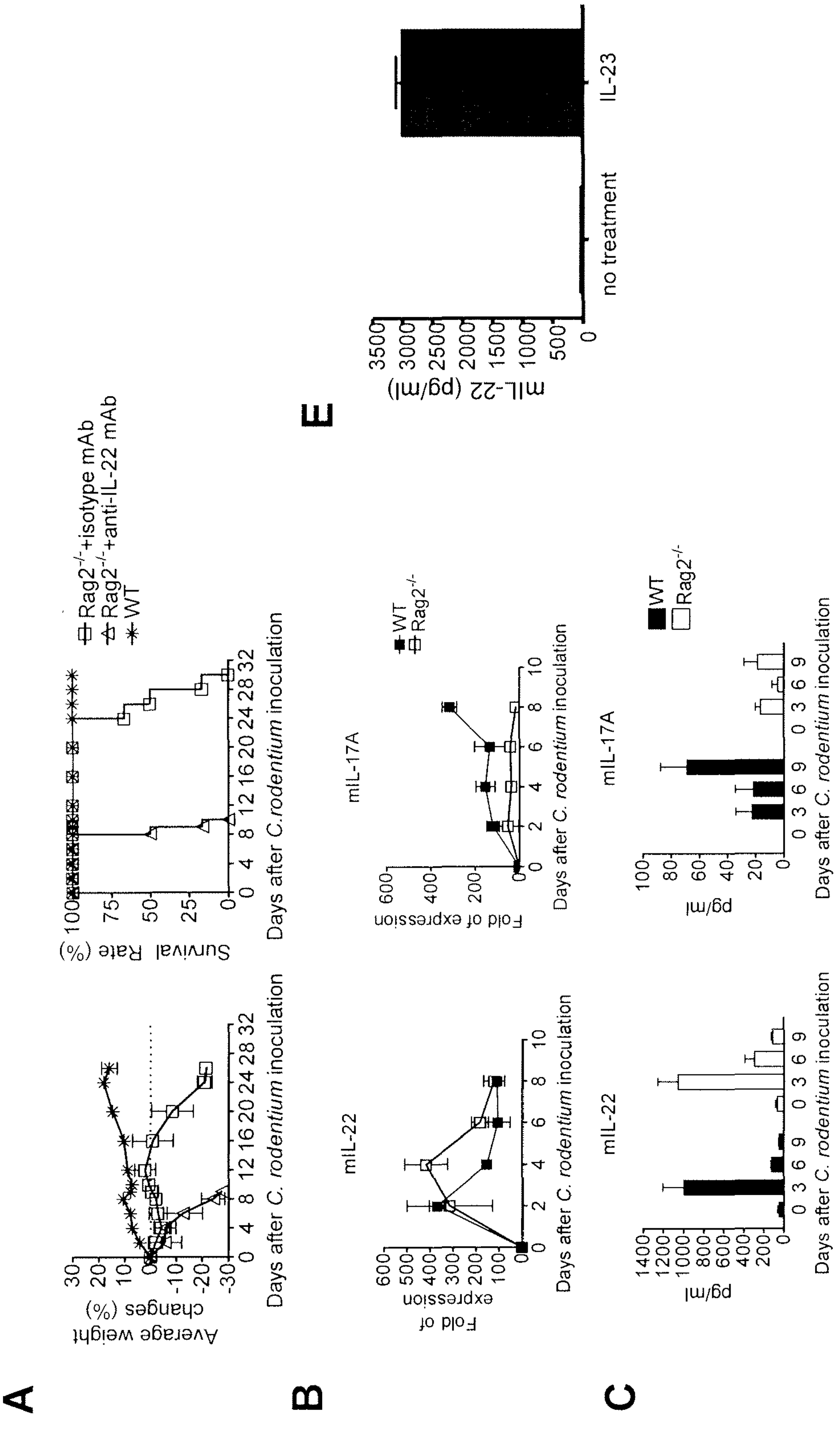
A
Average weight changes (%)
30
20
10
0
-10
-20
-30
0 4 8 12 16 20 24 28 32
Days after C. rodentium inoculation
Survival Rate (%)
100
75
50
25
0
0 4 8 12 16 20 24 28 32
Days after C. rodentium inoculation
Rag2-/-+isotype mAb
Rag2-/-+anti-IL-22 mAb
WT
B
mIL-22
Fold of expression
600
500
400
300
200
100
0
0 2 4 6 8 10
Days after C. rodentium inoculation
mIL-17A
Fold of expression
600
400
200
0
0 2 4 6 8 10
Days after C. rodentium inoculation
WT
Rag2-/-
C
mIL-22
pg/ml
1400
1200
1000
800
600
400
200
0
0 3 6 9 0 3 6 9
Days after C. rodentium inoculation
mIL-17A
pg/ml
100
80
60
40
20
0
0 3 6 9 0 3 6 9
Days after C. rodentium inoculation
WT
Rag2-/-
E
mIL-22 (pg/ml)
3500
3000
2500
2000
1500
1000
500
0
no treatment
IL-23

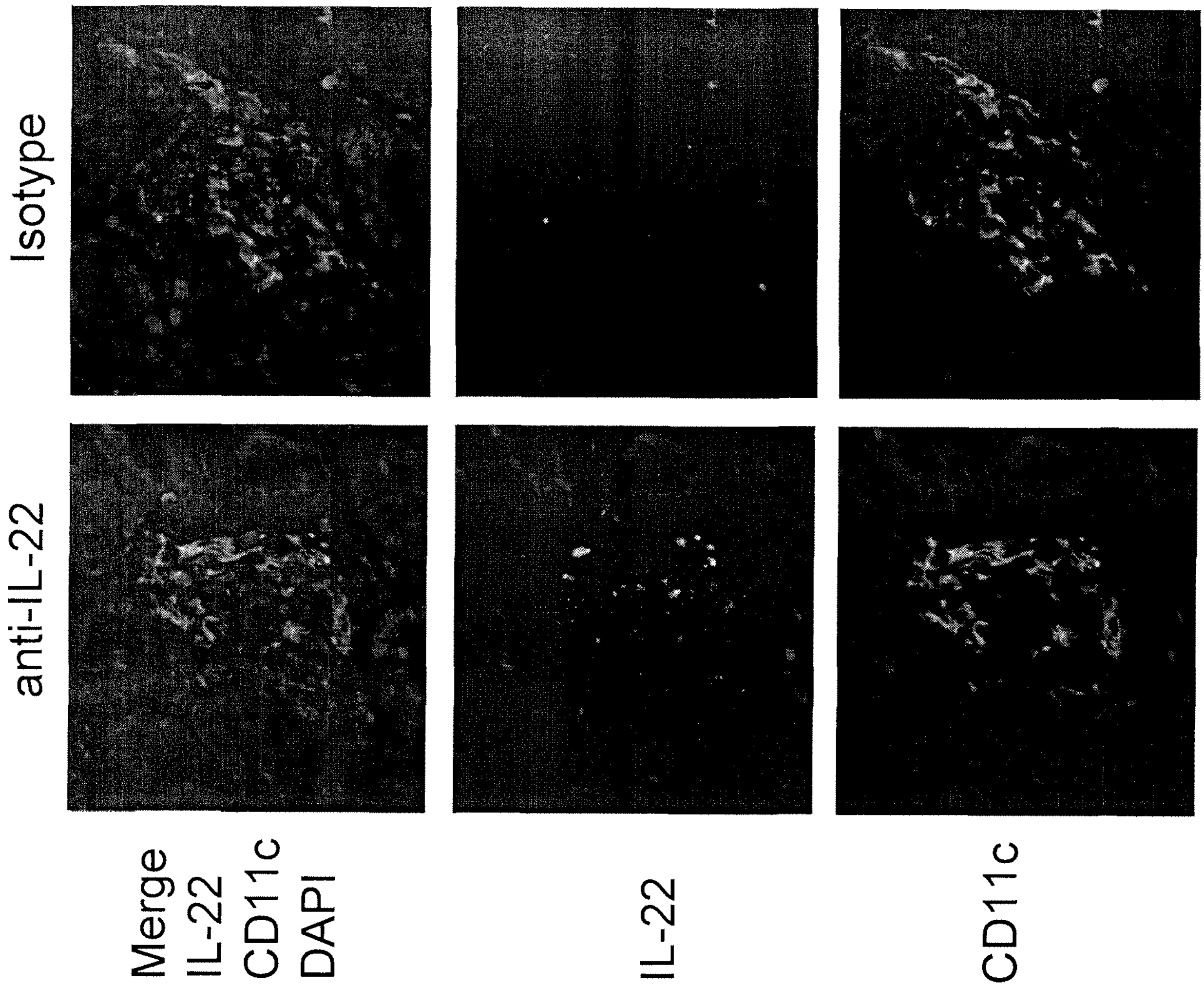


Fig. 13D

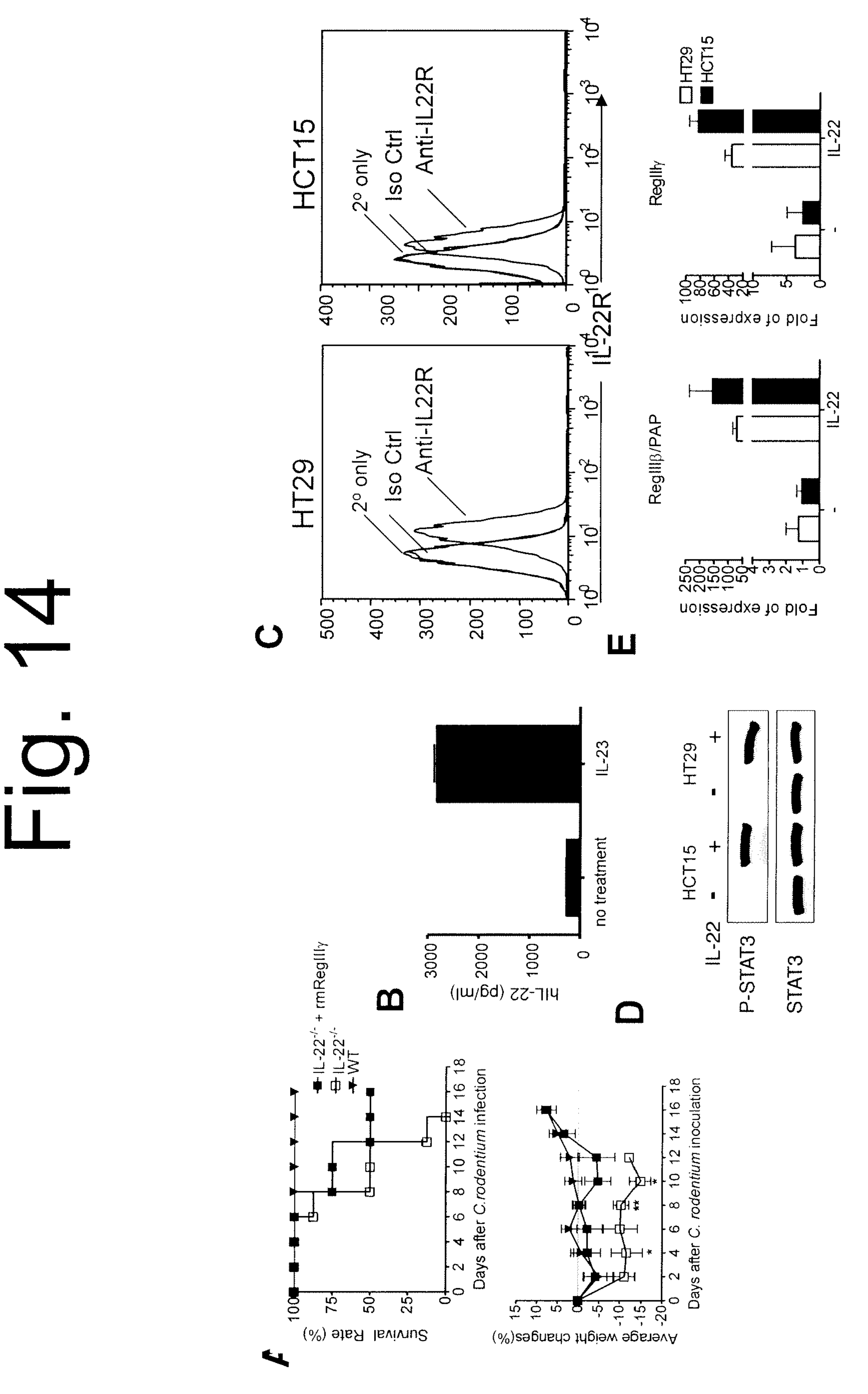
Fig. 14
A
Survival Rate (%)
Days after C.rodentium infection
IL-22-/- + rmRegIIIγ
IL-22-/-
WT
Average weight changes(%)
Days after C. rodentium inoculation
B
hIL-22 (pg/ml)
no treatment
IL-23
C
HT29
HCT15
2° only
Iso Ctrl
Anti-IL22R
IL-22R
D
IL-22
HCT15
HT29
P-STAT3
STAT3
E
Fold of expression
RegIIIβ/PAP
RegIIIγ
HT29
HCT15

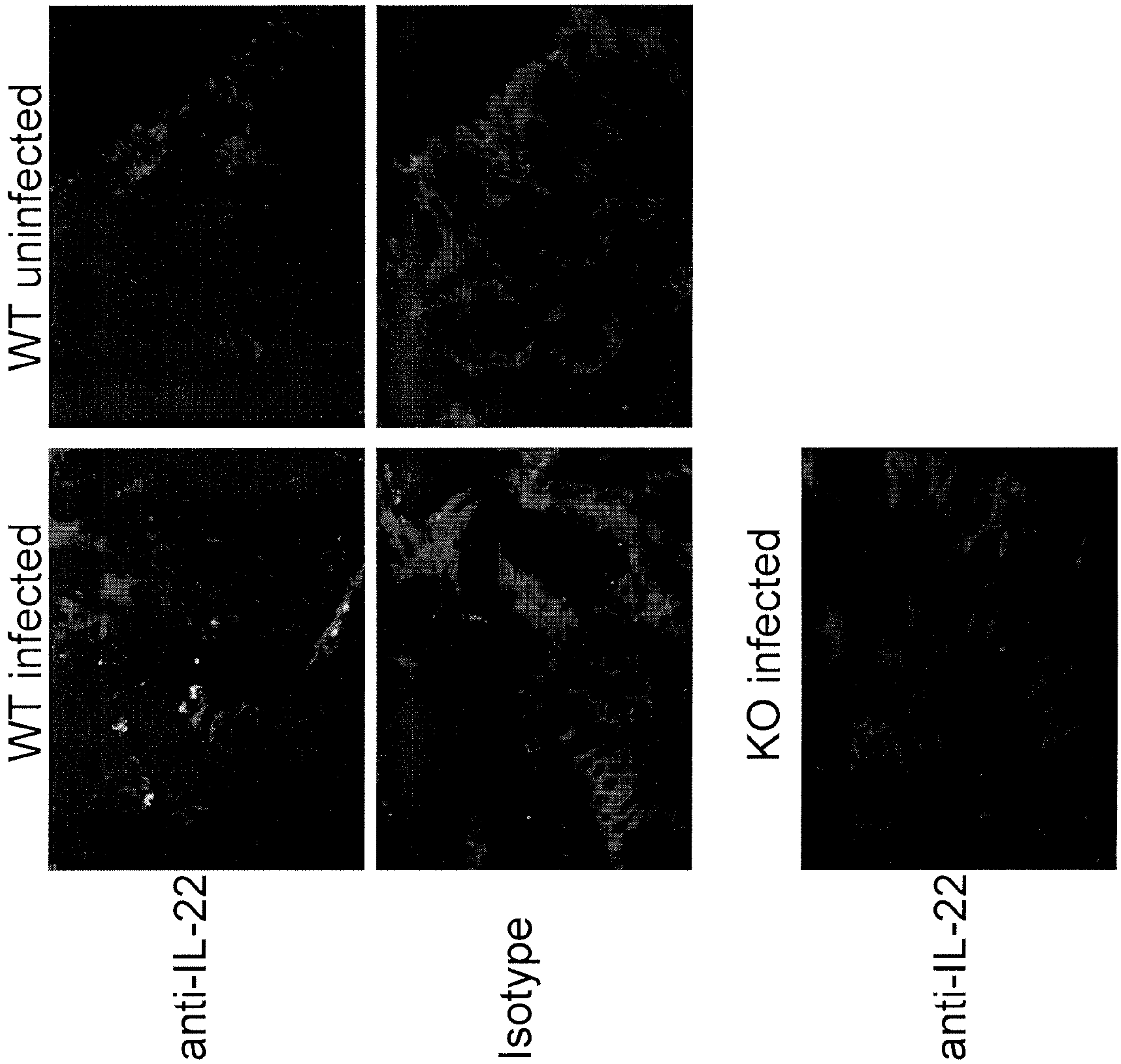
WT uninfected
WT infected
KO infected
anti-IL-22
Isotype
anti-IL-22

Fig. 15A

Fig. 15B

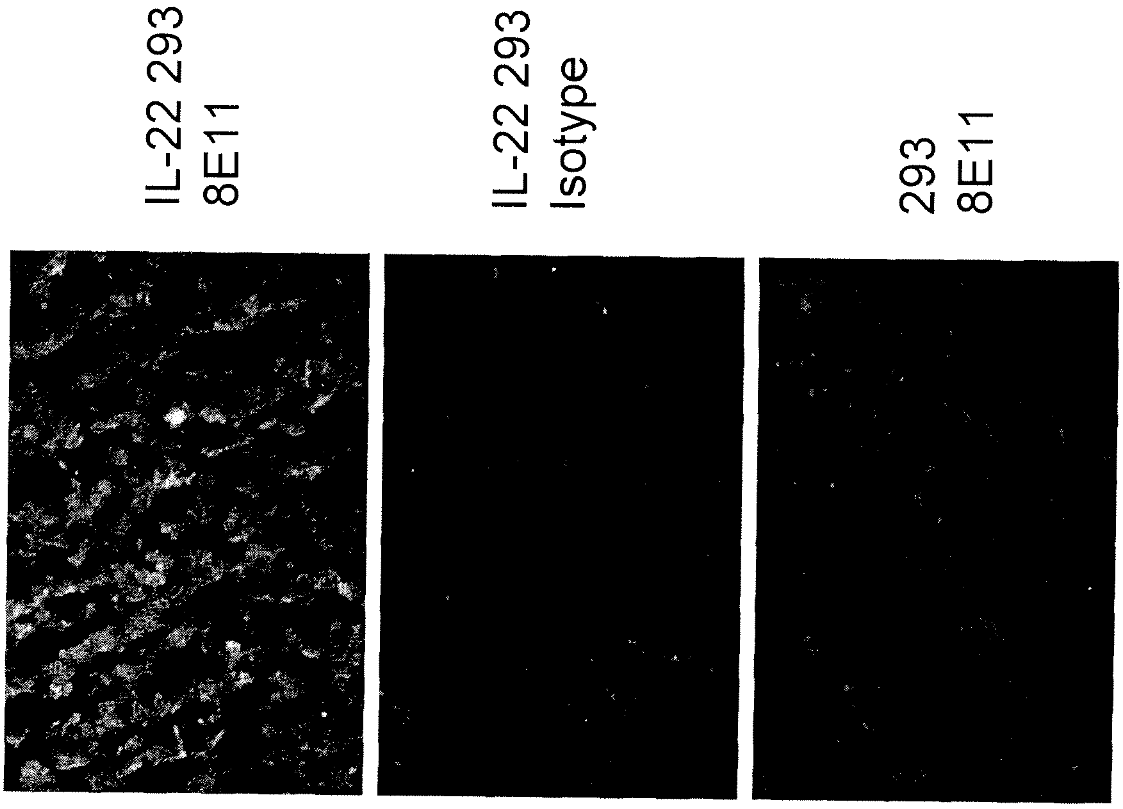

Fig. 16

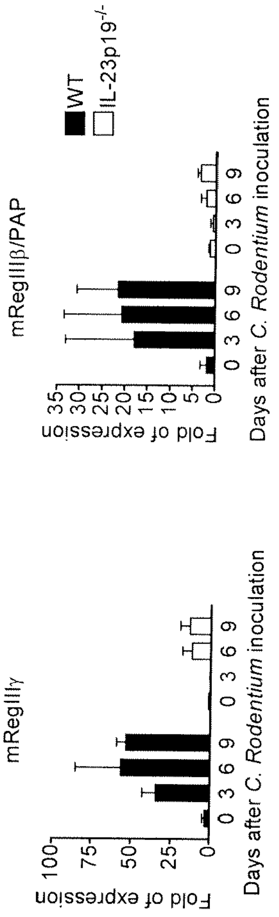
mRegIIIγ
Fold of expression
100
75
50
25
0
0 3 6 9 0 3 6 9
Days after C. Rodentium inoculation
mRegIIIβ/PAP
Fold of expression
35
30
25
20
15
10
5
0
0 3 6 9 0 3 6 9
Days after C. Rodentium inoculation
WT
IL-23p19-/-

Fig. 17

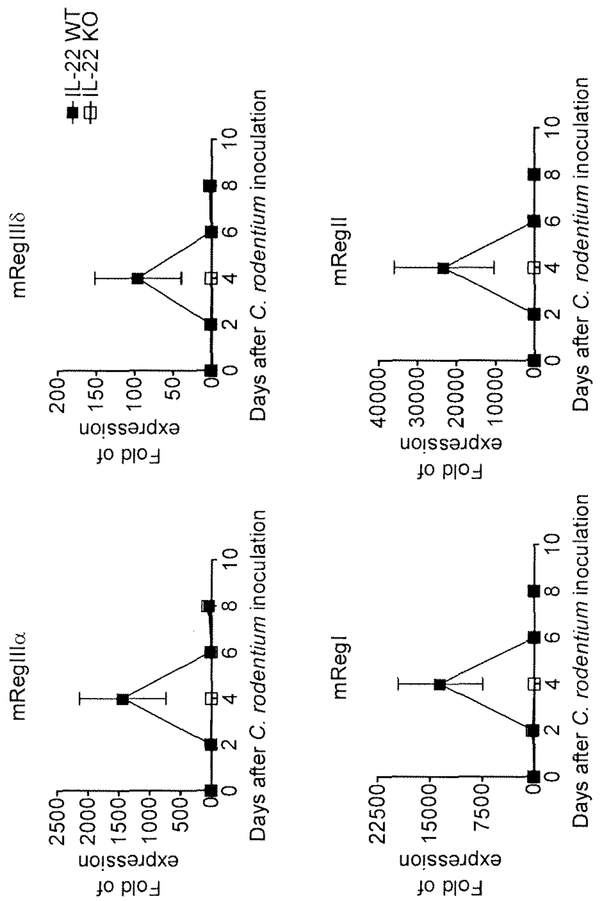
mRegIIIα
mRegIIIδ
mRegI
mRegII
IL-22 WT
IL-22 KO
Fold of expression
Days after *C. rodentium* inoculation
2500
2000
1500
1000
500
0
200
150
100
50
22500
15000
7500
40000
30000
20000
10000
2
4
6
8
10

Fig. 18

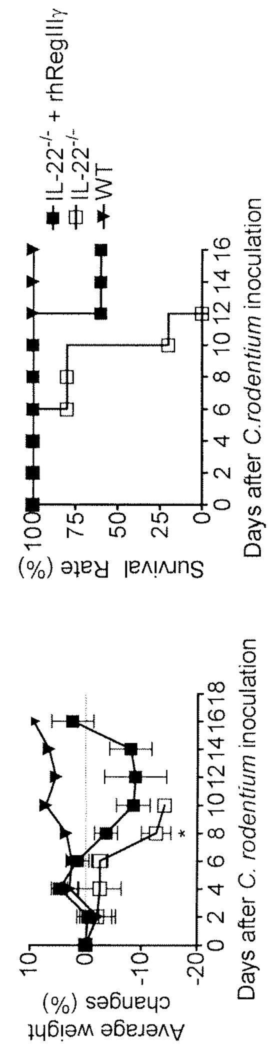
Average weight changes (%)
10
0
-10
-20
0 2 4 6 8 10 12 14 16 18
*
Days after C. rodentium inoculation
Survival Rate (%)
100
75
50
25
0
0 2 4 6 8 10 12 14 16
Days after C. rodentium inoculation
IL-22-/- + rhRegIIIγ
IL-22-/-
WT

Fig. 19A

| Probeset | P-value | Log2 FC | ID | Symbol |
|---|---|---|---|---|
| A_51_P256827 | 0 | 5.674 | NM_013650 | S100a8 |
| A_52_P700056 | 0.001 | 3.722 | BC089618 | |
| A_51_P402943 | 0.001 | 3.637 | NM_009114 | S100a9 |
| A_51_P235945 | 0.007 | 2.668 | NM_017370 | Hp |
| A_51_P169671 | 0.042 | 2.523 | NM_011036 | Pap |
| A_52_P472324 | 0.012 | 2.351 | NM_011414 | Slpi |
| A_52_P329207 | 0.006 | 2.262 | NM_007969 | Expi |
| A_52_P295432 | 0.015 | 2.111 | NM_009141 | Cxcl5 |
| A_52_P321831 | 0.007 | 2.062 | XM_894811 | Krtap3-1 |
| A_51_P279437 | 0.013 | 2.032 | NM_029662 | Mfsd2 |
| A_51_P246166 | 0.01 | 1.983 | NM_007969 | Expi |
| A_52_P230938 | 0.008 | 1.966 | NM_010741 | Ly6c |
| A_51_P384894 | 0.019 | 1.837 | NM_001001332 | Stfa1 |
| A_51_P156955 | 0.045 | 1.837 | NM_013459 | Cfd |
| A_51_P303160 | 0.03 | 1.821 | NM_007482 | Arg1 |
| A_51_P461703 | 0.034 | 1.804 | NM_031188 | Mup1 |
| A_51_P374476 | 0.019 | 1.742 | NM_008220 | Hbb-b1 |
| A_51_P481958 | 0.012 | 1.735 | NM_016674 | Cldn1 |
| A_51_P464703 | 0.019 | 1.727 | NM_021443 | Ccl8 |
| A_51_P132185 | 0.031 | 1.724 | NM_001001332 | Stfa1 |
| A_51_P496795 | 0.019 | 1.669 | NM_145555 | A330049M08Rik |
| A_51_P296109 | 0.01 | 1.63 | NM_028618 | Dmkn |
| A_51_P228768 | 0.043 | 1.591 | NM_011409 | Slfn3 |
| A_51_P466535 | 0.014 | 1.571 | AK172918 | Ttc9 |
| A_51_P387239 | 0.04 | 1.569 | NM_021792 | Iigp1 |
| A_51_P337308 | 0.03 | 1.547 | NM_011315 | Saa3 |
| A_51_P346938 | 0.031 | 1.541 | NM_029796 | Lrg1 |
| A_51_P504546 | 0.047 | 1.536 | NM_027406 | Aldh1l1 |
| A_51_P386870 | 0.026 | 1.53 | NM_011472 | Sprr2f |
| A_51_P216456 | 0.024 | 1.522 | NM_009311 | Tac1 |
| A_51_P212491 | 0.025 | 1.517 | NM_133232 | Pfkfb3 |
| A_52_P48681 | 0.018 | 1.514 | NM_016674 | Cldn1 |
| A_52_P424959 | 0.038 | 1.498 | NM_153457 | Rtn1 |
| A_52_P232813 | 0.047 | 1.484 | NM_203320 | Gm1960 |
| A_51_P181891 | 0.011 | 1.458 | NM_028113 | 2600011E07Rik |
| A_51_P249989 | 0.014 | 1.455 | NM_145133 | T2bp |
| A_52_P487686 | 0.042 | 1.405 | NM_001001332 | Stfa1 |
| A_52_P168962 | 0.023 | 1.387 | NM_007646 | Cd38 |
| A_51_P485312 | 0.026 | 1.372 | NM_013653 | Ccl5 |
| A_52_P99353 | 0.019 | 1.37 | AK009488 | |
| A_52_P252931 | 0.037 | 1.358 | NM_013505 | Dsc2 |
| A_52_P278538 | 0.039 | 1.349 | NM_008218 | Hba-a1 |
| A_52_P85132 | 0.034 | 1.331 | NM_172440 | Stxbp5l |
| A_52_P407198 | 0.023 | 1.322 | NM_183153 | A430060F13Rik |
| A_52_P638459 | 0.03 | 1.317 | BC033508 | Ccl5 |
| A_51_P335559 | 0.019 | 1.311 | AK034993 | |
| A_51_P240801 | 0.018 | 1.308 | BC046640 | 2610307O08Rik |
| A_52_P627816 | 0.032 | 1.292 | NM_019984 | Tgm1 |
| A_52_P656860 | 0.01 | 1.273 | NM_026232 | Slc25a30 |
| A_51_P180314 | 0.045 | 1.258 | AK011413 | Mup1 |
| A_51_P328870 | 0.011 | 1.249 | D86599 | Oxtr |
| A_51_P482908 | 0.041 | 1.237 | NM_025549 | Arrdc4 |
| A_51_P257951 | 0.041 | 1.235 | NM_020509 | Retnla |
| A_51_P441426 | 0.029 | 1.234 | NM_019932 | Cxcl4 |

# Fig. 19B

| Probeset | P-value | Log2 FC | ID | Symbol |
|---|---|---|---|---|
| A_51_P128463 | 0.049 | 1.232 | XM_485455 | Grrp1 |
| A_51_P239750 | 0.008 | 1.196 | NM_008380 | Inhba |
| A_51_P413866 | 0.044 | 1.188 | NM_008198 | Cfb |
| A_51_P462192 | 0.037 | 1.18 | NM_138648 | Olr1 |
| A_51_P291417 | 0.026 | 1.172 | NM_009378 | Thbd |
| A_51_P459176 | 0.013 | 1.162 | XM_899837 | 1700095J03Rik |
| A_51_P122321 | 0.012 | 1.161 | NM_133775 | 9230117N10Rik |
| A_51_P110301 | 0.023 | 1.158 | NM_009778 | C3 |
| A_51_P294807 | 0.039 | 1.154 | NM_013515 | Stom |
| A_51_P504991 | 0.027 | 1.151 | NM_181542 | Slfn10 |
| A_51_P440743 | 0.041 | 1.148 | NM_009886 | Celsr1 |
| A_51_P141012 | 0.019 | 1.137 | AK173296 | Slitrk1 |
| A_51_P445726 | 0.043 | 1.128 | NM_011095 | Lilrb3 |
| A_51_P397968 | 0.03 | 1.087 | AK087113 | |
| A_51_P464408 | 0.034 | 1.078 | NM_177314 | 5330439B14Rik |
| A_52_P597634 | 0.041 | 1.077 | NM_021457 | Fzd1 |
| A_51_P149714 | 0.038 | 1.066 | NM_026835 | Ms4a6d |
| A_52_P409833 | 0.027 | 1.04 | NM_008872 | Plat |
| A_51_P172853 | 0.03 | 1.04 | NM_009841 | Cd14 |
| A_52_P26161 | 0.026 | 1.039 | NM_008987 | Ptx3 |
| A_52_P164821 | 0.034 | 1.038 | XM_905096 | LOC631323 |
| A_52_P987411 | 0.036 | 1.038 | AK032717 | |
| A_51_P370552 | 0.037 | 1.037 | NM_030061 | 9230117E20Rik |
| A_52_P616392 | 0.017 | 1.032 | NM_183426 | Stno |
| A_51_P181899 | 0.027 | 1.021 | NM_028113 | 2600011E07Rik |
| A_52_P104075 | 0.037 | 1.021 | AK052629 | Frem1 |
| A_52_P621991 | 0.048 | 1.019 | NM_009684 | Apaf1 |
| A_52_P616949 | 0.036 | 1.014 | NM_027893 | Pvrl4 |
| A_51_P504988 | 0.022 | 1.01 | NM_172796 | Slfn9 |
| A_52_P87713 | 0.031 | 1.008 | NM_011593 | Timp1 |
| A_52_P1012710 | 0.034 | 1.005 | AK075639 | |
| A_52_P491718 | 0.049 | -1.002 | AK047841 | Mbtps2 |
| A_51_P191331 | 0.015 | -1.003 | NM_011379 | Sipa1 |
| A_52_P680941 | 0.028 | -1.003 | NM_010284 | Ghr |
| A_52_P1027837 | 0.038 | -1.003 | AK034309 | 9330175M20Rik |
| A_52_P787453 | 0.021 | -1.019 | AK045835 | |
| A_52_P484194 | 0.025 | -1.023 | NM_001025602 | Il1rl1 |
| A_52_P524426 | 0.03 | -1.023 | NM_001003815 | Epb4.1l1 |
| A_52_P828218 | 0.027 | -1.027 | NM_001030294 | Olfm4 |
| A_52_P674087 | 0.011 | -1.028 | AK036172 | Prtg |
| A_52_P826055 | 0.032 | -1.028 | AK134458 | 2310020J12Rik |
| A_52_P563505 | 0.022 | -1.033 | AK007074 | 1700095A21Rik |
| A_52_P44454 | 0.015 | -1.034 | P052591-1 | |
| A_51_P398037 | 0.018 | -1.039 | AK048407 | Scn3a |
| A_52_P1132297 | 0.017 | -1.058 | AK052006 | |
| A_52_P35598 | 0.034 | -1.064 | AK051891 | D230015P20Rik |
| A_51_P151983 | 0.009 | -1.066 | NM_019744 | Ncoa4 |
| A_51_P130719 | 0.014 | -1.066 | NM_023047 | Dpysl5 |
| A_52_P218833 | 0.018 | -1.066 | ENSMUST000000( | |
| A_52_P1084199 | 0.022 | -1.078 | AK036139 | 9630039A02Rik |
| A_51_P138496 | 0.021 | -1.08 | AK046037 | |
| A_52_P1124261 | 0.046 | -1.086 | AK081465 | |
| A_52_P158438 | 0.023 | -1.094 | AK006669 | 1700041C23Rik |
| A_52_P187046 | 0.022 | -1.098 | NM_022724 | Suv39h2 |

Fig. 19C

| Probeset | P-value | Log2 FC | ID | Symbol |
|---|---|---|---|---|
| A_52_P344098 | 0.027 | -1.098 | AK040876 | Pde7b |
| A_51_P314669 | 0.034 | -1.1 | NM_011128 | Pnliprp2 |
| A_52_P475356 | 0.02 | -1.107 | NM_011445 | Sox6 |
| A_52_P177454 | 0.043 | -1.114 | AK045474 | Rab34 |
| A_52_P972196 | 0.015 | -1.12 | AK084612 | |
| A_52_P1140731 | 0.024 | -1.122 | AK089673 | |
| A_51_P249302 | 0.017 | -1.125 | NM_011994 | Abcd2 |
| A_52_P866925 | 0.031 | -1.133 | AK006067 | 1700017I07Rik |
| A_52_P956057 | 0.026 | -1.17 | AK081935 | |
| A_52_P939063 | 0.012 | -1.171 | AK032576 | |
| A_51_P243750 | 0.037 | -1.175 | NM_012025 | Racgap1 |
| A_52_P205710 | 0.047 | -1.189 | NM_021611 | Mylc2pl |
| A_52_P1188576 | 0.017 | -1.197 | AK082439 | |
| A_51_P282523 | 0.032 | -1.201 | NM_054044 | Gpr124 |
| A_52_P642801 | 0.009 | -1.202 | XM_618738 | Lats1 |
| A_51_P312615 | 0.038 | -1.207 | NM_010377 | Hist1h1t |
| A_52_P214828 | 0.022 | -1.209 | NM_011253 | Rbmy1a1 |
| A_52_P158660 | 0.024 | -1.231 | XM_001002308 | 4833417A11Rik |
| A_51_P104255 | 0.012 | -1.237 | NM_001039226 | LOC625026 |
| A_52_P811599 | 0.035 | -1.244 | AK084144 | |
| A_52_P668855 | 0.016 | -1.246 | AK042778 | 9330154J02Rik |
| A_51_P399974 | 0.035 | -1.246 | NM_146997 | Olfr178 |
| A_52_P355709 | 0.007 | -1.247 | XM_973329 | LOC664837 |
| A_52_P207356 | 0.048 | -1.249 | AK052515 | Aspm |
| A_52_P1188835 | 0.034 | -1.256 | NM_001002780 | F830005K03Rik |
| A_51_P407915 | 0.013 | -1.263 | AK040806 | A530026G17 |
| A_51_P352743 | 0.045 | -1.285 | NM_001013616 | Trim6 |
| A_51_P191139 | 0.012 | -1.293 | AK050699 | D030005H02Rik |
| A_52_P1139473 | 0.007 | -1.294 | AK018751 | 0610037L18Rik |
| A_52_P69506 | 0.042 | -1.294 | NM_177566 | Arhgef15 |
| A_52_P134003 | 0.042 | -1.327 | NM_001033289 | Slc9a2 |
| A_52_P519943 | 0.004 | -1.337 | BC044804 | Fads6 |
| A_51_P366413 | 0.009 | -1.394 | AK033346 | 8030498J20Rik |
| A_52_P988314 | 0.029 | -1.401 | AK083509 | |
| A_52_P778486 | 0.029 | -1.404 | AK016521 | 4932412D23Rik |
| A_52_P162486 | 0.009 | -1.409 | NM_007542 | Bgn |
| A_51_P242952 | 0.032 | -1.411 | AK079868 | |
| A_52_P267243 | 0.039 | -1.413 | NM_010200 | Fgf13 |
| A_52_P328714 | 0.018 | -1.422 | AK133758 | Fbxw2 |
| A_51_P238040 | 0.018 | -1.424 | AK030263 | |
| A_51_P327166 | 0.006 | -1.426 | AK015801 | |
| A_51_P201035 | 0.027 | -1.443 | NM_145924 | Cenpi |
| A_52_P169595 | 0.034 | -1.455 | NM_177669 | A630098G03Rik |
| A_52_P507877 | 0.04 | -1.463 | NM_007729 | Col11a1 |
| A_52_P224818 | 0.012 | -1.468 | NM_172483 | Zfp180 |
| A_51_P270050 | 0.041 | -1.485 | AK078370 | 6530421E24Rik |
| A_51_P309099 | 0.02 | -1.489 | AK050657 | |
| A_51_P103261 | 0.034 | -1.533 | NM_009198 | Slc17a1 |
| A_52_P535430 | 0.021 | -1.561 | NM_207130 | Abca16 |
| A_52_P1083387 | 0.015 | -1.585 | AK005856 | 1700011B04Rik |
| A_51_P152873 | 0.014 | -1.71 | NM_207670 | Gripap1 |
| A_52_P569873 | 0.012 | -1.778 | XM_127913 | 4930455B06Rik |
| A_51_P396119 | 0.008 | -2.489 | AK047973 | Ttll5 |

# Fig.20

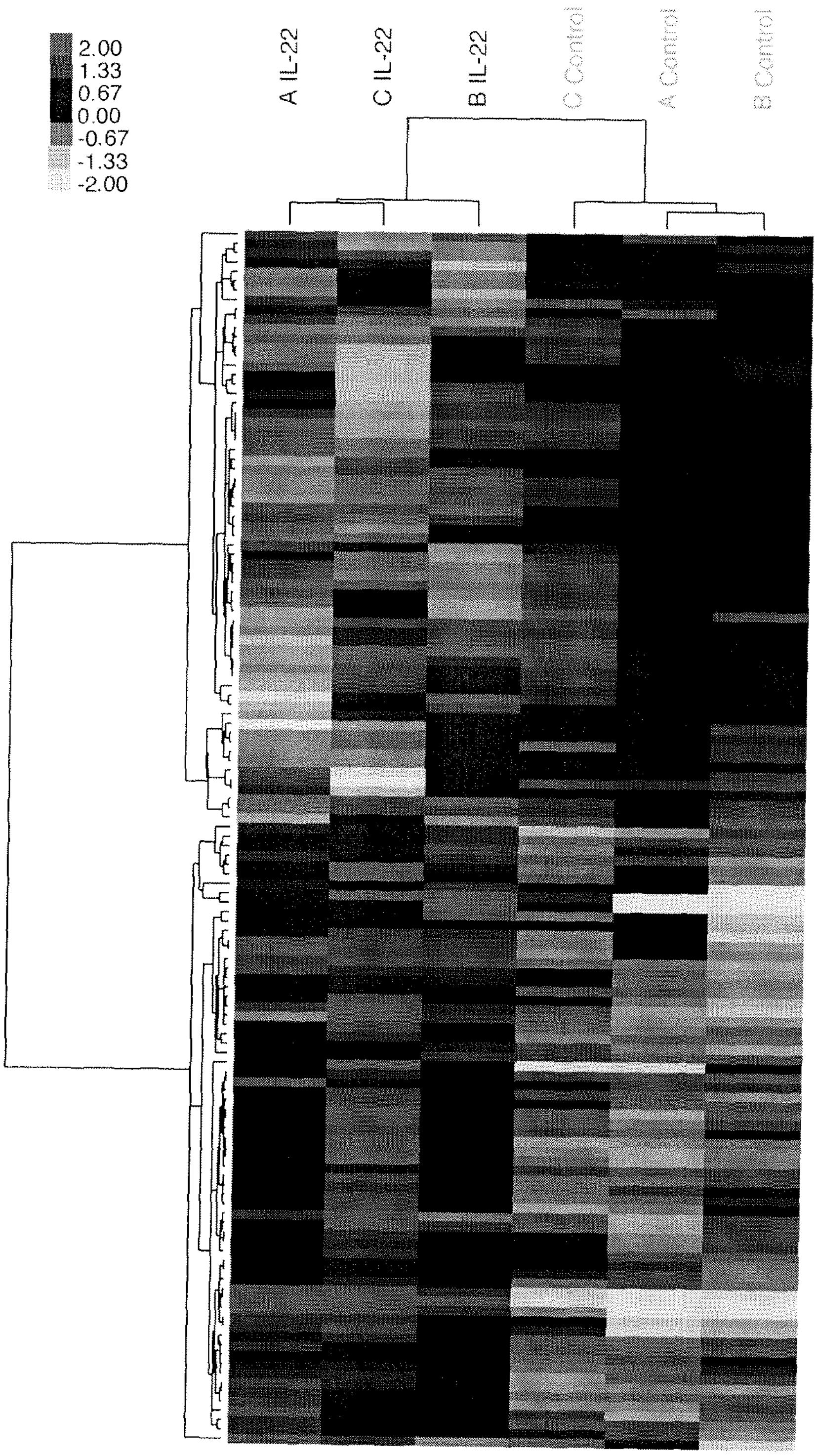
2.00
1.33
0.67
0.00
-0.67
-1.33
-2.00
A IL-22
C IL-22
B IL-22
C Control
A Control
B Control

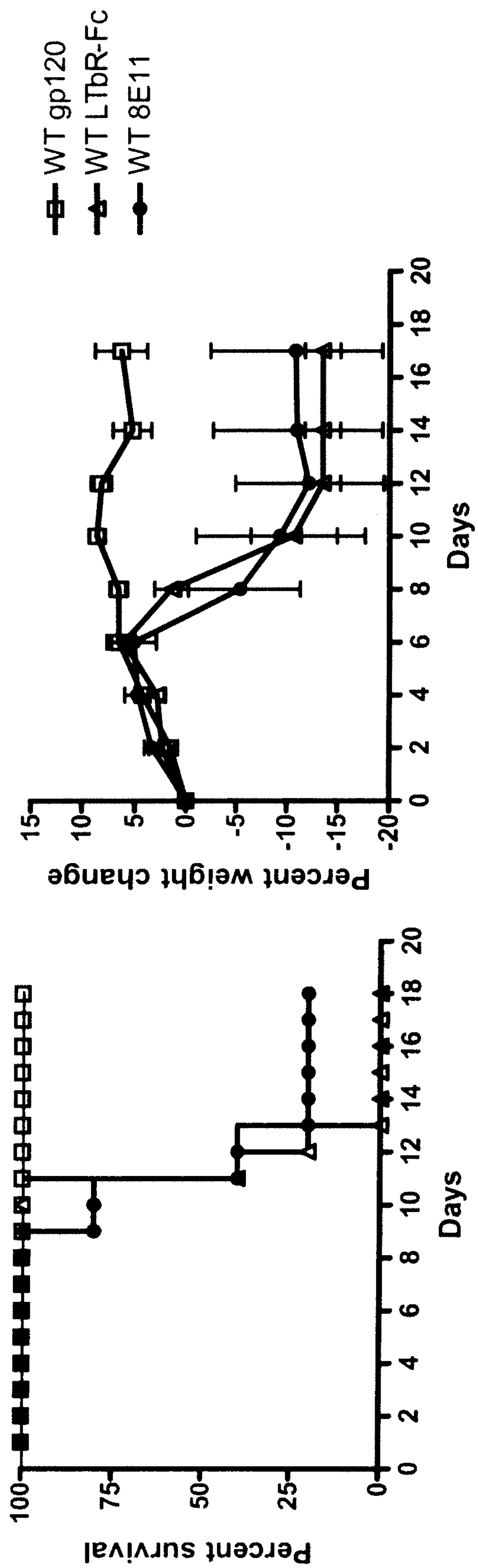
Percent survival
100
75
50
25
0
0
2
4
6
8
10
12
14
16
18
20
Days
Percent weight change
15
10
5
0
-5
-10
-15
-20
0
2
4
6
8
10
12
14
16
18
20
Days
WT gp120
WT LTbR-Fc
WT 8E11

Fig. 21

Fig. 22

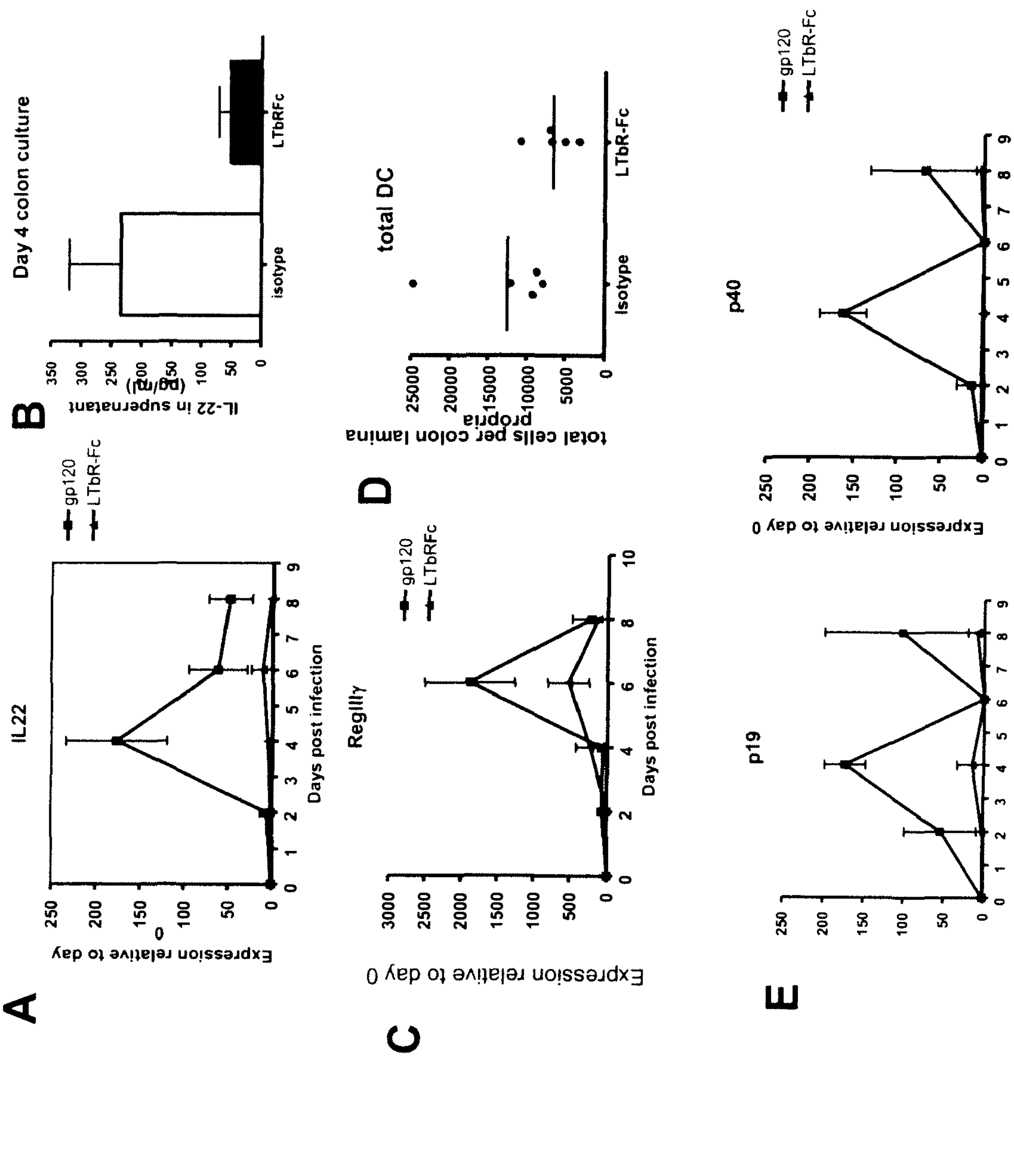
A
IL22
Expression relative to day 0
Days post infection
gp120
LTbR-Fc
B
Day 4 colon culture
IL-22 in supernatant (pg/ml)
isotype
LTbRFc
C
RegIIIγ
Expression relative to day 0
Days post infection
gp120
LTbRFc
D
total DC
total cells per colon lamina propria
Isotype
LTbR-Fc
E
p19
p40
Expression relative to day 0
Days post infection
gp120
LTbR-Fc

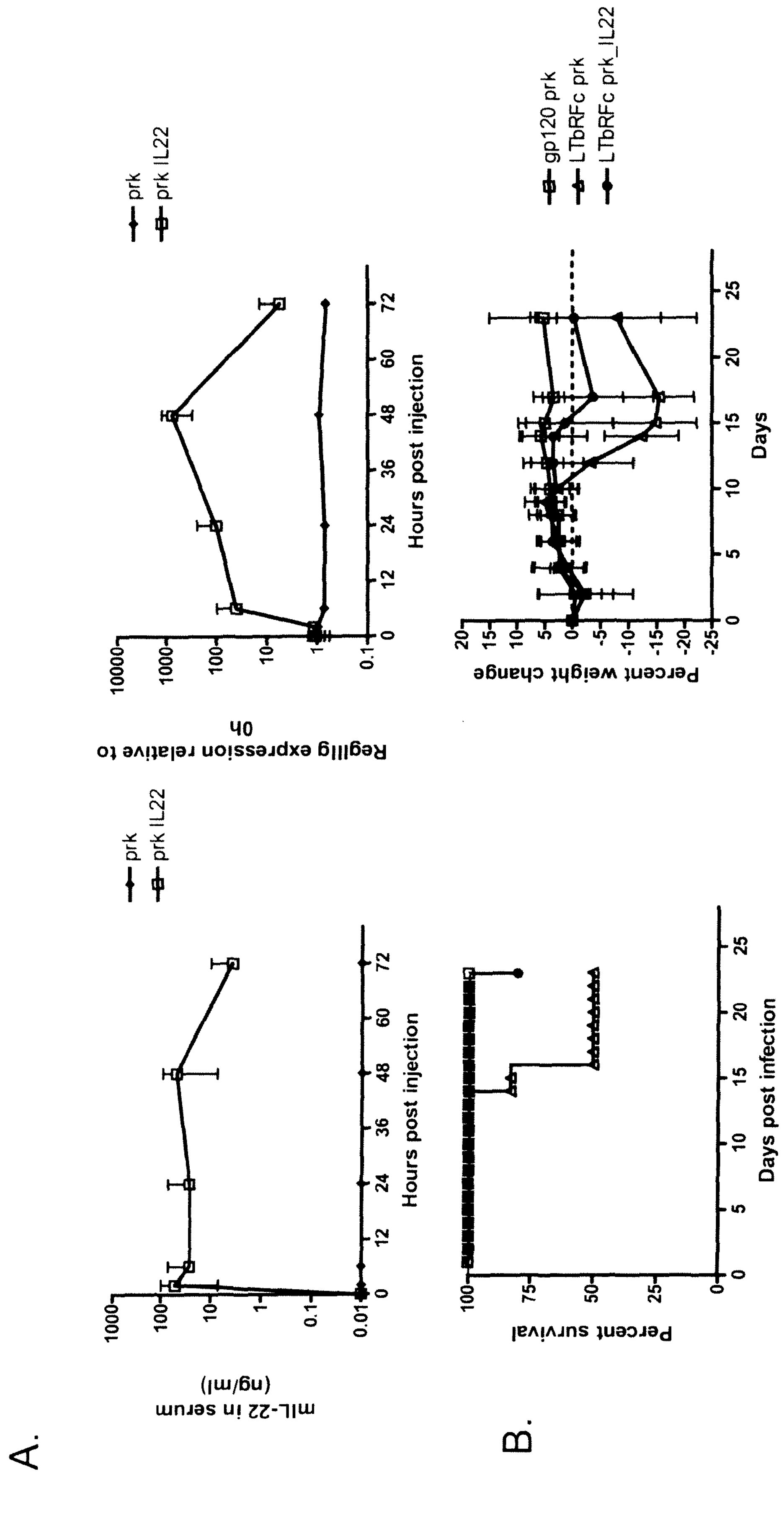
A.
mIL-22 in serum (ng/ml)
1000
100
10
1
0.1
0.01
Hours post injection
0
12
24
36
48
60
72
prk
prk IL22
Reglllg expression relative to 0h
10000
1000
100
10
1
0.1
Hours post injection
prk
prk IL22
B.
Percent survival
100
75
50
25
0
Days post infection
0
5
10
15
20
25
Percent weight change
20
15
10
5
0
-5
-10
-15
-20
-25
Days
gp120 prk
LTbRFc prk
LTbRFc prk_IL22

Fig. 23

Fig. 24

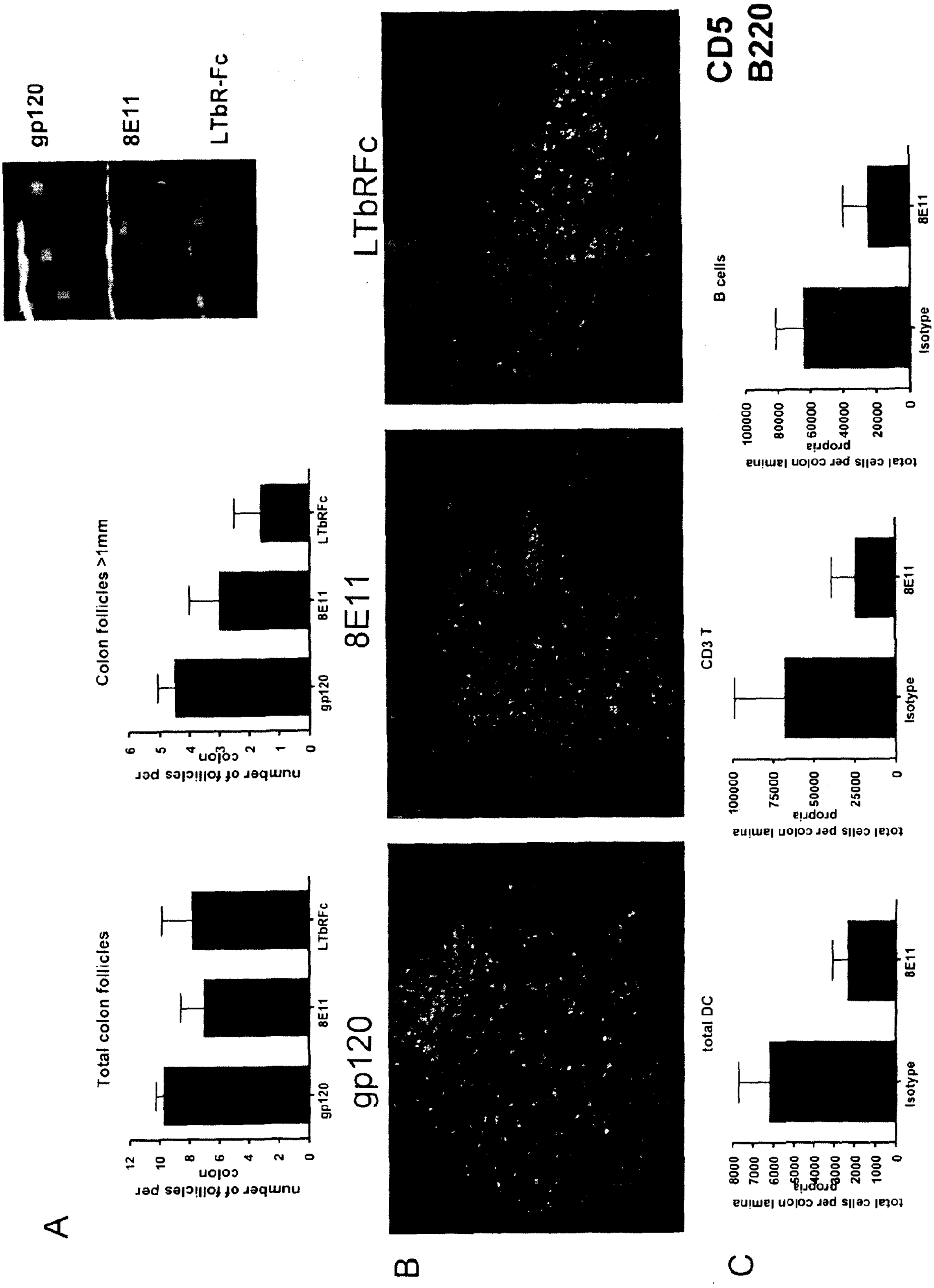
A
Total colon follicles
number of follicles per colon
gp120
8E11
LTbRFc
Colon follicles >1mm
gp120
8E11
LTbR-Fc
B
gp120
8E11
LTbRFc
CD5
B220
C
total DC
CD3 T
B cells
total cells per colon lamina propria
Isotype
8E11

Europäisches Patentamt
European Patent Office
Office européen des brevets

# EUROPEAN SEARCH REPORT

Application Number
EP 17 20 8162

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br>Y | US 2005/277593 A1 (DIECKGRAEFE BRIAN K [US] ET AL) 15 December 2005 (2005-12-15)<br>* abstract *<br>* paragraphs [0005], [0023], [0026], [0028] *<br>* example 1 *<br>* claim 33 *<br>----- | 1,2,4-8<br>1-8 | INV.<br>A61K38/20<br>A61K38/17<br>A61K38/16<br>A61P31/00 |
| X<br>Y | WO 2004/058307 A1 (WYETH CORP [US]; PETERSON RON L [US]; DORNER ANDREW J [US]) 15 July 2004 (2004-07-15)<br>* the whole document *<br>----- | 1,2,4,7<br>1-8 | |
| X<br>Y | WO 2004/003144 A2 (HUMAN GENOME SCIENCES INC [US]; ROSEN CRAIG A [US]) 8 January 2004 (2004-01-08)<br>* the whole document *<br>----- | 1-3,7<br>1-8 | |
| Y | CASH H L ET AL: "Symbiotic bacteria direct expression of an intestinal bactericidal lectin", SCIENCE, AMERICAN ASSOCIATION FOR THE ADVANCEMENT OF SCIENCE, WASHINGTON, DC; US, vol. 313, no. 5790, 25 August 2006 (2006-08-25), pages 1126-1130, XP002540223, ISSN: 0036-8075, DOI: 10.1126/SCIENCE.1127119<br>* page 1126, columns 3-6 *<br>* page 1127, columns 1-3; figure 1 *<br>* page 1128 *<br>* page 1129, column 2 *<br>* page 1130, columns 1,2 *<br>-----<br>-/-- | 1-8 | TECHNICAL FIELDS SEARCHED (IPC)<br>A61K<br>A61P |

1

The present search report has been drawn up for all claims

| Place of search | Date of completion of the search | Examiner |
|---|---|---|
| Munich | 13 June 2018 | Weisser, Dagmar |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 2

Europäisches Patentamt
European Patent Office
Office européen des brevets

# EUROPEAN SEARCH REPORT

Application Number
EP 17 20 8162

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | OGAWA HITOSHI ET AL: "Increased expression of HIP/PAP and regenerating gene III in human inflammatory bowel disease and a murine bacterial reconstitution model.", INFLAMMATORY BOWEL DISEASES MAY 2003, vol. 9, no. 3, May 2003 (2003-05), pages 162-170, XP002688917, ISSN: 1078-0998<br>* abstract *<br>* page 163, columns 3,4 *<br>* page 164, columns 2-4,6 *<br>* page 165, columns 2,3; figure 2 *<br>* page 167, columns 4-7 *<br>* page 169, column 4 *<br>----- | 1-8 | |
| Y | KEILBAUGH S A ET AL: "Activation of RegIII beta/gamma and interferon gamma expression in the intestinal tract of SCID mice: an innate response to bacterial colonisation of the gut", GUT, BRITISH MEDICAL ASSOCIATION, LONDON, UK, vol. 54, 1 January 2005 (2005-01-01), pages 623-629, XP002540221, ISSN: 0017-5749, DOI: 10.1136/GUT.2004.056028<br>* page 624, column 1 *<br>* page 625, columns 1-4 *<br>* page 626, column 2 *<br>* page 628, column 3 *<br>----- | 1-8 | TECHNICAL FIELDS SEARCHED (IPC) |

1

The present search report has been drawn up for all claims

| Place of search | Date of completion of the search | Examiner |
|---|---|---|
| Munich | 13 June 2018 | Weisser, Dagmar |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

page 2 of 2

## ANNEX TO THE EUROPEAN SEARCH REPORT ON EUROPEAN PATENT APPLICATION NO. EP 17 20 8162

This annex lists the patent family members relating to the patent documents cited in the above-mentioned European search report. The members are as contained in the European Patent Office EDP file on
The European Patent Office is in no way liable for these particulars which are merely given for the purpose of information.

13-06-2018

| Patent document cited in search report | Publication date | Patent family member(s) | Publication date |
|---|---|---|---|
| US 2005277593 A1 | 15-12-2005 | NONE | |
| WO 2004058307 A1 | 15-07-2004 | AU 2003301059 A1 | 22-07-2004 |
| | | US 2004176293 A1 | 09-09-2004 |
| | | US 2008206261 A1 | 28-08-2008 |
| | | WO 2004058307 A1 | 15-07-2004 |
| WO 2004003144 A2 | 08-01-2004 | AU 2003256299 A1 | 19-01-2004 |
| | | CA 2490280 A1 | 08-01-2004 |
| | | EP 1539235 A2 | 15-06-2005 |
| | | US 2005158313 A1 | 21-07-2005 |
| | | US 2007036792 A1 | 15-02-2007 |
| | | US 2010240875 A1 | 23-09-2010 |
| | | WO 2004003144 A2 | 08-01-2004 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 20050129614 A
- US 5824509 A
- US 5661004 A
- EP 404097 A
- WO 931161 A
- US 4816567 A
- WO 9824893 A
- WO 9634096 A
- WO 9633735 A
- WO 9110741 A
- US 5545807 A
- US 5545806 A
- US 5569825 A
- US 5625126 A
- US 5633425 A
- US 5661016 A
- US 4275149 A
- US 5364934 A
- US 5428130 A
- US 5869046 A
- WO 9316185 A
- US 5571894 A
- US 5587458 A
- US 5641870 A
- WO 9306213 A
- WO 9308829 A
- WO 9404690 A
- US 4676980 A
- WO 9100360 A
- WO 9200373 A
- EP 03089 A
- US 6248516 B1
- US 20030157108 A, Presta, L.
- US 20040093621 A
- WO 2003011878 A, Jean-Mairet
- US 6602684 B, Umana
- WO 199730087 A, Patel
- WO 199858964 A, Raju, S.
- WO 199922764 A, Raju, S.
- US 20050123546 A, Umana
- WO 200061739 A
- WO 200129246 A
- US 20030115614 A
- US 20020164328 A
- US 20040132140 A
- US 20040110704 A
- US 20040110282 A
- US 20040109865 A
- WO 2003085119 A
- WO 2003084570 A
- WO 2005035586 A
- WO 2005035778 A
- WO 2005053742 A
- US 20030157108 A1, Presta, L
- WO 2004056312 A1, Adams
- WO 9951642 A
- US 5648260 A
- US 5624821 A
- WO 9429351 A
- WO 0042072 A, Presta
- WO 2004056312 A, Lowman
- US 20050014934 A1, Hinton
- US 6194551 B1
- US 5731168 A
- WO 92200373 A
- US 5648237 A, Carter
- US 5840523 A, Simmons
- US 5639635 A
- US 6083715 A, Georgiou
- US 6027888 A, Georgiou
- US 5264365 A, Georgiou
- US 5508192 A, Georgiou
- US 4965199 A
- US 4419446 A
- US 4601978 A
- WO 9411026 A
- US 4767704 A
- US 4657866 A
- US 4927762 A
- US 4560655 A
- US 5122469 A
- WO 9003430 A
- WO 8700195 A
- US RE30985 E
- US 5556762 A
- US 5750373 A
- US 4708871 A
- US 4833092 A
- US 5223409 A
- US 5403484 A
- US 5571689 A
- US 5663143 A
- WO 8403506 A
- WO 8403564 A
- WO 0000823 A
- WO 0039585 A
- WO 9106629 A
- US 4485045 A

- US 4544545 A
- US 5013556 A
- US 3773919 A
- US 4376110 A

**Non-patent literature cited in the description**


- *The World Health Report,* 2004
- *MMWR Morb Mortal Wkly Rep,* 29 September 2006, vol. 55, 1045
- **R. L. SIEGLER.** *Pediatr Clin North Am,* December 1995, vol. 42, 1505
- **T. S. WHITTAM et al.** *Infect Immun,* May 1993, vol. 61, 1619
- **L. ECKMANN.** *Ann NY Acad Sci,* 01 August 2006, vol. 1072, 28
- **D. B. SCHAUER ; S. FALKOW.** *Infect Immun,* June 1993, vol. 61, 2486
- **T. T. MACDONALD ; G. MONTELEONE.** *Science,* 25 March 2005, vol. 307, 1920
- **A. TAKAHASHI et al.** *FEBS Lett,* 23 November 2001, vol. 508, 484
- **L. BRY ; M. B. BRENNER.** *J Immunol,* 01 January 2004, vol. 172, 433
- **Y. ZHENG et al.** *Nature,* 08 February 2007, vol. 445, 648
- **K. I. HAPPEL et al.** *J. Exp. Med.,* 19 September 2005, vol. 202, 761
- **SAMBROOK et al.** Molecular Cloning: A Laboratory Manual. Cold Spring Harbor Laboratory Press, 2001
- Current Protocols in Molecular Biology. 2003
- A Practical Approach. Methods in Enzymology. Academic Press, Inc, 1995
- Antibodies, A Laboratory Manual, and Animal Cell Culture. 1987
- Oligonucleotide Synthesis. 1984
- Methods in Molecular Biology. Humana Press
- Cell Biology: A Laboratory Notebook. Academic Press, 1998
- Animal Cell Culture. 1987
- **J. P. MATHER ; P. E. ROBERTS.** Introduction to Cell and Tissue Culture. Plenum Press, 1998
- Cell and Tissue Culture: Laboratory Procedures. J. Wiley and Sons, 1993
- Handbook of Experimental Immunology
- Gene Transfer Vectors for Mammalian Cells. 1987
- PCR: The Polymerase Chain Reaction. 1994
- Current Protocols in Immunology. 1991
- Short Protocols in Molecular Biology. Wiley and Sons, 1999
- **C. A. JANEWAY ; P. TRAVERS.** Immunobiology. 1997
- **P. FINCH.** Antibodies. 1997
- Antibodies: A Practical Approach. IRL Press, 1988
- Monoclonal Antibodies: A Practical Approach. Oxford University Press, 2000
- Using Antibodies: A Laboratory Manual. Cold Spring Harbor Laboratory Press, 1999
- The Antibodies. Harwood Academic Publishers, 1995
- Cancer: Principles and Practice of Oncology. J.B. Lippincott Company, 1993
- **PENNICA et al.** *Nature,* 1984, vol. 312 (20/27), 724-729
- **AGGARWAL et al.** *J. Biol. Chem.,* 1985, vol. 260, 2345-2354
- **KABAT et al.** Sequences of Proteins of Immunological Interest. National Institute of Health, 1991
- **ABBAS et al.** Cellular and Mol. Immunology. 2000
- **PLUCKTHUN.** The Pharmacology of Monoclonal Antibodies. Springer-Verlag, 1994, vol. 113, 269-315
- **HUDSON et al.** *Nat. Med.,* 2003, vol. 9, 129-134
- **HOLLINGER et al.** *Proc. Natl. Acad. Sci. USA,* 1993, vol. 90, 6444-6448
- **KOHLER et al.** *Nature,* 1975, vol. 256, 495
- **HARLOW et al.** Antibodies: A Laboratory Manual. Cold Spring Harbor Laboratory Press, 1988
- **HAMMERLING et al.** Monoclonal Antibodies and T-Cell Hybridomas. Elsevier, 1981, 563-681
- **CLACKSON et al.** *Nature,* 1991, vol. 352, 624-628
- **MARKS et al.** *J. Mol. Biol.,* 1992, vol. 222, 581-597
- **SIDHU et al.** *J. Mol. Biol.,* 2004, vol. 338 (2), 299-310
- **LEE et al.** *J. Mol. Biol.,* 2004, vol. 340 (5), 1073-1093
- **FELLOUSE.** *Proc. Natl. Acad. Sci. USA,* 2004, vol. 101 (34), 12467-12472
- **LEE et al.** *J. Immunol. Methods,* 2004, vol. 284 (1-2), 119-132
- **JAKOBOVITS et al.** *Proc. Natl. Acad. Sci. USA,* 1993, vol. 90, 2551
- **JAKOBOVITS et al.** *Nature,* 1993, vol. 362, 255-258
- **BRUGGEMANN et al.** *Year in Immunol.,* 1993, vol. 7, 33
- **MARKS et al.** *Bio.Technology,* 1992, vol. 10, 779-783
- **LONBERG et al.** *Nature,* 1994, vol. 368, 856-859
- **MORRISON.** *Nature,* 1994, vol. 368, 812-813
- **FISHWILD et al.** *Nature Biotechnol.,* 1996, vol. 14, 845-851

- **NEUBERGER.** *Nature Biotechnol.,* 1996, vol. 14, 826
- **LONBERG ; HUSZAR.** *Intern. Rev. Immunol.,* 1995, vol. 13, 65-93
- **MORRISON et al.** *Proc. Natl. Acad. Sci. USA,* 1984, vol. 81, 6851-6855
- **JONES et al.** *Nature,* 1986, vol. 321, 522-525
- **RIECHMANN et al.** *Nature,* 1988, vol. 332, 323-329
- **PRESTA.** *Curr. Op. Struct. Biol.,* 1992, vol. 2, 593-596
- **VASWANI ; HAMILTON.** *Ann. Allergy, Asthma & Immunol.,* 1998, vol. 1, 105-115
- **HARRIS.** *Biochem. Soc. Transactions,* 1995, vol. 23, 1035-1038
- **HURLE ; GROSS.** *Curr. Op. Biotech.,* 1994, vol. 5, 428-433
- **MARKS et al.** *Bio/Technology,* 1992, vol. 10, 779-783
- **BARBAS et al.** *Proc Nat. Acad. Sci. USA,* 1994, vol. 91, 3809-3813
- **SCHIER et al.** *Gene,* 1995, vol. 169, 147-155
- **YELTON et al.** *J. Immunol.,* 1995, vol. 155, 1994-2004
- **JACKSON et al.** *J. Immunol.,* 1995, vol. 154 (7), 3310-9
- **HAWKINS et al.** *J. Mol. Biol.,* 1992, vol. 226, 889-896
- **CHEN et al.** *J. Mol. Biol.,* 1999, vol. 293, 865-881
- **PRESTA et al.** *Cancer Res.,* 1997, vol. 57, 4593-4599
- **CHEN, Y. et al.** *J. Mol. Biol.,* 1999, vol. 293, 865-881
- Cell cycle regulation, oncogens, and antineoplastic drugs. **MURAKAMI et al.** The Molecular Basis of Cancer. WB Saunders, 1995, 13
- **CARTER et al.** *Nucl. Acids Res.,* 1986, vol. 13, 4331
- **ZOLLER et al.** *Nucl. Acids Res.,* 1987, vol. 10, 6487
- **WELLS et al.** *Gene,* 1985, vol. 34, 315
- **WELLS et al.** *Philos. Trans. R. Soc. London SerA,* 1986, vol. 317, 415
- **T.E. CREIGHTON.** Proteins: Structure and Molecular Properties. W.H. Freeman & Co, 1983, 79-86
- **FIELD et al.** *Mol. Cell. Biol.,* 1988, vol. 8, 2159-2165
- **EVAN et al.** *Molecular and Cellular Biology,* 1985, vol. 5, 3610-3616
- **PABORSKY et al.** *Protein Engineering,* 1990, vol. 3 (6), 547-553
- **HOPP et al.** *BioTechnology,* 1988, vol. 6, 1204-1210
- **MARTIN et al.** *Science,* 1992, vol. 255, 192-194
- **SKINNER et al.** *J. Biol. Chem.,* 1991, vol. 266, 15163-15166
- **LUTZ-FREYERMUTH et al.** *Proc. Natl. Acad. Sci. USA,* 1990, vol. 87, 6393-6397
- **STEWART et al.** Solid-Phase Peptide Synthesis. W.H. Freeman Co, 1969
- **MERRIFIELD.** *J. Am. Chem. Soc.,* 1963, vol. 85, 2149-2154
- **DEUTSCHER.** *Methods in Enzymology,* 1990, vol. 182
- **SCOPES.** Protein Purification: Principles and Practice. Springer-Verlag, 1982
- **THOMAS.** *Proc. Natl. Acad. Sci. USA,* 1980, vol. 77, 5201-5205
- **KOHLER ; MILSTEIN.** *Nature,* 1975, vol. 256, 495
- **GODING.** Monoclonal Antibodies: Principles and Practice. Academic Press, 1986, 59-103
- **KOZBOR.** *J. Immunol.,* 1984, vol. 133, 3001
- **BRODEUR et al.** Monoclonal Antibody Production Techniques and Applications. Marcel Dekker, Inc, 1987, 51-63
- **MUNSON ; POLLARD.** *Anal. Biochem.,* 1980, vol. 107, 220
- **HOOGENBOOM et al.** Methods in Molecular Biology. Human Press, 2001, vol. 178, 1-37
- **LEE et al.** *J. Mol. Biol.,* 2004, vol. 340, 1073-1093
- **KABAT et al.** Sequences of Proteins of Immunological Interest. NIH, 1991, vol. 1-3
- **MORIMOTO et al.** *Journal of Biochemical and Biophysical Methods,* 1992, vol. 24, 107-117
- **BRENNAN et al.** *Science,* 1985, vol. 229, 81
- **CARTER et al.** *Bio/Technology,* 1992, vol. 10, 163-167
- Antibody Engineering
- **RIECHMANN et al.** *Nature,* 1988, vol. 332, 323-327
- **VERHOEYEN et al.** *Science,* 1988, vol. 239, 1534-1536
- **SIMS et al.** *J. Immunol.,* 1993, vol. 151, 2296
- **CHOTHIA et al.** *J. Mol. Biol.,* 1987, vol. 196, 901
- **CARTER et al.** *Proc. Natl. Acad. Sci. USA,* 1992, vol. 89, 4285
- **PRESTA.** *J. Immunol.,* 1993, vol. 151, 2623
- **BOERNER et al.** *J. Immunol.,* 1991, vol. 147, 86
- **JAKOBOVITS et al.** *Proc. Natl. Acad. Sci USA,* 1993, vol. 90, 2551
- **JAKOBOVITS et al.** *Nature,* 1993, vol. 362, 255
- **BRUGGERMANN et al.** *Year in Immunol.,* 1993, vol. 7, 33
- **MILSTEIN ; CUELLO.** *Nature,* 1983, vol. 305, 537

- **TRAUNECKER et al.** *EMBO J.,* 1991, vol. 10, 3655
- **SURESH et al.** *Methods in Enzymology,* 1986, vol. 121, 210
- **SHALABY et al.** *J. Exp. Med.,* 1992, vol. 175, 217-225
- **KOSTELNY et al.** *J. Immunol.,* 1992, vol. 148 (5), 1547-1 553
- **GRUBER et al.** *J. Immunol.,* 1994, vol. 152, 5368
- **TUTT et al.** *J. Immunol.,* 1991, vol. 147, 60
- **CUNNINGHAM ; WELLS.** *Science,* 1989, vol. 244, 1081-1085
- **OKAZAKI et al.** *J. Mol. Biol.,* 2004, vol. 336, 1239-1249
- **YAMANE-OHNUKI et al.** *Biotech. Bioeng,* 2004, vol. 87, 614
- **RIPKA et al.** *Arch. Biochem. Biophys.,* 1986, vol. 249, 533-545
- **YAMANE-OHNUKI et al.** *Biotech. Bioeng.,* 2004, vol. 87, 614
- **DUNCAN ; WINTER.** *Nature,* 1988, vol. 322, 738-40
- **SHIELDS et al.** *J. Biol. Chem.,* 2001, vol. 9 (2), 6591-6604
- **GUYER et al.** *J. Immunol.,* 1976, vol. 1 17, 587
- **KIM et al.** *J. Immunol.,* 1994, vol. 24, 249
- **IDUSOGIE et al.** *J Immunol.,* 2000, vol. 164, 4178-4184
- **KAM et al.** *Proc. Natl. Acad. Sci.,* 2005, vol. 102, 11600-11605
- **SIEBENLIST et al.** *Cell,* 1980, vol. 20, 269
- **PROBA ; PLUCKTHUN.** *Gene,* 1995, vol. 159, 203
- **YANSURA et al.** *METHODS: A Companion to Methods in Enzymol.,* 1992, vol. 4, 151-158
- **BACHMANN.** Cellular and Molecular Biology. American Society for Microbiology, 1987, vol. 2, 1190-1219
- **BASS et al.** *Proteins,* 1990, vol. 8, 309-314
- **SIMMONS et al.** *J. Immunol. Methods,* 2002, vol. 263, 133-147
- **CHEN et al.** *J. Biol. Chem.,* 1999, vol. 274, 19601-19605
- **BOTHMANN ; PLUCKTHUN.** *J. Biol. Chem.,* 2000, vol. 275, 17100-17105
- **RAMM ; PLUCKTHUN.** *J. Biol. Chem.,* 2000, vol. 275, 17106-171 13
- **ARIE et al.** *Mol. Microbiol.,* 2001, vol. 39, 199-210
- **HARA et al.** *Microbial Drug Resistance,* 1996, vol. 2, 63-72
- **LINDMARK et al.** *J. Immunol. Meth.,* 1983, vol. 62, 1-13
- **REYES et al.** *Nature,* 1982, vol. 297, 598-601
- **YANIV.** *Nature,* 1982, vol. 297, 17-18
- **GRAHAM et al.** *J. Gen Virol.,* 1977, vol. 36, 59
- **URLAUB et al.** *Proc. Natl. Acad. Sci. USA,* 1980, vol. 77, 4216
- **MATHER.** *Biol. Reprod.,* 1980, vol. 23, 243-251
- **MATHER et al.** *Annals N.Y. Acad. Sci,* 1982, vol. 383, 44-68
- **HAM et al.** *Meth. Enz.,* 1979, vol. 58, 44
- **BARNES et al.** *Anal. Biochem.,* 1980, vol. 102, 255
- **LINDMARK et al.** *J. Immunol. Methods,* 1983, vol. 62, 1-13
- **GUSS et al.** *EMBO J.,* 1986, vol. 5, 15671575
- **GEYSEN et al.** *Proc. Natl. Acad. Sci. U.S.A.,* 1984, vol. 81, 3998-4002
- **GEYSEN et al.** *Proc. Natl. Acad. Sci. USA,* 1985, vol. 82, 178-182
- **GEYSEN et al.** *Synthetic Peptides as Antigens,* 1986, 130-149
- **GEYSEN et al.** *J. Immunol. Meth.,* 1987, vol. 102, 259-274
- **SCHOOFS et al.** *J. Immunol.,* 1988, vol. 140, 611-616
- **CWIRLA, S. E. et al.** *Proc. Natl. Acad. Sci. USA,* 1990, vol. 87, 6378
- **LOWMAN, H.B. et al.** *Biochemistry,* 1991, vol. 30, 10832
- **CLACKSON, T. et al.** *Nature,* 1991, vol. 352, 624
- **MARKS, J. D. et al.** *J. Mol. Biol.,* 1991, vol. 222, 581
- **KANG, A.S. et al.** *Proc. Natl. Acad. Sci. USA,* 1991, vol. 88, 8363
- **SMITH, G. P.** *Current Opin. Biotechnol.,* 1991, vol. 2, 668
- **DUMOUTIER L. et al.** Cloning and characterization of IL-22 binding protein, a natural antagonist of IL-10-related T cell-derived inducible factor/IL-22. *J. Immunol.,* 2001, vol. 166, 7090-7095
- **XU W. et al.** A soluble class 11 cytokine receptor, IL-22RA2, is a naturally occurring IL-22 antagonist. *Proc. Natl. Acad. Sci. U.S.A.,* 2001, vol. 98, 9511-9516
- **NOVINA et al.** *Nature,* 2004, vol. 430, 161-164
- Remington's Pharmaceutical Sciences. 1980
- **MARASCO et al.** *Proc. Natl. Acad. Sci. USA,* 1993, vol. 90, 7889-7893
- **EPSTEIN et al.** *Proc. Natl. Acad. Sci. USA,* 1985, vol. 82, 3688
- **HWANG et al.** *Proc. Natl Acad. Sci. USA,* 1980, vol. 77, 4030
- **MARTIN et al.** *J. Biol. Chem.,* 1982, vol. 257, 286-288
- **GABIZON et al.** *J. National Cancer Inst.,* 1989, vol. 81 (19), 1484
- **H. JR. ; SACHS, D. H.** Fundamental Immunology. Raven Press, 1989, 889-992

- **TANABE, M. et al.** *Transplantation,* 1994, vol. 58, 23
- **TINUBU, S. A. et al.** *J. Immunol.,* 1994, 4330-4338
- Current Protocols in Immunology. John Wiley & Sons, Inc, 1994
- **GRABBE, S. ; SCHWARZ, T.** *Immun. Today,* 1998, vol. 19 (1), 37-44
- **SCHON, M. P. et al.** *Nat. Med.,* 1997, vol. 3, 183
- **NICKOLOFF, B. J. et al.** *Am. J. Path.,* 1995, vol. 146, 580
- **BOYMAN et al.** *J Exp Med.,* 2004, vol. 199 (5), 731-6
- **THOMAS ; CAPECCHI.** *Cell,* 1987, vol. 51, 503
- **LI et al.** *Cell,* 1992, vol. 69, 915
- **BRADLEY.** Teratocarcinomas and Embryonic Stem Cells: A Practical Approach. IRL, 1987, 113-152
- **FIELDS ; SONG.** *Nature,* 1989, vol. 340, 245-246
- **CHIEN et al.** *Proc. Natl. Acad. Sci. USA,* 1991, vol. 88, 9578-9582
- **CHEVRAY ; NATHANS.** *Proc. Natl. Acad. Sci. USA,* 1991, vol. 89, 5789-5793
- **ZOLA.** Monoclonal Antibodies: A Manual of Techniques. CRC Press, Inc, 1987, 147-158
- **D. TOY et al.** *J Immunol,* 01 July 2006, vol. 177, 36
- **J. WITOWSKI ; K. KSIAZEK ; A. JORRES.** *Cell Mol Life Sci,* March 2004, vol. 61, 567
- **S. M. SA et al.** *J Immunol,* 15 February 2007, vol. 178, 2229
- **J.-C. RENAULD.** *Nature Reviews Immunology,* 2003, vol. 3, 667
- **C. MAASER et al.** *Infect. Immun.,* 01 June 2004, vol. 72, 3315
- **S. LECART et al.** *Int. Immunol.,* 01 November 2002, vol. 14, 1351
- **T. GANZ.** *Science,* 15 October 1999, vol. 286, 420
- **H. L. CASH ; C. V. WHITHAM ; L. V. HOOPER.** *Protein Expression and Purification,* 2006, vol. 48, 151
- **S. A. KEILBAUGH et al.** *Gut,* 01 May 2005, vol. 54, 623
- **H. OGAWA et al.** *Inflammatory Bowel Diseases,* 2003, vol. 9, 162
- **H. OGAWA ; K. FUKUSHIMA ; I. SASAKI ; S. MATSUNO.** *Am J Physiol Gastrointest Liver Physiol,* September 2000, vol. 279, G492
- **S. L. PULL ; J. M. DOHERTY ; J. C. MILLS ; J. I. GORDON ; T. S. STAPPENBECK.** *PNAS,* 04 January 2005, vol. 102, 99
- **V. MOUCADEL et al.** *Eur J Cell Biol,* February 2001, vol. 80, 156

SEQUENCE LISTING

<110> Genentech, Inc.

<120> COMPOSITIONS AND METHODS FOR TREATMENT OF MICROBIAL DISORDERS

<130> SMW/FP7296189

<140> EP
<141> 2008-11-07

<150> EP12169572.0
<151> 2008-11-07

<150> EP08848478.7
<151> 2008-11-07

<150> PCT/US2008/082890
<151> 2008-11-07

<150> US60/986,170
<151> 2007-11-07

<150> US61/013,620
<151> 2007-12-13

<150> US61/015,620
<151> 2007-12-20

<160> 118

<170> PatentIn version 3.5

<210> 1
<211> 1131
<212> DNA
<213> Homo sapiens

<400> 1
cattctgccc tcgagcccac cgggaacgaa agagaagctc tatctcccct ccaggagccc 60

agctatgaac tccttctcca caagcgcctt cggtccagtt gccttctccc tggggctgct 120

cctggtgttg cctgctgcct tccctgcccc agtaccccca ggagaagatt ccaaagatgt 180

agccgcccca cacagacagc cactcacctc ttcagaacga attgacaaac aaattcggta 240

catcctcgac ggcatctcag ccctgagaaa ggagacatgt aacaagagta acatgtgtga 300

aagcagcaaa gaggcactgg cagaaaacaa cctgaacctt ccaaagatgg ctgaaaaaga 360

tggatgcttc caatctggat tcaatgagga gacttgcctg gtgaaaatca tcactggtct 420

tttggagttt gaggtatacc tagagtacct ccagaacaga tttgagagta gtgaggaaca 480

agccagagct gtgcagatga gtacaaaagt cctgatccag ttcctgcaga aaaaggcaaa 540

gaatctagat gcaataacca cccctgaccc aaccacaaat gccagcctgc tgacgaagct 600

gcaggcacag aaccagtggc tgcaggacat gacaactcat ctcattctgc gcagctttaa 660

ggagttcctg cagtccagcc tgagggctct tcggcaaatg tagcatgggc acctcagatt 720

gttgttgtta atgggcattc cttcttctgg tcagaaacct gtccactggg cacagaactt 780

atgttgttct ctatggagaa ctaaaagtat gagcgttagg acactatttt aattattttt 840

aatttattaa tatttaaata tgtgaagctg agttaattta tgtaagtcat atttatattt 900

ttaagaagta ccacttgaaa cattttatgt attagttttg aaataataat ggaaagtggc 960

tatgcagttt gaatatcctt tgtttcagag ccagatcatt tcttggaaag tgtaggctta 1020

cctcaaataa atggctaact tatacatatt tttaaagaaa tatttatatt gtatttatat 1080

aatgtataaa tggtttttat accaataaat ggcattttaa aaaattcagc a 1131

<210> 2
<211> 212
<212> PRT
<213> Homo sapiens

<400> 2
Met Asn Ser Phe Ser Thr Ser Ala Phe Gly Pro Val Ala Phe Ser Leu
1 5 10 15

Gly Leu Leu Leu Val Leu Pro Ala Ala Phe Pro Ala Pro Val Pro Pro
20 25 30

Gly Glu Asp Ser Lys Asp Val Ala Ala Pro His Arg Gln Pro Leu Thr
35 40 45

Ser Ser Glu Arg Ile Asp Lys Gln Ile Arg Tyr Ile Leu Asp Gly Ile
50 55 60

Ser Ala Leu Arg Lys Glu Thr Cys Asn Lys Ser Asn Met Cys Glu Ser
65 70 75 80

Ser Lys Glu Ala Leu Ala Glu Asn Asn Leu Asn Leu Pro Lys Met Ala
85 90 95

Glu Lys Asp Gly Cys Phe Gln Ser Gly Phe Asn Glu Glu Thr Cys Leu
100 105 110

Val Lys Ile Ile Thr Gly Leu Leu Glu Phe Glu Val Tyr Leu Glu Tyr
115 120 125

Leu Gln Asn Arg Phe Glu Ser Ser Glu Glu Gln Ala Arg Ala Val Gln
130 135 140

Met Ser Thr Lys Val Leu Ile Gln Phe Leu Gln Lys Lys Ala Lys Asn
145 150 155 160

Leu Asp Ala Ile Thr Thr Pro Asp Pro Thr Thr Asn Ala Ser Leu Leu
165 170 175

```
Thr Lys Leu Gln Ala Gln Asn Gln Trp Leu Gln Asp Met Thr Thr His
            180                 185                 190

Leu Ile Leu Arg Ser Phe Lys Glu Phe Leu Gln Ser Ser Leu Arg Ala
        195                 200                 205

Leu Arg Gln Met
    210
```

<210> 3
<211> 2347
<212> DNA
<213> Homo sapiens

<400> 3

```
ctgtttcagg gccattggac tctccgtcct gcccagagca agatgtgtca ccagcagttg      60
gtcatctctt ggttttccct ggtttttctg gcatctcccc tcgtggccat atgggaactg     120
aagaaagatg tttatgtcgt agaattggat tggtatccgg atgcccctgg agaaatggtg     180
gtcctcacct gtgacacccc tgaagaagat ggtatcacct ggaccttgga ccagagcagt     240
gaggtcttag gctctggcaa aaccctgacc atccaagtca aagagtttgg agatgctggc     300
cagtacacct gtcacaaagg aggcgaggtt ctaagccatt cgctcctgct gcttcacaaa     360
aaggaagatg gaatttggtc cactgatatt ttaaaggacc agaaagaacc caaaaataag     420
acctttctaa gatgcgaggc caagaattat tctggacgtt tcacctgctg gtggctgacg     480
acaatcagta ctgatttgac attcagtgtc aaaagcagca gaggctcttc tgacccccaa     540
ggggtgacgt gcggagctgc tacactctct gcagagagag tcagagggga caacaaggag     600
tatgagtact cagtggagtg ccaggaggac agtgcctgcc cagctgctga ggagagtctg     660
cccattgagg tcatggtgga tgccgttcac aagctcaagt atgaaaacta caccagcagc     720
ttcttcatca gggacatcat caaacctgac ccacccaaga acttgcagct gaagccatta     780
aagaattctc ggcaggtgga ggtcagctgg gagtaccctg acacctggag tactccacat     840
tcctacttct ccctgacatt ctgcgttcag gtccagggca agagcaagag agaaaagaaa     900
gatagagtct tcacggacaa gacctcagcc acggtcatct gccgcaaaaa tgccagcatt     960
agcgtgcggg cccaggaccg ctactatagc tcatcttgga gcgaatgggc atctgtgccc    1020
tgcagttagg ttctgatcca ggatgaaaat ttggaggaaa agtggaagat attaagcaaa    1080
atgtttaaag acacaacgga atagacccaa aaagataatt tctatctgat ttgctttaaa    1140
acgttttttt aggatcacaa tgatatcttt gctgtatttg tatagttaga tgctaaatgc    1200
tcattgaaac aatcagctaa tttatgtata gattttccag ctctcaagtt gccatgggcc    1260
ttcatgctat ttaaatattt aagtaattta tgtatttatt agtatattac tgttatttaa    1320
```

```
cgtttgtctg ccaggatgta tggaatgttt catactctta tgacctgatc catcaggatc     1380
agtccctatt atgcaaaatg tgaatttaat tttatttgta ctgacaactt ttcaagcaag     1440
gctgcaagta catcagtttt atgacaatca ggaagaatgc agtgttctga taccagtgcc     1500
atcatacact tgtgatggat gggaacgcaa gagatactta catggaaacc tgacaatgca     1560
aacctgttga gaagatccag gagaacaaga tgctagttcc catgtctgtg aagacttcct     1620
ggagatggtg ttgataaagc aatttagggc cacttacact tctaagcaag tttaatcttt     1680
ggatgcctga attttaaaag ggctagaaaa aaatgattga ccagcctggg aaacataaca     1740
agaccccgtc tctacaaaaa aaatttaaaa ttagccaggc gtggtggctc atgcttgtgg     1800
tcccagctgt tcaggaggat gaggcaggag gatctcttga gcccaggagg tcaaggctat     1860
ggtgagccgt gattgtgcca ctgcatacca gcctaggtga cagaatgaga ccctgtctca     1920
aaaaaaaaaa tgattgaaat taaaattcag ctttagcttc catggcagtc ctcacccccca     1980
cctctctaaa agacacagga ggatgacaca gaaacaccgt aagtgtctgg aaggcaaaaa     2040
gatcttaaga ttcaagagag aggacaagta gttatggcta aggacatgaa attgtcagaa     2100
tggcaggtgg cttcttaaca gccctgtgag aagcagacag atgcaaagaa aatctggaat     2160
ccctttctca ttagcatgaa tgaacctgat acacaattat gaccagaaaa tatggctcca     2220
tgaaggtgct acttttaagt aatgtatgtg cgctctgtaa agtgattaca tttgtttcct     2280
gtttgtttat ttatttattt atttttgcat tctgaggctg aactaataaa aactcttctt     2340
tgtaatc                                                               2347
```

```
<210> 4
<211> 328
<212> PRT
<213> Homo sapiens

<400> 4
Met Cys His Gln Gln Leu Val Ile Ser Trp Phe Ser Leu Val Phe Leu
1               5                   10                  15

Ala Ser Pro Leu Val Ala Ile Trp Glu Leu Lys Lys Asp Val Tyr Val
            20                  25                  30

Val Glu Leu Asp Trp Tyr Pro Asp Ala Pro Gly Glu Met Val Val Leu
        35                  40                  45

Thr Cys Asp Thr Pro Glu Glu Asp Gly Ile Thr Trp Thr Leu Asp Gln
    50                  55                  60

Ser Ser Glu Val Leu Gly Ser Gly Lys Thr Leu Thr Ile Gln Val Lys
65                  70                  75                  80
```

```
Glu Phe Gly Asp Ala Gly Gln Tyr Thr Cys His Lys Gly Gly Glu Val
                85                  90                  95

Leu Ser His Ser Leu Leu Leu Leu His Lys Lys Glu Asp Gly Ile Trp
            100                 105                 110

Ser Thr Asp Ile Leu Lys Asp Gln Lys Glu Pro Lys Asn Lys Thr Phe
        115                 120                 125

Leu Arg Cys Glu Ala Lys Asn Tyr Ser Gly Arg Phe Thr Cys Trp Trp
    130                 135                 140

Leu Thr Thr Ile Ser Thr Asp Leu Thr Phe Ser Val Lys Ser Ser Arg
145                 150                 155                 160

Gly Ser Ser Asp Pro Gln Gly Val Thr Cys Gly Ala Ala Thr Leu Ser
                165                 170                 175

Ala Glu Arg Val Arg Gly Asp Asn Lys Glu Tyr Glu Tyr Ser Val Glu
            180                 185                 190

Cys Gln Glu Asp Ser Ala Cys Pro Ala Ala Glu Glu Ser Leu Pro Ile
        195                 200                 205

Glu Val Met Val Asp Ala Val His Lys Leu Lys Tyr Glu Asn Tyr Thr
    210                 215                 220

Ser Ser Phe Phe Ile Arg Asp Ile Ile Lys Pro Asp Pro Pro Lys Asn
225                 230                 235                 240

Leu Gln Leu Lys Pro Leu Lys Asn Ser Arg Gln Val Glu Val Ser Trp
                245                 250                 255

Glu Tyr Pro Asp Thr Trp Ser Thr Pro His Ser Tyr Phe Ser Leu Thr
            260                 265                 270

Phe Cys Val Gln Val Gln Gly Lys Ser Lys Arg Glu Lys Lys Asp Arg
        275                 280                 285

Val Phe Thr Asp Lys Thr Ser Ala Thr Val Ile Cys Arg Lys Asn Ala
    290                 295                 300

Ser Ile Ser Val Arg Ala Gln Asp Arg Tyr Tyr Ser Ser Ser Trp Ser
305                 310                 315                 320

Glu Trp Ala Ser Val Pro Cys Ser
                325
```

<210> 5
<211> 1145
<212> DNA
<213> Homo sapiens

<400> 5
attctctccc cagcttgctg agccctttgc tcccctggcg actgcctgga cagtcagcaa 60

ggaattgtct cccagtgcat tttgccctcc tggctgccaa ctctggctgc taaagcggct 120

gccacctgct gcagtctaca cagcttcggg aagaggaaag gaacctcaga ccttccagat 180

cgcttcctct cgcaacaaac tatttgtcgc aggaataaag atggctgctg aaccagtaga 240

agacaattgc atcaactttg tggcaatgaa atttattgac aatacgcttt actttatagc 300

tgaagatgat gaaaacctgg aatcagatta ctttggcaag cttgaatcta aattatcagt 360

cataagaaat ttgaatgacc aagttctctt cattgaccaa ggaaatcggc ctctatttga 420

agatatgact gattctgact gtagagataa tgcaccccgg accatattta ttataagtat 480

gtataaagat agccagccta gaggtatggc tgtaactatc tctgtgaagt gtgagaaaat 540

ttcaactctc tcctgtgaga acaaaattat ttcctttaag gaaatgaatc ctcctgataa 600

catcaaggat acaaaaagtg acatcatatt ctttcagaga agtgtcccag gacatgataa 660

taagatgcaa tttgaatctt catcatacga aggatacttt ctagcttgtg aaaaagagag 720

agaccttttt aaactcattt tgaaaaaaga ggatgaattg ggggatagat ctataatgtt 780

cactgttcaa aacgaagact agctattaaa atttcatgcc gggcgcagtg gctcacgcct 840

gtaatcccag ccctttggga ggctgaggcg ggcagatcac cagaggtcag gtgttcaaga 900

ccagcctgac caacatggtg aaacctcatc tctactaaaa atacaaaaaa ttagctgagt 960

gtagtgacgc atgccctcaa tcccagctac tcaagaggct gaggcaggag aatcacttgc 1020

actccggagg tagaggttgt ggtgagccga gattgcacca ttgcgctcta gcctgggcaa 1080

caacagcaaa actccatctc aaaaaataaa ataaataaat aaacaaataa aaaattcata 1140

atgtg 1145

<210> 6
<211> 193
<212> PRT
<213> Homo sapiens

<400> 6
Met Ala Ala Glu Pro Val Glu Asp Asn Cys Ile Asn Phe Val Ala Met
1 5 10 15

Lys Phe Ile Asp Asn Thr Leu Tyr Phe Ile Ala Glu Asp Asp Glu Asn
20 25 30

```
Leu Glu Ser Asp Tyr Phe Gly Lys Leu Glu Ser Lys Leu Ser Val Ile
        35                  40                  45

Arg Asn Leu Asn Asp Gln Val Leu Phe Ile Asp Gln Gly Asn Arg Pro
    50                  55                  60

Leu Phe Glu Asp Met Thr Asp Ser Asp Cys Arg Asp Asn Ala Pro Arg
65                  70                  75                  80

Thr Ile Phe Ile Ile Ser Met Tyr Lys Asp Ser Gln Pro Arg Gly Met
                85                  90                  95

Ala Val Thr Ile Ser Val Lys Cys Glu Lys Ile Ser Thr Leu Ser Cys
            100                 105                 110

Glu Asn Lys Ile Ile Ser Phe Lys Glu Met Asn Pro Pro Asp Asn Ile
        115                 120                 125

Lys Asp Thr Lys Ser Asp Ile Ile Phe Phe Gln Arg Ser Val Pro Gly
    130                 135                 140

His Asp Asn Lys Met Gln Phe Glu Ser Ser Ser Tyr Glu Gly Tyr Phe
145                 150                 155                 160

Leu Ala Cys Glu Lys Glu Arg Asp Leu Phe Lys Leu Ile Leu Lys Lys
                165                 170                 175

Glu Asp Glu Leu Gly Asp Arg Ser Ile Met Phe Thr Val Gln Asn Glu
            180                 185                 190

Asp


<210> 7
<211> 1147
<212> DNA
<213> Homo sapiens

<400> 7
cgaccaggtt ctccttcccc agtcaccagt tgctcgagtt agaattgtct gcaatggccg        60

ccctgcagaa atctgtgagc tctttcctta tggggaccct ggccaccagc tgcctccttc       120

tcttggccct cttggtacag ggaggagcag ctgcgcccat cagctcccac tgcaggcttg       180

acaagtccaa cttccagcag ccctatatca ccaaccgcac cttcatgctg gctaaggagg       240

ctagcttggc tgataacaac acagacgttc gtctcattgg ggagaaactg ttccacggag       300

tcagtatgag tgagcgctgc tatctgatga agcaggtgct gaacttcacc cttgaagaag       360

tgctgttccc tcaatctgat aggttccagc cttatatgca ggaggtggtg cccttcctgg       420
```

```
ccaggctcag caacaggcta agcacatgtc atattgaagg tgatgacctg catatccaga      480

ggaatgtgca aaagctgaag gacacagtga aaaagcttgg agagagtgga gagatcaaag      540

caattggaga actggatttg ctgtttatgt ctctgagaaa tgcctgcatt tgaccagagc      600

aaagctgaaa aatgaataac taaccccctt tccctgctag aaataacaat tagatgcccc      660

aaagcgattt tttttaacca aaaggaagat gggaagccaa actccatcat gatgggtgga      720

ttccaaatga acccctgcgt tagttacaaa ggaaaccaat gccacttttg tttataagac      780

cagaaggtag actttctaag catagatatt tattgataac atttcattgt aactggtgtt      840

ctatacacag aaaacaattt attttttaaa taattgtctt tttccataaa aaagattact      900

ttccattcct ttaggggaaa aaacccctaa atagcttcat gtttccataa tcagtacttt      960

atatttataa atgtatttat tattattata agactgcatt ttatttatat cattttatta     1020

atatggattt atttatagaa acatcattcg atattgctac ttgagtgtaa ggctaatatt     1080

gatatttatg acaataatta tagagctata acatgtttat ttgacctcaa taaacacttg     1140

gatatcc                                                                1147


<210> 8
<211> 179
<212> PRT
<213> Homo sapiens

<400> 8
Met Ala Ala Leu Gln Lys Ser Val Ser Ser Phe Leu Met Gly Thr Leu
1               5                   10                  15


Ala Thr Ser Cys Leu Leu Leu Leu Ala Leu Leu Val Gln Gly Gly Ala
            20                  25                  30


Ala Ala Pro Ile Ser Ser His Cys Arg Leu Asp Lys Ser Asn Phe Gln
        35                  40                  45


Gln Pro Tyr Ile Thr Asn Arg Thr Phe Met Leu Ala Lys Glu Ala Ser
    50                  55                  60


Leu Ala Asp Asn Asn Thr Asp Val Arg Leu Ile Gly Glu Lys Leu Phe
65                  70                  75                  80


His Gly Val Ser Met Ser Glu Arg Cys Tyr Leu Met Lys Gln Val Leu
                85                  90                  95


Asn Phe Thr Leu Glu Glu Val Leu Phe Pro Gln Ser Asp Arg Phe Gln
            100                 105                 110


Pro Tyr Met Gln Glu Val Val Pro Phe Leu Ala Arg Leu Ser Asn Arg
```

115 120 125

Leu Ser Thr Cys His Ile Glu Gly Asp Asp Leu His Ile Gln Arg Asn
130 135 140

Val Gln Lys Leu Lys Asp Thr Val Lys Lys Leu Gly Glu Ser Gly Glu
145 150 155 160

Ile Lys Ala Ile Gly Glu Leu Asp Leu Leu Phe Met Ser Leu Arg Asn
165 170 175

Ala Cys Ile

<210> 9
<211> 1049
<212> DNA
<213> Homo sapiens

<400> 9
aaaacaacag gaagcagctt acaaactcgg tgaacaactg agggaaccaa accagagacg 60

cgctgaacag agagaatcag gctcaaagca agtggaagtg ggcagagatt ccaccaggac 120

tggtgcaagg cgcagagcca gccagatttg agaagaaggc aaaaagatgc tggggagcag 180

agctgtaatg ctgctgttgc tgctgccctg gacagctcag ggcagagctg tgcctggggg 240

cagcagccct gcctggactc agtgccagca gctttcacag aagctctgca cactggcctg 300

gagtgcacat ccactagtgg gacacatgga tctaagagaa gagggagatg aagagactac 360

aaatgatgtt ccccatatcc agtgtggaga tggctgtgac ccccaaggac tcagggacaa 420

cagtcagttc tgcttgcaaa ggatccacca gggtctgatt ttttatgaga agctgctagg 480

atcggatatt ttcacagggg agccttctct gctccctgat agccctgtgg gccagcttca 540

tgcctcccta ctgggcctca gccaactcct gcagcctgag ggtcaccact gggagactca 600

gcagattcca agcctcagtc ccagccagcc atggcagcgt ctccttctcc gcttcaaaat 660

ccttcgcagc ctccaggcct ttgtggctgt agccgcccgg gtctttgccc atggagcagc 720

aaccctgagt ccctaaaggc agcagctcaa ggatggcact cagatctcca tggcccagca 780

aggccaagat aaatctacca ccccaggcac ctgtgagcca acaggttaat tagtccatta 840

attttagtgg gacctgcata tgttgaaaat taccaatact gactgacatg tgatgctgac 900

ctatgataag gttgagtatt tattagatgg gaagggaaat ttggggatta tttatcctcc 960

tggggacagt ttggggagga ttatttattg tatttatatt gaattatgta cttttttcaa 1020

taaagtctta tttttgtggc taaaaaaaa 1049

<210> 10

<211> 189
<212> PRT
<213> Homo sapiens

<400> 10

```
Met Leu Gly Ser Arg Ala Val Met Leu Leu Leu Leu Leu Pro Trp Thr
1               5                   10                  15

Ala Gln Gly Arg Ala Val Pro Gly Gly Ser Ser Pro Ala Trp Thr Gln
            20                  25                  30

Cys Gln Gln Leu Ser Gln Lys Leu Cys Thr Leu Ala Trp Ser Ala His
        35                  40                  45

Pro Leu Val Gly His Met Asp Leu Arg Glu Glu Gly Asp Glu Glu Thr
    50                  55                  60

Thr Asn Asp Val Pro His Ile Gln Cys Gly Asp Gly Cys Asp Pro Gln
65                  70                  75                  80

Gly Leu Arg Asp Asn Ser Gln Phe Cys Leu Gln Arg Ile His Gln Gly
                85                  90                  95

Leu Ile Phe Tyr Glu Lys Leu Leu Gly Ser Asp Ile Phe Thr Gly Glu
            100                 105                 110

Pro Ser Leu Leu Pro Asp Ser Pro Val Gly Gln Leu His Ala Ser Leu
        115                 120                 125

Leu Gly Leu Ser Gln Leu Leu Gln Pro Glu Gly His His Trp Glu Thr
    130                 135                 140

Gln Gln Ile Pro Ser Leu Ser Pro Ser Gln Pro Trp Gln Arg Leu Leu
145                 150                 155                 160

Leu Arg Phe Lys Ile Leu Arg Ser Leu Gln Ala Phe Val Ala Val Ala
                165                 170                 175

Ala Arg Val Phe Ala His Gly Ala Ala Thr Leu Ser Pro
            180                 185
```

<210> 11
<211> 808
<212> DNA
<213> Homo sapiens

<400> 11

```
gatataaagc tcctacagct acctggcctg agaagccaac tcagactcag ccaacagaga        60

ttgttgattt gcctcttaag caagagattc attgcagctc agcatggctc agaccagctc       120
```

```
atacttcatg ctgatctcct gcctgatgtt tctgtctcag agccaaggcc aagaggccca      180
gacagagttg ccccaggccc ggatcagctg cccagaaggc accaatgcct atcgctccta      240
ctgctactac tttaatgaag accgtgagac ctgggttgat gcagatctct attgccagaa      300
catgaattcg ggcaacctgg tgtctgtgct cacccaggcc gagggtgcct ttgtggcctc      360
actgattaag gagagtggca ctgatgactt caatgtctgg attggcctcc atgaccccaa      420
aaagaaccgc cgctggcact ggagcagtgg gtccctggtc tcctacaagt cctggggcat      480
tggagcccca agcagtgtta atcctggcta ctgtgtgagc ctgacctcaa gcacaggatt      540
ccagaaatgg aaggatgtgc cttgtgaaga caagttctcc tttgtctgca agttcaaaaa      600
ctagaggcaa ctggaaaata catgtctaga actgatccag caattacaac ggagtcaaaa      660
attaaaccgg accatctctc caactcaact caacctggac actctcttct ctgctgagtt      720
tgccttgtta atcttcaata gttttaccta ccccagtctt tggaacccta aataataaaa      780
ataaacatgt ttccactatt gtgctgtc                                          808
```

<210> 12
<211> 166
<212> PRT
<213> Homo sapiens

<400> 12

```
Met Ala Gln Thr Ser Ser Tyr Phe Met Leu Ile Ser Cys Leu Met Phe
1               5                   10                  15

Leu Ser Gln Ser Gln Gly Gln Glu Ala Gln Thr Glu Leu Pro Gln Ala
            20                  25                  30

Arg Ile Ser Cys Pro Glu Gly Thr Asn Ala Tyr Arg Ser Tyr Cys Tyr
        35                  40                  45

Tyr Phe Asn Glu Asp Arg Glu Thr Trp Val Asp Ala Asp Leu Tyr Cys
    50                  55                  60

Gln Asn Met Asn Ser Gly Asn Leu Val Ser Val Leu Thr Gln Ala Glu
65                  70                  75                  80

Gly Ala Phe Val Ala Ser Leu Ile Lys Glu Ser Gly Thr Asp Asp Phe
                85                  90                  95

Asn Val Trp Ile Gly Leu His Asp Pro Lys Lys Asn Arg Arg Trp His
            100                 105                 110

Trp Ser Ser Gly Ser Leu Val Ser Tyr Lys Ser Trp Gly Ile Gly Ala
        115                 120                 125
```

Pro Ser Ser Val Asn Pro Gly Tyr Cys Val Ser Leu Thr Ser Ser Thr
    130                 135                 140

Gly Phe Gln Lys Trp Lys Asp Val Pro Cys Glu Asp Lys Phe Ser Phe
145                 150                 155                 160

Val Cys Lys Phe Lys Asn
                165

<210> 13
<211> 773
<212> DNA
<213> Homo sapiens

<400> 13
aagccacctc aagtggacaa ggcacttacc aacagagatt gctgatttgc tccttaagca 60

agagattcac tgccgctaag catggctcag accaactcgt tcttcatgct gatctcctcc 120

ctgatgttcc tgtctctgag ccaaggccag gagtcccaga cagagctgcc taatccccga 180

atcagctgcc cagaaggcac caatgcctat cgctcctact gctactactt taatgaagac 240

cctgagacct gggttgatgc agatctctat tgccagaaca tgaattcagg caacctggtg 300

tctgtgctca cccaggcgga gggtgccttc gtggcctcac tgattaagga gagtagcact 360

gatgacagca atgtctggat tggcctccat gacccaaaaa agaaccgccg ctggcactgg 420

agtagtgggt ccctggtctc ctacaagtcc tgggacactg gatccccgag cagtgctaat 480

gctggctact gtgcaagcct gacttcatgc tcaggattca agaaatggaa ggatgaatct 540

tgtgagaaga agttctcctt tgtttgcaag ttcaaaaact agaggaagct gaaaaatgga 600

tgtctagaac tggtcctgca attactatga agtcaaaaat taaactagac tatgtctcca 660

actcagttca gaccatctcc tccctaatga gtttgcatcg ctgatcttca gtaccttcac 720

ctgtctcagt ctctagagcc ctgaaaaata aaaacaaact tatttttatc cag 773

<210> 14
<211> 166
<212> PRT
<213> Homo sapiens

<400> 14
Met Ala Gln Thr Asn Ser Phe Phe Met Leu Ile Ser Ser Leu Met Phe
1               5                   10                  15

Leu Ser Leu Ser Gln Gly Gln Glu Ser Gln Thr Glu Leu Pro Asn Pro
            20                  25                  30

Arg Ile Ser Cys Pro Glu Gly Thr Asn Ala Tyr Arg Ser Tyr Cys Tyr
        35                  40                  45

Tyr Phe Asn Glu Asp Pro Glu Thr Trp Val Asp Ala Asp Leu Tyr Cys
50 55 60

Gln Asn Met Asn Ser Gly Asn Leu Val Ser Val Leu Thr Gln Ala Glu
65 70 75 80

Gly Ala Phe Val Ala Ser Leu Ile Lys Glu Ser Ser Thr Asp Asp Ser
85 90 95

Asn Val Trp Ile Gly Leu His Asp Pro Lys Lys Asn Arg Arg Trp His
100 105 110

Trp Ser Ser Gly Ser Leu Val Ser Tyr Lys Ser Trp Asp Thr Gly Ser
115 120 125

Pro Ser Ser Ala Asn Ala Gly Tyr Cys Ala Ser Leu Thr Ser Cys Ser
130 135 140

Gly Phe Lys Lys Trp Lys Asp Glu Ser Cys Glu Lys Lys Phe Ser Phe
145 150 155 160

Val Cys Lys Phe Lys Asn
165

<210> 15
<211> 807
<212> DNA
<213> Homo sapiens

<400> 15
aaaccatacc atatcccacc agagagtgac tcctgattgc ctcctcaagt cgcagacact 60

atgctgcctc ccatggccct gcccagtgta tcttggatgc tgctttcctg cctcatgctg 120

ctgtctcagg ttcaaggtga agaaccccag agggaactgc cctctgcacg gatccgctgt 180

cccaaaggct ccaaggccta tggctcccac tgctatgcct tgtttttgtc accaaaatcc 240

tggacagatg cagatctggc ctgccagaag cggccctctg gaaacctggt gtctgtgctc 300

agtggggctg agggatcctt cgtgtcctcc ctggtgaaga gcattggtaa cagctactca 360

tacgtctgga ttgggctcca tgaccccaca cagggcaccg agcccaatgg agaaggttgg 420

gagtggagta gcagtgatgt gatgaattac tttgcatggg agagaaatcc ctccaccatc 480

tcaagccccg gccactgtgc gagcctgtcg agaagcacag catttctgag gtggaaagat 540

tataactgta atgtgaggtt accctatgtc tgcaagttca ctgactagtg caggagggaa 600

gtcagcagcc tgtgtttggt gtgcaactca tcatgggcat gagaccagtg tgaggactca 660

ccctggaaga gaatattcgc ttaattcccc caacctgacc acctcattct tatctttctt 720

ctgtttcttc ctccccgctg tcatttcagt ctcttcattt tgtcatacgg cctaaggctt 780

taaagagcaa taaaattttt agtctgc 807

<210> 16
<211> 175
<212> PRT
<213> Homo sapiens

<400> 16
Met Leu Pro Pro Met Ala Leu Pro Ser Val Ser Trp Met Leu Leu Ser
1               5                   10                  15

Cys Leu Met Leu Leu Ser Gln Val Gln Gly Glu Glu Pro Gln Arg Glu
            20                  25                  30

Leu Pro Ser Ala Arg Ile Arg Cys Pro Lys Gly Ser Lys Ala Tyr Gly
        35                  40                  45

Ser His Cys Tyr Ala Leu Phe Leu Ser Pro Lys Ser Trp Thr Asp Ala
    50                  55                  60

Asp Leu Ala Cys Gln Lys Arg Pro Ser Gly Asn Leu Val Ser Val Leu
65                  70                  75                  80

Ser Gly Ala Glu Gly Ser Phe Val Ser Ser Leu Val Lys Ser Ile Gly
                85                  90                  95

Asn Ser Tyr Ser Tyr Val Trp Ile Gly Leu His Asp Pro Thr Gln Gly
            100                 105                 110

Thr Glu Pro Asn Gly Glu Gly Trp Glu Trp Ser Ser Ser Asp Val Met
        115                 120                 125

Asn Tyr Phe Ala Trp Glu Arg Asn Pro Ser Thr Ile Ser Ser Pro Gly
    130                 135                 140

His Cys Ala Ser Leu Ser Arg Ser Thr Ala Phe Leu Arg Trp Lys Asp
145                 150                 155                 160

Tyr Asn Cys Asn Val Arg Leu Pro Tyr Val Cys Lys Phe Thr Asp
                165                 170                 175

<210> 17
<211> 784
<212> DNA
<213> Homo sapiens

<400> 17
aaaccatacc atatcccacc agagagtcgc agacactatg ctgcctccca tggccctgcc 60

cagtgtatct tggatgctgc tttcctgcct catgctgctg tctcaggttc aaggtgaaga 120

accccagagg gaactgccct ctgcacggat ccgctgtccc aaaggctcca aggcctatgg 180

ctcccactgc tatgccttgt ttttgtcacc aaaatcctgg acagatgcag atctggcctg 240

ccagaagcgg ccctctggaa acctggtgtc tgtgctcagt ggggctgagg gatccttcgt 300

gtcctccctg gtgaagagca ttggtaacag ctactcatac gtctggattg ggctccatga 360

ccccacacag ggcaccgagc ccaatggaga aggttgggag tggagtagca gtgatgtgat 420

gaattacttt gcatgggaga gaaatccctc caccatctca agccccggcc actgtgcgag 480

cctgtcgaga agcacagcat ttctgaggtg gaaagattat aactgtaatg tgaggttacc 540

ctatgtctgc aagttcactg actagtgcag gagggaagtc agcagcctgt gtttggtgtg 600

caactcatca tgggcatgag accagtgtga ggactcaccc tggaagagaa tattcgctta 660

attcccccaa cctgaccacc tcattcttat ctttcttctg tttcttcctc cccgctgtca 720

tttcagtctc ttcattttgt catacggcct aaggctttaa agagcaataa aatttttagt 780

ctgc 784

<210> 18
<211> 175
<212> PRT
<213> Homo sapiens

<400> 18

```
Met Leu Pro Pro Met Ala Leu Pro Ser Val Ser Trp Met Leu Leu Ser
1               5                   10                  15

Cys Leu Met Leu Leu Ser Gln Val Gln Gly Glu Glu Pro Gln Arg Glu
            20                  25                  30

Leu Pro Ser Ala Arg Ile Arg Cys Pro Lys Gly Ser Lys Ala Tyr Gly
        35                  40                  45

Ser His Cys Tyr Ala Leu Phe Leu Ser Pro Lys Ser Trp Thr Asp Ala
    50                  55                  60

Asp Leu Ala Cys Gln Lys Arg Pro Ser Gly Asn Leu Val Ser Val Leu
65                  70                  75                  80

Ser Gly Ala Glu Gly Ser Phe Val Ser Ser Leu Val Lys Ser Ile Gly
                85                  90                  95

Asn Ser Tyr Ser Tyr Val Trp Ile Gly Leu His Asp Pro Thr Gln Gly
            100                 105                 110

Thr Glu Pro Asn Gly Glu Gly Trp Glu Trp Ser Ser Ser Asp Val Met
        115                 120                 125
```

Asn Tyr Phe Ala Trp Glu Arg Asn Pro Ser Thr Ile Ser Ser Pro Gly
    130                 135                 140

His Cys Ala Ser Leu Ser Arg Ser Thr Ala Phe Leu Arg Trp Lys Asp
145                 150                 155                 160

Tyr Asn Cys Asn Val Arg Leu Pro Tyr Val Cys Lys Phe Thr Asp
                165                 170                 175

<210> 19
<211> 1002
<212> DNA
<213> Homo sapiens

<400> 19
gggagggtcc cttcctcagg gagcacagga actctgagac tcagcaaggg tgtcctggga 60

gggctcgggg atgggagagt acacagattc acaactcatt cagaactgta gaagatgatg 120

gatgtgacca agatcacttt agtcctaggg gactagagaa ggaaaatgac atgaggcagt 180

ggggtatctg tgtgttctcc cactgaccac gctttcttta gtgactcctg attgcctcct 240

caagtcgcag acactatgct gcctcccatg gccctgccca gtgtatcttg gatgctgctt 300

tcctgcctca tgctgctgtc tcaggttcaa ggtgaagaac cccagaggga actgccctct 360

gcacggatcc gctgtcccaa aggctccaag gcctatggct cccactgcta tgccttgttt 420

ttgtcaccaa aatcctggac agatgcagat ctggcctgcc agaagcggcc ctctggaaac 480

ctggtgtctg tgctcagtgg ggctgaggga tccttcgtgt cctccctggt gaagagcatt 540

ggtaacagct actcatacgt ctggattggg ctccatgacc ccacacaggg caccgagccc 600

aatggagaag gttgggagtg gagtagcagt gatgtgatga attactttgc atgggagaga 660

aatccctcca ccatctcaag ccccggccac tgtgcgagcc tgtcgagaag cacagcattt 720

ctgaggtgga aagattataa ctgtaatgtg aggttaccct atgtctgcaa gttcactgac 780

tagtgcagga gggaagtcag cagcctgtgt ttggtgtgca actcatcatg ggcatgagac 840

cagtgtgagg actcaccctg gaagagaata ttcgcttaat tcccccaacc tgaccacctc 900

attcttatct ttcttctgtt tcttcctccc cgctgtcatt tcagtctctt cattttgtca 960

tacggcctaa ggctttaaag agcaataaaa tttttagtct gc 1002

<210> 20
<211> 175
<212> PRT
<213> Homo sapiens

<400> 20
Met Leu Pro Pro Met Ala Leu Pro Ser Val Ser Trp Met Leu Leu Ser
1               5                   10                  15

Cys Leu Met Leu Leu Ser Gln Val Gln Gly Glu Glu Pro Gln Arg Glu
20 25 30

Leu Pro Ser Ala Arg Ile Arg Cys Pro Lys Gly Ser Lys Ala Tyr Gly
35 40 45

Ser His Cys Tyr Ala Leu Phe Leu Ser Pro Lys Ser Trp Thr Asp Ala
50 55 60

Asp Leu Ala Cys Gln Lys Arg Pro Ser Gly Asn Leu Val Ser Val Leu
65 70 75 80

Ser Gly Ala Glu Gly Ser Phe Val Ser Ser Leu Val Lys Ser Ile Gly
85 90 95

Asn Ser Tyr Ser Tyr Val Trp Ile Gly Leu His Asp Pro Thr Gln Gly
100 105 110

Thr Glu Pro Asn Gly Glu Gly Trp Glu Trp Ser Ser Ser Asp Val Met
115 120 125

Asn Tyr Phe Ala Trp Glu Arg Asn Pro Ser Thr Ile Ser Ser Pro Gly
130 135 140

His Cys Ala Ser Leu Ser Arg Ser Thr Ala Phe Leu Arg Trp Lys Asp
145 150 155 160

Tyr Asn Cys Asn Val Arg Leu Pro Tyr Val Cys Lys Phe Thr Asp
165 170 175

<210> 21
<211> 847
<212> DNA
<213> Homo sapiens

<400> 21
ccatccctga gatcttttta taaaaaaccc agtctttgct gaccagacaa agcataccag 60
atctcaccag agagtcgcag acactatgct gcctcccatg gccctgccca gtgtgtcctg 120
gatgctgctt tcctgcctca ttctcctgtg tcaggttcaa ggtgaagaaa cccagaagga 180
actgccctct ccacggatca gctgtcccaa aggctccaag gcctatggct ccccctgcta 240
tgccttgttt ttgtcaccaa aatcctggat ggatgcagat ctggcttgcc agaagcggcc 300
ctctggaaaa ctggtgtctg tgctcagtgg ggctgaggga tccttcgtgt cctccctggt 360
gaggagcatt agtaacagct attcatacat ctggattggg ctccatgacc ccacacaggg 420
ctctgagcct gatggagatg gatgggagtg gagtagcact gatgtgatga attactttgc 480

atgggagaaa aatccctcca ccatcttaaa ccctggccac tgtgggagcc tgtcaagaag 540

cacaggattt ctgaagtgga aagattataa ctgtgatgca aagttaccct atgtctgcaa 600

gttcaaggac tagggcaggt gggaagtcag cagcctgagc ttggcgtgca gctcatcatg 660

gacatgagac cagtgtgaag actcaccctg gaagagaata ttctccccaa actgccctac 720

ctgactacct tgtcatgatc ctccttcttt ttcctttttc ttcaccttca tttcaggctt 780

ttctctgtct tccatgtctt gagatctcag agaataataa taaaaatgtt actttatact 840

taaaaaa 847

<210> 22
<211> 175
<212> PRT
<213> Homo sapiens

<400> 22
```
Met Leu Pro Pro Met Ala Leu Pro Ser Val Ser Trp Met Leu Leu Ser
1               5                   10                  15

Cys Leu Ile Leu Leu Cys Gln Val Gln Gly Glu Glu Thr Gln Lys Glu
            20                  25                  30

Leu Pro Ser Pro Arg Ile Ser Cys Pro Lys Gly Ser Lys Ala Tyr Gly
        35                  40                  45

Ser Pro Cys Tyr Ala Leu Phe Leu Ser Pro Lys Ser Trp Met Asp Ala
    50                  55                  60

Asp Leu Ala Cys Gln Lys Arg Pro Ser Gly Lys Leu Val Ser Val Leu
65                  70                  75                  80

Ser Gly Ala Glu Gly Ser Phe Val Ser Ser Leu Val Arg Ser Ile Ser
                85                  90                  95

Asn Ser Tyr Ser Tyr Ile Trp Ile Gly Leu His Asp Pro Thr Gln Gly
            100                 105                 110

Ser Glu Pro Asp Gly Asp Gly Trp Glu Trp Ser Ser Thr Asp Val Met
        115                 120                 125

Asn Tyr Phe Ala Trp Glu Lys Asn Pro Ser Thr Ile Leu Asn Pro Gly
    130                 135                 140

His Cys Gly Ser Leu Ser Arg Ser Thr Gly Phe Leu Lys Trp Lys Asp
145                 150                 155                 160

Tyr Asn Cys Asp Ala Lys Leu Pro Tyr Val Cys Lys Phe Lys Asp
                165                 170                 175
```

<210> 23
<211> 947
<212> DNA
<213> Homo sapiens

<400> 23
ccatccctga gatcttttta taaaaaaccc agtctttgct gaccagacaa agcataccag 60

atctcaccag agagtcctag gggactacag aaggaaaaag acaagaggca gtaggatatc 120

tgtgtgtcct cccgctgacc acacttcctt tagtgacccg attgcctcct caagtcgcag 180

acactatgct gcctcccatg gccctgccca gtgtgtcctg gatgctgctt tcctgcctca 240

ttctcctgtg tcaggttcaa ggtgaagaaa cccagaagga actgccctct ccacggatca 300

gctgtcccaa aggctccaag gcctatggct ccccctgcta tgccttgttt ttgtcaccaa 360

aatcctggat ggatgcagat ctggcttgcc agaagcggcc ctctggaaaa ctggtgtctg 420

tgctcagtgg ggctgaggga tccttcgtgt cctccctggt gaggagcatt agtaacagct 480

attcatacat ctggattggg ctccatgacc ccacacaggg ctctgagcct gatggagatg 540

gatgggagtg gagtagcact gatgtgatga attactttgc atgggagaaa aatccctcca 600

ccatcttaaa ccctggccac tgtgggagcc tgtcaagaag cacaggattt ctgaagtgga 660

aagattataa ctgtgatgca aagttaccct atgtctgcaa gttcaaggac tagggcaggt 720

gggaagtcag cagcctgagc ttggcgtgca gctcatcatg gacatgagac cagtgtgaag 780

actcaccctg gaagagaata ttctccccaa actgccctac ctgactacct tgtcatgatc 840

ctccttcttt ttcctttttc ttcaccttca tttcaggctt ttctctgtct tccatgtctt 900

gagatctcag agaataataa taaaaatgtt actttatacg taaaaaa 947

<210> 24
<211> 175
<212> PRT
<213> Homo sapiens

<400> 24
Met Leu Pro Pro Met Ala Leu Pro Ser Val Ser Trp Met Leu Leu Ser
1 5 10 15

Cys Leu Ile Leu Leu Cys Gln Val Gln Gly Glu Glu Thr Gln Lys Glu
20 25 30

Leu Pro Ser Pro Arg Ile Ser Cys Pro Lys Gly Ser Lys Ala Tyr Gly
35 40 45

Ser Pro Cys Tyr Ala Leu Phe Leu Ser Pro Lys Ser Trp Met Asp Ala
50 55 60

Asp Leu Ala Cys Gln Lys Arg Pro Ser Gly Lys Leu Val Ser Val Leu
65 70 75 80

Ser Gly Ala Glu Gly Ser Phe Val Ser Ser Leu Val Arg Ser Ile Ser
85 90 95

Asn Ser Tyr Ser Tyr Ile Trp Ile Gly Leu His Asp Pro Thr Gln Gly
100 105 110

Ser Glu Pro Asp Gly Asp Gly Trp Glu Trp Ser Ser Thr Asp Val Met
115 120 125

Asn Tyr Phe Ala Trp Glu Lys Asn Pro Ser Thr Ile Leu Asn Pro Gly
130 135 140

His Cys Gly Ser Leu Ser Arg Ser Thr Gly Phe Leu Lys Trp Lys Asp
145 150 155 160

Tyr Asn Cys Asp Ala Lys Leu Pro Tyr Val Cys Lys Phe Lys Asp
165 170 175

<210> 25
<211> 1285
<212> DNA
<213> Homo sapiens

<400> 25
ataagacttt tatggatgga ttgtttttct caaataatat tatcgctttg tgactaaagt 60

aaagattatt aattcctgag gcaagaagat ataaaagctc cagaaacgtt gactgggacc 120

actggagaca ctgaagaagg caggggccct tagagtcttg gttgccaaac agatttgcag 180

atcaaggaga acccaggagt ttcaaagaag cgctagtaag gtctctgaga tccttgcact 240

agctacatcc tcagggtagg aggaagatgg cttccagaag catgcggctg ctcctattgc 300

tgagctgcct ggccaaaaca ggagtcctgg gtgatatcat catgagaccc agctgtgctc 360

ctggatggtt ttaccacaag tccaattgct atggttactt caggaagctg aggaactggt 420

ctgatgccga gctcgagtgt cagtcttacg gaaacggagc ccacctggca tctatcctga 480

gtttaaagga agccagcacc atagcagagt acataagtgg ctatcagaga agccagccga 540

tatggattgg cctgcacgac ccacagaaga ggcagcagtg gcagtggatt gatggggcca 600

tgtatctgta cagatcctgg tctggcaagt ccatgggtgg gaacaagcac tgtgctgaga 660

tgagctccaa taacaacttt ttaacttgga gcagcaacga atgcaacaag cgccaacact 720

tcctgtgcaa gtaccgacca tagagcaaga atcaagattc tgctaactcc tgcacagccc 780

cgtcctcttc ctttctgcta gcctggctaa atctgctcat tatttcagag gggaaaccta 840

gcaaactaag agtgataagg gccctactac actggctttt ttaggcttag agacagaaac 900

```
tttagcattg gcccagtagt ggcttctagc tctaaatgtt tgccccgcca tccctttcca      960
cagtatcctt cttccctcct cccctgtctc tggctgtctc gagcagtcta gaagagtgca     1020
tctccagcct atgaaacagc tgggtctttg gccataagaa gtaaagattt gaagacagaa     1080
ggaagaaact caggagtaag cttctagacc ccttcagctt ctacaccctt ctgccctctc     1140
tccattgcct gcaccccacc ccagccactc aactcctgct tgtttttcct ttggccatag     1200
gaaggtttac cagtagaatc cttgctaggt tgatgtgggc catacattcc tttaataaac     1260
cattgtgtac ataagaaaaa aaaaa                                           1285

<210> 26
<211> 158
<212> PRT
<213> Homo sapiens

<400> 26
Met Ala Ser Arg Ser Met Arg Leu Leu Leu Leu Leu Ser Cys Leu Ala
1               5                   10                  15

Lys Thr Gly Val Leu Gly Asp Ile Ile Met Arg Pro Ser Cys Ala Pro
            20                  25                  30

Gly Trp Phe Tyr His Lys Ser Asn Cys Tyr Gly Tyr Phe Arg Lys Leu
        35                  40                  45

Arg Asn Trp Ser Asp Ala Glu Leu Glu Cys Gln Ser Tyr Gly Asn Gly
    50                  55                  60

Ala His Leu Ala Ser Ile Leu Ser Leu Lys Glu Ala Ser Thr Ile Ala
65                  70                  75                  80

Glu Tyr Ile Ser Gly Tyr Gln Arg Ser Gln Pro Ile Trp Ile Gly Leu
                85                  90                  95

His Asp Pro Gln Lys Arg Gln Gln Trp Gln Trp Ile Asp Gly Ala Met
            100                 105                 110

Tyr Leu Tyr Arg Ser Trp Ser Gly Lys Ser Met Gly Gly Asn Lys His
        115                 120                 125

Cys Ala Glu Met Ser Ser Asn Asn Asn Phe Leu Thr Trp Ser Ser Asn
    130                 135                 140

Glu Cys Asn Lys Arg Gln His Phe Leu Cys Lys Tyr Arg Pro
145                 150                 155

<210> 27
```

<211> 1087
<212> DNA
<213> Mus musculus

<400> 27
ccaagaacga tagtcaattc cagaaaccgc tatgaagttc ctctctgcaa gagacttcca 60

tccagttgcc ttcttgggac tgatgctggt gacaaccacg gccttcccta cttcacaagt 120

ccggagagga gacttcacag aggataccac tcccaacaga cctgtctata ccacttcaca 180

agtcggaggc ttaattacac atgttctctg ggaaatcgtg gaaatgagaa aagagttgtg 240

caatggcaat tctgattgta tgaacaacga tgatgcactt gcagaaaaca atctgaaact 300

tccagagata caaagaaatg atggatgcta ccaaactgga tataatcagg aaatttgcct 360

attgaaaatt tcctctggtc ttctggagta ccatagctac ctggagtaca tgaagaacaa 420

cttaaaagat aacaagaaag acaaagccag agtccttcag agagatacag aaactctaat 480

tcatatcttc aaccaagagg taaaagattt acataaaata gtccttccta ccccaatttc 540

caatgctctc ctaacagata agctggagtc acagaaggag tggctaagga ccaagaccat 600

ccaattcatc ttgaaatcac ttgaagaatt tctaaaagtc actttgagat ctactcggca 660

aacctagtgc gttatgccta agcatatcag tttgtggaca ttcctcactg tggtcagaaa 720

atatatcctg ttgtcaggta tctgacttat gttgttctct acgaagaact gacaatatga 780

atgttgggac actattttaa ttatttttaa tttattgata atttaaataa gtaaacttta 840

agttaattta tgattgatat ttattatttt tatgaagtgt cacttgaaat gttatatgtt 900

atagttttga aatgataacc taaaaatcta tttgatataa atattctgtt acctagccag 960

atggtttctt ggaatgtata agtttacctc aatgaattgc taatttaaat atgtttttaa 1020

agaaatcttt gtgatgtatt tttataatgt ttagactgtc ttcaaacaaa taaattatat 1080

tatattt 1087

<210> 28
<211> 211
<212> PRT
<213> Mus musculus

<400> 28
Met Lys Phe Leu Ser Ala Arg Asp Phe His Pro Val Ala Phe Leu Gly
1 5 10 15

Leu Met Leu Val Thr Thr Thr Ala Phe Pro Thr Ser Gln Val Arg Arg
20 25 30

Gly Asp Phe Thr Glu Asp Thr Thr Pro Asn Arg Pro Val Tyr Thr Thr
35 40 45

Ser Gln Val Gly Gly Leu Ile Thr His Val Leu Trp Glu Ile Val Glu

50 55 60

Met Arg Lys Glu Leu Cys Asn Gly Asn Ser Asp Cys Met Asn Asn Asp
65 70 75 80

Asp Ala Leu Ala Glu Asn Asn Leu Lys Leu Pro Glu Ile Gln Arg Asn
85 90 95

Asp Gly Cys Tyr Gln Thr Gly Tyr Asn Gln Glu Ile Cys Leu Leu Lys
100 105 110

Ile Ser Ser Gly Leu Leu Glu Tyr His Ser Tyr Leu Glu Tyr Met Lys
115 120 125

Asn Asn Leu Lys Asp Asn Lys Lys Asp Lys Ala Arg Val Leu Gln Arg
130 135 140

Asp Thr Glu Thr Leu Ile His Ile Phe Asn Gln Glu Val Lys Asp Leu
145 150 155 160

His Lys Ile Val Leu Pro Thr Pro Ile Ser Asn Ala Leu Leu Thr Asp
165 170 175

Lys Leu Glu Ser Gln Lys Glu Trp Leu Arg Thr Lys Thr Ile Gln Phe
180 185 190

Ile Leu Lys Ser Leu Glu Glu Phe Leu Lys Val Thr Leu Arg Ser Thr
195 200 205

Arg Gln Thr
210

<210> 29
<211> 1951
<212> DNA
<213> Mus musculus

<400> 29
agaaggaaca gtgggtgtcc aggcacatca gaccaggcag ctcgcagcaa agcaaggtaa 60

gttctctcct cttccctgtc gctaactccc tgcatctaga ggctgtccag attcagactc 120

caggggacag gctacccctg aaccaggcag cgtgggagtg ggatgtgtcc tcagaagcta 180

accatctcct ggtttgccat cgttttgctg gtgtctccac tcatggccat gtgggagctg 240

gagaaagacg tttatgttgt agaggtggac tggactcccg atgcccctgg agaaacagtg 300

aacctcacct gtgacacgcc tgaagaagat gacatcacct ggacctcaga ccagagacat 360

ggagtcatag gctctggaaa gaccctgacc atcactgtca aagagtttct agatgctggc 420

```
cagtacacct gccacaaagg aggcgagact ctgagccact cacatctgct gctccacaag      480
aaggaaaatg gaatttggtc cactgaaatt ttaaaaaatt tcaaaaacaa gactttcctg      540
aagtgtgaag caccaaatta ctccggacgg ttcacgtgct catggctggt gcaaagaaac      600
atggacttga agttcaacat caagagcagt agcagttccc ctgactctcg ggcagtgaca      660
tgtggaatgg cgtctctgtc tgcagagaag gtcacactgg accaaaggga ctatgagaag      720
tattcagtgt cctgccagga ggatgtcacc tgcccaactg ccgaggagac cctgcccatt      780
gaactggcgt tggaagcacg gcagcagaat aaatatgaga actacagcac cagcttcttc      840
atcagggaca tcatcaaacc agacccgccc aagaacttgc agatgaagcc tttgaagaac      900
tcacaggtgg aggtcagctg ggagtaccct gactcctgga gcactcccca ttcctacttc      960
tccctcaagt tctttgttcg aatccagcgc aagaaagaaa agatgaagga gacagaggag     1020
gggtgtaacc agaaaggtgc gttcctcgta gagaagacat ctaccgaagt ccaatgcaaa     1080
ggcgggaatg tctgcgtgca agctcaggat cgctattaca attcctcatg cagcaagtgg     1140
gcatgtgttc cctgcagggt ccgatcctag gatgcaacgt tggaaaggaa agaaaagtgg     1200
aagacattaa ggaagaaaaa tttaaactca ggatggaaga gtcccccaaa agctgtcttc     1260
tgcttggttg gctttttcca gttttcctaa gttcatcatg acacctttgc tgatttctac     1320
atgtaaatgt taaatgcccg cagagccagg gagctaatgt atgcatagat attctagcat     1380
tccacttggc cttatgctgt tgaaatattt aagtaattta tgtatttatt aatttatttc     1440
tgcatttcac atttgtatac caagatgtat tgaatatttc atgtgctcgt ggcctgatcc     1500
actgggacca ggccctatta tgcaaattgt gagcttgtta tcttcttcaa cagctcttca     1560
atcagggctg cgtaggtaca ttagcttttg tgacaaccaa taagaacata atattctgac     1620
acaagcagtg ttacatattt gtgaccagta aagacatagg tggtatttgg agacatgaag     1680
aagctgtaaa gttgactctg aagagtttag cactagtttc aacaccaaga aagacttttt     1740
agaagtgata ttgataagaa accagggcct tctttagaag ggtacctaaa tttaaaagaa     1800
ttttgaaagg ctgggtatcg gtggtatatg cttttaattc cagcactcag gagaccaagg     1860
caggcagatc tctgtgagtt tgaggacagc ctggtgtaca gagggagttc cagcacagcc     1920
agtgccacac agaaattctg tctcaaaaac a                                    1951
```

<210> 30
<211> 335
<212> PRT
<213> Mus musculus

<400> 30

```
Met Cys Pro Gln Lys Leu Thr Ile Ser Trp Phe Ala Ile Val Leu Leu
1               5                   10                  15
```

```
Val Ser Pro Leu Met Ala Met Trp Glu Leu Glu Lys Asp Val Tyr Val
            20                  25                  30

Val Glu Val Asp Trp Thr Pro Asp Ala Pro Gly Glu Thr Val Asn Leu
        35                  40                  45

Thr Cys Asp Thr Pro Glu Glu Asp Asp Ile Thr Trp Thr Ser Asp Gln
    50                  55                  60

Arg His Gly Val Ile Gly Ser Gly Lys Thr Leu Thr Ile Thr Val Lys
65                  70                  75                  80

Glu Phe Leu Asp Ala Gly Gln Tyr Thr Cys His Lys Gly Gly Glu Thr
                85                  90                  95

Leu Ser His Ser His Leu Leu Leu His Lys Lys Glu Asn Gly Ile Trp
            100                 105                 110

Ser Thr Glu Ile Leu Lys Asn Phe Lys Asn Lys Thr Phe Leu Lys Cys
        115                 120                 125

Glu Ala Pro Asn Tyr Ser Gly Arg Phe Thr Cys Ser Trp Leu Val Gln
    130                 135                 140

Arg Asn Met Asp Leu Lys Phe Asn Ile Lys Ser Ser Ser Ser Ser Pro
145                 150                 155                 160

Asp Ser Arg Ala Val Thr Cys Gly Met Ala Ser Leu Ser Ala Glu Lys
                165                 170                 175

Val Thr Leu Asp Gln Arg Asp Tyr Glu Lys Tyr Ser Val Ser Cys Gln
            180                 185                 190

Glu Asp Val Thr Cys Pro Thr Ala Glu Glu Thr Leu Pro Ile Glu Leu
        195                 200                 205

Ala Leu Glu Ala Arg Gln Gln Asn Lys Tyr Glu Asn Tyr Ser Thr Ser
    210                 215                 220

Phe Phe Ile Arg Asp Ile Ile Lys Pro Asp Pro Pro Lys Asn Leu Gln
225                 230                 235                 240

Met Lys Pro Leu Lys Asn Ser Gln Val Glu Val Ser Trp Glu Tyr Pro
                245                 250                 255

Asp Ser Trp Ser Thr Pro His Ser Tyr Phe Ser Leu Lys Phe Phe Val
            260                 265                 270
```

```
Arg Ile Gln Arg Lys Lys Glu Lys Met Lys Glu Thr Glu Glu Gly Cys
        275                 280                 285

Asn Gln Lys Gly Ala Phe Leu Val Glu Lys Thr Ser Thr Glu Val Gln
    290                 295                 300

Cys Lys Gly Gly Asn Val Cys Val Gln Ala Gln Asp Arg Tyr Tyr Asn
305                 310                 315                 320

Ser Ser Cys Ser Lys Trp Ala Cys Val Pro Cys Arg Val Arg Ser
                325                 330                 335
```

<210> 31
<211> 866
<212> DNA
<213> Mus musculus

<400> 31

```
ggcacagctg gacctggtgg gggttctctg tggttccatg ctttctggac tcctgcctgc      60
tggctggagc tgctgacagg cctgacatct tctgcaacct ccagcatcag gacaaagaaa     120
gccgcctcaa accttccaaa tcacttcctc ttggcccagg aacaatggct gccatgtcag     180
aagactcttg cgtcaacttc aaggaaatga tgtttattga caacacgctt tactttatac     240
ctgaagaaaa tggagacctg gaatcagaca actttggccg acttcactgt acaaccgcag     300
taatacggaa tataaatgac caagttctct tcgttgacaa aagacagcct gtgttcgagg     360
atatgactga tattgatcaa agtgccagtg aaccccagac cagactgata atatacatgt     420
acaaagacag tgaagtaaga ggactggctg tgaccctctc tgtgaaggat agtaaaatgt     480
ctaccctctc ctgtaagaac aagatcattt cctttgagga aatggatcca cctgaaaata     540
ttgatgatat acaaagtgat ctcatattct ttcagaaacg tgttccagga cacaacaaga     600
tggagtttga atcttcactg tatgaaggac actttcttgc ttgccaaaag gaagatgatg     660
ctttcaaact cattctgaaa aaaaaggatg aaaatgggga taaatctgta atgttcactc     720
tcactaactt acatcaaagt taggtgggga gggtttgtgt tccagaaaga tgattagcac     780
acatgcgcct tgtgatgacc tcgcctgtat ttccataaca gaatacccga ggctgcatga     840
tttatagagt aaacacgttt atttgt                                          866
```

<210> 32
<211> 192
<212> PRT
<213> Mus musculus

<400> 32

```
Met Ala Ala Met Ser Glu Asp Ser Cys Val Asn Phe Lys Glu Met Met
1               5                   10                  15
```

```
Phe Ile Asp Asn Thr Leu Tyr Phe Ile Pro Glu Glu Asn Gly Asp Leu
            20                  25                  30

Glu Ser Asp Asn Phe Gly Arg Leu His Cys Thr Thr Ala Val Ile Arg
        35                  40                  45

Asn Ile Asn Asp Gln Val Leu Phe Val Asp Lys Arg Gln Pro Val Phe
    50                  55                  60

Glu Asp Met Thr Asp Ile Asp Gln Ser Ala Ser Glu Pro Gln Thr Arg
65                  70                  75                  80

Leu Ile Ile Tyr Met Tyr Lys Asp Ser Glu Val Arg Gly Leu Ala Val
                85                  90                  95

Thr Leu Ser Val Lys Asp Ser Lys Met Ser Thr Leu Ser Cys Lys Asn
            100                 105                 110

Lys Ile Ile Ser Phe Glu Glu Met Asp Pro Pro Glu Asn Ile Asp Asp
        115                 120                 125

Ile Gln Ser Asp Leu Ile Phe Phe Gln Lys Arg Val Pro Gly His Asn
    130                 135                 140

Lys Met Glu Phe Glu Ser Ser Leu Tyr Glu Gly His Phe Leu Ala Cys
145                 150                 155                 160

Gln Lys Glu Asp Asp Ala Phe Lys Leu Ile Leu Lys Lys Lys Asp Glu
                165                 170                 175

Asn Gly Asp Lys Ser Val Met Phe Thr Leu Thr Asn Leu His Gln Ser
            180                 185                 190
```

```
<210> 33
<211> 1121
<212> DNA
<213> Mus musculus

<400> 33
cctaaacagg ctctcctctc acttatcaac tgttgacact tgtgcgatct ctgatggctg      60

tcctgcagaa atctatgagt ttttccctta tggggacttt ggccgccagc tgcctgcttc     120

tcattgccct gtgggcccag gaggcaaatg cgctgcccgt caacacccgg tgcaagcttg     180

aggtgtccaa cttccagcag ccgtacatcg tcaaccgcac ctttatgctg gccaaggagg     240

ccagccttgc agataacaac acagacgtcc ggctcatcgg ggagaaactg ttccgaggag     300

tcagtgctaa agatcagtgc tacctgatga agcaggtgct caacttcacc ctggaagacg     360
```

```
ttctgctccc ccagtcagac aggttccagc cctacatgca ggaggtggta cctttcctga      420

ccaaactcag caatcagctc agctcctgtc acatcagcgg tgacgaccag aacatccaga      480

agaatgtcag aaggctgaag gagacagtga aaaagcttgg agagagtgga gagatcaagg      540

cgattgggga actggacctg ctgtttatgt ctctgagaaa tgcttgcgtc tgagcgagaa      600

gaagctagaa aacgaagaac tgctccttcc tgccttctaa aaagaacaat aagatccctg      660

aatggacttt tttactaaag gaaagtgaga agctaacgtc catcatcatt agaagatttc      720

acatgaaacc tggctcagtt gaaaaagaaa atagtgtcaa gttgtccatg agaccagagg      780

tagacttgat aaccacaaag attcattgac aatattttat tgtcactgat gatacaacag      840

aaaaataatg tactttaaaa aattgtttga aaggaggtta cctctcattc ctttagaaaa      900

aaagcttatg taacttcatt tccatatcca atattttata tatgtaagtt tatttattat      960

aagtatacat tttatttatg tcagtttatt aatatggatt tatttataga aacattatct     1020

gctattgata tttagtataa ggcaaataat atttatgaca ataactatgg aaacaagata     1080

tcttaggctt taataaacac atggatatca taaaaaaaaa a                         1121


<210> 34
<211> 179
<212> PRT
<213> Mus musculus

<400> 34
Met Ala Val Leu Gln Lys Ser Met Ser Phe Ser Leu Met Gly Thr Leu
1               5                   10                  15


Ala Ala Ser Cys Leu Leu Leu Ile Ala Leu Trp Ala Gln Glu Ala Asn
            20                  25                  30


Ala Leu Pro Val Asn Thr Arg Cys Lys Leu Glu Val Ser Asn Phe Gln
        35                  40                  45


Gln Pro Tyr Ile Val Asn Arg Thr Phe Met Leu Ala Lys Glu Ala Ser
    50                  55                  60


Leu Ala Asp Asn Asn Thr Asp Val Arg Leu Ile Gly Glu Lys Leu Phe
65                  70                  75                  80


Arg Gly Val Ser Ala Lys Asp Gln Cys Tyr Leu Met Lys Gln Val Leu
                85                  90                  95


Asn Phe Thr Leu Glu Asp Val Leu Leu Pro Gln Ser Asp Arg Phe Gln
            100                 105                 110


Pro Tyr Met Gln Glu Val Val Pro Phe Leu Thr Lys Leu Ser Asn Gln
        115                 120                 125
```

Leu Ser Ser Cys His Ile Ser Gly Asp Asp Gln Asn Ile Gln Lys Asn
130 135 140

Val Arg Arg Leu Lys Glu Thr Val Lys Lys Leu Gly Glu Ser Gly Glu
145 150 155 160

Ile Lys Ala Ile Gly Glu Leu Asp Leu Leu Phe Met Ser Leu Arg Asn
165 170 175

Ala Cys Val

<210> 35
<211> 1359
<212> DNA
<213> Mus musculus

<400> 35
cgcttagaag tcggactaca gagttagact cagaaccaaa ggaggtggat agggggtcca 60

caggcctggt gcagatcaca gagccagcca gatctgagaa gcagggaaca agatgctgga 120

ttgcagagca gtaataatgc tatggctgtt gccctgggtc actcagggcc tggctgtgcc 180

taggagtagc agtcctgact gggctcagtg ccagcagctc tctcggaatc tctgcatgct 240

agcctggaac gcacatgcac cagcgggaca tatgaatcta ctaagagaag aagaggatga 300

agagactaaa aataatgtgc cccgtatcca gtgtgaagat ggttgtgacc cacaaggact 360

caaggacaac agccagttct gcttgcaaag gatccgccaa ggtctggctt tttataagca 420

cctgcttgac tctgacatct tcaaagggga gcctgctcta ctccctgata gccccatgga 480

gcaacttcac acctccctac taggactcag ccaactcctc cagccagagg atcacccccg 540

ggagacccaa cagatgccca gcctgagttc tagtcagcag tggcagcgcc cccttctccg 600

ttccaagatc cttcgaagcc tccaggcctt tttggccata gctgcccggg tctttgccca 660

cggagcagca actctgactg agcccttagt gccaacagct taaggatgcc caggttccca 720

tggctaccat gataagacta atctatcagc ccagacatct accagttaat taacccatta 780

ggacttgtgc tgttcttgtt ttgtttgttt tgcgtgaagg gcaaggacac cattattaaa 840

gagaaaagaa acaaacccca gagcaggcag ctggctagag aaaggagctg gagaagaaga 900

ataaagtctc gagcccttgg ccttggaagc gggcaagcag ctgcgtggcc tgaggggaag 960

ggggcggtgg catcgagaaa ctgtgagaaa acccagagca tcagaaaaag tgagcccagg 1020

ctttggccat tatctgtaag aaaaacaaga aaaggggaac attatacttt cctgggtggc 1080

tcagggaaat gtgcagatgc acagtactcc agacagcagc tctgtacctg cctgctctgt 1140

ccctcagttc taacagaatc tagtcactaa gaactaacag gactaccaat acgaactgac 1200

aaatactacc actatgacct gtgacaaagc tgcatattta ttaagtggga agggaacttt 1260

tgatattatt tatccttgta acagtataga tgatggttat ttattctatt tataaggaat 1320

tatgtatttt ttttttcaat aaagatttat ttatgtggc 1359

<210> 36
<211> 196
<212> PRT
<213> Mus musculus

<400> 36

```
Met Leu Asp Cys Arg Ala Val Ile Met Leu Trp Leu Leu Pro Trp Val
1               5                   10                  15

Thr Gln Gly Leu Ala Val Pro Arg Ser Ser Ser Pro Asp Trp Ala Gln
            20                  25                  30

Cys Gln Gln Leu Ser Arg Asn Leu Cys Met Leu Ala Trp Asn Ala His
        35                  40                  45

Ala Pro Ala Gly His Met Asn Leu Leu Arg Glu Glu Glu Asp Glu Glu
    50                  55                  60

Thr Lys Asn Asn Val Pro Arg Ile Gln Cys Glu Asp Gly Cys Asp Pro
65                  70                  75                  80

Gln Gly Leu Lys Asp Asn Ser Gln Phe Cys Leu Gln Arg Ile Arg Gln
                85                  90                  95

Gly Leu Ala Phe Tyr Lys His Leu Leu Asp Ser Asp Ile Phe Lys Gly
            100                 105                 110

Glu Pro Ala Leu Leu Pro Asp Ser Pro Met Glu Gln Leu His Thr Ser
        115                 120                 125

Leu Leu Gly Leu Ser Gln Leu Leu Gln Pro Glu Asp His Pro Arg Glu
    130                 135                 140

Thr Gln Gln Met Pro Ser Leu Ser Ser Ser Gln Gln Trp Gln Arg Pro
145                 150                 155                 160

Leu Leu Arg Ser Lys Ile Leu Arg Ser Leu Gln Ala Phe Leu Ala Ile
                165                 170                 175

Ala Ala Arg Val Phe Ala His Gly Ala Ala Thr Leu Thr Glu Pro Leu
            180                 185                 190

Val Pro Thr Ala
```

195

<210> 37
<211> 759
<212> DNA
<213> Mus musculus

<400> 37
acaccatcca gatctctgga agacagacaa gatgctgcct ccaacagcct gctccgtcat 60

gtcctggatg ctgctctcct gcctgatgct cttatctcag gttcaaggtg aagactccct 120

gaagaatata ccctccgcac gcattagttg ccccaagggc tcccaggctt atggctccta 180

ctgctatgcc ttgtttcaga taccacagac ctggtttgat gcagaactgg cctgccaaaa 240

gaggcctgga ggacacctcg tatctgtgct caatagcgct gaggcttcat tcttgtcctc 300

catggtgaag agaacaggaa acagctacca atacacttgg attgggctcc atgaccccac 360

tctgggtgca gaacccaatg gaggtggatg ggaatggagt aacaatgacg tgatgaatta 420

ctttaactgg gagaggaacc catctactgc cttagaccgt gctttctgtg gcagcttgtc 480

aagagcttct ggatttctaa aatggagaga tatgacatgt gaggtgaagt tgccctatgt 540

ctgcaaattt actggttaaa cttatcagac agcaaacatc ccgaatttgt cttgaagagc 600

atcatggaca agggacaaaa tgtgaagact cacctagaaa aagcattttc tatctacagt 660

ccacattaga gccttaatct gctctttcca tatctgtctt tagtcctttt ggtataagtt 720

tgggctcaat tctaaaataa aaataagctt tctgtcaca 759

<210> 38
<211> 175
<212> PRT
<213> Mus musculus

<400> 38
Met Leu Pro Pro Thr Ala Cys Ser Val Met Ser Trp Met Leu Leu Ser
1 5 10 15

Cys Leu Met Leu Leu Ser Gln Val Gln Gly Glu Asp Ser Leu Lys Asn
20 25 30

Ile Pro Ser Ala Arg Ile Ser Cys Pro Lys Gly Ser Gln Ala Tyr Gly
35 40 45

Ser Tyr Cys Tyr Ala Leu Phe Gln Ile Pro Gln Thr Trp Phe Asp Ala
50 55 60

Glu Leu Ala Cys Gln Lys Arg Pro Gly Gly His Leu Val Ser Val Leu
65 70 75 80

Asn Ser Ala Glu Ala Ser Phe Leu Ser Ser Met Val Lys Arg Thr Gly

85 90 95

Asn Ser Tyr Gln Tyr Thr Trp Ile Gly Leu His Asp Pro Thr Leu Gly
100 105 110

Ala Glu Pro Asn Gly Gly Gly Trp Glu Trp Ser Asn Asn Asp Val Met
115 120 125

Asn Tyr Phe Asn Trp Glu Arg Asn Pro Ser Thr Ala Leu Asp Arg Ala
130 135 140

Phe Cys Gly Ser Leu Ser Arg Ala Ser Gly Phe Leu Lys Trp Arg Asp
145 150 155 160

Met Thr Cys Glu Val Lys Leu Pro Tyr Val Cys Lys Phe Thr Gly
165 170 175

<210> 39
<211> 769
<212> DNA
<213> Mus musculus

<400> 39
aagacacctt ggtctcagcc tgcagagatc gttgagttgc atcctaagca aaagactgtc 60

tgctgctcag catggctagg aacgcctact tcatcctgct ctcatgcctg atcgtcctgt 120

ctccaagcca aggccaggaa gctgaagaag acctgccatc tgccaggatc agttgcccag 180

aaggttccaa tgcctacagc tcctattgtt actacttcac tgaagaccgt ttaacttggg 240

ctgatgcaga tctttttgc cagaacatga attcaggcta cctggtgtca gttctcagtc 300

aggctgaggg caactttgtg gcctctctga ttaaggagag tggcactaca gacgccaatg 360

tctggactgg actccatgat cccaaaagga atcgtcgctg gcactggagc agtgggtctc 420

tgtttctcta caaatcctgg gcaactgggt ctcctaacag ttccaatcgt ggctactgtg 480

tatctctgac ttcaaacaca ggatacaaga aatggaagga tgacaactgt gatgcccaat 540

actcatttgt ctgcaagttc aaaggctgaa gtcacctgaa aaaaatagt catataaagc 600

aaaattgaaa ttactatagt gtcagaaatt aaattggacc atctatcaaa agcaaattag 660

atcctgtctt cctggagaga cattcttgct tcactgtcct atggtacctg tatctccatt 720

attttctgga aatttgcaca actgaaataa aaacaccttt acaatgttg 769

<210> 40
<211> 165
<212> PRT
<213> Mus musculus

<400> 40
Met Ala Arg Asn Ala Tyr Phe Ile Leu Leu Ser Cys Leu Ile Val Leu

```
1               5                   10                  15

Ser Pro Ser Gln Gly Gln Glu Ala Glu Glu Asp Leu Pro Ser Ala Arg
            20                  25                  30

Ile Ser Cys Pro Glu Gly Ser Asn Ala Tyr Ser Ser Tyr Cys Tyr Tyr
        35                  40                  45

Phe Thr Glu Asp Arg Leu Thr Trp Ala Asp Ala Asp Leu Phe Cys Gln
    50                  55                  60

Asn Met Asn Ser Gly Tyr Leu Val Ser Val Leu Ser Gln Ala Glu Gly
65                  70                  75                  80

Asn Phe Val Ala Ser Leu Ile Lys Glu Ser Gly Thr Thr Asp Ala Asn
                85                  90                  95

Val Trp Thr Gly Leu His Asp Pro Lys Arg Asn Arg Arg Trp His Trp
            100                 105                 110

Ser Ser Gly Ser Leu Phe Leu Tyr Lys Ser Trp Ala Thr Gly Ser Pro
        115                 120                 125

Asn Ser Ser Asn Arg Gly Tyr Cys Val Ser Leu Thr Ser Asn Thr Gly
    130                 135                 140

Tyr Lys Lys Trp Lys Asp Asp Asn Cys Asp Ala Gln Tyr Ser Phe Val
145                 150                 155                 160

Cys Lys Phe Lys Gly
                165
```

<210> 41
<211> 692
<212> DNA
<213> Mus musculus

<400> 41

```
agtattcatt attcctagct gacagaaatt attgatttag aatttaaatc gaagactgtc        60
tgctgatcag catggctcag aacaatgtat accttatcct gttcttatgc ctgatgttcc       120
tgtcatacag ccaaggccag gtagctgaag aagacttccc cttggctgaa aaagaccttc       180
cttctgccaa aatcaactgc ccagagggtg ccaacgccta tggttcctac tgttattatc       240
taattgaaga ccgtttgacc tgggggggagg ctgatctctt ttgccagaac atgaatgcag      300
gtcacctggt gtcaatactc agccaggctg agagcaactt tgtggcctcg ctggttaagg       360
agagtggtac tacagcttcc aatgtctgga ctggacttca tgaccctaaa agtaaccgtc       420
```

```
gttggcactg gagcagtggc tccctatttc tcttcaagtc atgggccact ggagctccaa      480

gcactgccaa ccgtggttat tgtgtatcgc tgacttcaaa cacagcatac aaaaaatgga      540

aggacgaaaa ctgtgaggca cagtactcct ttgtctgcaa gttcagagcc taaagtcacc      600

tgaagaacag atgtgcagaa ctctgttact atatattata aatgaaagaa gaccatctat      660

ctgcaaaata aatactcttt cctgtaataa ct                                    692


<210> 42
<211> 173
<212> PRT
<213> Mus musculus

<400> 42
Met Ala Gln Asn Asn Val Tyr Leu Ile Leu Phe Leu Cys Leu Met Phe
1               5                   10                  15


Leu Ser Tyr Ser Gln Gly Gln Val Ala Glu Glu Asp Phe Pro Leu Ala
            20                  25                  30


Glu Lys Asp Leu Pro Ser Ala Lys Ile Asn Cys Pro Glu Gly Ala Asn
        35                  40                  45


Ala Tyr Gly Ser Tyr Cys Tyr Tyr Leu Ile Glu Asp Arg Leu Thr Trp
    50                  55                  60


Gly Glu Ala Asp Leu Phe Cys Gln Asn Met Asn Ala Gly His Leu Val
65                  70                  75                  80


Ser Ile Leu Ser Gln Ala Glu Ser Asn Phe Val Ala Ser Leu Val Lys
                85                  90                  95


Glu Ser Gly Thr Thr Ala Ser Asn Val Trp Thr Gly Leu His Asp Pro
            100                 105                 110


Lys Ser Asn Arg Arg Trp His Trp Ser Ser Gly Ser Leu Phe Leu Phe
        115                 120                 125


Lys Ser Trp Ala Thr Gly Ala Pro Ser Thr Ala Asn Arg Gly Tyr Cys
    130                 135                 140


Val Ser Leu Thr Ser Asn Thr Ala Tyr Lys Lys Trp Lys Asp Glu Asn
145                 150                 155                 160


Cys Glu Ala Gln Tyr Ser Phe Val Cys Lys Phe Arg Ala
                165                 170


<210> 43
<211> 837
```

<212> DNA
<213> Mus musculus

<400> 43
gcccttcctt caaatcctat cataaagcag tcacctttgt cctgacaaac catctcagat 60

ctctacaaga gagacaagat gctgcctcac ctggtcctca acagtatttc ctggatgctg 120

ctctcctgcc tgttgtttgt atttcaggta caaggtgaag acttccagaa ggaagtgccc 180

tctccacgta ccagctgccc catgggttac aaggcttatc gctcccactg ctatgcctta 240

gttatgacac ctaaatcctg gtttcaagca gatctggtct gccagaagag accctcagga 300

catctcgtgt ctattcttag tggaggtgag gcttcctttg tgtcctcctt ggtgaacggc 360

agagtggaca actaccaaga catctggatt gggctccatg atccaacaat gggtcaacaa 420

cccaatggag gtggatggga gtggagtaac tccgatgtgc tgaattatct taactgggat 480

ggggatcctt cctctactgt caaccgtggt cactgtggga gtctgacagc aagttcaggg 540

tttctgaagt ggggagacta ttactgcgat gggacattac catttgtctg caagttcaag 600

cagtagacaa gcagcatcca gcatttatca tgaagctccc catgacaagg gatgaaatac 660

aagaattcac ccggcaaggc tgtacttgct ttacagttgt gcatcagact tattctggtt 720

ttctgtcctc tttcatccat ctccttcccc ttacttcagg cttttcaata tagttcctgc 780

tttgcaatct tgcagataaa taataaatac aacattttgg ttttactttt gtgtttt 837

<210> 44
<211> 175
<212> PRT
<213> Mus musculus

<400> 44
Met Leu Pro His Leu Val Leu Asn Ser Ile Ser Trp Met Leu Leu Ser
1               5                   10                  15

Cys Leu Leu Phe Val Phe Gln Val Gln Gly Glu Asp Phe Gln Lys Glu
            20                  25                  30

Val Pro Ser Pro Arg Thr Ser Cys Pro Met Gly Tyr Lys Ala Tyr Arg
        35                  40                  45

Ser His Cys Tyr Ala Leu Val Met Thr Pro Lys Ser Trp Phe Gln Ala
    50                  55                  60

Asp Leu Val Cys Gln Lys Arg Pro Ser Gly His Leu Val Ser Ile Leu
65                  70                  75                  80

Ser Gly Gly Glu Ala Ser Phe Val Ser Ser Leu Val Asn Gly Arg Val
                85                  90                  95

Asp Asn Tyr Gln Asp Ile Trp Ile Gly Leu His Asp Pro Thr Met Gly
            100                 105                 110

Gln Gln Pro Asn Gly Gly Gly Trp Glu Trp Ser Asn Ser Asp Val Leu
        115                 120                 125

Asn Tyr Leu Asn Trp Asp Gly Asp Pro Ser Ser Thr Val Asn Arg Gly
    130                 135                 140

His Cys Gly Ser Leu Thr Ala Ser Ser Gly Phe Leu Lys Trp Gly Asp
145                 150                 155                 160

Tyr Tyr Cys Asp Gly Thr Leu Pro Phe Val Cys Lys Phe Lys Gln
                165                 170                 175

<210> 45
<211> 788
<212> DNA
<213> Mus musculus

<400> 45
cccggggcga aatcacctct gagctgtcaa agcattgcag acctctgtat agacagatat 60

accatggtgt ctcacaagac ccttcatagc atgtcctgga tgctactgtg ttgcctgatg 120

tccctttctt gggtacaagg ggaacaatcc cagaaaaaac tgtcttctcc acgcatcagc 180

tgtccccaag aagcccaagc ttatggctcc tattgctatt tactgattct ggaaccacag 240

acctgggcta atgcagagat ccactgccag aagcatttct caggacacct ggcatttctg 300

ctcacttatg gggaaattat ctttgtgtcc tctctggtga aaaacagttt gaccacattc 360

ccatacatct ggattggact ccatgatctg tcacttggga gtttgcccaa tgaaaatgga 420

tggaagtgga gcagctctga ccctctgacc ttctataact gggagatccc accctccatg 480

tctgcacacc acggttactg cgcagctttg tctcaggcct caggttatca gaagtggaga 540

gattattatt gtgacccaac atttccctat gtctgcaaat tcaagggtta ggccagttct 600

gatttcaact gcctgaaagt atcctgaaga tcacatagac aaaggagcga gcatgatggc 660

tcaccaagaa agtccttctc acaccccgac accgaattcc tcatctcatc tctgctgttc 720

ttccataagt gtattctctg gggactctgg cctaaggatt cggagaacta taataaaatt 780

tagtcaat 788

<210> 46
<211> 175
<212> PRT
<213> Mus musculus

<400> 46
Met Val Ser His Lys Thr Leu His Ser Met Ser Trp Met Leu Leu Cys
1               5                   10                  15

```
Cys Leu Met Ser Leu Ser Trp Val Gln Gly Glu Gln Ser Gln Lys Lys
            20                  25                  30

Leu Ser Ser Pro Arg Ile Ser Cys Pro Gln Glu Ala Gln Ala Tyr Gly
        35                  40                  45

Ser Tyr Cys Tyr Leu Leu Ile Leu Glu Pro Gln Thr Trp Ala Asn Ala
    50                  55                  60

Glu Ile His Cys Gln Lys His Phe Ser Gly His Leu Ala Phe Leu Leu
65                  70                  75                  80

Thr Tyr Gly Glu Ile Ile Phe Val Ser Ser Leu Val Lys Asn Ser Leu
                85                  90                  95

Thr Thr Phe Pro Tyr Ile Trp Ile Gly Leu His Asp Leu Ser Leu Gly
            100                 105                 110

Ser Leu Pro Asn Glu Asn Gly Trp Lys Trp Ser Ser Ser Asp Pro Leu
        115                 120                 125

Thr Phe Tyr Asn Trp Glu Ile Pro Pro Ser Met Ser Ala His His Gly
    130                 135                 140

Tyr Cys Ala Ala Leu Ser Gln Ala Ser Gly Tyr Gln Lys Trp Arg Asp
145                 150                 155                 160

Tyr Tyr Cys Asp Pro Thr Phe Pro Tyr Val Cys Lys Phe Lys Gly
                165                 170                 175


<210> 47
<211> 1024
<212> DNA
<213> Mus musculus

<400> 47
ccgactcaag ctgaaaaagg cagggttctc ggagtgttgc ttccaatcag attgcaggtc      60

taggagaatc cacaaaaaag agaagaaaag aaaagaaaaa aactggaacg ggctctgagg     120

gccttgaaat caaagcattg aagtatcatc tccatcgaaa gaggaagatg gcttacaaag     180

gcgtgcggct actcttactg ctgagctggg tagctggccc cgaagtcctg agcgatatct     240

tgagacccag ctgtgcccca ggatggtttt actataggtc ccactgctat ggatacttcc     300

ggaagctaag aaactggtct catgctgagc tggagtgtca gtcatatgga aatggatccc     360

atctggcatc tgtcttgaat caaaaggaag ccagtgtcat atcaaagtac ataactggct     420

atcagagaaa cctgcctgtg tggattggcc tgcatgaccc acaaaagaag caattatggc     480
```

agtggactga tgggtctaca aacctgtaca gacgctggaa tcccaggaca aagagtgaag 540

ccaggcattg cgctgagatg aaccccaagg ataaattctt aacttggaac aaaaatggat 600

gtgccaaccg ccaacacttc ctgtgcaagt ataagacata gagcaaaaat caagcgtcta 660

ccagccttgc acaaactctt cccacttccc tctcacctgg tggctgatct aatcattatc 720

ccagagtaaa cactgtagca aacattgagg aggcctccag ggcactggct atcaagccct 780

gcttagcatg gtgggacagt ggcttccggt ctcagagttt agcatggtgg gacggtgact 840

tccggtctca gagattagca tggtgggaca agggcttctg gtctccgtgt tcactctaca 900

atcctttctg gtactcccct tccctctcat tgtcttaaac agcaatgctt aacaagctag 960

aaatgtgctt tcttgactac tgcgtctctg tcaaaccagt aaagttttgg agccaagaaa 1020

cagc 1024

<210> 48
<211> 157
<212> PRT
<213> Mus musculus

<400> 48
Met Ala Tyr Lys Gly Val Arg Leu Leu Leu Leu Leu Ser Trp Val Ala
1               5                   10                  15

Gly Pro Glu Val Leu Ser Asp Ile Leu Arg Pro Ser Cys Ala Pro Gly
            20                  25                  30

Trp Phe Tyr Tyr Arg Ser His Cys Tyr Gly Tyr Phe Arg Lys Leu Arg
        35                  40                  45

Asn Trp Ser His Ala Glu Leu Glu Cys Gln Ser Tyr Gly Asn Gly Ser
    50                  55                  60

His Leu Ala Ser Val Leu Asn Gln Lys Glu Ala Ser Val Ile Ser Lys
65                  70                  75                  80

Tyr Ile Thr Gly Tyr Gln Arg Asn Leu Pro Val Trp Ile Gly Leu His
                85                  90                  95

Asp Pro Gln Lys Lys Gln Leu Trp Gln Trp Thr Asp Gly Ser Thr Asn
            100                 105                 110

Leu Tyr Arg Arg Trp Asn Pro Arg Thr Lys Ser Glu Ala Arg His Cys
        115                 120                 125

Ala Glu Met Asn Pro Lys Asp Lys Phe Leu Thr Trp Asn Lys Asn Gly
    130                 135                 140

Cys Ala Asn Arg Gln His Phe Leu Cys Lys Tyr Lys Thr
145 150 155

<210> 49
<211> 618
<212> DNA
<213> Homo sapiens

<400> 49
atgacaccac ctgaacgtct cttcctccca agggtgtgtg gcaccaccct acacctcctc 60

cttctggggc tgctgctggt tctgctgcct ggggcccagg ggctccctgg tgttggcctc 120

acaccttcag ctgcccagac tgcccgtcag caccccaaga tgcatcttgc ccacagcacc 180

ctcaaacctg ctgctcacct cattggagac cccagcaagc agaactcact gctctggaga 240

gcaaacacgg accgtgcctt cctccaggat ggtttctcct tgagcaacaa ttctctcctg 300

gtccccacca gtggcatcta cttcgtctac tcccaggtgg tcttctctgg gaaagcctac 360

tctcccaagg ccacctcctc cccactctac ctggcccatg aggtccagct cttctcctcc 420

cagtacccct tccatgtgcc tctcctcagc tcccagaaga tggtgtatcc agggctgcag 480

gaaccctggc tgcactcgat gtaccacggg gctgcgttcc agctcaccca gggagaccag 540

ctatccaccc acacagatgg catcccccac ctagtcctca gccctagtac tgtcttcttt 600

ggagccttcg ctctgtag 618

<210> 50
<211> 205
<212> PRT
<213> Homo sapiens

<400> 50
Met Thr Pro Pro Glu Arg Leu Phe Leu Pro Arg Val Cys Gly Thr Thr
1 5 10 15

Leu His Leu Leu Leu Leu Gly Leu Leu Leu Val Leu Leu Pro Gly Ala
20 25 30

Gln Gly Leu Pro Gly Val Gly Leu Thr Pro Ser Ala Ala Gln Thr Ala
35 40 45

Arg Gln His Pro Lys Met His Leu Ala His Ser Thr Leu Lys Pro Ala
50 55 60

Ala His Leu Ile Gly Asp Pro Ser Lys Gln Asn Ser Leu Leu Trp Arg
65 70 75 80

Ala Asn Thr Asp Arg Ala Phe Leu Gln Asp Gly Phe Ser Leu Ser Asn
85 90 95

Asn Ser Leu Leu Val Pro Thr Ser Gly Ile Tyr Phe Val Tyr Ser Gln
            100                 105                 110

Val Val Phe Ser Gly Lys Ala Tyr Ser Pro Lys Ala Thr Ser Ser Pro
        115                 120                 125

Leu Tyr Leu Ala His Glu Val Gln Leu Phe Ser Ser Gln Tyr Pro Phe
    130                 135                 140

His Val Pro Leu Leu Ser Ser Gln Lys Met Val Tyr Pro Gly Leu Gln
145                 150                 155                 160

Glu Pro Trp Leu His Ser Met Tyr His Gly Ala Ala Phe Gln Leu Thr
                165                 170                 175

Gln Gly Asp Gln Leu Ser Thr His Thr Asp Gly Ile Pro His Leu Val
            180                 185                 190

Leu Ser Pro Ser Thr Val Phe Phe Gly Ala Phe Ala Leu
        195                 200                 205

<210> 51
<211> 735
<212> DNA
<213> Homo sapiens

<400> 51
atgggggcac tggggctgga gggcaggggt gggaggctcc aggggagggg ttccctcctg 60

ctagctgtgg caggagccac ttctctggtg accttgttgc tggcggtgcc tatcactgtc 120

ctggctgtgc tggccttagt gccccaggat cagggaggac tggtaacgga gacggccgac 180

cccggggcac aggcccagca aggactgggg tttcagaagc tgccagagga ggagccagaa 240

acagatctca gccccgggct cccagctgcc cacctcatag gcgctccgct gaaggggcag 300

gggctaggct gggagacgac gaaggaacag gcgtttctga cgagcgggac gcagttctcg 360

gacgccgagg ggctggcgct cccgcaggac ggcctctatt acctctactg tctcgtcggc 420

taccggggcc gggcgccccc tggcggcggg gacccccagg gccgctcggt cacgctgcgc 480

agctctctgt accgggcggg gggcgcctac gggccgggca ctcccgagct gctgctcgag 540

ggcgccgaga cggtgactcc agtgctggac ccggccagga gacaagggta cgggcctctc 600

tggtacacga gcgtggggtt cggcggcctg gtgcagctcc ggaggggcga gagggtgtac 660

gtcaacatca gtcaccccga tatggtggac ttcgcgagag ggaagacctt ctttggggcc 720

gtgatggtgg ggtga 735

<210> 52

```
<211> 240
<212> PRT
<213> Homo sapiens

<400> 52
Met Gly Ala Leu Gly Leu Glu Gly Arg Gly Gly Arg Leu Gln Gly Arg
1               5                   10                  15

Gly Ser Leu Leu Leu Ala Val Ala Gly Ala Thr Ser Leu Val Thr Leu
            20                  25                  30

Leu Leu Ala Val Pro Ile Thr Val Leu Ala Val Leu Ala Leu Val Pro
        35                  40                  45

Gln Asp Gln Gly Gly Leu Val Thr Glu Thr Ala Asp Pro Gly Ala Gln
    50                  55                  60

Ala Gln Gln Gly Leu Gly Phe Gln Lys Leu Pro Glu Glu Glu Pro Glu
65                  70                  75                  80

Thr Asp Leu Ser Pro Gly Leu Pro Ala Ala His Leu Ile Gly Ala Pro
                85                  90                  95

Leu Lys Gly Gln Gly Leu Gly Trp Glu Thr Thr Lys Glu Gln Ala Phe
            100                 105                 110

Leu Thr Ser Gly Thr Gln Phe Ser Asp Ala Glu Gly Leu Ala Leu Pro
        115                 120                 125

Gln Asp Gly Tyr Leu Tyr Cys Leu Val Gly Tyr Arg Gly Arg Ala Pro
    130                 135                 140

Pro Gly Gly Gly Asp Pro Gln Gly Arg Ser Val Thr Leu Arg Ser Ser
145                 150                 155                 160

Leu Tyr Arg Ala Gly Gly Ala Tyr Gly Pro Gly Thr Pro Glu Leu Leu
                165                 170                 175

Leu Glu Gly Ala Glu Thr Val Thr Pro Val Leu Asp Pro Ala Arg Arg
            180                 185                 190

Gln Gly Tyr Gly Pro Leu Trp Tyr Thr Ser Val Gly Phe Gly Gly Leu
        195                 200                 205

Val Gln Leu Arg Arg Gly Glu Arg Val Tyr Val Asn Ile Ser His Pro
    210                 215                 220

Asp Met Val Asp Phe Arg Lys Thr Phe Phe Gly Ala Val Met Val Gly
225                 230                 235                 240
```

<210> 53
<211> 609
<212> DNA
<213> Mus musculus

<400> 53
atgacactgc tcggccgtct ccacctcttg agggtgcttg gcacccctcc tgtcttcctc 60

ctggggctgc tgctggccct gcctctaggg gcccagggac tctctggtgt ccgcttctcc 120

gctgccagga cagcccatcc actccctcag aagcacttga cccatggcat cctgaaacct 180

gctgctcacc ttgttgggta ccccagcaag cagaactcac tgctctggag agcaagcacg 240

gatcgtgcct ttctccgaca tggcttctct ttgagcaaca actccctcct gatccccacc 300

agtggcctct actttgtcta ctcccaggtg gttttctctg gagaaagctg ctcccccagg 360

gccattccca ctcccatcta cctggcacac gaggtccagc tcttttcctc ccaatacccc 420

ttccatgtgc ctctcctcag tgcgcagaag tctgtgtatc cgggacttca aggaccgtgg 480

gtgcgctcaa tgtaccaggg ggctgtgttc ctgctcagta agggagacca gctgtccacc 540

cacaccgacg gcatctccca tctacacttc agccccagca gtgtattctt tggagccttt 600

gcactgtag 609

<210> 54
<211> 200
<212> PRT
<213> Mus musculus

<400> 54
Met Thr Leu Leu Gly Arg Leu His Leu Leu Arg Val Leu Gly Thr Pro
1 5 10 15

Pro Val Phe Leu Leu Gly Leu Leu Leu Ala Leu Pro Leu Gly Ala Gln
20 25 30

Gly Leu Ser Gly Val Arg Phe Ser Ala Ala Arg Thr Ala His Pro Leu
35 40 45

Pro Gln Lys His Leu Thr His Gly Ile Leu Lys Pro Ala Ala His Leu
50 55 60

Val Gly Tyr Pro Ser Lys Gln Asn Ser Leu Leu Trp Arg Ala Ser Thr
65 70 75 80

Asp Arg Ala Phe Leu Arg His Gly Phe Ser Leu Ser Asn Asn Ser Leu
85 90 95

Leu Ile Pro Thr Ser Gly Phe Val Tyr Ser Gln Val Val Phe Ser Gly
100 105 110

Glu Ser Cys Ser Pro Arg Ala Ile Pro Thr Pro Ile Tyr Leu Ala His
115 120 125

Glu Val Gln Leu Phe Ser Ser Gln Tyr Pro Phe His Val Pro Leu Leu
130 135 140

Ser Ala Gln Lys Ser Val Tyr Pro Gly Leu Gln Gly Pro Trp Val Arg
145 150 155 160

Ser Met Tyr Gln Gly Ala Val Phe Leu Leu Ser Lys Gly Asp Gln Leu
165 170 175

Ser Thr His Thr Asp Gly Ile Ser His Leu His Phe Ser Pro Ser Ser
180 185 190

Val Phe Phe Gly Ala Phe Ala Leu
195 200

<210> 55
<211> 609
<212> DNA
<213> Mus musculus

<400> 55
atgacactgc tcggccgtct ccacctcttg agggtgcttg gcacccctcc tgtcttcctc 60

ctggggctgc tgctggccct gcctctaggg gcccagggac tctctggtgt ccgcttctcc 120

gctgccagga cagcccatcc actccctcag aagcacttga cccatggcat cctgaaacct 180

gctgctcacc ttgttgggta ccccagcaag cagaactcac tgctctggag agcaagcacg 240

gatcgtgcct ttctccgaca tggcttctct ttgagcaaca actccctcct gatccccacc 300

agtggcctct actttgtcta ctcccaggtg gttttctctg gagaaagctg ctcccccagg 360

gccattccca ctcccatcta cctggcacac gaggtccagc tcttttcctc ccaatacccc 420

ttccatgtgc ctctcctcag tgcgcagaag tctgtgtatc cgggacttca aggaccgtgg 480

gtgcgctcaa tgtaccaggg ggctgtgttc ctgctcagta agggagacca gctgtccacc 540

cacaccgacg gcatctccca tctacacttc agccccagca gtgtattctt tggagccttt 600

gcactgtag 609

<210> 56
<211> 306
<212> PRT
<213> Mus musculus

<400> 56
Met Gly Thr Arg Gly Leu Gln Gly Leu Gly Gly Arg Pro Gln Gly Arg
1 5 10 15

```
Gly Cys Leu Leu Leu Ala Val Ala Gly Ala Thr Ser Leu Val Thr Leu
            20                  25                  30

Leu Leu Ala Val Pro Ile Thr Val Leu Ala Val Leu Ala Leu Val Pro
        35                  40                  45

Gln Asp Gln Gly Arg Arg Val Glu Lys Ile Ile Gly Ser Gly Ala Gln
    50                  55                  60

Ala Gln Lys Arg Leu Asp Asp Ser Lys Pro Ser Cys Ile Leu Pro Ser
65                  70                  75                  80

Pro Ser Ser Leu Ser Glu Thr Pro Asp Pro Arg Leu His Pro Gln Arg
                85                  90                  95

Ser Asn Ala Ser Arg Asn Leu Ala Ser Thr Ser Gln Gly Pro Val Ala
            100                 105                 110

Gln Ser Ser Arg Glu Ala Ser Ala Trp Met Thr Ile Leu Ser Pro Ala
        115                 120                 125

Ala Asp Ser Thr Pro Asp Pro Gly Val Gln Gln Leu Pro Lys Gly Glu
    130                 135                 140

Pro Glu Thr Asp Leu Asn Pro Glu Leu Pro Ala Ala His Leu Ile Gly
145                 150                 155                 160

Ala Trp Met Ser Gly Gln Gly Leu Ser Trp Glu Ala Ser Gln Glu Glu
                165                 170                 175

Ala Phe Leu Arg Ser Gly Ala Gln Phe Ser Pro Thr His Gly Leu Ala
            180                 185                 190

Leu Pro Gln Asp Gly Val Tyr Tyr Leu Tyr Cys His Val Gly Tyr Arg
        195                 200                 205

Gly Arg Thr Pro Pro Ala Gly Arg Ser Arg Ala Arg Ser Leu Thr Leu
    210                 215                 220

Arg Ser Ala Leu Tyr Arg Ala Gly Gly Ala Tyr Gly Arg Gly Ser Pro
225                 230                 235                 240

Glu Leu Leu Leu Glu Gly Ala Glu Thr Val Thr Pro Val Val Asp Pro
                245                 250                 255

Ile Gly Tyr Gly Ser Leu Trp Tyr Thr Ser Val Gly Phe Gly Gly Leu
```

260 265 270

Ala Gln Leu Arg Ser Gly Glu Arg Val Tyr Val Asn Ile Ser His Pro
275 280 285

Asp Met Val Asp Tyr Arg Arg Gly Lys Thr Phe Phe Gly Ala Val Met
290 295 300

Val Gly
305

<210> 57
<211> 672
<212> DNA
<213> Homo sapiens

<400> 57
atgctcctgc cttgggccac ctctgccccc ggcctggcct gggggcctct ggtgctgggc 60

ctcttcgggc tcctggcagc atcgcagccc caggcggtgc ctccatatgc gtcggagaac 120

cagacctgca gggaccagga aaaggaatac tatgagcccc agcaccgcat ctgctgctcc 180

cgctgcccgc caggcaccta tgtctcagct aaatgtagcc gcatccggga cacagtttgt 240

gccacatgtg ccgagaattc ctacaacgag cactggaact acctgaccat ctgccagctg 300

tgccgcccct gtgacccagt gatgggcctc gaggagattg ccccctgcac aagcaaacgg 360

aagacccagt gccgctgcca gccgggaatg ttctgtgctg cctgggccct cgagtgtaca 420

cactgcgagc tactttctga ctgcccgcct ggcactgaag ccgagctcaa agatgaagtt 480

gggaagggta acaaccactg cgtcccctgc aaggcagggc acttccagaa tacctcctcc 540

cccagcgccc gctgccagcc ccacaccagg tgtgagaacc aaggtctggt ggaggcagct 600

ccaggcactg cccagtccga cacaacctgc aaaaatccat tagagccact gcccccagag 660

atgtcaggag gg 672

<210> 58
<211> 706
<212> DNA
<213> Homo sapiens

<400> 58
cgcgcccagg tcaccgacaa agctgcgcac tatactctgt gcccaccgtg cccagcacct 60

gaactcctgg ggggaccgtc agtcttcctc ttccccccaa aacccaagga caccctcatg 120

atctcccgga cccctgaggt cacatgcgtg gtggtggacg tgagccacga agaccctgag 180

gtcaagttca actggtacgt ggacggcgtg gaggtgcata atgccaagac aaagccgcgg 240

gaggagcagt acaacagcac gtaccgtgtg gtcagcgtcc tcaccgtcct gcaccaggac 300

tggctgaatg gcaaggagta caagtgcaag gtctccaaca aagccctccc agcccccatc 360

gagaaaacca tctccaaagc caaagggcag ccccgagaac cacaggtgta caccctgccc 420

ccatcccggg aagagatgac caagaaccag gtcagcctga cctgcctggt caaaggcttc 480

tatcccagcg acatcgccgt ggagtgggag agcaatgggc agccggagaa caactacaag 540

accacgcctc ccgtgctgga ctccgacggc tccttcttcc tctacagcaa gctcaccgtg 600

gacaagagca ggtggcagca ggggaacgtc ttctcatgct ccgtgatgca tgaggctctg 660

cacaaccact acacgcagaa gagcctctcc ctgtctccgg gtaaat 706

<210> 59
<211> 640
<212> DNA
<213> Mus musculus

<400> 59
atgcgcctgc cccgggcctc ctctccctgc ggcctggcct gggggccact cctgctggga 60

ctcagcgggc ttctggtggc ctctcagccc cagctggtgc cccttatcg catagaaaac 120

cagacttgct gggaccagga caaggaatac tacgagccca tgcacgacgt ctgctgctcc 180

cgctgtcccc caggcgagtt tgtctttgcg gtatgcagcc gcagccaaga cacggtttgc 240

aagacttgcc cccataattc ctataatgaa cactggaacc atctctccac ctgccagctg 300

tgccgcccct gtgacattgt gctgggcttt gaggaggttg ccccttgcac cagcgatcgg 360

aaagccgagt gccgctgtca gccggggatg tcctgtgtgt atctggacaa tgagtgtgtg 420

cactgtgagg aggagcggct tgtactctgc cagcctggca cagaagccga ggtcacagat 480

gaaattatgg atactgacgt caactgtgtc ccctgtaagc cgggacactt ccagaacact 540

tcctcccctc gagcccgctg tcaaccccat accagatgtg agatccaggg cctggtggag 600

gcagctccag gtacctccta ctcggatacc atctgtaaaa 640

<210> 60
<211> 718
<212> DNA
<213> Mus musculus

<400> 60
ctcgaggacc cacaatcaag ccctgtcctc catgcaaatg cccagcacct aacctcttgg 60

gtggaccatc cgtcttcatc ttccctccaa agatcaagga tgtactcatg atctccctga 120

gccccatagt cacatgtgtg gtggtggatg tgagcgagga tgacccagat gtccagatca 180

gctggtttgt gaacaacgtg gaagtacaca cagctcagac acaaacccat agagaggatt 240

acgacagtac tctacgcgtg gtcagtgccc tccccatcca gcaccaggac tggatgagtg 300

gcaaggagtt caaatgcaag gtcaacaaca aagacctccc agcgcccatc gagagaacca 360

tctcaaaacc caaagggtca gtaagagctc cacaggtata tgtcttgcct ccaccagaag 420

aagagatgac taagaaacag gtcactctga cctgcatggt cacagacttc atgcctgaag 480

acatttacgt ggagtggacc aacaacggga aaacagagct aaactacaag aacactgaac 540

cagtcctgga ctctgatggt tcttacttca tgtacagcaa gctgagagtg gaaaagaaga 600

actgggtgga aagaaatagc tactcctgtt cagtggtcca cgagggtctg cacaatcacc 660

acacgactaa gagcttctcc cggactccgg gtaaatgaaa gcttggccgc catggccc 718

<210> 61
<211> 178
<212> PRT
<213> Homo sapiens

<400> 61
Met Lys His Gln His Gln His Gln His Gln His Gln His Gln Met His
1               5                   10                  15

Gln Ala Gln Thr Ala Arg Gln His Pro Lys Met His Leu Ala His Ser
            20                  25                  30

Thr Leu Lys Pro Ala Ala His Leu Ile Gly Asp Pro Ser Lys Gln Asn
        35                  40                  45

Ser Leu Leu Trp Arg Ala Asn Thr Asp Arg Ala Phe Leu Gln Asp Gly
    50                  55                  60

Phe Ser Leu Ser Asn Asn Ser Leu Leu Val Pro Thr Ser Gly Ile Tyr
65                  70                  75                  80

Phe Val Tyr Ser Gln Val Val Phe Ser Gly Lys Ala Tyr Ser Pro Lys
                85                  90                  95

Ala Thr Ser Ser Pro Leu Tyr Leu Ala His Glu Val Gln Leu Phe Ser
            100                 105                 110

Ser Gln Tyr Pro Phe His Val Pro Leu Leu Ser Ser Gln Lys Met Val
        115                 120                 125

Tyr Pro Gly Leu Gln Glu Pro Trp Leu His Ser Met Tyr His Gly Ala
    130                 135                 140

Ala Phe Gln Leu Thr Gln Gly Asp Gln Leu Ser Thr His Thr Asp Gly
145                 150                 155                 160

Ile Pro His Leu Val Leu Ser Pro Ser Thr Val Phe Phe Gly Ala Phe
                165                 170                 175

Ala Leu

<210> 62
<211> 24
<212> DNA
<213> Artificial Sequence

<220>
<221> source
<223> /note="Description of Artificial Sequence: Synthetic primer"

<400> 62
gtggaagcta cttgatgagt aggg 24

<210> 63
<211> 24
<212> DNA
<213> Artificial Sequence

<220>
<221> source
<223> /note="Description of Artificial Sequence: Synthetic primer"

<400> 63
agatgcgaaa atggagatta aaag 24

<210> 64
<211> 23
<212> DNA
<213> Artificial Sequence

<220>
<221> source
<223> /note="Description of Artificial Sequence: Synthetic primer"

<400> 64
ctacccgtga tattgctgaa gag 23

<210> 65
<211> 20
<212> DNA
<213> Artificial Sequence

<220>
<221> source
<223> /note="Description of Artificial Sequence: Synthetic primer"

<400> 65
gagcctgaag aagctggaaa 20

<210> 66
<211> 20
<212> DNA
<213> Artificial Sequence

<220>
<221> source
<223> /note="Description of Artificial Sequence: Synthetic primer"

<400> 66
caagtgttgg cagagatgga 20

<210> 67
<211> 20
<212> DNA
<213> Artificial Sequence

<220>
<221> source
<223> /note="Description of Artificial Sequence: Synthetic primer"

<400> 67
tcgccttctt gacgagttct 20

<210> 68
<211> 20
<212> DNA
<213> Artificial Sequence

<220>
<221> source
<223> /note="Description of Artificial Sequence: Synthetic primer"

<400> 68
tccgaggagt cagtgctaaa 20

<210> 69
<211> 20
<212> DNA
<213> Artificial Sequence

<220>
<221> source
<223> /note="Description of Artificial Sequence: Synthetic primer"

<400> 69
agaacgtctt ccagggtgaa 20

<210> 70
<211> 25
<212> DNA
<213> Artificial Sequence

<220>
<221> source
<223> /note="Description of Artificial Sequence: Synthetic probe"

<400> 70
tgagcacctg cttcatcagg tagca 25

<210> 71
<211> 19
<212> DNA
<213> Artificial Sequence

<220>
<221> source
<223> /note="Description of Artificial Sequence: Synthetic primer"

<400> 71
gctccagaag gccctcaga 19

<210> 72
<211> 19
<212> DNA
<213> Artificial Sequence

<220>
<221> source
<223> /note="Description of Artificial Sequence: Synthetic primer"

<400> 72
ctttccctcc gcattgaca 19

<210> 73
<211> 21
<212> DNA
<213> Artificial Sequence

<220>
<221> source
<223> /note="Description of Artificial Sequence: Synthetic probe"

<400> 73
acctcaaccg ttccacgtca c 21

<210> 74
<211> 19
<212> DNA
<213> Artificial Sequence

<220>
<221> source
<223> /note="Description of Artificial Sequence: Synthetic primer"

<400> 74
gcatcctcat ggagcacat 19

<210> 75
<211> 18
<212> DNA
<213> Artificial Sequence

```
<220>
<221> source
<223> /note="Description of Artificial Sequence: Synthetic
      primer"

<400> 75
ctggtcagcc aggagctt                                                18


<210> 76
<211> 21
<212> DNA
<213> Artificial Sequence

<220>
<221> source
<223> /note="Description of Artificial Sequence: Synthetic
      probe"

<400> 76
cttgcgggcc ttgtctgcct t                                            21


<210> 77
<211> 20
<212> DNA
<213> Artificial Sequence

<220>
<221> source
<223> /note="Description of Artificial Sequence: Synthetic
      primer"

<400> 77
agcctggatt gacaggaatc                                              20


<210> 78
<211> 21
<212> DNA
<213> Artificial Sequence

<220>
<221> source
<223> /note="Description of Artificial Sequence: Synthetic
      primer"

<400> 78
gataatcaga cgaggcgttt c                                            21


<210> 79
<211> 25
<212> DNA
<213> Artificial Sequence

<220>
<221> source
<223> /note="Description of Artificial Sequence: Synthetic
      probe"

<400> 79
tctggaaact cctgcagcct gacac                                        25
```

<210> 80
<211> 22
<212> DNA
<213> Artificial Sequence

<220>
<221> source
<223> /note="Description of Artificial Sequence: Synthetic primer"

<400> 80
tttgggagaa ctaggcattc tt 22

<210> 81
<211> 21
<212> DNA
<213> Artificial Sequence

<220>
<221> source
<223> /note="Description of Artificial Sequence: Synthetic primer"

<400> 81
tcttggacag gagtgttctc a 21

<210> 82
<211> 31
<212> DNA
<213> Artificial Sequence

<220>
<221> source
<223> /note="Description of Artificial Sequence: Synthetic probe"

<400> 82
cagcctctcc actttcatct atagcatctc c 31

<210> 83
<211> 19
<212> DNA
<213> Artificial Sequence

<220>
<221> source
<223> /note="Description of Artificial Sequence: Synthetic primer"

<400> 83
gctctccatg ccatttcaa 19

<210> 84
<211> 18
<212> DNA
<213> Artificial Sequence

<220>
<221> source
<223> /note="Description of Artificial Sequence: Synthetic primer"

<400> 84
tggccaaggg tctgaagt 18

<210> 85
<211> 25
<212> DNA
<213> Artificial Sequence

<220>
<221> source
<223> /note="Description of Artificial Sequence: Synthetic probe"

<400> 85
tgtacatccc tgctgtcctc aaggc 25

<210> 86
<211> 24
<212> DNA
<213> Artificial Sequence

<220>
<221> source
<223> /note="Description of Artificial Sequence: Synthetic primer"

<400> 86
tccaatgctc tcctaacaga taag 24

<210> 87
<211> 22
<212> DNA
<213> Artificial Sequence

<220>
<221> source
<223> /note="Description of Artificial Sequence: Synthetic primer"

<400> 87
caagatgaat tggatggtct tg 22

<210> 88
<211> 27
<212> DNA
<213> Artificial Sequence

<220>
<221> source
<223> /note="Description of Artificial Sequence: Synthetic probe"

<400> 88
tccttagcca ctccttctgt gactcca 27

<210> 89
<211> 26
<212> DNA
<213> Artificial Sequence

<220>
<221> source
<223> /note="Description of Artificial Sequence: Synthetic primer"

<400> 89
tgtcctcagt ttgtgcagaa tataaa 26

<210> 90
<211> 20
<212> DNA
<213> Artificial Sequence

<220>
<221> source
<223> /note="Description of Artificial Sequence: Synthetic primer"

<400> 90
tcaccatcgc aaggaactcc 20

<210> 91
<211> 36
<212> DNA
<213> Artificial Sequence

<220>
<221> source
<223> /note="Description of Artificial Sequence: Synthetic probe"

<400> 91
cgaaaacttg ttcagagaat tggacatcaa tagtga 36

<210> 92
<211> 19
<212> DNA
<213> Artificial Sequence

<220>
<221> source
<223> /note="Description of Artificial Sequence: Synthetic primer"

<400> 92
ggtggaagca cagttggca 19

<210> 93
<211> 21
<212> DNA
<213> Artificial Sequence

<220>
<221> source
<223> /note="Description of Artificial Sequence: Synthetic primer"

<400> 93
gtgtccaggt cctccatgat g 21

<210> 94
<211> 36
<212> DNA
<213> Artificial Sequence

<220>
<221> source
<223> /note="Description of Artificial Sequence: Synthetic probe"

<400> 94
tgaagaaaga gaagagaaat gaagccctca taaatg 36

<210> 95
<211> 20
<212> DNA
<213> Artificial Sequence

<220>
<221> source
<223> /note="Description of Artificial Sequence: Synthetic primer"

<400> 95
atggctccta ttgctatgcc 20

<210> 96
<211> 19
<212> DNA
<213> Artificial Sequence

<220>
<221> source
<223> /note="Description of Artificial Sequence: Synthetic primer"

<400> 96
gatgtcctga gggcctctt 19

<210> 97
<211> 25
<212> DNA
<213> Artificial Sequence

<220>
<221> source
<223> /note="Description of Artificial Sequence: Synthetic probe"

<400> 97
tggcaggcca tatctgcatc atacc 25

<210> 98
<211> 20
<212> DNA
<213> Artificial Sequence

<220>
<221> source
<223> /note="Description of Artificial Sequence: Synthetic primer"

<400> 98
atggctccta ctgctatgcc 20

<210> 99
<211> 19
<212> DNA
<213> Artificial Sequence

<220>
<221> source
<223> /note="Description of Artificial Sequence: Synthetic primer"

<400> 99
gtgtcctcca ggcctcttt 19

<210> 100
<211> 21
<212> DNA
<213> Artificial Sequence

<220>
<221> source
<223> /note="Description of Artificial Sequence: Synthetic probe"

<400> 100
tgatgcagaa ctggcctgcc a 21

<210> 101
<211> 22
<212> DNA
<213> Artificial Sequence

<220>
<221> source
<223> /note="Description of Artificial Sequence: Synthetic primer"

<400> 101
acatctaccg aagtccaatg ca 22

<210> 102
<211> 24
<212> DNA
<213> Artificial Sequence

<220>
<221> source
<223> /note="Description of Artificial Sequence: Synthetic primer"

<400> 102
ggaattgtaa tagcgatcct gagc 24

<210> 103
<211> 21
<212> DNA
<213> Artificial Sequence

<220>
<221> source
<223> /note="Description of Artificial Sequence: Synthetic probe"

<400> 103
tgcacgcaga cattcccgcc t 21

<210> 104
<211> 18
<212> DNA
<213> Artificial Sequence

<220>
<221> source
<223> /note="Description of Artificial Sequence: Synthetic primer"

<400> 104
ggtggctcag ggaaatgt 18

<210> 105
<211> 20
<212> DNA
<213> Artificial Sequence

<220>
<221> source
<223> /note="Description of Artificial Sequence: Synthetic primer"

<400> 105
gacagagcag gcaggtacag 20

<210> 106
<211> 27
<212> DNA
<213> Artificial Sequence

<220>
<221> source
<223> /note="Description of Artificial Sequence: Synthetic probe"

<400> 106
cagatgcaca gtactccaga cagcagc 27

<210> 107
<211> 20
<212> DNA
<213> Artificial Sequence

<220>
<221> source
<223> /note="Description of Artificial Sequence: Synthetic primer"

<400> 107
caggtgcttc cagtccgtct 20

<210> 108
<211> 21
<212> DNA
<213> Artificial Sequence

<220>
<221> source
<223> /note="Description of Artificial Sequence: Synthetic primer"

<400> 108
ctctcctgga atccccaaag t 21

<210> 109
<211> 24
<212> DNA
<213> Artificial Sequence

<220>
<221> source
<223> /note="Description of Artificial Sequence: Synthetic probe"

<400> 109
cagcacagat gccaacggcc tcat 24

<210> 110
<211> 18
<212> DNA
<213> Artificial Sequence

<220>
<221> source
<223> /note="Description of Artificial Sequence: Synthetic primer"

<400> 110
ctggccgctt cgggacgc 18

<210> 111
<211> 24
<212> DNA
<213> Artificial Sequence

<220>
<221> source
<223> /note="Description of Artificial Sequence: Synthetic
primer"

<400> 111
aaccacagaa gacacaagga actg 24

<210> 112
<211> 20
<212> DNA
<213> Artificial Sequence

<220>
<221> source
<223> /note="Description of Artificial Sequence: Synthetic
probe"

<400> 112
tctgctgctg gccgcttcgg 20

<210> 113
<211> 19
<212> DNA
<213> Artificial Sequence

<220>
<221> source
<223> /note="Description of Artificial Sequence: Synthetic
primer"

<400> 113
gctggactcc cttgtgtgt 19

<210> 114
<211> 19
<212> DNA
<213> Artificial Sequence

<220>
<221> source
<223> /note="Description of Artificial Sequence: Synthetic
primer"

<400> 114
cacatggcct cagtctcaa 19

<210> 115
<211> 20
<212> DNA
<213> Artificial Sequence

<220>
<221> source
<223> /note="Description of Artificial Sequence: Synthetic
probe"

<400> 115
cgcgggaccc tcatcctttg 20

```
<210> 116
<211> 21
<212> DNA
<213> Artificial Sequence

<220>
<221> source
<223> /note="Description of Artificial Sequence: Synthetic
      primer"

<400> 116
tccacagcac ctgaaggagt t                                          21


<210> 117
<211> 21
<212> DNA
<213> Artificial Sequence

<220>
<221> source
<223> /note="Description of Artificial Sequence: Synthetic
      primer"

<400> 117
ggagggaagg agaacagcag a                                          21


<210> 118
<211> 20
<212> DNA
<213> Artificial Sequence

<220>
<221> source
<223> /note="Description of Artificial Sequence: Synthetic
      probe"

<400> 118
tgggccaccc ccatcacagc                                            20
```